# EUROPEAN PATENT APPLICATION

(11) **EP 2 666 478 A2**
(43) Date of publication of application: **27.11.2013**
(21) Application number: 13172506.1
(22) Date of filing: 10.04.2007
(51) Int. Cl.: A61K 39/395, C07K 16/18, C07K 16/24, C07K 16/00

(54) **Uses and compositions for treatment of psoriasis**

(30) Priority: 10.04.2006 US 790909 P; 30.05.2006 US 809770 P; 20.06.2006 US 815489 P; 29.06.2006 US 817891 P; 25.08.2006 US 840122 P; 02.02.2007 US 899262 P; 02.04.2007 US 909683 P
(62) Divisional of application: 07755413.7
(71) Applicant: Abbott Biotechnology Ltd, HM 11 Hamilton (BM)
(72) Inventor: Willian, Mary, Kaye, Lake Forest, IL 60045 (US); Okun, Martin, M., Wilmette, IL 60091-2119 (US); Hoffman, Rebecca, S., Wilmette, IL 60091 (US); Gu, Yihua, Vernon Hills, IL 60061 (US); Camez, Anne, 68723 Schwetzingen (DE); Melilli, Lisa E., Norwalk, CT 06851-5421 (US); Gordon, Kenneth B., Stokie, IL 60077 (US)
(74) Representative: Modiano, Micaela Nadia

(57) **Abstract**

The invention provides methods, uses and compositions for the treatment of psoriasis. The invention describes methods and uses for treating psoriasis, wherein a TNFa inhibitor, such as a human TNFa antibody, or antigen-binding portion thereof, is used to treat psoriasis in a subject. Also described are methods for determining the efficacy of a TNFa inhibitor for treatment psoriasis in a subject.

SEQUENCE LISTING

<110> Abbott Biotechnology Ltd.

<120> USES AND COMPOSITIONS FOR TREATMENT OF PSORIASIS

<130> 117813/28421

<140> EP07755413.7
<141> 2008-11-10

<150> 60/790909
<151> 2006-04-10

<150> 60/809770
<151> 2006-05-30

<150> 60/815489
<151> 2006-06-30

<150> 60/899262
<151> 2007-02-02

<150> 60/817891
<151> 2006-06-29

<150> 60/840122
<151> 2006-08-25

<150> 60/909683
<151> 2007-0402

<160> 37

<170> FastSEQ for Windows Version 4.0

<210> 1
<211> 107
<212> PRT
<213> Artificial Sequence

<220>
<223> adalimumab light chain variable region

<400> 1

<210> 2
<211> 121
<212> PRT

<213> Artificial Sequence

<220>
<223> adalimumab heavy chain variable region

<400> 2

<210> 3
<211> 9
<212> PRT
<213> Artificial Sequence

<220>
<223> adalimumab light chain variable region CDR3

<221> VARIANT
<222> 9
<223> Xaa = Thr or Ala

<400> 3
Gln Arg Tyr Asn Arg Ala Pro Tyr Xaa
1               5

<210> 4
<211> 12
<212> PRT
<213> Artificial Sequence

<220>
<223> adalimumab heavy chain variable region CDR3

<221> VARIANT
<222> 12
<223> Xaa = Tyr or Asn

<400> 4
Val Ser Tyr Leu Ser Thr Ala Ser Ser Leu Asp Xaa
1               5                   10

<210> 5
<211> 7
<212> PRT
<213> Artificial Sequence

<220>
<223> adalimumab light chain variable region CDR2

<400> 5
Ala Ala Ser Thr Leu Gln Ser
1               5

<210> 6
<211> 17
<212> PRT
<213> Artificial Sequence

<220>
<223> adalimumab heavy chain variable region CDR2

<400> 6
Ala Ile Thr Trp Asn Ser Gly His Ile Asp Tyr Ala Asp Ser Val Glu
1               5                   10                  15
Gly

<210> 7
<211> 11
<212> PRT
<213> Artificial Sequence

<220>
<223> adalimumab light chain variable region CDR1

<400> 7
Arg Ala Ser Gln Gly Ile Arg Asn Tyr Leu Ala
1               5                   10

<210> 8
<211> 5
<212> PRT
<213> Artificial Sequence

<220>
<223> adalimumab heavy chain variable region CDR1

<400> 8
Asp Tyr Ala Met His
1               5

<210> 9
<211> 107
<212> PRT
<213> Artificial Sequence

<220>
<223> 2SD4 light chain variable region

<400> 9
Asp Ile Gln Met Thr Gln Ser Pro Ser Ser Leu Ser Ala Ser Ile Gly
1               5                   10                  15
Asp Arg Val Thr Ile Thr Cys Arg Ala Ser Gln Gly Ile Arg Asn Tyr
            20                  25                  30
Leu Ala Trp Tyr Gln Gln Lys Pro Gly Lys Ala Pro Lys Leu Leu Ile
        35                  40                  45
Tyr Ala Ala Ser Thr Leu Gln Ser Gly Val Pro Ser Arg Phe Ser Gly
    50                  55                  60
Ser Gly Ser Gly Thr Asp Phe Thr Leu Thr Ile Ser Ser Leu Gln Pro
65                  70                  75                  80

Glu Asp Val Ala Thr Tyr Tyr Cys Gln Lys Tyr Asn Ser Ala Pro Tyr
                85                  90                  95

Ala Phe Gly Gln Gly Thr Lys Val Glu Ile Lys
            100                 105

<210> 10
<211> 121
<212> PRT
<213> Artificial Sequence

<220>
<223> 2SD4 heavy chain variable region

<400> 10
Gln Val Gln Leu Val Glu Ser Gly Gly Gly Leu Val Gln Pro Gly Arg
 1               5                  10                  15
Ser Leu Arg Leu Ser Cys Ala Ala Ser Gly Phe Thr Phe Asp Asp Tyr
            20                  25                  30
Ala Met His Trp Val Arg Gln Ala Pro Gly Lys Gly Leu Asp Trp Val
        35                  40                  45
Ser Ala Ile Thr Trp Asn Ser Gly His Ile Asp Tyr Ala Asp Ser Val
    50                  55                  60
Glu Gly Arg Phe Ala Val Ser Arg Asp Asn Ala Lys Asn Ala Leu Tyr
65                  70                  75                  80
Leu Gln Met Asn Ser Leu Arg Pro Glu Asp Thr Ala Val Tyr Tyr Cys
                85                  90                  95
Thr Lys Ala Ser Tyr Leu Ser Thr Ser Ser Ser Leu Asp Asn Trp Gly
            100                 105                 110
Gln Gly Thr Leu Val Thr Val Ser Ser
        115                 120

<210> 11
<211> 9
<212> PRT
<213> Artificial Sequence

<220>
<223> 2SD4 light chain variable region CDR3

<400> 11
Gln Lys Tyr Asn Ser Ala Pro Tyr Ala
 1               5

<210> 12
<211> 9
<212> PRT
<213> Artificial Sequence

<220>
<223> EP B12  light chain variable region CDR3

<400> 12
Gln Lys Tyr Asn Arg Ala Pro Tyr Ala
 1               5

<210> 13
<211> 9
<212> PRT
<213> Artificial Sequence

<220>
<223> VL10E4 light chain variable region CDR3

<400> 13
Gln Lys Tyr Gln Arg Ala Pro Tyr Thr

1               5

<210> 14
<211> 9
<212> PRT
<213> Artificial Sequence

<220>
<223> VL100A9 light chain variable region CDR3

<400> 14
Gln Lys Tyr Ser Ser Ala Pro Tyr Thr
1               5

<210> 15
<211> 9
<212> PRT
<213> Artificial Sequence

<220>
<223> VLL100D2 light chain variable region CDR3

<400> 15
Gln Lys Tyr Asn Ser Ala Pro Tyr Thr
1               5

<210> 16
<211> 9
<212> PRT
<213> Artificial Sequence

<220>
<223> VLL0F4 light chain variable region CDR3

<400> 16
Gln Lys Tyr Asn Arg Ala Pro Tyr Thr
1               5

<210> 17
<211> 9
<212> PRT
<213> Artificial Sequence

<220>
<223> LOE5 light chain variable region CDR3

<400> 17
Gln Lys Tyr Asn Ser Ala Pro Tyr Tyr
1               5

<210> 18
<211> 9
<212> PRT
<213> Artificial Sequence

<220>
<223> VLLOG7 light chain variable region CDR3

<400> 18
Gln Lys Tyr Asn Ser Ala Pro Tyr Asn

1               5

<210> 19
<211> 9
<212> PRT
<213> Artificial Sequence

<220>
<223> VLLOG9 light chain variable region CDR3

<400> 19
Gln Lys Tyr Thr Ser Ala Pro Tyr Thr
1               5

<210> 20
<211> 9
<212> PRT
<213> Artificial Sequence

<220>
<223> VLLOH1 light chain variable region CDR3

<400> 20
Gln Lys Tyr Asn Arg Ala Pro Tyr Asn
1               5

<210> 21
<211> 9
<212> PRT
<213> Artificial Sequence

<220>
<223> VLLOH10 light chain variable region CDR3

<400> 21
Gln Lys Tyr Asn Ser Ala Ala Tyr Ser
1               5

<210> 22
<211> 9
<212> PRT
<213> Artificial Sequence

<220>
<223> VL1B7 light chain variable region CDR3

<400> 22
Gln Gln Tyr Asn Ser Ala Pro Asp Thr
1               5

<210> 23
<211> 9
<212> PRT
<213> Artificial Sequence

<220>
<223> VL1C1 light chain variable region CDR3

<400> 23
Gln Lys Tyr Asn Ser Asp Pro Tyr Thr
1               5

<210> 24

<211> 9
<212> PRT
<213> Artificial Sequence

<220>
<223> VL0.1F4 light chain variable region CDR3

<400> 24
Gln Lys Tyr Ile Ser Ala Pro Tyr Thr
1               5

<210> 25
<211> 9
<212> PRT
<213> Artificial Sequence

<220>
<223> VL0.1H8 light chain variable region CDR3

<400> 25
Gln Lys Tyr Asn Arg Pro Pro Tyr Thr
1               5

<210> 26
<211> 9

<212> PRT
<213> Artificial Sequence

<220>
<223> LOE7.A light chain variable region CDR3

<400> 26
Gln Arg Tyr Asn Arg Ala Pro Tyr Ala
1               5

<210> 27
<211> 12
<212> PRT
<213> Artificial Sequence

<220>
<223> 2SD4 heavy chain variable region CDR3

<400> 27
Ala Ser Tyr Leu Ser Thr Ser Ser Ser Leu Asp Asn
1               5                   10

<210> 28
<211> 12
<212> PRT
<213> Artificial Sequence

<220>
<223> VH1B11 heavy chain variable region CDR3

<400> 28
Ala Ser Tyr Leu Ser Thr Ser Ser Ser Leu Asp Lys
1               5                   10

<210> 29
<211> 12
<212> PRT

<213> Artificial Sequence

<220>
<223> VH1D8 heavy chain variable region CDR3

<400> 29
Ala Ser Tyr Leu Ser Thr Ser Ser Ser Leu Asp Tyr
1               5                   10

<210> 30
<211> 12
<212> PRT
<213> Artificial Sequence

<220>
<223> VH1A11 heavy chain variable region CDR3

<400> 30
Ala Ser Tyr Leu Ser Thr Ser Ser Ser Leu Asp Asp
1               5                   10

<210> 31
<211> 12
<212> PRT
<213> Artificial Sequence


<220>
<223> VH1B12 heavy chain variable region CDR3

<400> 31
Ala Ser Tyr Leu Ser Thr Ser Phe Ser Leu Asp Tyr
1               5                   10

<210> 32
<211> 12
<212> PRT
<213> Artificial Sequence

<220>
<223> VH1E4 heavy chain variable region CDR3

<400> 32
Ala Ser Tyr Leu Ser Thr Ser Ser Ser Leu His Tyr
1               5                   10

<210> 33
<211> 12
<212> PRT
<213> Artificial Sequence

<220>
<223> VH1F6 heavy chain variable region CDR3

<400> 33
Ala Ser Phe Leu Ser Thr Ser Ser Ser Leu Glu Tyr
1               5                   10

<210> 34
<211> 12
<212> PRT
<213> Artificial Sequence

<220>
<223> 3C-H2 heavy chain variable region CDR3

<400> 34
Ala Ser Tyr Leu Ser Thr Ala Ser Ser Leu Glu Tyr
1               5                   10

<210> 35
<211> 12
<212> PRT
<213> Artificial Sequence

<220>
<223> VH1-D2.N heavy chain variable region CDR3

<400> 35
Val Ser Tyr Leu Ser Thr Ala Ser Ser Leu Asp Asn
1               5                   10

<210> 36
<211> 321
<212> DNA
<213> Artificial Sequence

<220>
<223> adalimumab light chain variable region

<400> 36
gacatccaga tgacccagtc tccatcctcc ctgtctgcat ctgtagggga cagagtcacc 60
atcacttgtc gggcaagtca gggcatcaga aattacttag cctggtatca gcaaaaacca 120
gggaaagccc ctaagctcct gatctatgct gcatccactt tgcaatcagg ggtcccatct 180
cggttcagtg gcagtggatc tgggacagat ttcactctca ccatcagcag cctacagcct 240
gaagatgttg caacttatta ctgtcaaagg tataaccgtg caccgtatac ttttggccag 300
gggaccaagg tggaaatcaa a                                           321

<210> 37
<211> 363
<212> DNA
<213> Artificial Sequence

<220>
<223> adalimumab heavy chain variable region

<400> 37
gaggtgcagc tggtggagtc tgggggaggc ttggtacagc ccggcaggtc cctgagactc 60
tcctgtgcgg cctctggatt cacctttgat gattatgcca tgcactgggt ccggcaagct 120
ccagggaagg gcctggaatg ggtctcagct atcacttgga atagtggtca catagactat 180
gcggactctg tggagggccg attcaccatc tccagagaca acgccaagaa ctccctgtat 240
ctgcaaatga acagtctgag agctgaggat acggccgtat attactgtgc gaaagtctcg 300
taccttagca ccgcgtcctc ccttgactat tggggccaag gtaccctggt caccgtctcg 360
agt                                                               363

## Description

### RELATED APPLICATIONS

This application claims the benefit of priority to U.S. provisional patent application number 60/790909 filed on April 10, 2006; U.S. provisional patent application number 60/809770 filed on May 30, 2006; U.S. provisional patent application number 60/815489 filed on June 20, 2006; U.S. provisional patent application number 60/817891 filed on June 29, 2006; U.S. provisional patent application number 60/840122 filed on August 25, 2006; U.S. provisional patent application number 60/899262 filed on February 2, 2007; and U.S. provisional patent application number 60/909683 filed on April 2, 2007. The contents of all the above-mentioned priority applications are hereby incorporated by reference in their entirety.

### BACKGROUND OF THE INVENTION

Psoriasis is a chronic, immune-mediated disease affecting 1-3% of the population worldwide (Jacobson and Kimball, Epidemiology: Psoriasis In: Psoriasis and Psoriatic Arthritis (Eds: Gordon KB, Ruderman EM). Springer-Verlag Berlin Heidelberg, Germany;2005:47-56), with the greatest disease prevalence occurring in North America and Europe (Krueger and Duvic, J. Invest. Dermatol, 102:145-185, 1994). The most common form of psoriasis is plaque-type psoriasis, present in 65-86% of patients and characterized by the presence of thick, scaly plaques. Based on the National Psoriasis Foundation's definitions of moderate to severe psoriasis, the prevalence of moderate to severe psoriasis in the United States is estimated at 0.31 % of persons age 18 or older (Stem et al., J. Investig. Dermatol. Symp. Proc. 9:136-139, 2004). Patients with psoriasis report reduction in physical functioning and mental functioning comparable to that observed in patients with cancer, arthritis, hypertension, heart disease, diabetes, and depression (Rapp et al., J. Am. Acad. Dermatol. 41(3Pt1):401-407, 1999). In a US survey of the impact of psoriasis on quality of life, respondents reported difficulties in the workplace, difficulties socializing with family members and friends, exclusion from public facilities, difficulties in getting a job, and contemplation of suicide (Krueger et al., Arch. Dermatol., 137:280-284, 2001).

### SUMMARY OF THE INVENTION

There remains a need for an effective and safe treatment option for patients suffering from psoriasis. The instant invention provides improved methods and compositions for treating psoriasis.

The invention provides an article of manufacture comprising a packaging material; a TNFα inhibitor; and a label or package insert contained within the packaging material indicating that the standardized mortality rate for the TNFα inhibitor was calculated at about 0.67.

The invention includes an article of manufacture comprising a packaging material; a TNFα inhibitor; and a label or package insert contained within the packaging material indicating that patients receiving treatment with the TNFα inhibitor can be safely administered a pneumonococcal or influenza virus vaccine.

The invention also includes an article of manufacture comprising a packaging material; pneumonococcal or influenza virus vaccine; and a label or package insert contained within the packaging material indicating that patients receiving the pneumonococcal or influenza virus vaccine can be safely administered a TNFα inhibitor.

The invention further provides an article of manufacture comprising a packaging material; a TNFα inhibitor; and a label or package insert contained within the packaging material indicating that in studies of the TNFα inhibitor, observed malignancies included melanoma and granulose cell tumor of the ovary.

In one embodiment, the TNFα inhibitor is administered weekly. In another embodiment, the TNFa inhibitor is administered every other week.

The invention further provides a package comprising a TNFα inhibitor and a label, in a position which is visible to prospective purchasers, comprising a printed statement which informs prospective purchasers that the median apparent clearance (CL/F) of the TNFα inhibitor ranges from about 13.2 to about 15.0 mL/hr. In one embodiment of the invention, the package further informs prospective purchasers that concomitant therapy with either immunosuppressant 6 mercaptopurine or azathioprine has slightly lower or no impact on TNFα inhibitor CL/F. In one embodiment, the anti-TNFa antibody, or antigen-binding portion thereof, is a 40 mg dose.

The invention includes a method for determining the efficacy of a TNFα inhibitor for improving the functional limitations of human subjects having moderate to severe chronic plaque psoriasis comprising administering the TNFα inhibitor to a preselected patient population having moderate to severe chronic plaque psoriasis; and determining the efficacy of the TNFα inhibitor using a baseline Dermatology Life Quality Index (DLQI) score from the patient population and a DLQI score from a time period following administration of the TNFα inhibitor, wherein a DLQI score of no or small impact in at least about 83% of the patient population indicates that TNFα inhibitor is efficacious for improving the functional limitations of human subjects having moderate to severe chronic plaque psoriasis. In one embodiment, the TNFα inhibitor has already been administered to the pre-selected patient population when the efficacy is determined.

The invention also provides a method for determining the efficacy of a TNFα inhibitor for improving the functional limitations of human subjects having moderate to severe psoriasis comprising determining the efficacy of the TNFα inhibitor using a baseline Dermatology Life Quality Index (DLQI) score from a preselected patient population and a DLQI score from the preselected patient population following administration of the TNFα inhibitor, wherein a resulting DLQI score of no or small impact in at least about 83% of the patient population indicates that TNFα inhibitor is efficacious for improving the functional limitations of human subjects having moderate to severe psoriasis.

In one embodiment, the TNFα inhibitor is administered weekly to the patient population. In another embodiment, the TNFα inhibitor is administered biweekly to the patient population. In still another embodiment, the TNFα inhibitor is administered in a multiple variable dose regimen. In one embodiment, the multiple variable dose regimen comprises an induction dose which is at least double the treatment dose. In one embodiment, the induction dose comprises about 80 mg. In one embodiment, the treatment dose comprises about 40 mg.

The invention provides an article of manufacture comprising: a packaging material; a TNFα inhibitor, and a label or package insert contained within the packaging material indicating that a history of systemic or biologic therapy does not adversely affect efficacy of the TNFα inhibitor in patients.

The invention also provides an article of manufacture comprising: a packaging material; a TNFα inhibitor, and a label or package insert contained within the packaging material indicating that administration of the TNFα inhibitor is safe in patients with a history of systemic or biologic therapy. In one embodiment, the patients have moderate to severe psoriasis.

The invention includes a method of treating a subtherapeutic response in a subject having moderate to severe plaque psoriasis comprising administering a TNFα inhibitor to the subject at an increased dosing rate which is about twice as frequent as the original dosing rate. In one embodiment, the increased dosing rate is weekly. In another embodiment, the subtherapeutic response comprises < PASI50 improvement from baseline (week 0) and a determined time period following baseline. In one embodiment, the determined time period following baseline is about 24 weeks.

The invention includes a package comprising a TNFα inhibitor and instructions for administering the TNFα inhibitor to a human subject for the treatment of adults with moderate to severe psoriasis who have had an inadequate response to conventional therapy.

The invention also includes a package comprising a TNFα inhibitor, wherein the package contains, on the label and in a position which is visible to prospective purchasers, a printed statement which informs prospective purchasers that the TNFα inhibitor is indicated for the treatment of adults with moderate to severe psoriasis who have had an inadequate response to conventional therapy.

The invention further provides a package comprising a TNFα inhibitor, wherein the package contains, on the label and in a position which is visible to prospective purchasers, a printed statement which informs prospective purchasers that the recommended dose of the TNFα inhibitor for patients with psoriasis is 40 mg TNFα inhibitor administered every other week as a single dose via subcutaneous injection.

The invention also includes a package comprising a TNFα inhibitor, wherein the package contains, on the label and in a position which is visible to prospective purchasers, a printed statement which informs prospective purchasers that available data suggest that the clinical response is usually achieved within 12 weeks of treatment; and continued therapy should be carefully reconsidered in a patient not responding within this time period.

The invention provides a package comprising adalimumab, wherein the package contains, on the label and in a position which is visible to prospective purchasers, a printed statement which informs prospective purchasers that the proportion of patients who discontinued treatment due to adverse events during the double-blind, controlled portion of Studies I - IX was 5.1% for patients taking the adalimumab and 3.2% for control treated patients.

The invention also provides a package comprising a TNFα inhibitor, wherein the package contains, on the label and in a position which is visible to prospective purchasers, a printed statement which informs prospective purchasers that the TNFα inhibitor has been shown to have an uncommon undesirable effect in clinical studies selected from the group consisting of vaginal infection (including fungal), hyperglycaemia, dysphonia, pharyngeal erythema, wheezing, skin reaction, skin exfoliation, spasm, rheumatoid nodule, shoulder pain, and feeling hot.

The invention further provides a package comprising adalimumab, wherein the package contains, on the label and in a position which is visible to prospective purchasers, a printed statement which informs prospective purchasers of at least one of the following notifications: in the nine controlled trials, 17% of patients treated with adalimumab developed injection site reactions (erythema and/or itching, haemorrhage, pain or swelling), compared to 10% of patients receiving placebo or active control; in the nine controlled trials, the rate of infection was 1.52 per patient year in the adalimumab treated patients and 1.40 per patient year in the placebo and active control-treated patients; in the nine controlled trials, 29 malignancies were reported in 2370 adalimumab treated patients with 1779 patient-years of exposure ( 16.3 per 1000 patient years), and 6 malignancies were reported in 1309 control treated patients observed with 872 patient-years of exposure (6.9 per 1000 patient years); this included 2 lymphomas in the adalimumab treated patients (1.1 per 1000 patient years) and 1 lymphoma in the control treated patients (1.1 per 1000 patient years); and two patients out of 3834 treated with adalimumab in all rheumatoid arthritis, psoriatic arthritis and ankylosing spondylitis studies developed clinical signs suggestive of new-onset lupuslike syndrome.

In one embodiment of the invention, the TNFα inhibitor is selected from the group consisting of an anti-TNFα antibody, or an antigen-binding portion thereof, a TNF fusion protein, or a recombinant TNF binding protein. In one embodiment, the TNF fusion protein is etanercept In another embodiment of the invention, the anti-TNFα antibody, or antigen-binding portion thereof, is selected from the group consisting of a chimeric antibody, a humanized antibody, and a multivalent antibody.

In one embodiment of the invention, the anti-TNFα antibody, or antigen-binding portion thereof, is a human antibody.

In another embodiment, the anti-TNFα antibody, or antigen-binding portion thereof, is an isolated human antibody that dissociates from human TNFα with a K_{d} of 1 x 10⁻⁸ M or less and a K_{off} rate constant of 1 x 10⁻³ s⁻¹ or less, both determined by surface plasmon resonance, and neutralizes human TNFα cytotoxicity in a standard *in vitro* L929 assay with an IC₅₀ of 1 x 10⁻⁷ M or less.

In one embodiment of the invention, the anti-TNFα antibody is an isolated human antibody, or antigen-binding portion thereof, with the following characteristics:
a) dissociates from human TNFα with a K_{off} rate constant of 1 x 10⁻³ s⁻¹ or less, as determined by surface plasmon resonance;
b) has a light chain CDR3 domain comprising the amino acid sequence of SEQ ID NO: 3, or modified from SEQ ID NO: 3 by a single alanine substitution at position 1, 4, 5, 7 or 8 or by one to five conservative amino acid substitutions at positions 1, 3, 4, 6, 7, 8 and/or 9;
c) has a heavy chain CDR3 domain comprising the amino acid sequence of SEQ ID NO: 4, or modified from SEQ ID NO: 4 by a single alanine substitution at position 2, 3, 4, 5, 6, 8, 9, 10 or 11 or by one to five conservative amino acid substitutions at positions 2, 3, 4, 5, 6, 8, 9, 10, 11 and/or 12.

In one embodiment of the invention, the anti-TNFα antibody is an isolated human antibody, or an antigen binding portion thereof, with a light chain variable region (LCVR) comprising the amino acid sequence of SEQ ID NO: 1 and a heavy chain variable region (HCVR) comprising the amino acid sequence of SEQ ID NO: 2. In one embodiment, the anti-TNFα antibody, or antigen-binding portion thereof, is adalimumab.

In one embodiment, the anti-TNFα antibody, or antigen-binding portion thereof, is a 40 mg dose.

In another embodiment, the anti-TNFα antibody, or antigen-binding portion thereof, is administered subcutaneously.

The another embodiment of the invention, the anti- TNFα antibody, or antigen-binding portion thereof, is infliximab or golimumab.

The invention includes a method for determining the efficacy of a TNFα inhibitor for improving the functional limitations of human subjects having moderate to severe psoriasis comprising
a) administering the TNFα inhibitor to a preselected patient population having moderate to severe psoriasis; and
b) determining the efficacy of the TNFα inhibitor using a baseline Dermatology Life Quality Index (DLQI) score from the patient population and a DLQI score from a time period following administration of the TNFα inhibitor, wherein a DLQI score of no or small impact in at least about 83% of the patient population indicates that TNF 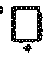 inhibitor is efficacious for improving the functional limitations of human subjects having moderate to psoriasis.

The invention also includes a method for determining the efficacy of a TNFα inhibitor for treatment of psoriasis in a subject having a PASI of ≥ 10 and a DLQI >10 comprising determining the efficacy of the TNFα inhibitor in a patient population having a PASI of ≥ 10 and a DLQI >10, using a baseline PASI score of the patient population before administration of the TNFα inhibitor and a PASI score following administration of the TNFα inhibitor, wherein a PASI75 response in at least about 44% of the patient population following administration of the TNFα inhibitor indicates that the TNFα inhibitor is is efficacious for treatment of psoriasis in a subject having a PASI ≥10 and a DLQI> 10.

The invention further provides a method for determining the efficacy of a TNFα inhibitor for treatment of psoriasis in a subject having a PASI of ≥ 10 and a DLQI > 10 comprising determining the efficacy of the TNFα inhibitor in a patient population having a PASI of ≥ 10 and a DLQI > 10, using a baseline PGA score of the patient population before administration of the TNFα inhibitor and a PGA score following administration of the TNFα inhibitor, wherein a PGA score of "clear" or "almost clear" in at least about 33% of the patient population following administration of the TNFα inhibitor indicates that the TNFα inhibitor is is efficacious for treatment of psoriasis in a subject having a PASI ≥ 10 and a DLQI > 10.

The invention describes a method for determining the efficacy of a TNFα inhibitor for treatment of psoriasis in a subject having a PASI of ≥ 10 and a DLQI >10 comprising determining the efficacy of the TNFα inhibitor in a patient population having a PASI of ≥ 10 and a DLQI >10, using a baseline DLQI score of the patient population before administration of the TNFα inhibitor and a DLQI score following administration of the TNFα inhibitor, wherein a decrease in the DLQI score of at least about 12 indicates that the TNFα inhibitor is is efficacious for treatment of psoriasis in a subject having a PASI ≥ 10 and a DLQI > 10.

The invention further provides a method for determining the efficacy of a TNFα inhibitor for improving the general health of human subjects having moderate to severe psoriasis comprising administering the TNFα inhibitor to a preselected patient population having moderate to severe chronic plaque psoriasis; and determining the efficacy of the TNFα inhibitor using a baseline SF-36 (Short Form 36 Health Survey) score from the patient population and an SF-36 score from a time period following administration of the TNFα inhibitor, wherein an improvement in the SF-36 score indicates that TNFα inhibitor is efficacious for improving the functional limitations of human subjects having moderate to severe psoriasis. The change in the SF-36 scoremay be selected from any of the eight domains of the SF-36 instrument, including physical function, physical role limitations, vitality, general health perceptions, bodily pain, social function, emotional role limitations, and mental health. In one embodiment, an improvement of at least 16 in the bodily pain domain indicates that the TNFα inhibitor is efficacious for treatment of moderate to severe chronic plaque psoriasis. The invention further provides a method of improving an SF-36 score of a patient having moderate to severe chronic plaque psoriasis comprising administering to the patient a TNFα inhibitor.

In one embodiment, the moderate to severe psoriasis is moderate to severe chronic plaque psoriasis.

In one embodiment, the TNFα inhibitor is administered weekly to the patient population.

In one embodiment, the multiple variable dose regimen comprises an induction dose which is at least double the treatment dose.

In one embodiment, the induction dose comprises about 80 mg.

In one embodiment, the treatment dose comprises about 40 mg.

In one embodiment, the patient also has psoriatic arthritis.

The invention includes an article of manufacture comprising:
a) a packaging material;
b) a TNFα inhibitor, and
c) a label or package insert contained within the packaging material indicating that a history of systemic or biologic therapy does not adversely effect efficacy of the TNFα inhibitor in patients.

The invention also includes an article of manufacture comprising:
a) a packaging material;
b) a TNFα inhibitor, and
c) a label or package insert contained within the packaging material indicating that administration of the TNFα inhibitor is safe in patients with a history of systemic or biologic therapy.

In one embodiment, the patients have moderate to severe psoriasis.

The invention further provides an article of manufacture comprising:
a) a packaging material;
b) a TNFα inhibitor, and
c) a label or package insert contained within the packaging material indicating that a use of TNF blockers have been associated with reactivation of hepatitis B virus (HBV) in patients who are chronic carriers of the virus.

The invention also includes an article of manufacture comprising:
a) a packaging material;
b) a TNFα inhibitor, and
c) a label or package insert contained within the packaging material indicating that an adverse event which has been reported in the use of the TNFa inhibitor is angioneurotic edema.

In one embodiment, the TNFα inhibitor is selected from the group consisting of an anti- TNFα antibody, or an antigen-binding portion thereof, a TNF fusion protein, or a recombinant TNF binding protein.

In one embodiment, the TNF fusion protein is etanercept.

In one embodiment, the anti- TNFα antibody, or antigen-binding portion thereof, is selected from the group consisting of a chimeric antibody, a humanized antibody, and a multivalent antibody.

The invention provides a method of preventing reactivation of latent tuberculosis (LTB) in a patient prior to administration of a TNFα inhibitor to the subject comprising prescreening the patient for TB, wherein the patient is administered isoniazid (INH) if the patient is identified as being high-risk based on the prescreening results. In one embodiment, the prescreening method is selected from the group consisting of a clinical interview, a PPD test, a chest x-ray, or any combination thereof. In a further embodiment, the patient is administered isoniazid (INH) if the patient is identified as being high-risk if the prescreening result is a positive PPD test.

The invention also provides a method of promoting the safety a TNFα inhibitor for the treatment of a disorder in which TNFα activity is detrimental comprising conveying to a recipient or a medical agent that studies have indicated that prescreening a recipient prior to initial administration of the TNFα inhibitor significantly reduces the chance of latent TB reactivation.

The invention includes a method of achieving a PASI 100 and an improvement in the quality of life in a subject having psoriatic arthritis comprising administering a TNFα inhibitor to the subject such that a PAS 100 score and DLQI score of 0 or 1 is achieved.

The invention further provides an article of manufacture comprising: a packaging material; an autoinjector pen filled with a TNFα inhibitor; and a label or package insert contained within the packaging material indicating that the bioequivalence of the TNFα inhibitor is similar regardless of whether the injection site is the thigh or abdomen.

In one embodiment of the invention, the TNFα inhibitor is selected from the group consisting of an anti-TNFα antibody, or an antigen-binding portion thereof, a TNF fusion protein, or a recombinant TNF binding protein. In one embodiment, the TNF fusion protein is etanercept. In another embodiment of the invention, the anti-TNFα antibody, or antigen-binding portion thereof, is selected from the group consisting of a chimeric antibody, a humanized antibody, and a multivalent antibody.

In one embodiment of the invention, the anti-TNFα antibody, or antigen-binding portion thereof, is a human antibody.

The efficacy of a TNFα inhibitor for treating psoriasis in a patient population, *i.e.,* PASI 75 response (also referred to herein as a PASI / PASI75 score), may be evaluated by determining the percentage of the patient population in treatment of psoriasis has been effective following administration of the TNFα inhibitor.

In one embodiment, the invention provides a method of determining the efficacy of a TNFα inhibitor for treating psoriasis in a subject comprising determining a Psoriasis Area Severity Index (PASI) score of a patient population having psoriasis and who was administered the TNFα inhibitor, wherein a PASI 75 response is achieved in at least about 32% of the patient population indicates that the TNFα inhibitor is an effective TNFα inhibitor for the treatment of psoriasis in a subject. In one embodiment, the method further comprises administering the effective TNFα inhibitor to a subject to treat psoriasis. The invention provides a method of treating psoriasis in a subject comprising administering an effective amount of a TNFα inhibitor to the subject such that treatment of psoriasis is maintained, wherein the effective human TNFα antibody was previously identified as achieving a PASI 75 response in at least about 32% of a patient population having psoriasis and a baseline PASI < 10.

In one embodiment, the invention provides a method of treatnig psoriasis in a subject comprising administering an effective amount of a human TNFα antibody to the subject such that psoriasis is treated, wherein the effective human TNFα antibody was previously identified as achieving a PASI 75 response in at least about 32% of a patient population having psoriasis and a baseline PASI < 10.

In one embodiment, a PASI 75 response is achieved in at least about 32% of the patient population indicates that the human TNFα antibody is an effective human TNFα antibody for the treatment of psoriasis in a subject. In one embodiment, a PASI 75 response is achieved in at least about 40% of the patient population indicates that the human TNFα antibody is an effective human TNFα antibody for the treatment of psoriasis in a subject. In one embodiment, a PASI 75 response is achieved in at least about 50% of the patient population indicates that the human TNFα antibody is an effective human TNFα antibody for the treatment of psoriasis in a subject. In one embodiment, a PASI 75 response is achieved in at least about 60% of the patient population indicates that the human TNFα antibody is an effective human TNFα antibody for the treatment of psoriasis in a subject. In one embodiment, a PASI 75 response is achieved in at least about 70% of the patient population indicates that the human TNFα antibody is an effective human TNFα antibody for the treatment of psoriasis in a subject.

Numbers intermediate to the above recited percentages, *e.g*., 32%, 33%, 34%. 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, and 89%, as well as all other numbers recited herein, are also intended to be part of this invention. Ranges of values using a combination of any of the above recited values as upper and/or lower limits are intended to be included in the scope of the invention. For example, in one embodiment a PASI 75 response score of in at least between 32% and 90% of the patient population indicates that the TNFα inhibitor is an effective TNFα inhibitor for the treatment of psoriasis in a subject.

In one embodiment the invention provides a method of determining the efficacy of a TNFα inhibitor for achieving a clinical response in psoriasis in a subject comprising determining a PASI 90 response of a patient population having psoriasis and a baseline PASI < 10 and who was administered the human TNFα antibody, wherein a PASI 90 response is achieved in at least about 24% of the patient population indicates that the human TNFα antibody is an effective human TNFα antibody for achieving a clinical resopnse in psoriasis in a subject.

In one embodiment, the invention provides a method for achieving a clinical response in psoriasis in a subject comprising administering an effective amount of a human TNFα antibody to the subject such that a clinical response in psoriasis is achieved, wherein the effective amount of the human TNFα antibody was previously identified as achieving a PASI 90 response in at least about 24% of a patient population having psoriasis and a baseline PASI < 10.

In one embodiment, a PASI 90 response is achieved in at least about 25% of the patient population indicates that the human TNFα antibody is an effective human TNFα antibody for achieving a clinical resopnse in psoriasis in a subject. In one embodiment, a PASI 90 response is achieved in at least about 30% of the patient population indicates that the human TNFα antibody is an effective human TNFα antibody for achieving a clinical resopnse in psoriasis in a subject. In one embodiment, a PASI 90 response is achieved in at least about 40% of the patient population indicates that the human TNFα antibody is an effective human TNFα antibody for achieving a clinical resopnse in psoriasis in a subject. In one embodiment, a PASI 90 response is achieved in at least about 50% of the patient population indicates that the human TNFα antibody is an effective human TNFα antibody for achieving a clinical resopnse in psoriasis in a subject. In one embodiment, a PASI 90 response is achieved in at least about 60% of the patient population indicates that the human TNFα antibody is an effective human TNFα antibody for achieving a clinical resopnse in psoriasis in a subject. In one embodiment, a PASI 90 response is achieved in at least about 62% of the patient population indicates that the human TNFα antibody is an effective human TNFα antibody for achieving a clinical resopnse in psoriasis in a subject.

Numbers intermediate to the above recited percentages, *e.g*., 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, and 61%, as well as all other numbers recited herein, are also intended to be part of this invention. Ranges of values using a combination of any of the above recited values as upper and/or lower limits are intended to be included in the scope of the invention. For example, in one embodiment a PASI 90 response score of in at least between 24% and 62% of the patient population indicates that the TNFα inhibitor is an effective TNFα inhibitor for the treatment of psoriasis in a subject.

In one embodiment the invention provides a method of determining the efficacy of a TNFα inhibitor for achieving a clinical response in psoriasis in a subject comprising determining a PASI 100 response of a patient population having psoriasis and a baseline PASI < 10 and who was administered the human TNFα antibody, wherein a PASI 100 response is achieved in at least about 11% of the patient population indicates that the human TNFα antibody is an effective human TNFα antibody for achieving a clinical response in psoriasis in a subject.

In one embodiment, a PASI 100 response is achieved in at least about 15% of the patient population indicates that the human TNFα antibody is an effective human TNFα antibody for achieving a clinical resopnse in psoriasis in a subject. In one embodiment, a PASI 100 response is achieved in at least about 20% of the patient population indicates that the human TNFα antibody is an effective human TNFα antibody for achieving a clinical resopnse in psoriasis in a subject. In one embodiment, a PASI 100 response is achieved in at least about 25% of the patient population indicates that the human TNFα antibody is an effective human TNFα antibody for achieving a clinical resopnse in psoriasis in a subject. In one embodiment, a PASI 100 response is achieved in at least about 30% of the patient population indicates that the human TNFα antibody is an effective human TNFα antibody for achieving a clinical resopnse in psoriasis in a subject. In one embodiment, a PASI 100 response is achieved in at least about 35% of the patient population indicates that the human TNFα antibody is an effective human TNFα antibody for achieving a clinical resopnse in psoriasis in a subject.

Numbers intermediate to the above recited percentages, *e.g*., 15%, 16%, 17%, 18%, 19%, 20%, 21%, 22%, 23%, 24%, 25%, 26%, 27%, 28%, 29%, 30%, 31%, 32%, 33%, and 34% as well as all other numbers recited herein, are also intended to be part of this invention. Ranges of values using a combination of any of the above recited values as upper and/or lower limits are intended to be included in the scope of the invention. For example, in one embodiment a PASI 100 response score of in at least between 15% and 35% of the patient population indicates that the TNFα inhibitor is an effective TNFα inhibitor for the treatment of psoriasis in a subject.

In one embodiment the invention provides a method of determining the efficacy of a TNFα inhibitor for achieving a clinical response in psoriasis in a subject comprising determining a Physician's Global Assessment (PGA) score of a patient population having psoriasis who was administered the human TNFα antibody, wherein a PGA score of "clear" or "almost clear" in at least about 45% of the patient population indicates that the human TNFα antibody is an effective human TNFα antibody for treating psoriasis in a subject.

In one embodiment, the invention provides a method of treating psoriasis in a subject comprisign administering an effective amount of a human TNFα antibody to the subject, wherein the effective human TNFα antibody was previously identified as maintaining a PGA score of "clear" or "almost clear" in at least about 76% of a patient population having psoriasis.

In one embodiment, a PGA score of "clear" or "almost clear" in at least about 45% of a patient population having psoriasis indicates that the human TNFα antibody is an effective human TNFα antibody for treating psoriasis in a subject. In one embodiment, a PGA score of "clear" or "almost clear" in at least about 76% of a patient population having psoriasis indicates that the human TNFα antibody is an effective human TNFα antibody for treating psoriasis in a subject.

Numbers intermediate to the above recited percentages, e.g., 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, and 75%,, as well as all other numbers recited herein, are also intended to be part of this invention. Ranges of values using a combination of any of the above recited values as upper and/or lower limits are intended to be included in the scope of the invention. For example, in one embodiment a PGA score of "clear" or "almost clear" in at least between 45% and 76% of the patient population indicates that the TNFα inhibitor is an effective TNFα inhibitor for the treatment of psoriasis in a subject.

It should be noted that the Examples provided herein represent different methods of determining the efficacy of a TNFα inhibitor, such as a human TNFα antibody, or antigen-binding portion thereof. As such, data and results described in the Examples section which shows efficacy of a TNFα inhibitor, *e.g*., ability to maintain remission of psoriasis, are included in the methods of determining efficacy of the invention.

Time points for determining efficacy will be understood by those of skill in the art to depend on the type of efficacy being determined, e.g., maintenance of remission. In one embodiment, measurements in scores, e.g., the PASI response or PGA score of a subject, may be measured against a subject's baseline score. Generally, a baseline refers to a measurement or score of a patient before treatment, i.e. week 0. Other time points may also be included as a starting point in determining efficacy, however. For example, in determining the efficacy of a TNFα inhibitor for treating psoriasis in a patient population, a determination of the percentage of the patient population who were achieved a response, *i.e*., PASI 75 response, may be determined based on a time point from when remission was induced.

Patient populations described in the methods of the invention are generally selected based on common characteristics, such as, but not limited to, subjects diagnosed with psoriasis who are in remission as a result of being on a dosing regimen comprising a TNFα inhibitor. Such a patient population would be appropriate for determining the efficacy of the TNFα inhibitor for maintaining remission in psoriasis in the given patient population. In one embodiment, the patient population is an adult population, e.g., older than 17 years of age or older than 18 years of age.

In one embodiment, the methods of the invention for determining whether a TNFα inhibitor is an effective TNFα inhibitor, include determining changes, improvements, measurements, etc., in psoriasis using appropriate indices known in the art, *e.g*., PASI, PGA, DLQI, status of psoriasis related disorders, *etc.* from a patient population who has already been administered the TNFα inhibitor. Such a patient population may be pre-selected according to common characteristics, *e.g*., psoriasis, loss of response to infliximab, and may have already been given the TNFα inhibitor. Administration of the TNFα inhibitor may or may not be performed by the same person of ordinary skill who is determining the efficacy of the TNFα inhibitor in accordance with the teachings of the specificaiton.

In one embodiment, the methods of the invention comprise administering the TNFα inhibitor to the subjects of a patient population and determining the efficacy of the TNFα inhibitor by determining changes, improvements, measurements, etc., using psoriasis indices known in the art, in the patient population in comparison to the Examples set forth below. For example, in one embodiment the invention includes a method for determining efficacy of a TNFα inhibitor for the treatment of psoriasis comprising administering the TNFα inhibitor to a preselected patient population having psoriasis; and determining the effectiveness of the TNFα inhibitor by using a mean baseline Psoriasis Area Severity Index (PASI) response of the patient population and a mean PASI response following administration of the TNFα inhibitor, wherein a PASI 75 response achieved in at least about 77% of the patient population indicates that the TNFα inhibitor is effective for the treatment of psoriasis.

Methods of the invention relating to determining efficacy, *i.e.,* determining whether a TNFα inhibitor is an effective TNFα inhibitor, may also be applied to specific patient populations within the overall patient population who together have specific, common characteristics, *i.e*., a subpopulation. For example, the patient population may comprise patients on concomitant immunosuppressant (IMM) treatment with the TNFα inhibitor. In another example, the patient population may comprises patients not on concomitant IMM treatment.

In addition, while the above methods are described in terms of patient populations, methods of efficacy described herein may also be applied to individual subjects. For example, a method for determining efficacy may comprise determining whether a subject who has psoriasis, and who is on a dosage regimen comprising a human TNFα antibody, is able to achieve a PASI 75 response to determing if the human TNFα antibody is an effective human TNFα antibody. In one embodiment, if the subject is able to achieve a PASI 75 response for at least about 24 weeks, then the human TNFα antibody is effective at treating psoriasis.

In aspect embodiment, the invention provides a method of treating psoriasis in a subject comprising administering an initial loading dose of a human TNFα antibody to the subject at week 0, administering a second dose of the human TNFα antibody to the subject, wherein the second dose is about half the dose amount of the loading dose, and administering at least one a maintenance dose to the subject, wherein the maintenance dose is about half the dose amount of the second dose, such that psoriasis is treated.

In another embodiment of the invention, the initial dose is given in its entirety on one day or is divided over 2 days. In another embodiment of the invention, the second dose is administered to the subject about two weeks after the first dose. In one embodiment of the invention, the maintenance dose is administered to the subject about two weeks after the second dose. In one embodiment of the invention, the maintenance dose is administered on a biweekly dosing regimen.

In one aspect, the invention provides a method of determining the efficacy of a human TNFα antibody for treating psoriasis in a subject comprising determining a Psoriasis Area Severity Index (PASI) 75 response of a patient population having psoriasis and a baseline PASI < 10 and who was administered the human TNFα antibody, wherein a PASI 75 response is achieved in at least about 77% of the patient population indicates that the human TNFα antibody is an effective human TNFα antibody for the treatment of psoriasis in a subject.

In one embodiment, the invention further comprises administering the effective human TNFα antibody to a subject to treat psoriasis. In another embodiment, a PASI 75 response is achieved in at least about 80% of the patient population indicates that the human TNFα antibody is an effective human TNFα antibody for the treatment of psoriasis in a subject. In one embodiment, a PASI 75 response is achieved in at least about 85% of the patient population indicates that the human TNFα antibody is an effective human TNFα antibody for the treatment of psoriasis in a subject. In another embodiment, a PASI 75 response is achieved in at least about 88% of the patient population indicates that the human TNFα antibody is an effective human TNFα antibody for the treatment of psoriasis in a subject. In one embodiment, a PASI 75 response is achieved in at least about 90% of the patient population indicates that the human TNFα antibody is an effective human TNFα antibody for the treatment of psoriasis in a subject.

In one aspect, the invention provides a method of treating psoriasis in a subject comprising administering an effective amount of a human TNFα antibody to the subject such that psoriasis is treated, wherein the effective human TNFα antibody was previously identified as achieving a PASI 75 response in at least about 77% of a patient population having psoriasis and a baseline PASI < 10.

In one embodiment, the effective human TNFα antibody was previously identified as achieving a PASI 75 response in at least about 80% of a patient population having psoriasis and a baseline PASI < 10. In one embodiment, the effective amount of the human TNFα antibody was previously identified as achieving a PASI 75 response in at least about 85% of a patient population having psoriasis and a baseline PASI < 10. In one embodiment, the effective amount of the human TNFα antibody was previously identified as achieving a PASI 75 response in at least about 88% of a patient population having psoriasis and a baseline PASI < 10. In one embodiment, the effective amount of the human TNFα antibody was previously identified as achieving a PASI 75 response in at least about 90% of a patient population having psoriasis and a baseline PASI < 10.

In one aspect, the invention provides a method of determining the efficacy of a human TNFα antibody for achieving a clinical response in psoriasis in a subject comprising determining a Psoriasis Area Severity Index (PASI) 90 response of a patient population having psoriasis and a baseline PASI < 10 and who was administered the human TNFα antibody, wherein a PASI 90 response is achieved in at least about 63% of the patient population indicates that the human TNFα antibody is an effective human TNFα antibody for achieving a clinical response in psoriasis in a subject.

In one embodiment, the method further comprises administering the effective human TNFα antibody to a subject to achieve a clinical response in psoriasis. In one embodiment, a PASI 90 response is achieved in at least about 65% of the patient population indicates that the human TNFα antibody is an effective human TNFα antibody for achieving a clinical response in psoriasis in a subject. In one embodiment, a PASI 90 response is achieved in at least about 68% of the patient population indicates that the human TNFα antibody is an effective human TNFα antibody for achieving a clinical response in psoriasis in a subject. In another embodiment, a PASI 90 response is achieved in at least about 70% of the patient population indicates that the human TNFα antibody is an effective human TNFα antibody for achieving a clinical response in psoriasis in a subject. In one embodiment, a PASI 90 response is achieved in at least about 75% of the patient population indicates that the human TNFα antibody is an effective human TNFα antibody for achieving a clinical response in psoriasis in a subject. In one embodiment, a PASI 90 response is achieved in at least about 80% of the patient population indicates that the human TNFα antibody is an effective human TNFα antibody for achieving a clinical response in psoriasis in a subject.

In one aspect, the invention provides a method of achieving a clinical response in psoriasis in a subject comprising administering an effective amount of a human TNFα antibody to the subject such that a clinical response in psoriasis is achieved, wherein the effective amount of the human TNFα antibody was previously identified as achieving a PASI 90 response in at least about 63% of a patient population having psoriasis and a baseline PASI < 10.

In one embodiment, the effective amount of the human TNFα antibody was previously identified as achieving a PASI 90 response in at least about 65% of a patient population having psoriasis and a baseline PASI < 10. In another embodiment, the effective amount of the human TNFα antibody was previously identified as as achieving a PASI 90 response in at least about 68% of a patient population having psoriasis and a baseline PASI < 10. In one embodiment, the effective amount of the human TNFα antibody was previously identified as as achieving a PASI 90 response in at least about 70% of a patient population having psoriasis and a baseline PASI < 10. In one embodiment, the effective amount of the human TNFα antibody was previously identified as as achieving a PASI 90 response in at least about 75% of a patient population having psoriasis and a baseline PASI < 10. In one embodiment, the effective amount of the human TNFα antibody was previously identified as as achieving a PASI 90 response in at least about 80% of a patient population having psoriasis and a baseline PASI < 10.

In one aspect, the invention provides a method of determining the efficacy of a human TNFα antibody, or antigen-binding portion thereof, for achieving a clinical response in psoriasis in a subject comprising determining a Psoriasis Area Severity Index (PASI) 100 response of a patient population having psoriasis and who was administered the human TNFα antibody, or antigen-binding portion thereof, wherein a PASI 100 response is achieved in at least about 36% of the patient population indicates that the human TNFα antibody, or antigen-binding portion thereof, is an effective human TNFα antibody, or antigen-binding portion thereof, for achieving a clinical response in psoriasis in a subject.

In one embodiment, the method comprises administering the effective human TNFα antibody, or antigen-binding portion thereof, to a subject. In one embodiment, a PASI 100 response is achieved in at least about 38% of the patient population indicates that the human TNFα antibody, or antigen-binding portion thereof, is an effective human TNFα antibody, or antigen-binding portion thereof, for achieving a clinical response in psoriasis in a subject. In one embodiment, a PASI 100 response is achieved in at least about 40% of the patient population indicates that the human TNFα antibody, or antigen-binding portion thereof, is an effective human TNFα antibody, or antigen-binding portion thereof, for achieving a clinical response in psoriasis in a subject. In one embodiment, a PASI 100 response is achieved in at least about 45% of the patient population indicates that the human TNFα antibody, or antigen-binding portion thereof, is an effective human TNFα antibody, or antigen-binding portion thereof, for achieving a clinical response in psoriasis in a subject. In one embodiment, a PASI 100 response is achieved in at least about 48% of the patient population indicates that the human TNFα antibody, or antigen-binding portion thereof, is an effective human TNFα antibody, or antigen-binding portion thereof, for achieving a clinical response in psoriasis in a subject. In one embodiment, a PASI 100 response is achieved in at least about 50% of the patient population indicates that the human TNFα antibody, or antigen-binding portion thereof, is an effective human TNFα antibody, or antigen-binding portion thereof, for achieving a clinical response in psoriasis in a subject.

In one aspect, the invention provides a method of determining the efficacy of a human TNFα antibody to treat psoriasis in a subject comprising determining a Physician's Global Assessment (PGA) score of a patient population having psoriasis who was administered the human TNFα antibody, wherein a PGA score of "clear" or "almost clear" in at least about 77% of the patient population indicates that the human TNFα antibody is an effective human TNFα antibody for treating psoriasis in a subject. In one embodiment, the method comprises administering the effective human TNFα antibody to a subject having psoriasis. In one embodiment, a PGA score of "clear" or "almost clear" in at least about 80% of the patient population indicates that the human TNFα antibody is an effective human TNFα antibody for treating psoriasis in a subject.

In one aspect, the invention provides a method of treating psoriasis in a subject comprising administering an effective amount of a human TNFα antibody to the subject, wherein the effective human TNFα antibody was previously identified as maintaining an PGA score of "clear" or "almost clear" in at least about 77% of a patient population having psoriasis. In one embodiment, the effective human TNFα antibody was previously identified as maintaining an PGA score of "clear" or "almost clear" in at least about 80% of a patient population having psoriasis.

In one embodiment, the human TNFα antibody is a human TNFα antibody or antigen-binding portion thereof. In another embodiment, the human TNFα antibody, or an antigen-binding portion thereof, dissociates from human TNFα with a K_{d} of 1 x 10⁻⁸ M or less and a K_{off} rate constant of 1 x 10⁻³ s⁻¹ or less, both determined by surface plasmon resonance, and neutralizes human TNFα cytotoxicity in a standard *in vitro* L929 assay with an IC₅₀ of 1 x 10⁻⁷ M or less. In one embodiment, the human TNFα antibody, or an antigen-binding portion thereof, has the following characteristics:
a) dissociates from human TNFα with a K_{off} rate constant of 1 x 10⁻³ s⁻¹ or less, as determined by surface plasmon resonance;
b) has a light chain CDR3 domain comprising the amino acid sequence of SEQ ID NO: 3, or modified from SEQ ID NO: 3 by a single alanine substitution at position 1, 4, 5, 7 or 8 or by one to five conservative amino acid substitutions at positions 1, 3, 4, 6, 7, 8 and/or 9;
c) has a heavy chain CDR3 domain comprising the amino acid sequence of SEQ ID NO: 4, or modified from SEQ ID NO: 4 by a single alanine substitution at position 2, 3, 4, 5, 6, 8, 9, 10 or 11 or by one to five conservative amino acid substitutions at positions 2, 3, 4, 5, 6, 8, 9, 10, 11 and/or 12.

In one embodiment, the human TNFα antibody, or an antigen-binding portion thereof, comprises a light chain variable region (LCVR) having a CDR3 domain comprising the amino acid sequence of SEQ ID NO: 3, or modified from SEQ ID NO: 3 by a single alanine substitution at position 1, 4, 5, 7 or 8, and comprises a heavy chain variable region (HCVR) having a CDR3 domain comprising the amino acid sequence of SEQ ID NO: 4, or modified from SEQ ID NO: 4 by a single alanine substitution at position 2, 3, 4, 5, 6, 8, 9, 10 or 11. In one embodiment, the human TNFα antibody, or an antigen-binding portion thereof, comprises a light chain variable region (LCVR) comprising the amino acid sequence of SEQ ID NO: I and a heavy chain variable region (HCVR) comprising the amino acid sequence of SEQ ID NO: 2.

In another embodiment, the human TNFα antibody, or an antigen-binding portion thereof, is adalimumab.

In one embodiment, the human TNFα antibody, or an antigen-binding portion thereof, was administered to the patient population on a biweekly dosing regimen. In one embodiment, the human TNFα antibody, or an antigen-binding portion thereof, was administered in a dose of about 40 mg.

In one aspect, the invention provides a method of achieving a clinical response in psoriasis in a subject comprising administering an effective amount of a human TNFα antibody, or antigen-binding portion thereof, to the subject such that a clinical response in psoriasis is achieved, wherein the effective human TNFα antibody, or antigen-binding portion thereof, was previously identified as achieving a PASI 75 response in at least about 77% of a patient population having psoriasis.

In one embodiment, the effective human TNFα antibody, or antigen-binding portion thereof, was previously identified as achieving a PASI 75 response in at least about 78% of a patient population having psoriasis. In one embodiment, the effective amount of the human TNFα antibody, or antigen-binding portion thereof, was previously identified as achieving a PASI 75 response in at least about 80% of a patient population having psoriasis. In one embodiment, the effective amount of the human TNFα antibody, or antigen-binding portion thereof, was previously identified as achieving a PASI 75 response in at least about 85% of a patient population having psoriasis. In one embodiment, the effective amount of the human TNFα antibody, or antigen-binding portion thereof, was previously identified as achieving a PASI 75 response in at least about 88% of a patient population having psoriasis. In one embodiment, the effective amount of the human TNFα antibody, or antigen-binding portion thereof, was previously identified as achieving a PASI 75 response in at least about 90% of a patient population having psoriasis.

In one aspect, the invention further provides the of a human TNFα antibody, or antigen-binding portion thereof, in the manufacture of a medicament for treating psoriasis in a subject.

In another aspect, the invention further provides a method of treating psoriasis in a subject comprising administering an initial loading dose of a human TNFα antibody or antigen-binding portion thereof, to the subject at week 0, administering a second dose of the human TNFα antibody or antigen-binding portion thereof, to the subject, wherein the second dose is about half the dose amount of the loading dose, and administering at least one a maintenance dose of the human TNFα antibody or antigen-binding portion thereof, to the subject, wherein the maintenance dose is about half the dose amount of the second dose, such that psoriasis is treated.

In one embodiment, the initial dose is given in its entirety on one day or is divided over 2 days. In one embodiment, the second dose is administered to the subject about two weeks after the first dose. In one embodiment, the maintenance dose is administered to the subject about two weeks after the second dose. In one embodiment, the maintenance dose is administered on a biweekly dosing regimen.

In one embodiment, the human TNFα antibody, or an antigen-binding portion thereof, dissociates from human TNFα with a K_{d} of 1 x 10⁻⁸ M or less and a K_{off} rate constant of 1 x 10⁻³ s⁻¹ or less, both determined by surface plasmon resonance, and neutralizes human TNFα cytotoxicity in a standard *in vitro* L929 assay with an IC₅₀ of 1 x 10⁻⁷ M or less.

In one embodiment, the human TNFα antibody, or an antigen-binding portion thereof, has the following characteristics:
a) dissociates from human TNFα with a K_{off} rate constant of 1 x 10⁻³ s⁻¹ or less, as determined by surface plasmon resonance;
b) has a light chain CDR3 domain comprising the amino acid sequence of SEQ ID NO: 3, or modified from SEQ ID NO: 3 by a single alanine substitution at position 1, 4, 5, 7 or 8 or by one to five conservative amino acid substitutions at positions 1, 3, 4, 6, 7, 8 and/or 9;
c) has a heavy chain CDR3 domain comprising the amino acid sequence of SEQ ID NO: 4, or modified from SEQ ID NO: 4 by a single alanine substitution at position 2, 3, 4, 5, 6, 8, 9, 10 or 11 or by one to five conservative amino acid substitutions at positions 2, 3, 4, 5, 6, 8, 9, 10, 11 and/or 12.

In one embodiment, the human TNFα antibody, or an antigen-binding portion thereof, comprises a light chain variable region (LCVR) having a CDR3 domain comprising the amino acid sequence of SEQ ID NO: 3, or modified from SEQ ID NO: 3 by a single alanine substitution at position 1, 4, 5, 7 or 8, and comprises a heavy chain variable region (HCVR) having a CDR3 domain comprising the amino acid sequence of SEQ ID NO: 4, or modified from SEQ ID NO: 4 by a single alanine substitution at position 2, 3, 4, 5, 6, 8, 9, 10 or 11. In another embodiment, the human TNFα antibody, or an antigen-binding portion thereof, comprises a light chain variable region (LCVR) comprising the amino acid sequence of SEQ ID NO: 1 and a heavy chain variable region (HCVR) comprising the amino acid sequence of SEQ ID NO: 2.

In one embodiment, the human TNFα antibody, or an antigen-binding portion thereof, is adalimumab.

In one aspect, the invention provides an article of manufacture comprising a human TNFα antibody and a package insert, wherein the package insert indicates the recommended human TNFα antibody dose regimen for adult patients with psoriasis is 80 mg at week 0, 80 mg at week 1, followed by 40 mg every other week beginning at week 3.

In one embodiment, the human TNFα antibody is a human TNFα antibody, or an antigen-binding portion thereof. In another embodiment, the human TNFα antibody, or an antigen-binding portion thereof, dissociates from human TNFα with a K_{d} of 1 x 10⁻⁸ M or less and a K_{off} rate constant of 1 x 10⁻³ s⁻¹ or less, both determined by surface plasmon resonance, and neutralizes human TNFα cytotoxicity in a standard *in vitro* L929 assay with an IC₅₀ of 1 x 10⁻⁷ M or less. In one embodiment, the human TNFα antibody, or an antigen-binding portion thereof, comprises a light chain variable region (LCVR) having a CDR3 domain comprising the amino acid sequence of SEQ ID NO: 3, or modified from SEQ ID NO: 3 by a single alanine substitution at position 1, 4, 5, 7 or 8, and comprises a heavy chain variable region (HCVR) having a CDR3 domain comprising the amino acid sequence of SEQ ID NO: 4, or modified from SEQ ID NO: 4 by a single alanine substitution at position 2, 3, 4, 5, 6, 8, 9, 10 or 11. In another embodiment, the human TNFα antibody, or an antigen-binding portion thereof, is adalimumab.

In one aspect, the invention provides an article of manufacture which comprising adalimumab and a package insert, wherein the package insert indicates that the adalimumab may be used to treat psoriasis in patients who have had an inadequate response to conventional therapy and/or who have lost response to or are intolerant to infliximab.

In another aspect, the invention provides an article of manufacture comprising a) a packaging material; b) a human TNFα antibody, or antigen-binding portion thereof; and c) a package insert contained within the packaging material indicating that aminosalicylates, corticosteroids, and/or immunomodulatory agent, e.g., 6-mercaptopurine and azathioprine, may be continued during treatment with the TNFα antibody, or antigen-binding portion thereof.

In another aspect, the invention provides an article of manufacture comprising:
a) a packaging material; b) a human TNFα antibody, and c) a label or package insert contained within the packaging material indicating that a history of systemic or biologic therapy does not adversely effect efficacy of the human TNFα antibody in patients and/or that administration of the human TNFα antibody is safe in patients with a history of systemic or biologic therapy.

In one aspect, the invention provides an article of manufacture comprising:
a) a packaging material; b) a human TNFα antibody, and c) a label or package insert contained within the packaging material indicating that a use of TNF blockers have been associated with reactivation of hepatitis B virus (HBV) in patients who are chronic carriers of the virus.

In one aspect, the invention provides an article of manufacture comprising:
a) a packaging material; b) a human TNFα antibody, and c)a label or package insert contained within the packaging material indicating that an adverse event which has been reported in the use of the human TNFα antibody is angioneurotic edema.

In one aspect, the invention provides an article of manufacture comprising:
a) a packaging material; b) a human TNFα antibody, and c) a label or package insert contained within the packaging material indicating that the human TNFα antibody may be used as a first line treatment for the treatment of psoriasis.

In one aspect, the invention provides an article of manufacture comprising:
a) a packaging material; b) a human TNFα antibody, and c) a label or package insert contained within the packaging material indicating that the human TNFα antibody may be used for the treatment of psoriasis without methotrexate.

In another aspect, the invention provides an article of manufacture comprising:
a) a packaging material; b) a human TNFα antibody, and c) a label or package insert contained within the packaging material indicating that the human TNFα antibody was found to be more effective than methotrexate as a first line treatment and/or that the human TNFα antibody has significantly superior efficacy for the treatment of moderate to severe psoriasis versus methotrexate.

In one aspect, the invention provides a method of decreasing a DLQI score of a subject having psoriasis by at least about 10 points comprising administering a human TNFα antibody to the subject, such that the DLQI score is decreased by at least about 10 points.

In another aspect, the invention provides a method of treating a subtherapeutic response in a subject having psoriasis comprising administering a human TNFα antibody to the subject at an increased dosing rate which is about twice as frequent as the original dosing rate. In one embodiment, the increased dosing rate is weekly. In another embodiment, the subtherapeutic response comprises < PASI 50 improvement from baseline (week 0) and a determined time period following baseline. In one embodiment, the determined time period following baseline is at least about 24 weeks.

In one aspect, the invention provides a method of decreasing a DLQI score of a subject having psoriasis from a large/extremely large score to a no or small impact score comprising administering a human TNFα antibody to the subject, such that the DLQI score is decreased from the large/extremely large score to the no or small impact score.

In another aspect, the invention provides a method of decreasing a DLQI score of a subject having psoriasis by at least about 10 points comprising administering a human TNFα antibody to the subject, such that the DLQI score is decreased by at least about 10 points.

In one aspect, the invention provides a method of decreasing a PASI score of a subject having psoriasis by at least about 8 points comprising administering a human TNFα antibody to the subject, such that the PASI score is decreased by at least about 8 points.

In one aspect, the invention provides a method of decreasing a PGA score of a subject having psoriasis by at least about 2 points comprising administering a human TNFα antibody to the subject, such that the PGA score is decreased by at least about 2 points.

In one aspect, the invention provides a method for determining the efficacy of a TNFα inhibitor for improving the functional limitations of a subject having psoriasis comprising determining an improvement in a Dermatology Life Quality Index (DLQI) score from a patient population who was administered the TNFα inhibitor, wherein a DLQI score of no or small impact in at least about 83% of the patient population indicates that the TNFα inhibitor is efficacious for improving the functional limitations of human subjects having psoriasis.

In one embodiment, the subject has a PASI score of ≥ 10 and a DLQI score >10.

In one aspect, the invention provides the use of a human TNFα antibody in the manufacture of a medicament for the treatment of psoriasis in a subject comprising administering an initial loading dose of a human TNFα antibody to the subject at week 0, administering a second dose of the human TNFα antibody to the subject, wherein the second dose is about half the dose amount of the loading dose, and administering at least one a maintenance dose to the subject, wherein the maintenance dose is about half the dose amount of the second dose, such that psoriasis is treated.

In one embodiment, the initial dose is given in its entirety on one day or is divided over 2 days. In another embodiment, the second dose is administered to the subject about two weeks after the first dose. In another embodiment, the maintenance dose is administered to the subj ect about two weeks after the second dose. In one embodiment, the maintenance dose is administered on a biweekly dosing regimen.

In one aspect, the invention provides the use of a human TNFα antibody in the manufacture of a medicament for the treatment of psoriasis comprising administering an effective amount of a human TNFα antibody to the subject such that psoriasis is treated, wherein the effective human TNFα antibody was previously identified as achieving a PASI 75 response in at least about 77% of a patient population having psoriasis and a baseline PASI < 10.

In embodiment, the effective human TNFα antibody was previously identified as achieving a PASI 75 response in at least about 80% of a patient population having psoriasis and a baseline PASI < 10. In one embodiment, the effective amount of the human TNFα antibody was previously identified as achieving a PASI 75 response in at least about 85% of a patient population having psoriasis and a baseline PASI < 10. In one embodiment, the effective amount of the human TNFα antibody was previously identified as achieving a PASI 75 response in at least about 88% of a patient population having psoriasis and a baseline PASI < 10. In one embodiment, the effective amount of the human TNFα antibody was previously identified as achieving a PASI 75 response in at least about 90% of a patient population having psoriasis and a baseline PASI < 10.

In one aspect, the invention provides the use of a human TNFα antibody in the manufacture of a medicament for the treatment of psoriasis comprising administering an effective amount of a human TNFα antibody to the subject, wherein the effective human TNFα antibody was previously identified as maintaining an PGA score of "clear" or "almost clear" in at least about 77% of a patient population having psoriasis.

In one embodiment, the effective human TNFα antibody was previously identified as maintaining an PGA score of "clear" or "almost clear" in at least about 80% of a patient population having psoriasis. In one embodiment, the human TNFα antibody is a human TNFα antibody or antigen-binding portion thereof. In one embodiment, the human TNFα antibody, or an antigen-binding portion thereof, dissociates from human TNFα with a K_{d} of 1 x 10⁻⁸ M or less and a K_{off} rate constant of 1 x 10⁻³ s⁻¹ or less, both determined by surface plasmon resonance, and neutralizes human TNFα cytotoxicity in a standard *in vitro* L929 assay with an IC₅₀ of 1 x 10⁻⁷ M or less. In one embodiment, the human TNFα antibody, or an antigen-binding portion thereof, has the following characteristics:
a) dissociates from human TNFα with a K_{off} rate constant of 1 x 10⁻³ s⁻¹ or less, as determined by surface plasmon resonance;
b) has a light chain CDR3 domain comprising the amino acid sequence of SEQ ID NO: 3, or modified from SEQ ID NO: 3 by a single alanine substitution at position 1, 4, 5, 7 or 8 or by one to five conservative amino acid substitutions at positions 1, 3, 4, 6, 7, 8 and/or 9;
c) has a heavy chain CDR3 domain comprising the amino acid sequence of SEQ ID NO: 4, or modified from SEQ ID NO: 4 by a single alanine substitution at position 2, 3, 4, 5, 6, 8, 9, 10 or 11 or by one to five conservative amino acid substitutions at positions 2, 3, 4, 5, 6, 8, 9, 10, 11 and/or 12.

In one embodiment, the human TNFα antibody, or an antigen-binding portion thereof, comprises a light chain variable region (LCVR) having a CDR3 domain comprising the amino acid sequence of SEQ ID NO: 3, or modified from SEQ ID NO: 3 by a single alanine substitution at position 1, 4, 5, 7 or 8, and comprises a heavy chain variable region (HCVR) having a CDR3 domain comprising the amino acid sequence of SEQ ID NO: 4, or modified from SEQ ID NO: 4 by a single alanine substitution at position 2, 3, 4, 5, 6, 8, 9, 10 or 11. In another embodiment, the human TNFα antibody, or an antigen-binding portion thereof, comprises a light chain variable region (LCVR) comprising the amino acid sequence of SEQ ID NO: 1 and a heavy chain variable region (HCVR) comprising the amino acid sequence of SEQ ID NO: 2.

In one embodiment, the human TNFα antibody, or an antigen-binding portion thereof, is adalimumab. In one embodiment, the human TNFα antibody, or an antigen-binding portion thereof, was administered to the patient population on a biweekly dosing regimen. In one embodiment, the human TNFα antibody, or an antigen-binding portion thereof, was administered in a dose of about 40 mg.

In one aspect, the invention provides the use of a human TNFα antibody, or antigen-binding portion thereof, in the manufacture of a medicament for treating psoriasis in a subject.

In one aspect, the invention provides the use of a human TNF α antibody in the manufacture of a medicament for the treatment of psoriasis comprising administering an initial loading dose of a human TNFα antibody or antigen-binding portion thereof, to the subject at week 0, administering a second dose of the human TNFα antibody or antigen-binding portion thereof, to the subject, wherein the second dose is about half the dose amount of the loading dose, and administering at least one a maintenance dose of the human TNFα antibody or antigen-binding portion thereof, to the subject, wherein the maintenance dose is about half the dose amount of the second dose, such that psoriasis is treated.

In one embodiment, the initial dose is given in its entirety on one day or is divided over 2 days. In one embodiment, the second dose is administered to the subject about two weeks after the first dose. In one embodiment, the maintenance dose is administered to the subject about two weeks after the second dose. In one embodiment, the maintenance dose is administered on a biweekly dosing regimen.

In one embodiment, the human TNFα antibody, or an antigen-binding portion thereof, dissociates from human TNFα with a K_{d} of 1 x 10⁻⁸ M or less and a K_{off} rate constant of 1 x 10⁻³ s⁻¹ or less, both determined by surface plasmon resonance, and neutralizes human TNFα cytotoxicity in a standard *in vitro* L929 assay with an IC₅₀ of 1 x 10⁻⁷ M or less.

In another embodiment, the human TNFα antibody, or an antigen-binding portion thereof, has the following characteristics:
a) dissociates from human TNFα with a K_{off} rate constant of 1 x 10⁻³ s⁻¹ or less, as determined by surface plasmon resonance;
b) has a light chain CDR3 domain comprising the amino acid sequence of SEQ ID NO: 3, or modified from SEQ ID NO: 3 by a single alanine substitution at position 1, 4, 5, 7 or 8 or by one to five conservative amino acid substitutions at positions 1, 3, 4, 6, 7, 8 and/or 9;
c) has a heavy chain CDR3 domain comprising the amino acid sequence of SEQ ID NO: 4, or modified from SEQ ID NO: 4 by a single alanine substitution at position 2, 3, 4, 5, 6, 8, 9, 10 or 11 or by one to five conservative amino acid substitutions at positions 2, 3, 4, 5, 6, 8, 9, 10, 11 and/or 12.

In one embodiment, the human TNFα antibody, or an antigen-binding portion thereof, comprises a light chain variable region (LCVR) having a CDR3 domain comprising the amino acid sequence of SEQ ID NO: 3, or modified from SEQ ID NO: 3 by a single alanine substitution at position 1, 4, 5, 7 or 8, and comprises a heavy chain variable region (HCVR) having a CDR3 domain comprising the amino acid sequence of SEQ ID NO: 4, or modified from SEQ ID NO: 4 by a single alanine substitution at position 2, 3, 4, 5, 6, 8, 9, 10 or 11. In one embodiment, the human TNFα antibody, or an antigen-binding portion thereof, comprises a light chain variable region (LCVR) comprising the amino acid sequence of SEQ ID NO: 1 and a heavy chain variable region (HCVR) comprising the amino acid sequence of SEQ ID NO: 2.

In one embodiment, the human TNFα antibody, or an antigen-binding portion thereof, is adalimumab.

In one aspect, the invention provides the use of a human TNFα antibody in the treatment of a subtherapeutic response in a subject having psoriasis comprising administering a human TNFα antibody to the subject at an increased dosing rate which is about twice as frequent as the original dosing rate.

In one embodiment, the increased dosing rate is weekly. In one embodiment, the subtherapeutic response comprises < PASI 50 improvement from baseline (week 0) and a determined time period following baseline. In one embodiment, the determined time period following baseline is at least about 24 weeks.

In one embodiment, the subject has a PASI score of ≥ 10 and a DLQI score >10.

### BRIEF DESCRIPTION OF THE DRAWINGS

***Figure 1*** shows the study design from Phase II clinical trial.
***Figure 2*** shows the PASI improvement in patients after dosage escalation.
***Figure 3*** shows the study design for Example 8.
***Figure 4*** shows a graph describing mean percentage PASI improvement
***Figure 5*** describes the study design described in Example 10.
***Figure 6*** graphically depicts the mean percentage PASI improvement through week 60.
***Figure 7*** describes the study design of Example 11.
***Figure 8*** describes the study design of Example 12.
***Figure 9*** describes patient disposition for Example 12.

### DETAILED DESCRIPTION OF THE INVENTION

### I. Definitions

The term "human TNFα" (abbreviated herein as hTNFα, or simply hTNF), as used herein, is intended to refer to a human cytokine that exists as a 17 kD secreted form and a 26 kD membrane associated form, the biologically active form of which is composed of a trimer of noncovalently bound 17 kD molecules. The structure of hTNFα is described further in, for example, Pennica, D., et al. (1984) Nature 312:724-729; Davis, J.M., et al. (1987) Biochemistry 26:1322-1326; and Jones, E.Y., et al. (1989) Nature 338:225-228. The term human TNFα is intended to include recombinant human TNFα (rhTNFα), which can be prepared by standard recombinant expression methods or purchased commercially (R & D Systems, Catalog No. 210-TA, Minneapolis, MN). TNFα is also referred to as TNF.

The term "TNFα inhibitor" includes agents which interfere with TNFα activity. The term also includes each of the anti-TNFα human antibodies and antibody portions described herein as well as those described in U.S. Patent Nos. 6,090,382; 6,258,562; 6,509,015, and in U.S. Patent Application Serial Nos. 09/801185 and 10/302356. In one embodiment, the TNFα inhibitor used in the invention is an anti-TNFα antibody, or a fragment thereof, including infliximab (Remicade^{®}, Johnson and Johnson; described in U.S. Patent No. 5,656,272, incorporated by reference herein), CDP571 (a humanized monoclonal anti-TNF-alpha IgG4 antibody), CDP 870 (a humanized monoclonal anti-TNF-alpha antibody fragment), an anti-TNF dAb (Peptech), CNTO 148 (golimumab; Medarex and Centocor, see WO 02/12502), and adalimumab (HUMIRA®^{®} Abbott Laboratories, a human anti-TNF mAb, described in US 6,090,382 as D2E7). Additional TNF antibodies which may be used in the invention are described in U.S. Patent Nos. 6,593,458; 6,498,237; 6,451,983; and 6,448,380, each of which is incorporated by reference herein. In another embodiment, the TNFα inhibitor is a TNF fusion protein, e.g., etanercept (Enbrel^{®}, Amgen; described in WO 91/03553 and WO 09/406476, incorporated by reference herein). In another embodiment, the TNFα inhibitor is a recombinant TNF binding protein (r-TBP-I) (Serono).

The term "antibody", as used herein, is intended to refer to immunoglobulin molecules comprised of four polypeptide chains, two heavy (H) chains and two light (L) chains inter-connected by disulfide bonds. Each heavy chain is comprised of a heavy chain variable region (abbreviated herein as HCVR or VH) and a heavy chain constant region. The heavy chain constant region is comprised of three domains, CH1, CH2 and CH3. Each light chain is comprised of a light chain variable region (abbreviated herein as LCVR or VL) and a light chain constant region. The light chain constant region is comprised of one domain, CL. The VH and VL regions can be further subdivided into regions of hypervariability, termed complementarity determining regions (CDR), interspersed with regions that are more conserved, termed framework regions (FR). Each VH and VL is composed of three CDRs and four FRs, arranged from amino-terminus to carboxy-terminus in the following order: FR1, CDR1, FR2, CDR2, FR3, CDR3, FR4. The antibodies of the invention are described in further detail in U.S. Patent Nos. 6,090,382; 6,258,562; and 6,509,015, each of which is incorporated herein by reference in its entirety.

The term "antigen-binding portion" or "antigen-binding fragment" of an antibody (or simply "antibody portion"), as used herein, refers to one or more fragments of an antibody that retain the ability to specifically bind to an antigen (e.g., hTNFα). It has been shown that the antigen-binding function of an antibody can be performed by fragments of a full-length antibody. Binding fragments include Fab, Fab', F(ab')₂, Fabc, Fv, single chains, and single-chain antibodies. Examples of binding fragments encompassed within the term "antigen-binding portion" of an antibody include (i) a Fab fragment, a monovalent fragment consisting of the VL, VH, CL and CH1 domains; (ii) a F(ab')₂ fragment, a bivalent fragment comprising two Fab fragments linked by a disulfide bridge at the hinge region; (iii) a Fd fragment consisting of the VH and CH1 domains; (iv) a Fv fragment consisting of the VL and VH domains of a single arm of an antibody, (v) a dAb fragment (Ward et al. (1989) Nature 341:544-546 ), which consists of a VH domain; and (vi) an isolated complementarity determining region (CDR). Furthermore, although the two domains of the Fv fragment, VL and VH, are coded for by separate genes, they can be joined, using recombinant methods, by a synthetic linker that enables them to be made as a single protein chain in which the VL and VH regions pair to form monovalent molecules (known as single chain Fv (scFv); see *e.g.,* Bird et al. (1988) Science 242:423-426; and Huston et al. (1988) Proc. Natl. Acad. Sci. USA 85:5879-5883). Such single chain antibodies are also intended to be encompassed within the term "antigen-binding portion" of an antibody. Other forms of single chain antibodies, such as diabodies are also encompassed. Diabodies are bivalent, bispecific antibodies in which VH and VL domains are expressed on a single polypeptide chain, but using a linker that is too short to allow for pairing between the two domains on the same chain, thereby forcing the domains to pair with complementary domains of another chain and creating two antigen binding sites (see *e.g.,* Holliger et al. (1993) Proc. Natl. Acad. Sci. USA 90:6444-6448; Poljak et al. (1994) Structure 2:1121-1123). The antibody portions of the invention are described in further detail in U.S. Patent Nos. 6,090,382, 6,258,562, 6,509,015, each of which is incorporated herein by reference in its entirety.

Still further, an antibody or antigen-binding portion thereof may be part of a larger immunoadhesion molecules, formed by covalent or noncovalent association of the antibody or antibody portion with one or more other proteins or peptides. Examples of such immunoadhesion molecules include use of the streptavidin core region to make a tetrameric scFv molecule (Kipriyanov, S.M., et al. (1995) Human Antibodies and Hybridomas 6:93-101) and use of a cysteine residue, a marker peptide and a C-terminal polyhistidine tag to make bivalent and biotinylated scFv molecules (Kipriyanov, S.M., et al. (1994) Mol. Immunol. 31:1047-1058). Antibody portions, such as Fab and F(ab')₂ fragments, can be prepared from whole antibodies using conventional techniques, such as papain or pepsin digestion, respectively, of whole antibodies. Moreover, antibodies, antibody portions and immunoadhesion molecules can be obtained using standard recombinant DNA techniques, as described herein.

A "conservative amino acid substitution", as used herein, is one in which one amino acid residue is replaced with another amino acid residue having a similar side chain. Families of amino acid residues having similar side chains have been defined in the art, including basic side chains (*e.g.,* lysine, arginine, histidine), acidic side chains (*e.g.,* aspartic acid, glutamic acid), uncharged polar side chains (*e.g.,* glycine, asparagine, glutamine, serine, threonine, tyrosine, cysteine), nonpolar side chains (*e.g.,* alanine, valine, leucine, isoleucine, proline, phenylalanine, methionine, tryptophan), beta-branched side chains (*e.g.,* threonine, valine, isoleucine) and aromatic side chains (*e.g.,* tyrosine, phenylalanine, tryptophan, histidine).

"Chimeric antibodies" refers to antibodies wherein one portion of each of the amino acid sequences of heavy and light chains is homologous to corresponding sequences in antibodies derived from a particular species or belonging to a particular class, while the remaining segment of the chains is homologous to corresponding sequences from another species. In one embodiment, the invention features a chimeric antibody or antigen-binding fragment, in which the variable regions of both light and heavy chains mimics the variable regions of antibodies derived from one species of mammals, while the constant portions are homologous to the sequences in antibodies derived from another species. In a preferred embodiment of the invention, chimeric antibodies are made by grafting CDRs from a mouse antibody onto the framework regions of a human antibody.

"Humanized antibodies" refer to antibodies which comprise at least one chain comprising variable region framework residues substantially from a human antibody chain (referred to as the acceptor immunoglobulin or antibody) and at least one complementarity determining region (CDR) substantially from a non-human-antibody (e.g., mouse). In addition to the grafting of the CDRs, humanized antibodies typically undergo further alterations in order to improve affinity and/or immmunogenicity.

The term "multivalent antibody" refers to an antibody comprising more than one antigen recognition site. For example, a "bivalent" antibody has two antigen recognition sites, whereas a "tetravalent" antibody has four antigen recognition sites. The terms "monospecific", "bispecific", "trispecific", "tetraspecific", etc. refer to the number of different antigen recognition site specificities (as opposed to the number of antigen recognition sites) present in a multivalent antibody. For example, a "monospecific" antibody's antigen recognition sites all bind the same epitope. A "bispecific" or "dual specific" antibody has at least one antigen recognition site that binds a first epitope and at least one antigen recognition site that binds a second epitope that is different from the first epitope. A "multivalent monospecific" antibody has multiple antigen recognition sites that all bind the same epitope. A "multivalent bispecific" antibody has multiple antigen recognition sites, some number of which bind a first epitope and some number of which bind a second epitope that is different from the first epitope

The term "human antibody", as used herein, is intended to include antibodies having variable and constant regions derived from human germline immunoglobulin sequences. The human antibodies of the invention may include amino acid residues not encoded by human germline immunoglobulin sequences (*e.g.,* mutations introduced by random or site-specific mutagenesis *in vitro* or by somatic mutation *in vivo),* for example in the CDRs and in particular CDR3. However, the term "human antibody", as used herein, is not intended to include antibodies in which CDR sequences derived from the germline of another mammalian species, such as a mouse, have been grafted onto human framework sequences.

The term "recombinant human antibody", as used herein, is intended to include all human antibodies that are prepared, expressed, created or isolated by recombinant means, such as antibodies expressed using a recombinant expression vector transfected into a host cell (described further below), antibodies isolated from a recombinant, combinatorial human antibody library (described further below), antibodies isolated from an animal (*e.g.,* a mouse) that is transgenic for human immunoglobulin genes (see *e.g.,* Taylor et al. (1992) Nucl. Acids Res. 20:6287) or antibodies prepared, expressed, created or isolated by any other means that involves splicing of human immunoglobulin gene sequences to other DNA sequences. Such recombinant human antibodies have variable and constant regions derived from human germline immunoglobulin sequences. In certain embodiments, however, such recombinant human antibodies are subjected to *in vitro* mutagenesis (or, when an animal transgenic for human Ig sequences is used, *in vivo* somatic mutagenesis) and thus the amino acid sequences of the VH and VL regions of the recombinant antibodies are sequences that, while derived from and related to human germline VH and VL sequences, may not naturally exist within the human antibody germline repertoire *in vivo.*

Such chimeric, humanized, human, and dual specific antibodies can be produced by recombinant DNA techniques known in the art, for example using methods described in PCT International Application No. PCT/US86/02269; European Patent Application No. 184,187; European Patent Application No. 171,496; European Patent Application No. 173,494; PCT International Publication No. WO 86/01533; U.S. Pat. No. 4,816,567; European Patent Application No. 125,023; Better et al. (1988) Science 240:1041-1043*;* Liu et al. (1987) Proc. Natl. Acad. Sci. USA 84:3439-3443; Liu et al. (1987) J. Immunol. 139:3521-3526; Sun et al. (1987) Proc. Natl. Acad. Sci. USA 84:214-218; Nishimura et al. (1987) Cancer Res. 47:999-1005; Wood et al. (1985) Nature 314:446-449; Shaw et al. (1988) J. Natl. Cancer Inst. 80:1553-1559); Morrison (1985) Science 229:1202-1207; Oi et al. (1986) BioTechniques 4:214; U.S. Pat. No. 5,225,539; Jones et al. (1986) Nature 321:552-525; Verhoeyan et al. (1988) Science 239:1534; and Beidler et al. (1988) J. Immunol. 141:4053-4060, Queen et al., Proc. Natl. Acad. Sci. USA 86:10029-10033 (1989), US 5,530,101, US 5,585,089, US 5,693,761, US 5,693,762, Selick et al.*,* WO 90/07861, and Winter, US 5,225,539.

An "isolated antibody", as used herein, is intended to refer to an antibody that is substantially free of other antibodies having different antigenic specificities (*e.g*., an isolated antibody that specifically binds hTNFα is substantially free of antibodies that specifically bind antigens other than hTNFα). An isolated antibody that specifically binds TNFα may, however, have cross-reactivity to other antigens, such as TNFα molecules from other species. Moreover, an isolated antibody may be substantially free of other cellular material and/or chemicals.

A "neutralizing antibody", as used herein (or an "antibody that neutralized hTNFα activity"), is intended to refer to an antibody whose binding to hTNFα results in inhibition of the biological activity of hTNFα. This inhibition of the biological activity of hTNFα can be assessed by measuring one or more indicators of hTNFα biological activity, such as hTNFα-induced cytotoxicity (either *in vitro* or *in vivo),* hTNFα-induced cellular activation and hTNFα binding to hTNFα receptors. These indicators of hTNFα biological activity can be assessed by one or more of several standard *in vitro* or *in vivo* assays known in the art (see U.S. Patent No. 6,090,382). Preferably, the ability of an antibody to neutralize hTNFα activity is assessed by inhibition of hTNFα-induced cytotoxicity of L929 cells. As an additional or alternative parameter of hTNFα activity, the ability of an antibody to inhibit hTNFα-induced expression of ELAM-1 on HUVEC, as a measure of hTNFα-induced cellular activation, can be assessed.

The term "surface plasmon resonance", as used herein, refers to an optical phenomenon that allows for the analysis of real-time biospecific interactions by detection of alterations in protein concentrations within a biosensor matrix, for example using the BIAcore system (Pharmacia Biosensor AB, Uppsala, Sweden and Piscataway, NJ). For further descriptions, see Example 1 of U.S. Patent 6,258,562 and Jönsson et al. (1993) Ann. Biol. Clin. 51:19; Jönsson et al. (1991) Biotechniques 11:620-627; Johnsson et al. (1995) J. Mol. Recognit. 8:125; and Johnnson et al. (1991) Anal.Biochem.198:268*.*

The term "K_{off}", as used herein, is intended to refer to the off rate constant for dissociation of an antibody from the antibody/antigen complex.

The term "K_{d}", as used herein, is intended to refer to the dissociation constant of a particular antibody-antigen interaction.

The term "IC₅₀" as used herein, is intended to refer to the concentration of the inhibitor required to inhibit the biological endpoint of interest, *e.g.,* neutralize cytotoxicity activity.

The term "dose," as used herein, refers to an amount of TNFα inhibitor which is administered to a subject.

The term "dosing", as used herein, refers to the administration of a substance (*e.g.,* an anti-TNFα antibody) to achieve a therapeutic objective (*e.g.,* treatment of psoriasis).

A "dosing regimen" describes a treatment schedule for a TNFα inhibitor, *e.g*., a treatment schedule over a prolonged period of time and/or throughout the course of treatment, *e.g.* administering a first dose of a TNFα inhibitor at week 0 followed by a second dose of a TNFα inhibitor on a biweekly dosing regimen.

The term "multiple-variable dose" includes different doses of a TNFα inhibitor which are administered to a subject for therapeutic treatment. "Multiple-variable dose regimen" or "multiple-variable dose therapy" describes a treatment schedule which is based on administering different amounts of TNFα inhibitor at various time points throughout the course of treatment. Multiple-variable dose regimens are described in PCT application no. PCT/US05/12007 and US 20060009385, which is incorporated by reference herein.

The term "maintenance therapy" or "maintenance dosing regime" refers to a treatment schedule for a subject or patient diagnosed with a disorder/disease, *e.g*., psoriasis, to enable them to maintain their health in a given state, *e.g,* remission. Generally, the first goal of treatment of psoriasis is to induce remission in the subject in need thereof. The next challenge is to keep the subject in remission. Maintenance doses may be used in a maintenance therapy for maintaining remission in a subject who has achieved remission of a disease or who has reached a state of the disease which is advantageous, *e.g.* reduction in symptoms. In one embodiment, a maintenance therapy of the invention is used for a subject or patient diagnosed with a disorder/disease, *e.g*., psoriasis to enable them to maintain their health in a state which is completely free of symptoms associated with the disease. In one embodiment, a maintenance therapy of the invention is used for a subject or patient diagnosed with a disorder/disease, *e.g*., psoriasis, to enable them to maintain their health in a state which is substantially free of symptoms associated with the disease. In one embodiment, a maintenance therapy of the invention is used for a subject or patient diagnosed with a disorder/disease, *e.g.,* psroaisis, to enable them to maintain their health in a state where there is a significant reduction in symptoms associated with the disease.

The term "induction dose" or "loading dose," used interchangeably herein, refers to the first dose of TNFα inhibitor which is initially used to induce remission of psoriasis. Often, the loading dose is larger in comparison to the subsequent maintenance or treatment dose. The induction dose can be a single dose or, alternatively, a set of doses. In one embodiment, an induction dose is subsequently followed by administration of smaller doses of TNFα inhibitor, *e.g*., the treatment or maintenance dose. The induction dose is administered during the induction or loading phase of therapy. In one embodiment of the invention, the induction dose is at least twice the given amount of the treatment dose. In one embodiment of the invention, the induction dose is 80 mg.

The term "treatment phase" or "maintenance phase", as used herein, refers to a period of treatment comprising administration of a TNFα inhibitor to a subject in order to maintain a desired therapeutic effect, *i.e.,* maintaining remission of psoriasis.

The term "maintenance dose" or "treatment dose" is the amount of TNFα inhibitor taken by a subject to maintain or continue a desired therapeutic effect. A maintenance dose can be a single dose or, alternatively, a set of doses. A maintenance dose is administered during the treatment or maintenance phase of therapy. In one embodiment, amaintenance dose(s) is smaller than the induction dose(s) and can be equal to each other when administered in succession. In one embodiment, the invention provides a maintenance dose of 40 mg of adalimumab administered subcutaneously to a subject who is in remission, every other week, or biweekly. In one embodiment, the maintenance dose is administered every other week beginning at week 4 of treatment.

The terms "biweekly dosing regimen", "biweekly dosing", and "biweekly administration", as used herein, refer to the time course of administering a substance (*e.g.,* an anti-TNFα antibody) to a subject to achieve a therapeutic objective, *e.g,* throughout the course of treatment. The biweekly dosing regimen is not intended to include a weekly dosing regimen. Preferably, the substance is administered every 9-19 days, more preferably, every 11-17 days, even more preferably, every 13-15 days, and most preferably, every 14 days. In one embodiment, the biweekly dosing regimen is initiated in a subject at week 0 of treatment. In another embodiment, a maintenance dose is administered on a biweekly dosing regimen. In one embodiment, both the loading and maintenance doses are administered according to a biweekly dosing regimen. In one embodiment, biweekly dosing includes a dosing regimen wherein doses of a TNFα inhibitor are administered to a subject every other week beginning at week 0. In one embodiment, biweekly dosing includes a dosing regimen where doses of a TNFα inhibitor are administered to a subject every other week consecutively for a given time period, *e.g*., 4 weeks, 8 weeks, 16, weeks, 24 weeks, 26 weeks, 32 weeks, 36 weeks, 42 weeks, 48 weeks, 52 weeks, 56 weeks, etc. Biweekly dosing methods are also described in US 20030235585, incorporated by reference herein.

The term "combination" as in the phrase "a first agent in combination with a second agent" includes co-administration of a first agent and a second agent, which for example may be dissolved or intermixed in the same pharmaceutically acceptable carrier, or administration of a first agent, followed by the second agent, or administration of the second agent, followed by the first agent. The present invention, therefore, includes methods of combination therapeutic treatment and combination pharmaceutical compositions.

The term "concomitant" as in the phrase "concomitant therapeutic treatment" includes administering an agent in the presence of a second agent. A concomitant therapeutic treatment method includes methods in which the first, second, third, or additional agents are co-administered. A concomitant therapeutic treatment method also includes methods in which the first or additional agents are administered in the presence of a second or additional agents, wherein the second or additional agents, for example, may have been previously administered. A concomitant therapeutic treatment method may be executed step-wise by different actors. For example, one actor may administer to a subject a first agent and a second actor may to administer to the subject a second agent, and the administering steps may be executed at the same time, or nearly the same time, or at distant times, so long as the first agent (and additional agents) are after administration in the presence of the second agent (and additional agents). The actor and the subject may be the same entity (*e.g.,* human).

The term "combination therapy", as used herein, refers to the administration of two or more therapeutic substances, *e.g*., an anti-TNFα antibody and another drug. The other drug(s) may be administered concomitant with, prior to, or following the administration of an anti-TNFα antibody.

The term "treatment," as used within the context of the present invention, is meant to include therapeutic treatment, as well as prophylactic or suppressive measures, for the treatment of psoriasis. For example, the term treatment may include administration of a TNFα inhibitor prior to or following the onset of psoriasis thereby preventing or removing signs of the disease or disorder. As another example, administration of a TNFα inhibitor after clinical manifestation of psoriasis to combat the symptoms and/or complications and disorders associated with psoriasis comprises "treatment" of the disease. Further, administration of the agent after onset and after clinical symptoms and/or complications have developed where administration affects clinical parameters of the disease or disorder and perhaps amelioration of the disease, comprises "treatment" of the psoriasis. In one embodiment, treatment of psoriasis in a subject comprises inducing and maintaining remission of psoriasis in a subject. In another embodiment, treatment of psoriasis in a subject comprises maintaining remission of psoriasis in a subject.

Those "in need of treatment" include mammals, such as humans, already having psoriasis, including those in which the disease or disorder is to be prevented.

Various aspects of the invention are described in further detail herein.

The invention provides improved uses and compositions for treating psoriasis disease with a TNFα inhibitor, *e.g.,* a human TNFα antibody, or an antigen-binding portion thereof. Compositions and articles of manufacture, including kits, relating to the methods and uses for treating psoriasis are also contemplated as part of the invention.

### II. TNF Inhibitors

A TNFα inhibitor which is used in the methods and compositions of the invention includes any agent which interferes with TNFα activity. In a preferred embodiment, the TNFα inhibitor can neutralize TNFα activity, particularly detrimental TNFα activity which is associated with psoriasis, and related complications and symptoms.

In one embodiment, the TNFα inhibitor used in the invention is an TNFα antibody (also referred to herein as a TNFα antibody), or an antigen-binding fragment thereof, including chimeric, humanized, and human antibodies. Examples of TNFα antibodies which may be used in the invention include, but not limited to, infliximab (Remicade^{®}, Johnson and Johnson; described in U.S. Patent No. 5,656,272, incorporated by reference herein), CDP571 (a humanized monoclonal anti-TNF-alpha IgG4 antibody), CDP 870 (a humanized monoclonal anti-TNF-alpha antibody fragment), an anti-TNF dAb (Peptech), CNTO 148 (golimumab; Medarex and Centocor, see WO 02/12502), and adalimumab (HUMIRA^{®} Abbott Laboratories, a human anti-TNF mAb, described in US 6,090,382 as D2E7). Additional TNF antibodies which may be used in the invention are described in U.S. Patent Nos. 6,593,458; 6,498,237; 6,451,983; and 6,448,380, each of which is incorporated by reference herein.

Other examples of TNFα inhibitors which may be used in the methods and compositions of the invention include etanercept (Enbrel, described in WO 91/03553 and WO 09/406476), soluble TNF receptor Type I, a pegylated soluble TNF receptor Type I (PEGs TNF-R1), p55TNFR1gG (Lenercept), and recombinant TNF binding protein (r-TBP-I) (Serono).

In one embodiment, the term "TNFα inhibitor" excludes infliximab. In one embodiment, the term "TNFα inhibitor" excludes adalimumab. In another embodiment, the term "TNFα inhibitor" excludes adalimumab and infliximab.

In one embodiment, the term "TNFα inhibitor" excludes etanercept, and, optionally, adalimumab, infliximab, and adalimumab and infliximab.

In one embodiment, the term "TNFα antibody" excludes infliximab. In one embodiment, the term "TNFα antibody" excludes adalimumab. In another embodiment, the term "TNFα antibody" excludes adalimumab and infliximab.

In one embodiment, the invention features uses and composition for treating or determining the efficacy of a TNFα inhibitor for the treatment of psoriasis, wherein the TNFα antibody is an isolated human antibody, or antigen-binding portion thereof, that binds to human TNFα with high affinity and a low off rate, and also has a high neutralizing capacity. Preferably, the human antibodies used in the invention are recombinant, neutralizing human anti-hTNFα antibodies. The most preferred recombinant, neutralizing antibody of the invention is referred to herein as D2E7, also referred to as HUMIRA^{®} or adalimumab (the amino acid sequence of the D2E7 VL region is shown in SEQ ID NO: 1; the amino acid sequence of the D2E7 VH region is shown in SEQ ID NO: 2). The properties of D2E7 (adalimumab / HUMIRA^{®}) have been described in Salfeld et al., U.S. Patent Nos. 6,090,382, 6,258,562, and 6,509,015, which are each incorporated by reference herein. The methods of the invention may also be performed using chimeric and humanized murine anti-hTNFα antibodies which have undergone clinical testing for treatment of rheumatoid arthritis (see e.g., Elliott, M.J., et al. (1994) Lancet 344:1125-1127; Elliot, M.J., et al. (1994) Lancet 344:1105-1110; Rankin, E.C., et al. (1995) Br. J. Rheumatol. 34:334-342).

In one embodiment, the method of the invention includes determining the efficacy of D2E7 antibodies and antibody portions, D2E7-related antibodies and antibody portions, or other human antibodies and antibody portions with equivalent properties to D2E7, such as high affinity binding to hTNFα with low dissociation kinetics and high neutralizing capacity, for the treatment of psoriasis. In one embodiment, the invention provides treatment with an isolated human antibody, or an antigen-binding portion thereof, that dissociates from human TNFα with a K_{d} of 1 x 10⁻⁸ M or less and a K_{off} rate constant of 1 x 10⁻³ s⁻¹ or less, both determined by surface plasmon resonance, and neutralizes human TNFα cytotoxicity in a standard *in vitro* L929 assay with an IC₅₀ of 1 x 10⁻⁷ M or less. More preferably, the isolated human antibody, or antigen-binding portion thereof, dissociates from human TNFα with a K_{off} of 5 x 10⁻⁴ s⁻¹ or less, or even more preferably, with a K_{off} of 1 x 10⁻⁴ s⁻¹ or less. More preferably, the isolated human antibody, or antigen-binding portion thereof, neutralizes human TNFα cytotoxicity in a standard *in vitro* L929 assay with an IC₅₀ of 1 x 10⁻⁸ M or less, even more preferably with an IC₅₀ of 1 x 10⁻⁹ M or less and still more preferably with an IC₅₀ of 1 x 10⁻¹⁰ M or less. In a preferred embodiment, the antibody is an isolated human recombinant antibody, or an antigen-binding portion thereof.

It is well known in the art that antibody heavy and light chain CDR3 domains play an important role in the binding specificity/affinity of an antibody for an antigen. Accordingly, in another aspect, the invention pertains to treating psoriasis by administering human antibodies that have slow dissociation kinetics for association with hTNFα and that have light and heavy chain CDR3 domains that structurally are identical to or related to those of D2E7. Position 9 of the D2E7 VL CDR3 can be occupied by Ala or Thr without substantially affecting the K_{off}. Accordingly, a consensus motif for the D2E7 VL CDR3 comprises the amino acid sequence: Q-R-Y-N-R-A-P-Y-(T/A) (SEQ ID NO: 3). Additionally, position 12 of the D2E7 VH CDR3 can be occupied by Tyr or Asn, without substantially affecting the K_{off}. Accordingly, a consensus motif for the D2E7 VH CDR3 comprises the amino acid sequence: V-S-Y-L-S-T-A-S-S-L-D-(Y/N) (SEQ ID NO: 4). Moreover, as demonstrated in Example 2 of U.S. Patent No. 6,090,382, the CDR3 domain of the D2E7 heavy and light chains is amenable to substitution with a single alanine residue (at position 1, 4, 5, 7 or 8 within the VL CDR3 or at position 2, 3, 4, 5, 6, 8, 9, 10 or 11 within the VH CDR3) without substantially affecting the K_{off}. Still further, the skilled artisan will appreciate that, given the amenability of the D2E7 VL and VH CDR3 domains to substitutions by alanine, substitution of other amino acids within the CDR3 domains may be possible while still retaining the low off rate constant of the antibody, in particular substitutions with conservative amino acids. Preferably, no more than one to five conservative amino acid substitutions are made within the D2E7 VL and/or VH CDR3 domains. More preferably, no more than one to three conservative amino acid substitutions are made within the D2E7 VL and/or VH CDR3 domains. Additionally, conservative amino acid substitutions should not be made at amino acid positions critical for binding to hTNFα. Positions 2 and 5 of the D2E7 VL CDR3 and positions 1 and 7 of the D2E7 VH CDR3 appear to be critical for interaction with hTNFα and thus, conservative amino acid substitutions preferably are not made at these positions (although an alanine substitution at position 5 of the D2E7 VL CDR3 is acceptable, as described above) *(see* U.S. Patent No. 6,090,382).

Accordingly, in another embodiment, the antibody or antigen-binding portion thereof preferably contains the following characteristics:
a) dissociates from human TNFα with a K_{off} rate constant of 1 x 10⁻³ s⁻¹ or less, as determined by surface plasmon resonance;
b) has a light chain CDR3 domain comprising the amino acid sequence of SEQ ID NO: 3, or modified from SEQ ID NO: 3 by a single alanine substitution at position 1, 4, 5, 7 or 8 or by one to five conservative amino acid substitutions at positions 1, 3, 4, 6, 7, 8 and/or 9;
c) has a heavy chain CDR3 domain comprising the amino acid sequence of SEQ ID NO: 4, or modified from SEQ ID NO: 4 by a single alanine substitution at position 2, 3, 4, 5, 6, 8, 9, 10 or 11 or by one to five conservative amino acid substitutions at positions 2, 3, 4, 5, 6, 8, 9, 10, 11 and/or 12.

More preferably, the antibody, or antigen-binding portion thereof, dissociates from human TNFα with a K_{off} of 5 x 10⁻⁴ s⁻¹ or less. Even more preferably, the antibody, or antigen-binding portion thereof, dissociates from human TNFα with a K_{off} of 1 x 10⁻⁴ s⁻¹ or less.

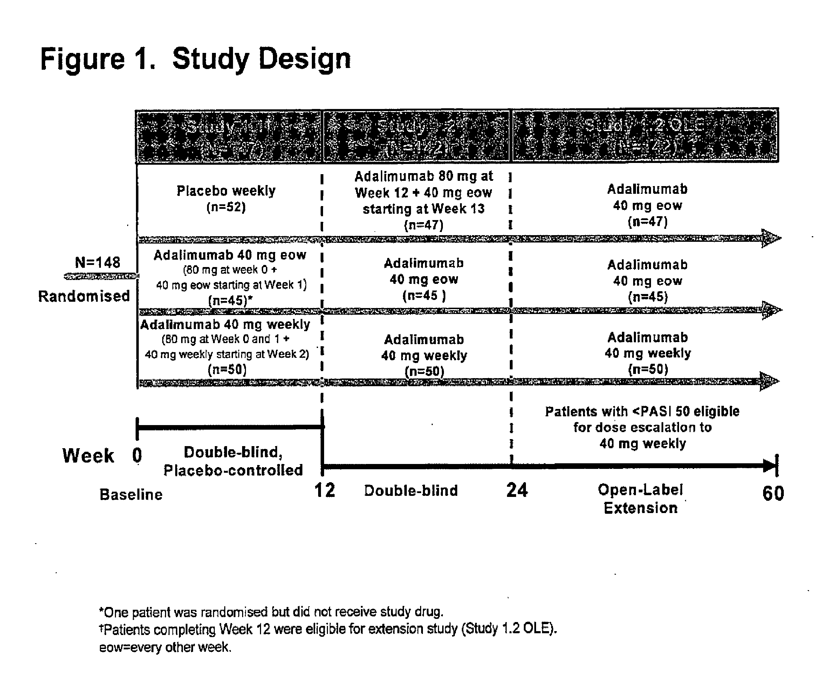

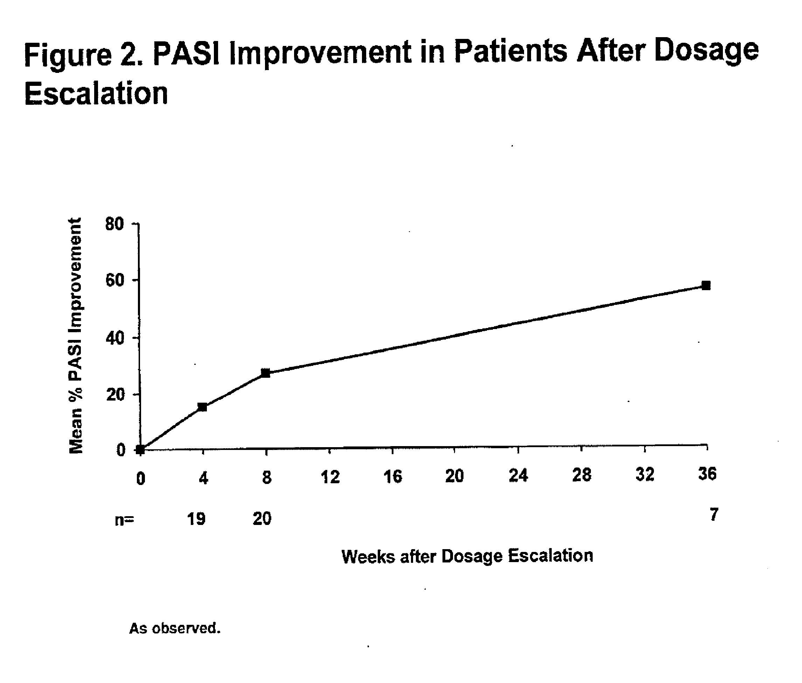

In yet another embodiment, the antibody or antigen-binding portion thereof preferably contains a light chain variable region (LCVR) having a CDR3 domain comprising the amino acid sequence of SEQ ID NO: 3, or modified from SEQ ID NO: 3 by a single alanine substitution at position 1, 4, 5, 7 or 8, and with a heavy chain variable region (HCVR) having a CDR3 domain comprising the amino acid sequence of SEQ ID NO: 4, or modified from SEQ ID NO: 4 by a single alanine substitution at position 2, 3, 4, 5, 6, 8, 9, 10 or 11. Preferably, the LCVR further has a CDR2 domain comprising the amino acid sequence of SEQ ID NO: 5 *(i.e.,* the D2E7 VL CDR2) and the HCVR further has a CDR2 domain comprising the amino acid sequence of SEQ ID NO: 6 (*i.e.,* the D2E7 VH CDR2). Even more preferably, the LCVR further has CDR1 domain comprising the amino acid sequence of SEQ ID NO: 7 *(i.e.,* the D2E7 VL CDR1) and the HCVR has a CDR1 domain comprising the amino acid sequence of SEQ ID NO: 8 *(i.e.,* the D2E7 VH CDR1). The framework regions for VL preferably are from the V_{κ}I human germline family, more preferably from the A20 human germline Vk gene and most preferably from the D2E7 VL framework sequences shown in Figures 1A and 1B of U.S. Patent No. 6,090,382. The framework regions for VH preferably are from the V_{H}3 human germline family, more preferably from the DP-31 human germline VH gene and most preferably from the D2E7 VH framework sequences shown in Figures 2A and 2B of U.S. Patent No. 6,090,382.

Accordingly, in another embodiment, the antibody or antigen-binding portion thereof preferably contains a light chain variable region (LCVR) comprising the amino acid sequence of SEQ ID NO: 1 (i.e., the D2E7 VL) and a heavy chain variable region (HCVR) comprising the amino acid sequence of SEQ ID NO: 2 (*i.e.,* the D2E7 VH). In certain embodiments, the antibody comprises a heavy chain constant region, such as an IgG1, IgG2, IgG3, IgG4, IgA, IgE, IgM or IgD constant region. Preferably, the heavy chain constant region is an IgG1 heavy chain constant region or an IgG4 heavy chain constant region. Furthermore, the antibody can comprise a light chain constant region, either a kappa light chain constant region or a lambda light chain constant region. Preferably, the antibody comprises a kappa light chain constant region. Alternatively, the antibody portion can be, for example, a Fab fragment or a single chain Fv fragment.

In still other embodiments, the invention includes uses of an isolated human antibody, or an antigen-binding portions thereof, containing D2E7-related VL and VH CDR3 domains. For example, antibodies, or antigen-binding portions thereof, with a light chain variable region (LCVR) having a CDR3 domain comprising an amino acid sequence selected from the group consisting of SEQ ID NO: 3, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25 and SEQ ID NO: 26 or with a heavy chain variable region (HCVR) having a CDR3 domain comprising an amino acid sequence selected from the group consisting of SEQ ID NO: 4, SEQ ID NO: 27, SEQ ID NO: 28, SEQ ID NO: 29, SEQ ID NO: 30, SEQ ID NO: 31, SEQ ID NO: 32, SEQ ID NO: 33, SEQ ID NO: 34 and SEQ ID NO: 35.

The TNFα antibody used in the methods and compositions of the invention may be modified for improved treatment of psoriasis. In some embodiments, the TNFα antibody or antigen binding fragments thereof, is chemically modified to provide a desired effect. For example, pegylation of antibodies and antibody fragments of the invention may be carried out by any of the pegylation reactions known in the art, as described, for example, in the following references: Focus on Growth Factors 3:4-10 (1992); EP 0 154 316; and EP 0 401 384 (each of which is incorporated by reference herein in its entirety). Preferably, the pegylation is carried out via an acylation reaction or an alkylation reaction with a reactive polyethylene glycol molecule (or an analogous reactive water-soluble polymer). A preferred water-soluble polymer for pegylation of the antibodies and antibody fragments of the invention is polyethylene glycol (PEG). As used herein, "polyethylene glycol" is meant to encompass any of the forms of PEG that have been used to derivatize other proteins, such as mono (C1-C10) alkoxy- or aryloxy-polyethylene glycol.

Methods for preparing pegylated antibodies and antibody fragments of the invention will generally comprise the steps of (a) reacting the antibody or antibody fragment with polyethylene glycol, such as a reactive ester or aldehyde derivative of PEG, under conditions whereby the antibody or antibody fragment becomes attached to one or more PEG groups, and (b) obtaining the reaction products. It will be apparent to one of ordinary skill in the art to select the optimal reaction conditions or the acylation reactions based on known parameters and the desired result.

Pegylated antibodies and antibody fragments may generally be used to treat psoriasis by administration of the TNFα antibodies and antibody fragments described herein. Generally the pegylated antibodies and antibody fragments have increased half-life, as compared to the nonpegylated antibodies and antibody fragments. The pegylated antibodies and antibody fragments may be employed alone, together, or in combination with other pharmaceutical compositions.

In yet another embodiment of the invention, TNFα antibodies or fragments thereof can be altered wherein the constant region of the antibody is modified to reduce at least one constant region-mediated biological effector function relative to an unmodified antibody. To modify an antibody of the invention such that it exhibits reduced binding to the Fc receptor, the immunoglobulin constant region segment of the antibody can be mutated at particular regions necessary for Fc receptor (FcR) interactions (see e.g., Canfield, S.M. and S.L. Morrison (1991) J. Exp. Med. 173:1483-1491; and Lund, J. et al. (1991) J. of Immunol. 147:2657-2662). Reduction in FcR binding ability of the antibody may also reduce other effector functions which rely on FcR interactions, such as opsonization and phagocytosis and antigen-dependent cellular cytotoxicity.

An antibody or antibody portion used in the methods of the invention can be derivatized or linked to another functional molecule (e.g., another peptide or protein). Accordingly, the antibodies and antibody portions of the invention are intended to include derivatized and otherwise modified forms of the human anti-hTNFα antibodies described herein, including immunoadhesion molecules. For example, an antibody or antibody portion of the invention can be functionally linked (by chemical coupling, genetic fusion, noncovalent association or otherwise) to one or more other molecular entities, such as another antibody (e.g., a bispecific antibody or a diabody), a detectable agent, a cytotoxic agent, a pharmaceutical agent, and/or a protein or peptide that can mediate associate of the antibody or antibody portion with another molecule (such as a streptavidin core region or a polyhistidine tag).

One type of derivatized antibody is produced by crosslinking two or more antibodies (of the same type or of different types, e.g., to create bispecific antibodies). Suitable crosslinkers include those that are heterobifunctional, having two distinctly reactive groups separated by an appropriate spacer (e.g., m-maleimidobenzoyl-N-hydroxysuccinimide ester) or homobifunctional (e.g., disuccinimidyl suberate). Such linkers are available from Pierce Chemical Company, Rockford, IL.

Useful detectable agents with which an antibody or antibody portion of the invention may be derivatized include fluorescent compounds. Exemplary fluorescent detectable agents include fluorescein, fluorescein isothiocyanate, rhodamine, 5-dimethylamine-1-napthalenesulfonyl chloride, phycoerythrin and the like. An antibody may also be derivatized with detectable enzymes, such as alkaline phosphatase, horseradish peroxidase, glucose oxidase and the like. When an antibody is derivatized with a detectable enzyme, it is detected by adding additional reagents that the enzyme uses to produce a detectable reaction product. For example, when the detectable agent horseradish peroxidase is present, the addition of hydrogen peroxide and diaminobenzidine leads to a colored reaction product, which is detectable. An antibody may also be derivatized with biotin, and detected through indirect measurement of avidin or streptavidin binding.

An antibody, or antibody portion, used in the methods and compositions of the invention, can be prepared by recombinant expression of immunoglobulin light and heavy chain genes in a host cell. To express an antibody recombinantly, a host cell is transfected with one or more recombinant expression vectors carrying DNA fragments encoding the immunoglobulin light and heavy chains of the antibody such that the light and heavy chains are expressed in the host cell and, preferably, secreted into the medium in which the host cells are cultured, from which medium the antibodies can be recovered. Standard recombinant DNA methodologies are used to obtain antibody heavy and light chain genes, incorporate these genes into recombinant expression vectors and introduce the vectors into host cells, such as those described in Sambrook, Fritsch and Maniatis (eds), Molecular Cloning; A Laboratory Manual, Second Edition, Cold Spring Harbor, N.Y., (1989), Ausubel, F.M. et al. (eds.) Current Protocols in Molecular Biology, Greene Publishing Associates, (1989) and in U.S. Patent No. 4,816,397 by Boss et al.

To express adalimumab (D2E7) or an adalimumab (D2E7)-related antibody, DNA fragments encoding the light and heavy chain variable regions are first obtained. These DNAs can be obtained by amplification and modification of germline light and heavy chain variable sequences using the polymerase chain reaction (PCR). Germline DNA sequences for human heavy and light chain variable region genes are known in the art (see e.g., the "Vbase" human germline sequence database; see also Kabat, E.A., et al. (1991) Sequences of Proteins of Immunological Interest, Fifth Edition, U.S. Department of Health and Human Services, NIH Publication No. 91-3242; Tomlinson, I.M., et al. (1992) "The Repertoire of Human Germline VH Sequences Reveals about Fifty Groups of VH Segments with Different Hypervariable Loops" J. Mol. Biol. 227:776-798; and Cox, J.P.L. et al. (1994) "A Directory of Human Germ-line V78 Segments Reveals a Strong Bias in their Usage" Eur. J. Immunol. 24:827-836; the contents of each of which are expressly incorporated herein by reference). To obtain a DNA fragment encoding the heavy chain variable region of D2E7, or a D2E7-related antibody, a member of the V_{H}3 family of human germline VH genes is amplified by standard PCR. Most preferably, the DP-31 VH germline sequence is amplified. To obtain a DNA fragment encoding the light chain variable region of D2E7, or a D2E7-related antibody, a member of the V_{κ}I family of human germline VL genes is amplified by standard PCR. Most preferably, the A20 VL germline sequence is amplified. PCR primers suitable for use in amplifying the DP-31 germline VH and A20 germline VL sequences can be designed based on the nucleotide sequences disclosed in the references cited *supra,* using standard methods.

Once the germline VH and VL fragments are obtained, these sequences can be mutated to encode the D2E7 or D2E7-related amino acid sequences disclosed herein. The amino acid sequences encoded by the germline VH and VL DNA sequences are first compared to the D2E7 or D2E7-related VH and VL amino acid sequences to identify amino acid residues in the D2E7 or D2E7-related sequence that differ from germline. Then, the appropriate nucleotides of the germline DNA sequences are mutated such that the mutated germline sequence encodes the D2E7 or D2E7-related amino acid sequence, using the genetic code to determine which nucleotide changes should be made. Mutagenesis of the germline sequences is carried out by standard methods, such as PCR-mediated mutagenesis (in which the mutated nucleotides are incorporated into the PCR primers such that the PCR product contains the mutations) or site-directed mutagenesis.

Moreover, it should be noted that if the "germline" sequences obtained by PCR amplification encode amino acid differences in the framework regions from the true germline configuration (i.e., differences in the amplified sequence as compared to the true germline sequence, for example as a result of somatic mutation), it may be desireable to change these amino acid differences back to the true germline sequences (i.e., "backmutation" of framework residues to the germline configuration).

Once DNA fragments encoding D2E7 or D2E7-related VH and VL segments are obtained (by amplification and mutagenesis of germline VH and VL genes, as described above), these DNA fragments can be further manipulated by standard recombinant DNA techniques, for example to convert the variable region genes to full-length antibody chain genes, to Fab fragment genes or to a scFv gene. In these manipulations, a VL- or VH-encoding DNA fragment is operatively linked to another DNA fragment encoding another protein, such as an antibody constant region or a flexible linker. The term "operatively linked", as used in this context, is intended to mean that the two DNA fragments are joined such that the amino acid sequences encoded by the two DNA fragments remain in-frame.

The isolated DNA encoding the VH region can be converted to a full-length heavy chain gene by operatively linking the VH-encoding DNA to another DNA molecule encoding heavy chain constant regions (CH 1, CH2 and CH3). The sequences of human heavy chain constant region genes are known in the art (see e.g., Kabat, E.A., et al. (1991) Sequences of Proteins of Immunological Interest, Fifth Edition, U.S. Department of Health and Human Services, NIH Publication No. 91-3242) and DNA fragments encompassing these regions can be obtained by standard PCR amplification. The heavy chain constant region can be an IgG1, IgG2, IgG3, IgG4, IgA, IgE, IgM or IgD constant region, but most preferably is an IgG1 or IgG4 constant region. For a Fab fragment heavy chain gene, the VH-encoding DNA can be operatively linked to another DNA molecule encoding only the heavy chain CH1 constant region.

The isolated DNA encoding the VL region can be converted to a full-length light chain gene (as well as a Fab light chain gene) by operatively linking the VL-encoding DNA to another DNA molecule encoding the light chain constant region, CL. The sequences of human light chain constant region genes are known in the art (see *e.g.,* Kabat, E.A., et al. (1991) Sequences of Proteins of Immunological Interest, Fifth Edition, U.S. Department of Health and Human Services, NIH Publication No. 91-3242) and DNA fragments encompassing these regions can be obtained by standard PCR amplification. The light chain constant region can be a kappa or lambda constant region, but most preferably is a kappa constant region.

To create a scFv gene, the VH- and VL-encoding DNA fragments are operatively linked to another fragment encoding a flexible linker, e.g., encoding the amino acid sequence (Gly₄-Ser)₃, such that the VH and VL sequences can be expressed as a contiguous single-chain protein, with the VL and VH regions joined by the flexible linker (see *e.g.,* Bird et al. (1988) Science 242:423-426; Huston et al. (1988) Proc. Natl. Acad. Sci. USA 85:5879-5883; McCafferty et al., Nature (1990) 348:552-554).

To express the antibodies, or antibody portions used in the invention, DNAs encoding partial or full-length light and heavy chains, obtained as described above, are inserted into expression vectors such that the genes are operatively linked to transcriptional and translational control sequences. In this context, the term "operatively linked" is intended to mean that an antibody gene is ligated into a vector such that transcriptional and translational control sequences within the vector serve their intended function of regulating the transcription and translation of the antibody gene. The expression vector and expression control sequences are chosen to be compatible with the expression host cell used. The antibody light chain gene and the antibody heavy chain gene can be inserted into separate vector or, more typically, both genes are inserted into the same expression vector. The antibody genes are inserted into the expression vector by standard methods (e.g., ligation of complementary restriction sites on the antibody gene fragment and vector, or blunt end ligation if no restriction sites are present). Prior to insertion of the D2E7 or D2E7-related light or heavy chain sequences, the expression vector may already carry antibody constant region sequences. For example, one approach to converting the D2E7 or D2E7-related VH and VL sequences to full-length antibody genes is to insert them into expression vectors already encoding heavy chain constant and light chain constant regions, respectively, such that the VH segment is operatively linked to the CH segment(s) within the vector and the VL segment is operatively linked to the CL segment within the vector. Additionally or alternatively, the recombinant expression vector can encode a signal peptide that facilitates secretion of the antibody chain from a host cell. The antibody chain gene can be cloned into the vector such that the signal peptide is linked in-frame to the amino terminus of the antibody chain gene. The signal peptide can be an immunoglobulin signal peptide or a heterologous signal peptide (i.e., a signal peptide from a non-immunoglobulin protein).

In addition to the antibody chain genes, the recombinant expression vectors of the invention carry regulatory sequences that control the expression of the antibody chain genes in a host cell. The term "regulatory sequence" is intended to include promoters, enhancers and other expression control elements (e.g., polyadenylation signals) that control the transcription or translation of the antibody chain genes. Such regulatory sequences are described, for example, in Goeddel; Gene Expression Technology: Methods in Enzymology 185, Academic Press, San Diego, CA (1990). It will be appreciated by those skilled in the art that the design of the expression vector, including the selection of regulatory sequences may depend on such factors as the choice of the host cell to be transformed, the level of expression of protein desired, *etc.* Preferred regulatory sequences for mammalian host cell expression include viral elements that direct high levels of protein expression in mammalian cells, such as promoters and/or enhancers derived from cytomegalovirus (CMV) (such as the CMV promoter/enhancer), Simian Virus 40 (SV40) (such as the SV40 promoter/enhancer), adenovirus, (e.g., the adenovirus major late promoter (AdMLP)) and polyoma. For further description of viral regulatory elements, and sequences thereof, see e.g., U.S. Patent No. 5,168,062 by Stinski, U.S. Patent No. 4,510,245 by Bell et al. and U.S. Patent No. 4,968,615 by Schaffner et al.

In addition to the antibody chain genes and regulatory sequences, the recombinant expression vectors used in the invention may carry additional sequences, such as sequences that regulate replication of the vector in host cells (e.g., origins of replication) and selectable marker genes. The selectable marker gene facilitates selection of host cells into which the vector has been introduced (see e.g., U.S. Patents Nos. 4,399,216, 4,634,665 and 5,179,017, all by Axel *et al.*). For example, typically the selectable marker gene confers resistance to drugs, such as G418, hygromycin or methotrexate, on a host cell into which the vector has been introduced. Preferred selectable marker genes include the dihydrofolate reductase (DHFR) gene (for use in dhfr⁻ host cells with methotrexate selection/amplification) and the *neo* gene (for G418 selection).

For expression of the light and heavy chains, the expression vector(s) encoding the heavy and light chains is transfected into a host cell by standard techniques. The various forms of the term "transfection" are intended to encompass a wide variety of techniques commonly used for the introduction of exogenous DNA into a prokaryotic or eukaryotic host cell, e.g., electroporation, calcium-phosphate precipitation, DEAE-dextran transfection and the like. Although it is theoretically possible to express the antibodies of the invention in either prokaryotic or eukaryotic host cells, expression of antibodies in eukaryotic cells, and most preferably mammalian host cells, is the most preferred because such eukaryotic cells, and in particular mammalian cells, are more likely than prokaryotic cells to assemble and secrete a properly folded and immunologically active antibody. Prokaryotic expression of antibody genes has been reported to be ineffective for production of high yields of active antibody (Boss, M.A. and Wood, C. R. (1985) Immunology Today 6:12-13).

Preferred mammalian host cells for expressing the recombinant antibodies of the invention include Chinese Hamster Ovary (CHO cells) (including dhfr- CHO cells, described in Urlaub and Chasin, (1980) Proc. Natl. Acad. Sci. USA 77:4216-4220, used with a DHFR selectable marker, e.g., as described in R.J. Kaufman and P.A. Sharp (1982) Mol. Biol. 159:601-62 1), NS0 myeloma cells, COS cells and SP2 cells. When recombinant expression vectors encoding antibody genes are introduced into mammalian host cells, the antibodies are produced by culturing the host cells for a period of time sufficient to allow for expression of the antibody in the host cells or, more preferably, secretion of the antibody into the culture medium in which the host cells are grown. Antibodies can be recovered from the culture medium using standard protein purification methods.

Host cells can also be used to produce portions of intact antibodies, such as Fab fragments or scFv molecules. It is understood that variations on the above procedure are within the scope of the present invention. For example, it may be desirable to transfect a host cell with DNA encoding either the light chain or the heavy chain (but not both) of an antibody of this invention. Recombinant DNA technology may also be used to remove some or all of the DNA encoding either or both of the light and heavy chains that is not necessary for binding to hTNFα. The molecules expressed from such truncated DNA molecules are also encompassed by the antibodies of the invention. In addition, bifunctional antibodies may be produced in which one heavy and one light chain are an antibody of the invention and the other heavy and light chain are specific for an antigen other than hTNFα by crosslinking an antibody of the invention to a second antibody by standard chemical crosslinking methods.

In a preferred system for recombinant expression of an antibody, or antigen-binding portion thereof, of the invention, a recombinant expression vector encoding both the antibody heavy chain and the antibody light chain is introduced into dhfr-CHO cells by calcium phosphate-mediated transfection. Within the recombinant expression vector, the antibody heavy and light chain genes are each operatively linked to CMV enhancer/AdMLP promoter regulatory elements to drive high levels of transcription of the genes. The recombinant expression vector also carries a DHFR gene, which allows for selection of CHO cells that have been transfected with the vector using methotrexate selection/amplification. The selected transformant host cells are culture to allow for expression of the antibody heavy and light chains and intact antibody is recovered from the culture medium. Standard molecular biology techniques are used to prepare the recombinant expression vector, transfect the host cells, select for transformants, culture the host cells and recover the antibody from the culture medium.

In view of the foregoing, nucleic acid, vector and host cell compositions that can be used for recombinant expression of the antibodies and antibody portions used in the invention include nucleic acids, and vectors comprising said nucleic acids, comprising the human TNFα antibody adalimumab (D2E7). The nucleotide sequence encoding the D2E7 light chain variable region is shown in SEQ ID NO: 36. The CDR1 domain of the LCVR encompasses nucleotides 70-102, the CDR2 domain encompasses nucleotides 148-168 and the CDR3 domain encompasses nucleotides 265-291. The nucleotide sequence encoding the D2E7 heavy chain variable region is shown in SEQ ID NO: 37. The CDR1 domain of the HCVR encompasses nucleotides 91-105, the CDR2 domain encompasses nucleotides 148-198 and the CDR3 domain encompasses nucleotides 295-330. It will be appreciated by the skilled artisan that nucleotide sequences encoding D2E7-related antibodies, or portions thereof (e.g., a CDR domain, such as a CDR3 domain), can be derived from the nucleotide sequences encoding the D2E7 LCVR and HCVR using the genetic code and standard molecular biology techniques.

Recombinant human antibodies of the invention in addition to D2E7 or an antigen binding portion thereof, or D2E7-related antibodies disclosed herein can be isolated by screening of a recombinant combinatorial antibody library, preferably a scFv phage display library, prepared using human VL and VH cDNAs prepared from mRNA derived from human lymphocytes. Methodologies for preparing and screening such libraries are known in the art. In addition to commercially available kits for generating phage display libraries (e.g., the Pharmacia *Recombinant Phage Antibody System,* catalog no. 27-9400-01; and the Stratagene *SurfZAP*™ phage display kit, catalog no. 240612), examples of methods and reagents particularly amenable for use in generating and screening antibody display libraries can be found in, for example, Ladner et al. U.S. Patent No. 5,223,409; Kang et al. PCT Publication No. WO 92/18619; Dower et al. PCT Publication No. WO 91/17271; Winter et al. PCT Publication No. WO 92/20791; Markland et al. PCT Publication No. WO 92/15679; Breitling et al. PCT Publication No. WO 93/01288; McCafferty et al. PCT Publication No. WO 92/01047; Garrard et al. PCT Publication No. WO 92/09690; Fuchs et al. (1991) Bio/Technology 9:1370-1372; Hay et al. (1992) Hum Antibod Hybridomas 3:81-65; Huse et al. (1989) Science 246:1275-1281; McCafferty et al., Nature (1990) 348:552-554; Griffiths et al. (1993) EMBO J 12:725-734; Hawkins et al. (1992) J Mol Biol 226:889-896; Clackson et al. (1991) Nature 352:624-628; Gram et al. (1992) PNAS 89:3576-3580; Garrard et al. (1991) Bio/Technology 9:1373-1377; Hoogenboom et al. (1991) Nuc Acid Res 19:4133-4137; and Barbas et al. (1991) PNAS 88:7978-7982.

In a preferred embodiment, to isolate human antibodies with high affinity and a low off rate constant for hTNFα, a murine anti-hTNFα antibody having high affinity and a low off rate constant for hTNFα (e.g., MAK 195, the hybridoma for which has deposit number ECACC 87 050801) is first used to select human heavy and light chain sequences having similar binding activity toward hTNFα, using the epitope imprinting methods described in Hoogenboom et al., PCT Publication No. WO 93/06213. The antibody libraries used in this method are preferably scFv libraries prepared and screened as described in McCafferty et al., PCT Publication No. WO 92/01047, McCafferty et al., Nature (1990) 348:552-554; and Griffiths et al., (1993) EMBO J 12:725-734. The scFv antibody libraries preferably are screened using recombinant human TNFα as the antigen.

Once initial human VL and VH segments are selected, "mix and match" experiments, in which different pairs of the initially selected VL and VH segments are screened for hTNFα binding, are performed to select preferred VL/VH pair combinations. Additionally, to further improve the affinity and/or lower the off rate constant for hTNFα binding, the VL and VH segments of the preferred VL/VH pair(s) can be randomly mutated, preferably within the CDR3 region of VH and/or VL, in a process analogous to the *in vivo* somatic mutation process responsible for affinity maturation of antibodies during a natural immune response. This *in vitro* affinity maturation can be accomplished by amplifying VH and VL regions using PCR primers complimentary to the VH CDR3 or VL CDR3, respectively, which primers have been "spiked" with a random mixture of the four nucleotide bases at certain positions such that the resultant PCR products encode VH and VL segments into which random mutations have been introduced into the VH and/or VL CDR3 regions. These randomly mutated VH and VL segments can be rescreened for binding to hTNFα and sequences that exhibit high affinity and a low off rate for hTNFα binding can be selected.

Following screening and isolation of an anti-hTNFα antibody of the invention from a recombinant immunoglobulin display library, nucleic acid encoding the selected antibody can be recovered from the display package (*e.g*., from the phage genome) and subcloned into other expression vectors by standard recombinant DNA techniques. If desired, the nucleic acid can be further manipulated to create other antibody forms of the invention (e.g., linked to nucleic acid encoding additional immunoglobulin domains, such as additional constant regions). To express a recombinant human antibody isolated by screening of a combinatorial library, the DNA encoding the antibody is cloned into a recombinant expression vector and introduced into a mammalian host cells, as described in further detail in above.

Methods of isolating human neutralizing antibodies with high affinity and a low off rate constant for hTNFα are described in U.S. Patent Nos. 6,090,382, 6,258,562, and 6,509,015, each of which is incorporated by reference herein.

Antibodies, antibody-portions, and other TNFα inhibitors for use in the methods of the invention, can be incorporated into pharmaceutical compositions suitable for administration to a subject. Typically, the pharmaceutical composition comprises an antibody, antibody portion, or other TNFα inhibitor, and a pharmaceutically acceptable carrier. As used herein, "pharmaceutically acceptable carrier" includes any and all solvents, dispersion media, coatings, antibacterial and antifungal agents, isotonic and absorption delaying agents, and the like that are physiologically compatible. Examples of pharmaceutically acceptable carriers include one or more of water, saline, phosphate buffered saline, dextrose, glycerol, ethanol and the like, as well as combinations thereof. In many cases, it is preferable to include isotonic agents, for example, sugars, polyalcohols such as mannitol, sorbitol, or sodium chloride in the composition. Pharmaceutically acceptable carriers may further comprise minor amounts of auxiliary substances such as wetting or emulsifying agents, preservatives or buffers, which enhance the shelf life or effectiveness of the antibody, antibody portion, or other TNFα inhibitor.

The compositions for use in the methods and compositions of the invention may be in a variety of forms. These include, for example, liquid, semi-solid and solid dosage forms, such as liquid solutions (*e.g*., injectable and infusible solutions), dispersions or suspensions, tablets, pills, powders, liposomes and suppositories. The preferred form depends on the intended mode of administration and therapeutic application. Typical preferred compositions are in the form of injectable or infusible solutions, such as compositions similar to those used for passive immunization of humans with other antibodies or other TNFα inhibitors. The preferred mode of administration is parenteral (*e.g*., intravenous, subcutaneous, intraperitoneal, intramuscular). In a preferred embodiment, the antibody or other TNFα inhibitor is administered by intravenous infusion or injection. In another preferred embodiment, the antibody or other TNFα inhibitor is administered by intramuscular or subcutaneous injection.

Therapeutic compositions typically must be sterile and stable under the conditions of manufacture and storage. The composition can be formulated as a solution, microemulsion, dispersion, liposome, or other ordered structure suitable to high drug concentration. Sterile injectable solutions can be prepared by incorporating the active compound (*i.e*., antibody, antibody portion, or other TNFα inhibitor) in the required amount in an appropriate solvent with one or a combination of ingredients enumerated above, as required, followed by filtered sterilization. Generally, dispersions are prepared by incorporating the active compound into a sterile vehicle that contains a basic dispersion medium and the required other ingredients from those enumerated above. In the case of sterile powders for the preparation of sterile injectable solutions, the preferred methods of preparation are vacuum drying and freeze-drying that yields a powder of the active ingredient plus any additional desired ingredient from a previously sterile-filtered solution thereof. The proper fluidity of a solution can be maintained, for example, by the use of a coating such as lecithin, by the maintenance of the required particle size in the case of dispersion and by the use of surfactants. Prolonged absorption of injectable compositions can be brought about by including in the composition an agent that delays absorption, for example, monostearate salts and gelatin.

In one embodiment, the invention includes pharmaceutical compositions comprising an effective TNFα inhibitor and a pharmaceutically acceptable carrier, wherein the effective TNFα inhibitor may be used to treat psoriasis.

In one embodiment, the antibody or antibody portion for use in the methods of the invention is incorporated into a pharmaceutical formulation as described in PCT/IB03/04502 and U.S. Appln. No. 20040033228, incorporated by reference herein. This formulation includes a concentration 50 mg/ml of the antibody D2E7 (adalimumab), wherein one pre-filled syringe contains 40 mg of antibody for subcutaneous injection.

The antibodies, antibody-portions, and other TNFα inhibitors of the present invention can be administered by a variety of methods known in the art, although for many therapeutic applications, the preferred route/mode of administration is parenteral, *e.g*., subcutaneous injection. In another embodiment, administration is via intravenous injection or infusion.

As will be appreciated by the skilled artisan, the route and/or mode of administration will vary depending upon the desired results. In certain embodiments, the active compound may be prepared with a carrier that will protect the compound against rapid release, such as a controlled release formulation, including implants, transdermal patches, and microencapsulated delivery systems. Biodegradable, biocompatible polymers can be used, such as ethylene vinyl acetate, polyanhydrides, polyglycolic acid, collagen, polyorthoesters, and polylactic acid. Many methods for the preparation of such formulations are patented or generally known to those skilled in the art. See, *e.g.,* Sustained and Controlled Release Drug Delivery Systems, Robinson, ed., Dekker, Inc., New York, 1978.

In one embodiment, the TNFα antibodies and inhibitors used in the invention are delivered to a subject subcutaneously. In one embodiment, the subject administers the TNFα inhibitor, including, but not limited to, TNFα antibody, or antigen-binding portion thereof, to himself/herself.

The TNFα antibodies and inhibitors used in the invention may also be administered in the form of protein crystal formulations which include a combination of protein crystals encapsulated within a polymeric carrier to form coated particles. The coated particles of the protein crystal formulation may have a spherical morphology and be microspheres of up to 500 micro meters in diameter or they may have some other morphology and be microparticulates. The enhanced concentration of protein crystals allows the antibody of the invention to be delivered subcutaneously. In one embodiment, the TNFα antibodies of the invention are delivered via a protein delivery system, wherein one or more of a protein crystal formulation or composition, is administered to a subject with a TNFα-related disorder. Compositions and methods of preparing stabilized formulations of whole antibody crystals or antibody fragment crystals are also described in WO 02/072636, which is incorporated by reference herein. In one embodiment, a formulation comprising the crystallized antibody fragments described in PCT/IB03/04502 and U.S. Appln. No. 20040033228, incorporated by reference herein, are used to treat rheumatoid arthritis using the treatment methods of the invention.

In certain embodiments, an antibody, antibody portion, or other TNFα inhibitor of the invention may be orally administered, for example, with an inert diluent or an assimilable edible carrier. The compound (and other ingredients, if desired) may also be enclosed in a hard or soft shell gelatin capsule, compressed into tablets, or incorporated directly into the subject's diet. For oral therapeutic administration, the compounds may be incorporated with excipients and used in the form of ingestible tablets, buccal tablets, troches, capsules, elixirs, suspensions, syrups, wafers, and the like. To administer a compound of the invention by other than parenteral administration, it may be necessary to coat the compound with, or co-administer the compound with, a material to prevent its inactivation.

Supplementary active compounds can also be incorporated into the compositions. In certain embodiments, an antibody or antibody portion for use in the methods of the invention is coformulated with and/or coadministered with one or more additional therapeutic agents, including an Psoriasis inhibitor or antagonist. For example, an anti-hTNFα antibody or antibody portion of the invention may be coformulated and/or coadministered with one or more additional antibodies that bind other targets associated with TNFα related disorders (*e.g*., antibodies that bind other cytokines or that bind cell surface molecules), one or more cytokines, soluble TNFα receptor (see *e.g.,* PCT Publication No. WO 94/06476) and/or one or more chemical agents that inhibit hTNFα production or activity (such as cyclohexane-ylidene derivatives as described in PCT Publication No. WO 93/19751) or any combination thereof. Furthermore, one or more antibodies of the invention may be used in combination with two or more of the foregoing therapeutic agents. Such combination therapies may advantageously utilize lower dosages of the administered therapeutic agents, thus avoiding possible side effects, complications or low level of response by the patient associated with the various monotherapies.

The pharmaceutical compositions of the invention may include a "therapeutically effective amount" or a "prophylactically effective amount" of an antibody or antibody portion of the invention. A "therapeutically effective amount" refers to an amount effective, at dosages and for periods of time necessary, to achieve the desired therapeutic result. A therapeutically effective amount of the antibody, antibody portion, or other TNFα inhibitor may vary according to factors such as the disease state, age, sex, and weight of the individual, and the ability of the antibody, antibody portion, other TNFα inhibitor to elicit a desired response in the individual. A therapeutically effective amount is also one in which any toxic or detrimental effects of the antibody, antibody portion, or other TNFα inhibitor are outweighed by the therapeutically beneficial effects. A "prophylactically effective amount" refers to an amount effective, at dosages and for periods of time necessary, to achieve the desired prophylactic result. Typically, since a prophylactic dose is used in subjects prior to or at an earlier stage of disease, the prophylactically effective amount will be less than the therapeutically effective amount.

Additional description regarding methods and uses of the invention comprising administration of a TNFα inhibitor are described in Part III of this specification.

The invention also pertains to packaged pharmaceutical compositions or kits for administering the anti-TNF antibodies of the invention for the treatment of Psoriasis. In one embodiment of the invention, the kit comprises a TNFα inhibitor, such as an antibody and instructions for administration of the TNFα inhibitor for treatment of Psoriasis. The instructions may describe how, *e.g*., subcutaneously, and when, *e.g.,* at week 0, week 2, week 4, etc., the different doses of TNFα inhibitor shall be administered to a subject for treatment.

Another aspect of the invention pertains to kits containing a pharmaceutical composition comprising a TNFα inhibitor, such as an antibody, and a pharmaceutically acceptable carrier and one or more pharmaceutical compositions each comprising an additional therapeutic agent useful for treating psoriasis, and a pharmaceutically acceptable carrier. Alternatively, the kit comprises a single pharmaceutical composition comprising an anti-TNFα antibody, one or more drugs useful for treating psoriasis, and a pharmaceutically acceptable carrier. The instructions may describe how, *e.g.,* subcutaneously, and when, *e.g.,* at week 0, week 2, week 4, etc., the different doses of TNFα inhibitor and/or the additional therapeutic agent shall be administered to a subject for treatment.

The kit may contain instructions for dosing of the pharmaceutical compositions for the treatment of psoriasis. Additional description regarding articles of manufacture of the invention are described in subsection III.

The package or kit alternatively can contain the TNFα inhibitor and it can be promoted for use, either within the package or through accompanying information, for the uses or treatment of the disorders described herein. The packaged pharmaceuticals or kits further can include a second agent (as described herein) packaged with or copromoted with instructions for using the second agent with a first agent (as described herein).

### III. Uses and Compositions for Treating Psoriasis

Tumor necrosis factor has been implicated in the pathophysiology of psoriasis (Takematsu *et al.* (1989) *Arch Dermatol Res.* 281:398; Victor and Gottlieb (2002) *J Drugs Dermatol.* 1(3):264). Psoriasis is described as a skin inflammation (irritation and redness) characterized by frequent episodes of redness, itching, and thick, dry, silvery scales on the skin. In particular, lesions are formed which involve primary and secondary alterations in epidermal proliferation, inflammatory responses of the skin, and an expression of regulatory molecules such as lymphokines and inflammatory factors. Psoriatic skin is morphologically characterized by an increased turnover of epidermal cells, thickened epidermis, abnormal keratinization, inflammatory cell infiltrates into the epidermis and polymorphonuclear leukocyte and lymphocyte infiltration into the epidermis layer resulting in an increase in the basal cell cycle. Psoriasis often involves the nails, which frequently exhibit pitting, separation of the nail, thickening, and discoloration. Psoriasis is often associated with other inflammatory disorders, for example arthritis, including rheumatoid arthritis, inflammatory bowel disease (IBD), and Crohn's disease.

Evidence of psoriasis is most commonly seen on the trunk, elbows, knees, scalp, skin folds, or fingernails, but it may affect any or all parts of the skin. Normally, it takes about a month for new skin cells to move up from the lower layers to the surface. In psoriasis, this process takes only a few days, resulting in a build-up of dead skin cells and formation of thick scales. Symptoms of psoriasis include: skin patches, that are dry or red, covered with silvery scales, raised patches of skin, accompanied by red borders, that may crack and become painful, and that are usually lovated on the elbows, knees, trunk, scalp, and hands; skin lesions, including pustules, cracking of the skin, and skin redness; joint pain or aching which may be associated with of arthritis, *e.g.*, psoriatic arthritis.

Treatment for psoriasis often includes a topical corticosteroids, vitamin D analogs, and topical or oral retinoids, or combinations thereof. In one embodiment, the TNFα inhibitor of the invention is administered in combination with or the presence of one of these common treatments. Additional therapeutic agents which can also be combined with the TNFα inhibitor of the invention for treatment of psoriasis are described in more detail below.

The diagnosis of psoriasis is usually based on the appearance of the skin. Additionally a skin biopsy, or scraping and culture of skin patches may be needed to rule out other skin disorders. An x-ray may be used to check for psoriatic arthritis if joint pain is present and persistent.

In one embodiment of the invention, a TNFα inhibitor is used to treat psoriasis, including chronic plaque psoriasis, guttate psoriasis, inverse psoriasis, pustular psoriasis, pemphigus vulgaris, erythrodermic psoriasis, psoriasis associated with inflammatory bowel disease (IBD), and psoriasis associated with rheumatoid arthritis (RA). Specific types of psoriasis included in the treatment methods of the invention are described in detail below:

### a. Chronic plaque psoriasis

Tumor necrosis factor has been implicated in the pathophysiology of chronic plaque psoriasis (Asadullah et al. (1999) Br J Dermatol. 141:94). Chronic plaque psoriasis (also referred to as psoriasis vulgaris) is the most common form of psoriasis. Chronic plaque psoriasis is characterized by raised reddened patches of skin, ranging from coin-sized to much larger. In chronic plaque psoriasis, the plaques may be single or multiple, they may vary in size from a few millimeters to several centimeters. The plaques are usually red with a scaly surface, and reflect light when gently scratched, creating a "silvery" effect. Lesions (which are often symmetrical) from chronic plaque psoriasis occur all over body, but with predilection for extensor surfaces, including the knees, elbows, lumbosacral regions, scalp, and nails. Occasionally chronic plaque psoriasis can occur on the penis, vulva and flexures, but scaling is usually absent. Diagnosis of patients with chronic plaque psoriasis is usually based on the clinical features described above. In particular, the distribution, color and typical silvery scaling of the lesion in chronic plaque psoriasis are characteristic of chronic plaque psoriasis.

### b. Guttate psoriasis

Guttate psoriasis refers to a form of psoriasis with characteristic water drop shaped scaly plaques. Flares of guttate psoriasis generally follow an infection, most notably a streptococcal throat infection. Diagnosis of guttate psoriasis is usually based on the appearance of the skin, and the fact that there is often a history of recent sore throat.

### c. Inverse psoriasis

Inverse psoriasis is a form of psoriasis in which the patient has smooth, usually moist areas of skin that are red and inflammed, which is unlike the scaling associated with plaque psoriasis. Inverse psoriasis is also referred to as intertiginous psoriasis or flexural psoriasis. Inverse psoriasis occurs mostly in the armpits, groin, under the breasts and in other skin folds around the genitals and buttocks, and, as a result of the locations of presentation, rubbing and sweating can irriate the affected areas.

### d. Pustular psoriasis

Pustular psoriasis is a form of psoriasis that causes pus-filled blisters that vary in size and location, but often occur on the hands and feet. The blisters may be localized, or spread over large areas of the body. Pustular psoriasis can be both tender and painful, can cause fevers.

### e. Other psoriasis disorders

Other examples of psoriatic disorders which can be treated with the TNFα antibody of the invention include erythrodermic psoriasis, vulgaris, psoriasis associated with IBD, and psoriasis associated with arthritis, including rheumatoid arthritis.

TNFα is an important cytokine in the pathogenesis of psoriasis, with elevated concentrations of TNFα playing a role in pathologic inflammation. Psoriasis is a chronic, inflammatory proliferative disease of the skin that affects 1-3% of the general population (Greaves and Weinstein (1995) N Engl J Med 332: 581). Treatment of moderate to severe psoriasis with systemic therapy such as methotrexate or cyclosporine or biologic therapy such as efalizumab can be limited by lack of efficacy or precluded by side effects. Ultraviolet light therapy is often inconvenient. The methods and uses described herein provide a means of determining the efficacy of a TNFα inhibotor for treating psoriasis. In one embodiment, the invention provides a method for treating psoriasis in a subject having psoriasis.

Severity of psoriasis may be determined according to standard clinical definitions. For example, the Psoriasis Area and Severity Index (PASI is used by dermatologists to assess psoriasis disease intensity. This index is based on the quantitative assessment of three typical signs of psoriatic lesions: erythema, infiltration, and desquamation, combined with the skin surface area involvement. Since its development in 1978, this instrument has been used throughout the world by clinical investigators (Fredriksson T, Petersson U: Severe psoriasis - oral therapy with a new retinoid. Dermatologica 1978; 157: 238-41). PASI is indicated as PASI 50 (a 50 percent improvement in PASI from baseline), PASI 75 (a 75 percent improvement in PASI from baseline), PASI 90 (a 90 percent improvement in PASI from baseline), and PASI 100 (a 100 percent improvement in PASI from baseline). The efficacy of a TNFa inhibitor for treatment of psoriatic arthritis in a patient population who has psoriasis, may be evaluated by determining the percentage of the patient population in whom a PASI 50, PASI 75, PASI 90, or PASI 100 response has been achieved following administration of the TNFa inhibitor.

The Physicians Global Assessment (PGA) is used to assess psoriasis activity and follow clinical response to treatment. It is a six-point score that summarizes the overall quality (erythema, scaling and thickness) and extent of plaques relative to the baseline assessment. A patient's response is rated as worse, poor (0-24%), fair (25-49%), good (50-74%), excellent (75-99%), or cleared (100%) (van der Kerkhof P. The psoriasis area and severity index and alternative approaches for the assessment of severity: persisting areas of confusion. Br J Dermatol 1997; 137:661-662). Other measures of improvements in the disease state of a subject having psoriasis include clinical responses, such as the Dermatology Life Quality Index (DLQI) and the Minnimum Clinically Important Difference (MCID), described in more detail below.

Methods of treatment described herein may include administration of a TNFα inhibitor to a subject to achieve a therapeutic goal, *e.g.,* treatment of psoriasis, increase in PASI response, maintenance of a level of PASI response, improvement in PASI score, and/or achievement of a PGA score of "clear" or "almost clear." Also included in the scope of the invention are uses of a TNFα inhibitor in the manufacture of a medicament to achieve a therapeutic goal, *e.g.,* treatment, of psoriasis, increase in PASI response, maintenance of a level of PASI response, and/or improvement in PASI score, and/or achievement of a PGA score of "clear" or "almost clear." Thus, where methods are described herein, it is also intended to be part of this invention that the use of the TNFα inhibitor in the manufacture of a medicament for the purpose of the method is also considered within the scope of the invention. Likewise, where a use of a TNFα inhibitor in the manufacture of a medicament for the purpose of achieving a therapeutic goal is described, methods of treatment resulting in the therapeutic goal are also intended to be part of the invention.

The invention also provides pharmacokinetic parameters which have been identified as providing a therapeutic benefit to a subject having psoriasis. Certain mean steady-state trough levels of a TNFα inhibitor have be identified as corresponding to therapeutic benefits for subject having psoriasis, including, but not limited to, treatment of psoriasis. The term "trough level" refers to the serum TNFα inhibitor concentration at a time after delivery of a previous dose and immediately prior to delivery of the next subsequent dose of drug in a series of doses. Generally, the trough serum concentration is a minimum sustained efficacious drug concentration in the series of drug administrations. Also, the trough serum concentration is frequently targeted as a minimum serum concentration for efficacy because it represents the serum concentration at which another dose of drug is to be administered as part of the treatment regimen.

In one embodiment, the invention provides a method of treating psoriasis in a subject in need thereof comprising administering a loading dose of a TNFα inhibitor, *e.g*., human TNFα antibody, or antigen-binding portion thereof, to the subject, wherein the loading dose provides a mean serum TNFα inhibitor trough level of about 12 µg/mL. Once treatment has been achieved, *e.g*., PASI 50 or PASI 75 response has been achieved, a maintenance dose(s) of the TNFα inhibitor, *e.g.,* human TNFα antibody, or antigen-binding portion thereof, may be administered to the subject in order to maintain treatment of psoriasis, wherein the maintenance dose provides a mean serum trough level of about 7 µg/mL of the TNFα inhibitor.

In one embodiment, the invention provides a method of treating of psoriasis in a subject comprising administering a maintenance dose of the TNFα inhibitor, *e.g.,* human TNFα antibody, or antigen-binding portion thereof, to the subject, wherein the maintenance dose provides a mean serum trough level of about 7 µg/mL of the TNFα inhibitor.

The invention also provides a method of treating psoriasis-related disorders, comprising administering a TNFα inhibitor to a subject. The TNFα inhibitors used in the present invention may be administered by a variety of methods known in the art, although for many therapeutic applications, the preferred route/mode of administration is parenteral, including intravenous or subcutaneous injection. In one embodiment, the invention provides a method of treating fistulas associated with psoriasis.

In one embodiment, induction and remission of psoriasis is achieved using multiple variable dosing methods of treatment. Examples of such multiple variable dosing regimens are described in PCT appln. no. PCT/US05/12007, incorporated by reference herein.

In one embodiment, maintenance of remission of psoriasis is achieved by administering a TNFα inhibitor to a subject in accordance with a biweekly dosing regimen. Biweekly dosing regimens can be used to treat disorders in which TNFα activity is detrimental, and are further described in US Appln. No. 10/163657 (US 20030235585), incorporated by reference herein.

In one embodiment, the invention provides a method of treating psoriasis in a subject comprising administering an initial loading dose of a TNFα inhibitor to the subject at week 0. In one embodiment, the initial dose is given in its entirety on one day or is divided over 2 days. In one embodiment, the initial dose is administered subcutaneously. Following administration of the initial loading dose, a second dose of the TNFα inhibitor may be administered to the subject, wherein the second dose is about half the dose amount of the initial loading dose. In one embodiment, the second dose is administered to the subject about two weeks after the first dose. In one embodiment, the second dose is administered subcutaneously. Subsequent doses may be administered following the second dose in order to achieve treatment of the subject. Such additional doses may, in one embodiment of the invention, comprise half the dose amount of the second dose. Once treatment is achieved, at least one a maintenance dose of the TNFα inhibitor is administered to the subject in order to maintain treatment of psoriasis. In one embodiment, the maintenance dose is about half the dose amount of the second dose. In one embodiment, the maintenance dose is administered to the subject about two weeks after the second dose. In one embodiment, the maintenance therapy for administering the TNFα inhibitor comprises a biweekly dosing regimen. In one embodiment, the maintenance dose is administered subcutaneously.

In one embodiment, the invention provides a method of treating psoriasis in a subject comprising administering an initial loading dose of a human TNFα antibody, or antigen-binding portion thereof, *e.g.,* adalimumab, to the subject at week 0. The initial dose may be given in its entirety on one day or may be divided over 2 days. In one embodiment, the initial dose of the human TNFα antibody, or antigen-binding portion thereof, comprises 80 mg. In one embodiment, the initial dose is administered subcutaneously. Following administration of the initial loading dose, a second dose of the human TNFα antibody, or antigen-binding portion thereof, *e.g*., adalimumab, is administered to the subject. In one embodiment, the second dose comprises 80 mg of the human TNFα antibody, or antigen-binding portion thereof. In one embodiment, the second dose is administered to the subject about one week after the first dose. In one embodiment, the second dose is administered subcutaneously. In order to maintain treatment psoriasis once it is achieved, at least one maintenance dose of the human TNFα antibody, or antigen-binding portion thereof, *e.g*., adalimumab, is administered to the subject. In one embodiment, the maintenance dose is about half the dose amount of the second dose. In one embodiment, the maintenance dose of the human TNFα antibody, or antigen-binding portion thereof, comprises 40 mg. In one embodiment, the maintenance therapy for administering the human TNFα antibody, or antigen-binding portion thereof, comprises a biweekly dosing regimen. In one embodiment, the maintenance dose is administered subcutaneously.

In another embodiment, the initial dose of the human TNFα antibody, or antigen-binding portion thereof, comprises 80 mg and may be given at week 0, followed by at least one maintenance dose of the human TNFα antibody, or antigen-binding portion thereof, comprising 40 mg, administered on a biweekly dosing regimen.

Dosage unit form as used herein refers to physically discrete units suited as unitary dosages for the mammalian subjects to be treated; each unit containing a predetermined quantity of active compound calculated to produce the desired therapeutic effect in association with the required pharmaceutical carrier. The specification for the dosage unit forms of the invention are dictated by and directly dependent on (a) the unique characteristics of the active compound and the particular therapeutic or prophylactic effect to be achieved, and (b) the limitations inherent in the art of compounding such an active compound for the treatment of sensitivity in individuals.

Dosage regimens described herein may be adjusted to provide the optimum desired response, *e.g*., maintaining remission of psoriasis, in consideration of the teachings herein. It is to be noted that dosage values may vary with the type and severity of psoriasis. It is to be further understood that for any particular subject, specific dosage regimens may be adjusted over time according to the teachings of the specification and the individual need and the professional judgment of the person administering or supervising the administration of the compositions, and that dosage amounts and ranges set forth herein are exemplary only and are not intended to limit the scope or practice of the claimed invention.

Examples of other methods and uses of TNFα inhibitors for the treatment of psoriasis are also described in U.S. Provisional Application Nos. 60/832370, 60/851830, and 60/857352, incorporated herein.

### Subpopulations

The invention provides uses and methods for treating certain subpopulations of psoriasis patients with a TNFα inhibitor.

In one embodiment, the invention provides a method of treating moderate to severe psoriasis in a subject comprising administering to the subject a TNFα inhibitor, such that moderate to severe psoriasis is treated. Subjects having moderate to severe psoriasis may be administered a TNFα inhibitor such that moderate to severe psoriasis is treated and advancement of the disease is prevented. The invention also provides use of a TNFα inhibitor in the manufacture of a medicament for the treatment of moderate to severe psoriasis in a subject who has moderate to severe psoriasis. In a preferred embodiment, a patient having moderate to severe psoriasis is defined as a patient having a PASI < 10. In another embodiment, a patient having moderate to severe psoriasis is defined as a patient having a PASI <15. In another embodiment, a patient having moderate to severe psoriasis is defined as a patient having a PASI <20. In another embodiment, a patient having moderate to severe psoriasis is defined as a patient having a PASI <25. In another embodiment, a patient having moderate to severe psoriasis is defined as a patient having a PASI <30. In another embodiment, a patient having moderate to severe psoriasis is defined as a patient having a PASI <35. In another embodiment, a patient having moderate to severe psoriasis is defined as a patient having a PASI <40.

Numbers below and intermediate to the above recited PASI responses, e.g., 0%, 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, and 39%, as well as all other numbers recited herein, are also intended to be part of this invention. Ranges of values using a combination of any of the above recited values as upper and/or lower limits are intended to be included in the scope of the invention. For example, in one embodiment a PASI 90 response score of in at least between 0% and 40% of the patient population indicates that the pateient has moderate to severe psoriasis.

The invention also provides a method for treating a subpopulation of psoriasis patients who are intolerant to or have lost response to a first TNFα inhibitor, *e.g.,* infliximab, for the treatment of psoriasis. Clinical trials have demonstrated the efficacy of infliximab, a chimeric monoclonal antibody to TNF, for treatment of patients with moderate to severe psoriasis. Infusions of infliximab, especially when given episodically, may result in the development of antibodies to infliximab, however, which in turn may lead to infusion reactions, loss of efficacy, and delayed hypersensitivity reactions (Baert et al. N Engl J Med 2003;348:601-608; Cheifetz et al. Am J Gastroenterol 2003;98:1315-1324; Farrell et al. Gastroenterology 2003;124:917-924; Hanauer et al. Gastroenterology 1999;116:A731; and Hanauer et al. Clin Gastroenterol Hepatol 2004;2:542-553). In certain instances, some patients who are administered a TNFα inhibitor for the treatment of psoriasis and respond to said treatment, may eventually lose their response to the first TNFα inhibitor. In other patient populations, intolerance to a certain TNFα inhibitor may be marked from the initial administration of the TNFα inhibitor. In one embodiment, the invention provides use of a TNFα inhibitor in the manufacture of a medicament for treating psoriasis in a subject who has lost response to or is intolerant to a different TNFα inhibitor. In one embodiment, the TNFα inhibitor which the subject has lost response to or is intolerant to is infliximab.

In one embodiment, the invention also provides methods and compositions for use in a subject who has not previously been administered infliximab. Thus, in one embodiment, the methods and compositions of the invention are directed to a subpopulation of psoriasis patients who have not previously received infliximab.

In one embodiment, the invention provides an article of manufacture comprising adalimumab and a package insert, wherein the package insert indicates that adalimumab may be used to treat psoriasis in patients who have had an inadequate response to conventional therapy and/or who have lost response to or are intolerant to infliximab.

### Articles of Manufacture

The invention also provides a packaged pharmaceutical composition wherein the TNFα inhibitor, *e.g.,* human TNFα antibody, is packaged within a kit or an article of manufacture. The kit or article of manufacture of the invention contains materials useful for the treatment, including induction and/or remission, prevention and/or diagnosis of psoriasis. The kit or article of manufacture comprises a container and a label or package insert or printed material on or associated with the container which provides information regarding use of the TNFα inhibitor, *e.g.,* a TNFα antibody, for the treatment of psoriasis.

A kit or an article of manufacture refers to a packaged product comprising components with which to administer a TNFα inhibitor for treatment of a psoriasis. The kit preferably comprises a box or container that holds the components of the kit. The box or container is affixed with a label or a Food and Drug Administration approved label, including a protocol for administering the TNFα inhibitor. The box or container holds components of the invention which are preferably contained within plastic, polyethylene, polypropylene, ethylene, or propylene vessels. The vessels can be capped-tubes or bottles. The kit can also include instructions for administering the TNFα antibody of the invention. In one embodiment the kit of the invention includes the formulation comprising the human antibody adalimumab (or D2E7), as described in PCT/IB03/04502 and U.S. Appln. No. 10/222140, incorporated by reference herein.

The term "package insert" is used to refer to instructions customarily included in commercial packages of therapeutic products, that contain information about the indications, usage, dosage, administration, contraindications and/or warnings concerning the use of such therapeutic products.

In one embodiment, the article of manufacture of the invention comprises (a) a first container with a composition contained therein, wherein the composition comprises a TNFα antibody; and (b) a package insert indicating that the TNFα antibody may be used for reducing signs and symptoms and inducing and maintaining remission of psoriasis. In a preferred embodiment, the label or package insert indicates that the TNFα inhibitor, e.g., a TNFα antibody, is used for treating psoriasis.

Suitable containers for the TNFα inhibitor, *e.g.,* a TNFα antibody, include, for example, bottles, vials, syringes, pens, etc. The containers may be formed from a variety of materials such as glass or plastic. The container holds a composition which is by itself or when combined with another composition effective for treating, preventing and/or diagnosing the condition and may have a sterile access port.

In one embodiment, the article of manufacture comprises a TNFα inhibitor, *e.g.,* a human TNFα antibody, and a label which indicates to a subject who will be administering the TNFα inhibitor about using the TNFα inhibitor for the treatment of psoriasis. The label may be anywhere within or on the article of manufacture. In one embodiment, the article of manufacture comprises a container, such as a box, which comprises the TNFα inhibitor and a package insert or label providing information pertaining to use of the TNFα inhibitor for the treatment of psoriasis. In another embodiment, the information is printed on a label which is on the outside of the article of manufacture, in a position which is visible to prospective purchasers.

In one embodiment, the package insert of the invention informs a reader, including a subject, *e.g.,* a purchaser, who will be administering the TNFα inhibitor for treatment, that the TNFα inhibitor, *e.g*., a TNFα antibody such as adalimumab, is an indicated treatment of psoriasis, including of moderately to severely active disease in adult patients.

In one embodiment, the package insert describes certain patient populations who may respond favorably to the TNFα inhibitor within the article of manufacture. For example, the package insert may indicate that the TNFα antibody, *e.g*., adalimumab, may be used to treat psoriasis in patients who have had an inadequate response to conventional therapy and/or who have lost response to or are intolerant to infliximab.

In another embodiment, the label of the invention indicates that adalimumab is indicated for treatment of moderately to severely active psoriasis in adult patients who have had an inadequate response to conventional therapy. In another embodiment, the label of the invention indicates that the TNFα inhibitor, *e.g*., a TNFα antibody such as adalimumab, is also indicated for treatment in adult patients with moderately to severely active psoriasis who have lost response to or are intolerant to infliximab.

In one embodiment, the package insert of the invention describes certain therapeutic benefits of the TNFα antibody, *e.g*., adalimumab, including specific symptoms of psoriasis which may be reduced by using the TNFα antibody, *e.g.,* adalimumab. It should be noted that the package insert may also contain information pertaining to other disorders which are treatable using the TNFα antibody, *e.g*., adalimumab. Information described herein which is provided in a package insert and pertains to other disorders, *i.e.,* diseases other than psoriasis, is also included within the scope of the invention. The package insert of the invention may indicate that extra TNFα in your body can attack normal healthy body tissues and cause inflammation especially in the tissues in your bones, cartilage, joints and digestive tract. The package insert of the invention may also indicate that adalimumab helps reduce the signs and symptoms of immune diseases, including rheumatoid and psoriatic arthritis (pain and swollen joints), ankylosing spondylitis (morning stiffness and back pain), and psoriasis (abdominal pain and diarrhea).

In another embodiment, the package insert of the invention describes the dose and administration of adalimumab, for the treatment of psoriasis. The label may indicate that the initiation of therapy includes a 80 mg dose at week 0 and 80 mg at week 1. The label may also indicate that the maintenance dosing for the treatment of psoriasis with adalimumab is 40 mg every other week. In one embodiment, the package insert indicates that the week 0 dose may be administered as 4 injections in one day or divided over 2 days. The label may also indicate that some patients with psoriasis may derive additional benefit by increasing frequency to 40 mg every week. In another embodiment, the package insert of the invention indicates that adalimumab is administered by subcutaneous injection.

In another embodiment, the label of the invention indicates that the recommended TNFα inhibitor, *e.g.,* a TNFα antibody such as adalimumab, dose regimen for adult patients with psoriasis is 80 mg at week 0 (dose can be administered as four injections in one day or as two injections per day for two consecutive days), followed by 40 mg every other week beginning at week 2. The label of the invention may also indicate that some patients may derive additional benefit from increasing the dosing frequency of the TNFα inhibitor, *e.g.,* a TNFα antibody such as adalimumab from 40 mg every other week to 40 mg every week.

In another embodiment, the label of the invention indicates that the recommended TNFα inhibitor, *e.g.,* a TNFα antibody such as adalimumab, dose regimen for adult patients with psoriasis is 40 mg at week 0 (dose can be administered as four injections in one day or as two injections per day for two consecutive days), followed by 40 mg every other week beginning at week 2. The label of the invention may also indicate that some patients may derive additional benefit from increasing the dosing frequency of the TNFα inhibitor, *e.g*., a TNFα antibody such as adalimumab from 40 mg every other week to 40 mg every week.

The package insert of the invention may also provide information to subjects who will be receiving adalimumab regarding combination uses for both safety and efficacy purposes. In another embodiment, the label of the invention indicates that aminosalicylates, corticosteroids, and/or immunomodulatory agents (e.g., 6-mercaptopurine and azathioprine) may be continued during treatment with the TNFα inhibitor, *e.g*., a TNFα antibody, including adalimumab. In one embodiment, the invention provides an article of manufacture comprising a packaging material; a TNFα antibody, or antigen-binding portion thereof; and a label or package insert contained within the packaging material indicating that aminosalicylates, corticosteroids, and/or immunomodulatory agent, e.g., 6-mercaptopurine and azathioprine, may be continued during treatment with the TNFα antibody, or antigen-binding portion thereof.

The package insert of the invention may contain warnings and precautions regarding the use of the TNFα inhibitor, *e.g*., a TNFα antibody such as adalimumab. In one embodiment, the information provided in the label describes malignancies. The label of the invention may indicate that during the controlled portions of TNFα antibody, such as adalimumab, trials in patients with rheumatoid arthritis, psoriatic arthritis, ankylosing spondylitis, Crohn's diases, and psoriasis, malignancies, other than lymphoma and non-melanoma skin cancer, were observed at a rate (95% confidence interval) of 0.6 (0.3, 1.0)/100 patient-years among 2887 adalimumab-treated patients versus a rate of 0.4 (0.2, 1.1)/100 patient-years among 1570 control patients (median duration of treatment of 5.7 months for adalimumab-treated patients and 5.5 months for control-treated patients). The label may also indicate that the size of the control group and limited duration of the controlled portions of studies precludes the ability to draw firm conclusions. In one embodiment, the label indicates that in the controlled and uncontrolled open-label portions of the clinical trials of adalimumab, the more frequently observed malignancies, other than lymphoma and non-melanoma skin cancer, were breast, colon, prostate, lung and melanoma. In one embodiment, the label indicates that these malignancies in adalimumab treated and control-treated patients were similar in type and number to what would be expected in the general population. The label may further indicate that during the controlled portions of adalimumab rheumatoid arthritis, psoriatic arthritis, ankylosing spondylitis, Crohn's diases, and psoriasis trials, the rate (95% confidence interval) of non-melanoma skin cancers was 0.8 (0.47, 1.24)/100 patient-years among adalimumab -treated patients 0.2 (0.05, 0.82)/100 patient-years among control patients. In one embodiment, the label indicates that the potential role of TNF blocking therapy in the development of malignancies is not known. In one embodiment, the label indicates that in the controlled portions of clinical trials of all the TNF-blocking agents, more cases of lymphoma have been observed among patients receiving TNF blockers compared to control patients. In one embodiment, the label indicates that in controlled trials in patients with rheumatoid arthritis, psoriatic arthritis, ankylosing spondylitis, and psoriasis, 2 lymphomas were observed among 2887 HUMIRA®-treated patients versus 1 among 1570 control patients. In one embodiment, the label of the invention indicates that in combining the controlled and uncontrolled open-label portions of these clinical trials with a median duration of approximately 2 years, including 4843 patients and over 13,000 patient-years of therapy, the observed rate of lymphomas is approximately 0.12/100 patient-years, and that this is approximately 3.5-fold higher than expected in the general population.

The label of the invention may contain information regarding the use of the TNFα inhibitor, *e.g.,* a TNFα antibody such as adalimumab, in clinical studies for psoriasis. In one embodiment, the label of the invention describes the studies described herein as Example 1, either as a whole or in portion. The label of the invention may also indicate that adalimumab has been studied in over 1400 patients with psoriasis in four placebo-controlled and two open-label extension studies. The label of the invention may also indicate that the safety profile for patients with psoriasis treated with HUMIRA® was similar to the safety profile seen in patients with rheumatoid arthritis.

The label of the invention may contain information regarding the pharmacodynamics of the TNFα inhibitor, *e.g*., a TNFα antibody such as adalimumab. In one embodiment, the label of the invention indicates that after treatment with adalimumab, a rapid decrease in levels of acute phase reactants of inflammation (C-reactive protein (CRP) and erythrocyte sedimentation rate (ESR) and serum cytokines (IL-6) was observed compared to baseline in patients with rheumatoid arthritis. In one embodiment, the label of the invention indicates that a rapid decrease in CRP levels was also observed in patients with psoriasis. The label may further indicate that serum levels of matrix metalloproteinases (MMP-1 and MMP-3) that produce tissue remodeling responsible for cartilage destruction were also decreased after adalimumab administration.

The label of the invention may also contain information regarding the pharmacokinetics of the TNFα inhibitor, *e.g.,* a TNFα antibody such as adalimumab. In one embodiment, the label of the invention indicates that in patients with psoriasis, the loading dose of 80 mg adalimumab on week 0 followed by 80 mg adalimumab on week 1 achieves serum adalimumab trough concentrations of approximately 12 µg/mL. The label of the invention may also indicate that mean steady-state trough levels of approximately 7 µg/mL were observed in psoriasis patients who received a maintenance dose of 40 mg adalimumab every other week

In one embodiment, the invention provides an article of manufacture comprising a TNFα inhibitor and a package insert, wherein the package insert indicates that in patients with psoriasis who have been administered the TNFα inhibitor, the loading dose on week 0 followed by a second dose on week 2 achieves serum adalimumab trough concentrations of approximately 12 µg/mL.

In one embodiment, an article of manufacture comprising a TNFα inhibitor and a package insert, wherein the package insert indicates that in patients with psoriasis who have been administered the TNFα inhibitor, the mean steady-state trough levels of approximately 7 µg/mL were observed in psoriasis patients who received a maintenance dose of the TNFα inhibitor every other week.

The label of the invention may also contain information regarding drug interactions of the TNFα inhibitor, *e.g.,* a TNFα antibody such as adalimumab, with other drugs. In one embodiment, the label indicates that methotrexate (MTX) reduced adalimumab apparent clearance after single and multiple dosing by 29% and 44% respectively, in patients with rheumatoid arthritis.

In one embodiment of the invention, the kit comprises a TNFα inhibitor, such as an antibody, an second pharmaceutical composition comprising an additional therapeutic agent, and instructions for administration of both agents for the treatment of psoriasis. The instructions may describe how, *e.g.,* subcutaneously, and when, *e.g.,* at week 0, week 2, and biweekly thereafter, doses of TNFα antibody and/or the additional therapeutic agent shall be administered to a subject for treatment.

Another aspect of the invention pertains to kits containing a pharmaceutical composition comprising an anti-TNFα antibody and a pharmaceutically acceptable carrier and one or more additional pharmaceutical compositions each comprising a drug useful for treating a TNFα related disorder and a pharmaceutically acceptable carrier. Alternatively, the kit comprises a single pharmaceutical composition comprising an anti-TNFa antibody, one or more drugs useful for treating a TNFα related disorder and a pharmaceutically acceptable carrier. The kits further contain instructions for dosing of the pharmaceutical compositions for the treatment of a TNFα related disorder.

The package or kit alternatively may contain the TNFα inhibitor and it may be promoted for use, either within the package or through accompanying information, for the uses or treatment of the disorders described herein. The packaged pharmaceuticals or kits further can include a second agent (as described herein) packaged with or copromoted with instructions for using the second agent with a first agent (as described herein).

### Additional therapeutic agents

TNFα inhibitors, including TNFα antibodies, or antigen binding portions thereof, may be used in the methods, uses, and compositions of the invention either alone or in combination with an additional therapeutic agent. It should be understood that the TNFα inhibitors can be used alone or in combination with an additional agent, e.g., a therapeutic agent, said additional agent being selected by the skilled artisan for its intended purpose. For example, the additional agent can be a therapeutic agent art-recognized as being useful to treat the disease or condition being treated by the TNFα inhibitors. The additional agent also can be an agent that imparts a beneficial attribute to the therapeutic composition, *e.g.,* an agent which effects the viscosity of the composition.

It should further be understood that the combinations which are to be included within this invention are those combinations useful for their intended purpose. The agents set forth below are illustrative for purposes and not intended to be limited. The combinations, which are part of this invention, can be the TNFα inhibitors of the present invention and at least one additional agent selected from the lists below. The combination can also include more than one additional agent, *e.g*., two or three additional agents if the combination is such that the formed composition can perform its intended function.

Non-limiting examples of therapeutic agents for Psoriasis with which an antibody, or antibody portion, of the invention can be combined include the following: small molecule inhibitor of KDR (ABT-123), small molecule inhibitor of Tie-2, calcipotriene, clobetasol propionate, triamcinolone acetonide, halobetasol propionate, tazarotene, methotrexate, fluocinonide, betamethasone diprop augmented, fluocinolone acetonide, acitretin, tar shampoo, betamethasone valerate, mometasone furoate, ketoconazole, pramoxine/fluocinolone, hydrocortisone valerate, flurandrenolide, urea, betamethasone, clobetasol propionate/emoll, fluticasone propionate, azithromycin, hydrocortisone, moisturizing formula, folic acid, desonide, pimecrolimus, coal tar, diflorasone diacetate, etanercept folate, lactic acid, methoxsalen, hc/bismuth subgal/znox/resor, methylprednisolone acetate, prednisone, sunscreen, halcinonide, salicylic acid, anthralin, clocortolone pivalate, coal extract, coal tar/salicylic acid, coal tar/salicylic acid/sulfur, desoximetasone, diazepam, emollient, fluocinonide/emollient, mineral oil/castor oil/na lact, mineral oil/peanut oil, petroleum/isopropyl myristate, psoralen, salicylic acid, soap/tribromsalan, thimerosal/boric acid, celecoxib, infliximab, cyclosporine, alefacept, efalizumab, tacrolimus, pimecrolimus, PUVA, UVB, sulfasalazine.

TNFα inhibitors described herein may be used in combination with additional therapeutic agents such as a Disease Modifying Anti-Rheumatic Drug (DMARD) or a Nonsteroidal Antiinflammatory Drug (NSAID) or a steroid or any combination thereof. Preferred examples of a DMARD are hydroxychloroquine, leflunomide, methotrexate, parenteral gold, oral gold and sulfasalazine. Preferred examples of non-steroidal antiinflammatory drug(s) also referred to as NSAIDS include drugs like ibuprofen. Other preferred combinations are corticosteroids including prednisolone; the well known side effects of steroid use can be reduced or even eliminated by tapering the steroid dose required when treating patients in combination with TNFα inhibitors of this invention.

Preferred combinations of therapeutic agents may interfere at different points in the autoimmune and subsequent inflammatory cascade; preferred examples include TNF antagonists such as soluble p55 or p75 TNF receptors, derivatives, thereof, (p75TNFR1gG (Enbrel™) or p55TNFR1gG (Lenercept), chimeric, humanized or human TNF antibodies, or a fragment thereof, including infliximab (Remicade^{®}, Johnson and Johnson; described in U.S. Patent No. 5,656,272, incorporated by reference herein), PSORIASIS P571 (a humanized monoclonal anti-TNF-alpha IgG4 antibody), PSORIASIS P 870 (a humanized monoclonal anti-TNF-alpha antibody fragment), an anti-TNF dAb (Peptech), CNTO 148 (golimumab; Medarex and Centocor, see WO 02/12502), and adalimumab (HUMIRA®^{®} Abbott Laboratories, a human anti-TNF mAb, described in US 6,090,382 as D2E7). Additional TNF antibodies which can be used in the invention are described in U.S. Patent Nos. 6,593,458; 6,498,237; 6,451,983; and 6,448,380, each of which is incorporated by reference herein. Other combinations including TNFα converting enzyme (TACE) inhibitors; IL-1 inhibitors (Interleukin-1-converting enzyme inhibitors, IL-1RA etc.) may be effective for the same reason. Other preferred combinations include Interleukin 11. Yet another preferred combination are other key players of the autoimmune response which may act parallel to, dependent on or in concert with TNFα inhibitors function; especially preferred are IL-18 antagonists including IL-18 antibodies or soluble IL-18 receptors, or IL-18 binding proteins. Yet another preferred combination are non-depleting anti-PSORIASIS 4 inhibitors. Yet other preferred combinations include antagonists of the co-stimulatory pathway PSORIASIS 80 (B7.1) or PSORIASIS 86 (B7.2) including antibodies, soluble receptors or antagonistic ligands.

The TNFα inhibitors used in the invention may also be combined with agents, such as methotrexate, 6-MP, azathioprine sulphasalazine, mesalazine, olsalazine chloroquinine/hydroxychloroquine, pencillamine, aurothiomalate (intramuscular and oral), azathioprine, cochicine, corticosteroids (oral, inhaled and local injection), beta-2 adrenoreceptor agonists (salbutamol, terbutaline, salmeteral), xanthines (theophylline, aminophylline), cromoglycate, nedocromil, ketotifen, ipratropium and oxitropium, cyclosporin, FK506, rapamycin, mycophenolate mofetil, leflunomide, NSAIDs, for example, ibuprofen, corticosteroids such as prednisolone, phosphodiesterase inhibitors, adensosine agonists, antithrombotic agents, complement inhibitors, adrenergic agents, agents which interfere with signalling by proinflammatory cytokines such as TNFα or IL-1 (e.g. IRAK, NIK, IKK , p38 or MAP kinase inhibitors), IL-1β converting enzyme inhibitors, TNFα converting enzyme (TACE) inhibitors, T-cell signalling inhibitors such as kinase inhibitors, metalloproteinase inhibitors, sulfasalazine, azathioprine, 6-mercaptopurines, angiotensin converting enzyme inhibitors, soluble cytokine receptors and derivatives thereof (e.g. soluble p55 or p75 TNF receptors and the derivatives p75TNFRIgG (Enbrel™ and p55TNFRIgG (Lenercept)), sIL-1RI, sIL-1RII, sIL-6R), antiinflammatory cytokines (e.g. IL-4, IL-10, IL-11, IL-13 and TGFβ), celecoxib, folic acid, hydroxychloroquine sulfate, rofecoxib, etanercept, infliximab, naproxen, valdecoxib, sulfasalazine, methylprednisolone, meloxicam, methylprednisolone acetate, gold sodium thiomalate, aspirin, triamcinolone acetonide, propoxyphene napsylate/apap, folate, nabumetone, diclofenac, piroxicam, etodolac, diclofenac sodium, oxaprozin, oxycodone hcl, hydrocodone bitartrate/apap, diclofenac sodium/misoprostol, fentanyl, anakinra, human recombinant, tramadol hcl, salsalate, sulindac, cyanocobalamin/fa/pyridoxine, acetaminophen, alendronate sodium, prednisolone, morphine sulfate, lidocaine hydrochloride, indomethacin, glucosamine sulf/chondroitin, amitriptyline hcl, sulfadiazine, oxycodone hcl/acetaminophen, olopatadine hcl, misoprostol, naproxen sodium, omeprazole, cyclophosphamide, rituximab, IL-1 TRAP, MRA, CTLA4-IG, IL-18 BP, anti-IL-18, Anti-IL15, BIRB-796, SCIO-469, VX-702, AMG-548, VX-740, Roflumilast, IC-485, PSORIASIS C-801, and Mesopram.

Non-limiting examples of therapeutic agents for psoriasis with which TNFα inhibitor of the invention can be combined include the following: budenoside; epidermal growth factor; corticosteroids; cyclosporin, sulfasalazine; aminosalicylates; 6-mercaptopurine; azathioprine; metronidazole; lipoxygenase inhibitors; mesalamine; olsalazine; balsalazide; antioxidants; thromboxane inhibitors; IL-1 receptor antagonists; anti-IL-1β monoclonal antibodies; anti-IL-6 monoclonal antibodies; growth factors; elastase inhibitors; pyridinyl-imidazole compounds; antibodies to or antagonists of other human cytokines or growth factors, for example, TNF, LT, IL-1, IL-2, IL-6 (including Actemra (tocilizumab), IL-7, IL-8, IL-15, IL-16, IL-17, IL-18, EMAP-II, GM-CSF, FGF, and PDGF. Antibodies of the invention, or antigen binding portions thereof, can be combined with antibodies to cell surface molecules such as CD2, CD3, CD4, CD8, CD25, CD28, CD30, CD40, CD45, CD69, CD80 (B7.1), CD86 (B7.2), CD90, CTLA or their ligands including CD154 (gp39 or CD40L).

The antibodies of the invention, or antigen binding portions thereof, may also be combined with agents, such as methotrexate, cyclosporin, FK506, rapamycin, mycophenolate mofetil, leflunomide, NSAIDs, for example, ibuprofen, corticosteroids such as prednisolone, phosphodiesterase inhibitors, adenosine agonists, antithrombotic agents, complement inhibitors, adrenergic agents, agents which interfere with signalling by proinflammatory cytokines such as TNFα or IL-1 (e.g. IRAK, NIK, IKK, p38 or MAP kinase inhibitors), IL-1β converting enzyme inhibitors, TNFα converting enzyme inhibitors, T-cell signalling inhibitors such as kinase inhibitors, metalloproteinase inhibitors, sulfasalazine, azathioprine, 6-mercaptopurines, angiotensin converting enzyme inhibitors, soluble cytokine receptors and derivatives thereof (e.g. soluble p55 or p75 TNF receptors, sIL-1RI, sIL-1RII, sIL-6R) and antiinflammatory cytokines (e.g. IL-4, IL-10, IL-11, IL-13 and TGFβ).

Additional examples of therapeutic agents for psoriasis in which a TNFα inhibitor can be combined include the following: combinations of TNF antagonists, for example, anti-TNF antibodies, D2E7 (PCT Publication No. WO 97/29131; HUMIRA®), CA2 (REMICADE), PSORIASIS P 571, TNFR-Ig constructs, (p75TNFRIgG (ENBREL) and p55TNFRIgG (LENERCEPT)) inhibitors and PDE4 inhibitors. TNFα inhibitors of the invention can be combined with corticosteroids, for example, budenoside and dexamethasone. TNFα inhibitors of the invention may also be combined with agents such as sulfasalazine, 5-aminosalicylic acid and olsalazine, and agents which interfere with synthesis or action of proinflammatory cytokines such as IL-1, for example, IL-1β converting enzyme inhibitors and IL-1ra. TNFα inhibitors may also be used with T cell signaling inhibitors, for example, tyrosine kinase inhibitors 6-mercaptopurines. TNFα inhibitors can be combined with IL-11. TNFα inhibitors can be combined with mesalamine, prednisone, azathioprine, mercaptopurine, infliximab, methylprednisolone sodium succinate, diphenoxylate/atrop sulfate, loperamide hydrochloride, methotrexate, omeprazole, folate, ciprofloxacin/dextrose-water, hydrocodone bitartrate/apap, tetracycline hydrochloride, fluocinonide, metronidazole, thimerosal/boric acid, cholestyramine/sucrose, ciprofloxacin hydrochloride, hyoscyamine sulfate, meperidine hydrochloride, midazolam hydrochloride, oxycodone hcl/acetaminophen, promethazine hydrochloride, sodium phosphate, sulfamethoxazole/trimethoprim, celecoxib, polycarbophil, propoxyphene napsylate, hydrocortisone, multivitamins, balsalazide disodium, codeine phosphate/apap, colesevelam hcl, cyanocobalamin, folic acid, levofloxacin, methylprednisolone, natalizumab and interferon-gamma

The TNFα inhibitors may also be combined with agents, such as alemtuzumab, dronabinol, Unimed, daclizumab, mitoxantrone, xaliproden hydrochloride, fampridine, glatiramer acetate, natalizumab, sinnabidol, a-immunokine NNSO3, ABR-215062, AnergiX.MS, chemokine receptor antagonists, BBR-2778, calagualine, CPI-1189, LEM (liposome encapsulated mitoxantrone), THC.CBD (cannabinoid agonist) MBP-8298, mesopram (PDE4 inhibitor), MNA-715, anti-IL-6 receptor antibody, neurovax, pirfenidone allotrap 1258 (RDP-1258), sTNF-R1, talampanel, teriflunomide,TGF-beta2, tiplimotide, VLA-4 antagonists (for example, TR-14035, VLA4 Ultrahaler, Antegran-ELAN/Biogen), interferon gamma antagonists, IL-4 agonists, and the humanized IL-6 antibody tocilizumab.

In yet another embodiment, the invention includes an article of manufacture or a method comprising the combination of a TNF inhibitor and an antibiotic or antiinfective agent. Antiinfective agents include those agents known in the art to treat viral, fungal, parasitic or bacterial infections. The term, "antibiotic," as used herein, refers to a chemical substance that inhibits the growth of, or kills, microorganisms. Encompassed by this term are antibiotic produced by a microorganism, as well as synthetic antibiotics (e.g., analogs) known in the art. Antibiotics include, but are not limited to, clarithromycin (Biaxin^{®}), ciprofloxacin (Cipro^{®}), and metronidazole (Flagyl^{®}).

Any one of the above-mentioned therapeutic agents, alone or in combination therewith, can be administered to a subject suffering from a TNFα-related disorder in which TNFα is detrimental, in combination with the TNFα antibody using a multiple variable dose treatment regimen. In one embodiment, any one of the above-mentioned therapeutic agents, alone or in combination therewith, can be administered to a subject suffering from an intestinal disorder in addition to a TNFα antibody to treat another TNFα-related disorder, such as rheumatoid arthritis. It should be understood that the additional therapeutic agents can be used in combination therapy as described above, but also may be used in other indications described herein wherein a beneficial effect is desired.

The combination of agents used within the methods and pharmaceutical compositions described herein may have a therapeutic additive or synergistic effect on the condition(s) or disease(s) targeted for treatment. The combination of agents used within the methods or pharmaceutical compositions described herein also may reduce a detrimental effect associated with at least one of the agents when administered alone or without the other agent(s) of the particular pharmaceutical composition. For example, the toxicity of side effects of one agent may be attenuated by another agent of the composition, thus allowing a higher dosage, improving patient compliance, and improving therapeutic outcome. The additive or synergistic effects, benefits, and advantages of the compositions apply to classes of therapeutic agents, either structural or functional classes, or to individual compounds themselves.

### IV. Efficacy of TNFα inhibitor

The invention also provides methods for determining whether a TNFα inhibitor is effective at treating psoriasis in a subject. Such methods may be used to determine the efficacy of a TNFα inhibitor, including those which are unknown or unconfirmed to have such efficacy. Using the methods described herein, effective TNFα inhibitors may be determined or confirmed, and, subsequently, used in the method of treating psoriasis. Further methods for determining whether a TNFα inhibitor is effective at treating psoriasis in a subject are described in U.S. Provisional Application Nos. 60/832370 (filed July 20, 2006), 60/851830 (filed October 6, 2006), and 60/857352 (filed November 6, 2006), each of which are incorporated herein by reference.

In one embodiment, the invention provides a method for determining the efficacy of a TNFα inhibitor, including a human TNFα antibody, for treating psoriasis in a subject, using the Psoriasis Area Severity Index (PASI). The Psoriasis Area and Severity Index (PASI) is used by dermatologists to assess psoriasis disease intensity. This index is based on the quantitative assessment of three typical signs of psoriatic lesions: erythema, infiltration, and desquamation, combined with the skin surface area involvement. Since its development in 1978, this instrument has been used throughout the world by clinical investigators (Fredriksson T, Petersson U: Severe psoriasis - oral therapy with a new retinoid. Dermatologica 1978; 157: 238-41.) PASI is indicated as PASI 50 (a 50 percent improvement in PASI from baseline), PASI 75 (a 75 percent improvement in PASI from baseline), PASI 90 (a 90 percent improvement in PASI from baseline), and PASI 100 (a 100 percent improvement in PASI from baseline). The efficacy of a TNFa inhibitor for treatment of psoriatic arthritis in a patient population who has psoriasis, may be evaluated by determining the percentage of the patient population in whom a PASI 50, PASI 75, PASI 90, or PASI 100 response has been achieved following administration of the TNFa inhibitor.

The Physicians Global Assessment (PGA) is used to assess psoriasis activity and follow clinical response to treatment. It is a six-point score that summarizes the overall quality (erythema, scaling and thickness) and extent of plaques relative to the baseline assessment. A patient's response is rated as worse, poor (0-24%), fair (25-49%), good (50-74%), excellent (75-99%), or cleared (100%) (van der Kerkhof P. The psoriasis area and severity index and alternative approaches for the assessment of severity: persisting areas of confusion. Br J Dermatol 1997; 137:661-662).

The DLQI is an additional validated instrument used to assess dermatologic-related functional limitations. Characteristics of the DLQI include:
- ten items on an overall scoring range of 0-30; higher scores represent greater quality of life impairment and lower scores represent lower quality of life impairment;
- well-established properties of reliability and validity for the DLQI total score in a dermatology setting (see Badia et al. (1999) Br J Dermatol 141:698; Finlay et al. (1994) Clin Exp Dermatol 19:210; and Shikier et al. (2003) Health and Quality of Life Outcomes 1:53);
- six subcategories: symptoms and feelings; daily activities; leisure; work/school; personal relationships; and treatment;
- all data are observed values. Patients who discontinued before the time point were not included in this analysis.
Ranges of DLQI scores can be evaluated for their correspondence to categories of disease impact.

The PASI, PGA, and DLQI scores may be used as an index for measuring efficacy of a TNFα inhibitor in a patient population having psoriasis, where attaining a certain percentage of patients within a population who were administered the TNFα inhibitor and who maintain clinical remission, *i.e.* PASI < 50 or PASI < 75, indicates that the TNFα inhibitor is effective for treating of psoriasis. In one embodiment, the invention provides a method for determining whether a human TNFα antibody is effective for treating psoriasis.

The efficacy of a TNFα inhibitor for treating psoriasis in a patient population, *i.e.,* PASI 75 response (also referred to herein as a PASI / PASI75 score), may be evaluated by determining the percentage of the patient population in treatment of psoriasis has been effective following administration of the TNFα inhibitor.

In one embodiment, the invention provides a method of determining the efficacy of a TNFα inhibitor for treating psoriasis in a subject comprising determining a Psoriasis Area Severity Index (PASI) score of a patient population having psoriasis and who was administered the TNFα inhibitor, wherein a PASI 75 response is achieved in at least about 77% of the patient population indicates that the TNFα inhibitor is an effective TNFα inhibitor for the treatment of psoriasis in a subject. In one embodiment, the method further comprises administering the effective TNFα inhibitor to a subject to treat psoriasis. The invention provides a method of treatingpsoriasis in a subject comprising administering an effective amount of a TNFα inhibitor to the subject such that treatment of psoriasis is maintained, wherein the effective human TNFα antibody was previously identified as achieving a PASI 75 response in at least about 77% of a patient population having psoriasis and a baseline PASI < 10.

In one embodiment, the invention provides a method of treatnig psoriasis in a subject comprising administering an effective amount of a human TNFα antibody to the subject such that psoriasis is treated, wherein the effective human TNFα antibody was previously identified as achieving a PASI 75 response in at least about 77% of a patient population having psoriasis and a baseline PASI < 10.

In one embodiment, a PASI 75 response is achieved in at least about 77% of the patient population indicates that the human TNFα antibody is an effective human TNFα antibody for the treatment of psoriasis in a subject. In one embodiment, a PASI 75 response is achieved in at least about 80% of the patient population indicates that the human TNFα antibody is an effective human TNFα antibody for the treatment of psoriasis in a subject. In one embodiment, a PASI 75 response is achieved in at least about 85% of the patient population indicates that the human TNFα antibody is an effective human TNFα antibody for the treatment of psoriasis in a subject. In one embodiment, a PASI 75 response is achieved in at least about 88% of the patient population indicates that the human TNFα antibody is an effective human TNFα antibody for the treatment of psoriasis in a subject. In one embodiment, a PASI 75 response is achieved in at least about 90% of the patient population indicates that the human TNFα antibody is an effective human TNFα antibody for the treatment of psoriasis in a subject.

Numbers intermediate to the above recited percentages, *e.g.,* 78%, 79%, 80%. 81%, 82%, 83%, 84%, 85%, 86%, 87%, 88%, and 89%, as well as all other numbers recited herein, are also intended to be part of this invention. Ranges of values using a combination of any of the above recited values as upper and/or lower limits are intended to be included in the scope of the invention. For example, in one embodiment a PASI 75 response score of in at least between 77% and 90% of the patient population indicates that the TNFα inhibitor is an effective TNFα inhibitor for the treatment of psoriasis in a subject.

In one embodiment, the invention provides a method of determining the efficacy of a TNFα inhibitor for treating psoriasis in a subject comprising determining a Psoriasis Area Severity Index (PASI) score of a patient population having psoriasis and who was administered the TNFα inhibitor, wherein a PASI 75 response is achieved in at least about 32% of the patient population indicates that the TNFα inhibitor is an effective TNFα inhibitor for the treatment of psoriasis in a subject. In one embodiment, the method further comprises administering the effective TNFα inhibitor to a subject to treat psoriasis. The invention provides a method of treating psoriasis in a subject comprising administering an effective amount of a TNFα inhibitor to the subject such that treatment of psoriasis is maintained, wherein the effective human TNFα antibody was previously identified as achieving a PASI 75 response in at least about 32% of a patient population having psoriasis and a baseline PASI < 10.

In one embodiment, the invention provides a method of treatnig psoriasis in a subject comprising administering an effective amount of a human TNFα antibody to the subject such that psoriasis is treated, wherein the effective human TNFα antibody was previously identified as achieving a PASI 75 response in at least about 32% of a patient population having psoriasis and a baseline PASI < 10.

In one embodiment, a PASI 75 response is achieved in at least about 32% of the patient population indicates that the human TNFα antibody is an effective human TNFα antibody for the treatment of psoriasis in a subject. In one embodiment, a PASI 75 response is achieved in at least about 40% of the patient population indicates that the human TNFα antibody is an effective human TNFα antibody for the treatment of psoriasis in a subject. In one embodiment, a PASI 75 response is achieved in at least about 50% of the patient population indicates that the human TNFα antibody is an effective human TNFα antibody for the treatment of psoriasis in a subject. In one embodiment, a PASI 75 response is achieved in at least about 60% of the patient population indicates that the human TNFα antibody is an effective human TNFα antibody for the treatment of psoriasis in a subject. In one embodiment, a PASI 75 response is achieved in at least about 70% of the patient population indicates that the human TNFα antibody is an effective human TNFα antibody for the treatment of psoriasis in a subject. In one embodiment, a PASI 75 response is achieved in at least about 80% of the patient population indicates that the human TNFα antibody is an effective human TNFα antibody for the treatment of psoriasis in a subject. In one embodiment, a PASI 75 response is achieved in at least about 90% of the patient population indicates that the human TNFα antibody is an effective human TNFα antibody for the treatment of psoriasis in a subject.

Numbers intermediate to the above recited percentages, e.g., 32%, 33%, 34%. 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, and 89%, as well as all other numbers recited herein, are also intended to be part of this invention. Ranges of values using a combination of any of the above recited values as upper and/or lower limits are intended to be included in the scope of the invention. For example, in one embodiment a PASI 75 response score of in at least between 32% and 90% of the patient population indicates that the TNFα inhibitor is an effective TNFα inhibitor for the treatment of psoriasis in a subject.

In one embodiment the invention provides a method of determining the efficacy of a TNFα inhibitor for achieving a clinical response in psoriasis in a subject comprising determining a PASI 90 response of a patient population having psoriasis and a baseline PASI < 10 and who was administered the human TNFα antibody, wherein a PASI 90 response is achieved in at least about 63% of the patient population indicates that the human TNFα antibody is an effective human TNFα antibody for achieving a clinical resopnse in psoriasis in a subject.

In one embodiment, the invention provides a method for achieving a clinical response in psoriasis in a subject comprising administering an effective amount of a human TNFα antibody to the subject such that a clinical response in psoriasis is achieved, wherein the effective amount of the human TNFα antibody was previously identified as achieving a PASI 90 response in at least about 63% of a patient population having psoriasis and a baseline PASI < 10.

In one embodiment, a PASI 90 response is achieved in at least about 63% of the patient population indicates that the human TNFα antibody is an effective human TNFα antibody for achieving a clinical resopnse in psoriasis in a subject. In one embodiment, a PASI 90 response is achieved in at least about 65% of the patient population indicates that the human TNFα antibody is an effective human TNFα antibody for achieving a clinical resopnse in psoriasis in a subject. In one embodiment, a PASI 90 response is achieved in at least about 68% of the patient population indicates that the human TNFα antibody is an effective human TNFα antibody for achieving a clinical resopnse in psoriasis in a subject. In one embodiment, a PASI 90 response is achieved in at least about 70% of the patient population indicates that the human TNFα antibody is an effective human TNFα antibody for achieving a clinical resopnse in psoriasis in a subject. In one embodiment, a PASI 90 response is achieved in at least about 75% of the patient population indicates that the human TNFα antibody is an effective human TNFα antibody for achieving a clinical resopnse in psoriasis in a subject. In one embodiment, a PASI 90 response is achieved in at least about 80% of the patient population indicates that the human TNFα antibody is an effective human TNFα antibody for achieving a clinical resopnse in psoriasis in a subject.

Numbers intermediate to the above recited percentages, e.g., 63%, 64%, 65%. 66%, 67%, 68%, 69%, 70%, 71%, 72%, 73%, 74%, 75%, 76%, 77%, 78%, 79%, and 80%, as well as all other numbers recited herein, are also intended to be part of this invention. Ranges of values using a combination of any of the above recited values as upper and/or lower limits are intended to be included in the scope of the invention. For example, in one embodiment a PASI 90 response score of in at least between 63% and 80% of the patient population indicates that the TNFα inhibitor is an effective TNFα inhibitor for the treatment of psoriasis in a subject.

In one embodiment the invention provides a method of determining the efficacy of a TNFα inhibitor for achieving a clinical response in psoriasis in a subject comprising determining a PASI 90 response of a patient population having psoriasis and a baseline PASI < 10 and who was administered the human TNFα antibody, wherein a PASI 90 response is achieved in at least about 24% of the patient population indicates that the human TNFα antibody is an effective human TNFα antibody for achieving a clinical resopnse in psoriasis in a subject.

In one embodiment, the invention provides a method for achieving a clinical response in psoriasis in a subject comprising administering an effective amount of a human TNFα antibody to the subject such that a clinical response in psoriasis is achieved, wherein the effective amount of the human TNFα antibody was previously identified as achieving a PASI 90 response in at least about 24% of a patient population having psoriasis and a baseline PASI < 10.

In one embodiment, a PASI 90 response is achieved in at least about 25% of the patient population indicates that the human TNFα antibody is an effective human TNFα antibody for achieving a clinical resopnse in psoriasis in a subject. In one embodiment, a PASI 90 response is achieved in at least about 30% of the patient population indicates that the human TNFα antibody is an effective human TNFα antibody for achieving a clinical resopnse in psoriasis in a subject. In one embodiment, a PASI 90 response is achieved in at least about 40% of the patient population indicates that the human TNFα antibody is an effective human TNFα antibody for achieving a clinical resopnse in psoriasis in a subject. In one embodiment, a PASI 90 response is achieved in at least about 50% of the patient population indicates that the human TNFα antibody is an effective human TNFα antibody for achieving a clinical resopnse in psoriasis in a subject. In one embodiment, a PASI 90 response is achieved in at least about 60% of the patient population indicates that the human TNFα antibody is an effective human TNFα antibody for achieving a clinical resopnse in psoriasis in a subject. In one embodiment, a PASI 90 response is achieved in at least about 62% of the patient population indicates that the human TNFα antibody is an effective human TNFα antibody for achieving a clinical resopnse in psoriasis in a subject.

Numbers intermediate to the above recited percentages, *e.g.,* 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, and 61%, as well as all other numbers recited herein, are also intended to be part of this invention. Ranges of values using a combination of any of the above recited values as upper and/or lower limits are intended to be included in the scope of the invention. For example, in one embodiment a PASI 90 response score of in at least between 24% and 62% of the patient population indicates that the TNFα inhibitor is an effective TNFα inhibitor for the treatment of psoriasis in a subject.

In one embodiment the invention provides a method of determining the efficacy of a TNFα inhibitor for achieving a clinical response in psoriasis in a subject comprising determining a PASI 100 response of a patient population having psoriasis and a baseline PASI < 10 and who was administered the human TNFα antibody, wherein a PASI 100 response is achieved in at least about 36% of the patient population indicates that the human TNFα antibody is an effective human TNFα antibody for achieving a clinical response in psoriasis in a subject.

In one embodiment, a PASI 100 response is achieved in at least about 38% of the patient population indicates that the human TNFα antibody is an effective human TNFα antibody for achieving a clinical resopnse in psoriasis in a subject. In one embodiment, a PASI 100 response is achieved in at least about 40% of the patient population indicates that the human TNFα antibody is an effective human TNFα antibody for achieving a clinical resopnse in psoriasis in a subject. In one embodiment, a PASI 100 response is achieved in at least about 45% of the patient population indicates that the human TNFα antibody is an effective human TNFα antibody for achieving a clinical resopnse in psoriasis in a subject. In one embodiment, a PASI 100 response is achieved in at least about 48% of the patient population indicates that the human TNFα antibody is an effective human TNFα antibody for achieving a clinical resopnse in psoriasis in a subject. In one embodiment, a PASI 100 response is achieved in at least about 50% of the patient population indicates that the human TNFα antibody is an effective human TNFα antibody for achieving a clinical resopnse in psoriasis in a subject.

Numbers intermediate to the above recited percentages, *e.g.,* 36%, 37%, 38%. 39%, 40%, 41%, 42%, 43%, 44%, 45%, 46%, 47%, 48%, 49%, and 50%, as well as all other numbers recited herein, are also intended to be part of this invention. Ranges of values using a combination of any of the above recited values as upper and/or lower limits are intended to be included in the scope of the invention. For example, in one embodiment a PASI 100 response score of in at least between 36% and 50% of the patient population indicates that the TNFα inhibitor is an effective TNFα inhibitor for the treatment of psoriasis in a subject.

In one embodiment the invention provides a method of determining the efficacy of a TNFα inhibitor for achieving a clinical response in psoriasis in a subject comprising determining a PASI 100 response of a patient population having psoriasis and a baseline PASI < 10 and who was administered the human TNFα antibody, wherein a PASI 100 response is achieved in at least about 11 % of the patient population indicates that the human TNFα antibody is an effective human TNFα antibody for achieving a clinical response in psoriasis in a subject.

In one embodiment, a PASI 100 response is achieved in at least about 15% of the patient population indicates that the human TNFα antibody is an effective human TNFα antibody for achieving a clinical resopnse in psoriasis in a subject. In one embodiment, a PASI 100 response is achieved in at least about 20% of the patient population indicates that the human TNFα antibody is an effective human TNFα antibody for achieving a clinical resopnse in psoriasis in a subject. In one embodiment, a PASI 100 response is achieved in at least about 25% of the patient population indicates that the human TNFα antibody is an effective human TNFα antibody for achieving a clinical resopnse in psoriasis in a subject. In one embodiment, a PASI 100 response is achieved in at least about 30% of the patient population indicates that the human TNFα antibody is an effective human TNFα antibody for achieving a clinical resopnse in psoriasis in a subject. In one embodiment, a PASI 100 response is achieved in at least about 35% of the patient population indicates that the human TNFα antibody is an effective human TNFα antibody for achieving a clinical resopnse in psoriasis in a subject.

Numbers intermediate to the above recited percentages, e.g., 15%, 16%, 17%, 18%, 19%, 20%, 21%, 22%, 23%, 24%, 25%, 26%, 27%, 28%, 29%, 30%, 31%, 32%, 33%, and 34% as well as all other numbers recited herein, are also intended to be part of this invention. Ranges of values using a combination of any of the above recited values as upper and/or lower limits are intended to be included in the scope of the invention. For example, in one embodiment a PASI 100 response score of in at least between 15% and 35% of the patient population indicates that the TNFα inhibitor is an effective TNFα inhibitor for the treatment of psoriasis in a subject.

In one embodiment the invention provides a method of determining the efficacy of a TNFα inhibitor for achieving a clinical response in psoriasis in a subject comprising determining a Physician's Global Assessment (PGA) score of a patient population having psoriasis who was administered the human TNFα antibody, wherein a PGA score of "clear" or "almost clear" in at least about 77% of the patient population indicates that the human TNFα antibody is an effective human TNFα antibody for treating psoriasis in a subject.

In one embodiment, the invention provides a method of treating psoriasis in a subject comprisign administering an effective amount of a human TNFα antibody to the subject, wherein the effective human TNFα antibody was previously identified as maintaining a PGA score of "clear" or "almost clear" in at least about 77% of a patient population having psoriasis.

In one embodiment, a PGA score of "clear" or "almost clear" in at least about 77% of a patient population having psoriasis indicates that the human TNFα antibody is an effective human TNFα antibody for treating psoriasis in a subject. In one embodiment, a PGA score of "clear" or "almost clear" in at least about 80% of a patient population having psoriasis indicates that the human TNFα antibody is an effective human TNFα antibody for treating psoriasis in a subject.

Numbers intermediate to the above recited percentages, e.g., 78%, and 79%, as well as all other numbers recited herein, are also intended to be part of this invention. Ranges of values using a combination of any of the above recited values as upper and/or lower limits are intended to be included in the scope of the invention. For example, in one embodiment a PGA score of "clear" or "almost clear" in at least between 77% and 90% of the patient population indicates that the TNFα inhibitor is an effective TNFα inhibitor for the treatment of psoriasis in a subject.

In one embodiment the invention provides a method of determining the efficacy of a TNFα inhibitor for achieving a clinical response in psoriasis in a subject comprising determining a Physician's Global Assessment (PGA) score of a patient population having psoriasis who was administered the human TNFα antibody, wherein a PGA score of "clear" or "almost clear" in at least about 45% of the patient population indicates that the human TNFα antibody is an effective human TNFα antibody for treating psoriasis in a subject.

In one embodiment, the invention provides a method of treating psoriasis in a subject comprisign administering an effective amount of a human TNFα antibody to the subject, wherein the effective human TNFα antibody was previously identified as maintaining a PGA score of "clear" or "almost clear" in at least about 76% of a patient population having psoriasis.

In one embodiment, a PGA score of "clear" or "almost clear" in at least about 45% of a patient population having psoriasis indicates that the human TNFα antibody is an effective human TNFα antibody for treating psoriasis in a subject. In one embodiment, a PGA score of "clear" or "almost clear" in at least about 76% of a patient population having psoriasis indicates that the human TNFα antibody is an effective human TNFα antibody for treating psoriasis in a subject.

Numbers intermediate to the above recited percentages, *e.g.,* 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, and 75%,, as well as all other numbers recited herein, are also intended to be part of this invention. Ranges of values using a combination of any of the above recited values as upper and/or lower limits are intended to be included in the scope of the invention. For example, in one embodiment a PGA score of "clear" or "almost clear" in at least between 45% and 76% of the patient population indicates that the TNFα inhibitor is an effective TNFα inhibitor for the treatment of psoriasis in a subject.

It should be noted that the Examples provided herein represent different methods of determining the efficacy of a TNFα inhibitor, such as a human TNFα antibody, or antigen-binding portion thereof. As such, data and results described in the Examples section which shows efficacy of a TNFα inhibitor, *e.g.,* ability to maintain remission of psoriasis, are included in the methods of determining efficacy of the invention.

Time points for determining efficacy will be understood by those of skill in the art to depend on the type of efficacy being determined, *e.g.,* maintenance of remission. In one embodiment, measurements in scores, *e.g.,* the PASI response or PGA score of a subject, may be measured against a subject's baseline score. Generally, a baseline refers to a measurement or score of a patient before treatment, *i.e.* week 0. Other time points may also be included as a starting point in determining efficacy, however. For example, in determining the efficacy of a TNFα inhibitor for treating psoriasis in a patient population, a determination of the percentage of the patient population who were achieved a response, *i.e.,* PASI 75 response, may be determined based on a time point from when remission was induced.

Patient populations described in the methods of the invention are generally selected based on common characteristics, such as, but not limited to, subjects diagnosed with psoriasis who are in remission as a result of being on a dosing regimen comprising a TNFα inhibitor. Such a patient population would be appropriate for determining the efficacy of the TNFα inhibitor for maintaining remission in psoriasis in the given patient population. In one embodiment, the patient population is an adult population, *e.g.,* older than 17 years of age or older than 18 years of age.

In one embodiment, the methods of the invention for determining whether a TNFα inhibitor is an effective TNFα inhibitor, include determining changes, improvements, measurements, etc., in psoriasis using appropriate indices known in the art, *e.g.,* PASI, PGA, DLQI, status of psoriasis related disorders, etc. from a patient population who has already been administered the TNFα inhibitor. Such a patient population may be pre-selected according to common characteristics, *e.g.,* psoriasis, loss of response to infliximab, and may have already been given the TNFα inhibitor. Administration of the TNFα inhibitor may or may not be performed by the same person of ordinary skill who is determining the efficacy of the TNFα inhibitor in accordance with the teachings of the specificaiton.

In one embodiment, the methods of the invention comprise administering the TNFα inhibitor to the subjects of a patient population and determining the efficacy of the TNFα inhibitor by determining changes, improvements, measurements, etc., using psoriasis indices known in the art, in the patient population in comparison to the Examples set forth below. For example, in one embodiment the invention includes a method for determining efficacy of a TNFα inhibitor for the treatment of psoriasis comprising administering the TNFα inhibitor to a preselected patient population having psoriasis; and determining the effectiveness of the TNFα inhibitor by using a mean baseline Psoriasis Area Severity Index (PASI) response of the patient population and a mean PASI response following administration of the TNFα inhibitor, wherein a PASI 75 response achieved in at least about 77% of the patient population indicates that the TNFα inhibitor is effective for the treatment of psoriasis.

Methods of the invention relating to determining efficacy, *i.e*., determining whether a TNFα inhibitor is an effective TNFα inhibitor, may also be applied to specific patient populations within the overall patient population who together have specific, common characteristics, i.e., a subpopulation. For example, the patient population may comprise patients on concomitant immunosuppressant (IMM) treatment with the TNFα inhibitor. In another example, the patient population may comprises patients not on concomitant IMM treatment.

In addition, while the above methods are described in terms of patient populations, methods of efficacy described herein may also be applied to individual subjects. For example, a method for determining efficacy may comprise determining whether a subject who has psoriasis, and who is on a dosage regimen comprising a human TNFα antibody, is able to achieve a PASI 75 response to determing if the human TNFα antibody is an effective human TNFα antibody. In one embodiment, if the subject is able to achieve a PASI 75 response for at least about 24 weeks, then the human TNFα antibody is effective at treating psoriasis.

The Examples and discoveries described herein are representative of a TNFα inhibitor, *i.e.,* adalimumab, which is effective for treating psoriasis. As such, the studies and results described in the Examples section herein may be used as a guideline for determining the efficacy of a TNFα inhibitor, *i.e.,* whether a TNFα inhibitor is an effective TNFα inhibitor for the treatment of psoriasis. In one embodiment, methods of determining efficacy described herein may be used to determine whether a TNFα inhibitor is bioequivalent to another TNFα inhibitor.

In one embodiment, the article of manufacture of the invention comprises instructions regarding how to determine the efficacy of the TNF inhibitor for the treatment of psoriasis.

The present invention is further illustrated by the following examples which should not be construed as limiting in any way.

### EXAMPLES

### Example 1: Rapid Improvement in Functional Limitations of Patients with Moderate to Severe Chronic Plaque Psoriasis Treated with Adalimumab

Psoriasis affects approximately 1-3% of the worldwide population. Moderate to severe disease is associated with psoriatic lesions on ≥ 5% body surface area. Clinical manifestations of moderate to severe psoriasis can severely limit a patient's physical function.

The Dermatology Life Quality Index (DLQI) was used to evaluate the improvement in functional limitations of patients with moderate to severe chronic plaque psoriasis treated with adalimumab. The DLQI is a validated instrument used to measure disease impact on daily function. DLQI was specified as an endpoint in a 12-week, Phase II placebo-controlled trial of two different dose regimens of adalimumab in moderate to severe plaque psoriasis. Study design is shown in Figure 1. The object of this study was to assess the effects of adalimumab on the impact of psoriasis in patients' lives.

Efficacy and safety of adalimumab was evaluated in a 48-week extension trial conducted at eighteen sites. The study was a randomized, double-blind, placebo-controlled, multi-center clinical trial, wherein patients were randomized to one of three treatment groups:
1. 80 mg adalimumab at baseline (week 0) and 40 mg at week 1 followed by 40 mg every other week (eow) starting at week 3 (referred to as ada 40 mg eow);
2. 80 mg adalimumab at baseline (week 0) and 80 mg at week 1 followed by 40 mg weekly starting at week 2 (referred to as ada 40 mg weekly); or
3. placebo administered weekly beginning at baseline (referred to as placebo).

Inclusion criteria included a diagnosis of moderate to severe chronic plaque psoriasis ≥ 1 year prior to entry, a psoriasis-affected body surface area (BSA) > 5%, and no previous use of TNF-antagonist therapy.

A total of 142 patients who completed the 12 week randomized trial enrolled in the extension trial. Patients initially randomized to active treatment continued on their assigned dose. Placebo patients were switched to receive adalimumab 80 mg the first week followed by adalimumab 40 mg eow. During the first 12 weeks of the extension trial, patients remained on blinded therapy.

The DLQI is a validated instrument used to assess dermatologic-related functional limitations. Characteristics of the DLQI include:
- ten items on an overall scoring range of 0-30; higher scores represent greater quality of life impairment and lower scores represent lower quality of life impairment;
- well-established properties of reliability and validity for the DLQI total score in a dermatology setting (see Badia et al. (1999) Br J Dermatol 141:698; Finlay et al. (1994) Clin Exp Dermatol 19:210; and Shikier et al. (2003) Health and Quality of Life Outcomes 1:53);
- six subcategories: symptoms and feelings; daily activities; leisure; work/school; personal relationships; and treatment;
- all data are observed values. Patients who discontinued before the time point were not included in this analysis.

Ranges of DLQI scores were evaluated for their correspondence to categories of disease impact and are described below in Table 1.

**Table 1. DLQI total score and disease impact on daily life (see Hongbo et al. (2004) Br J Dermatol 151 (suppl. 68)45; Hongbo et al. (2005) Invest Dermatol)**

| DLQI Total Score | Disease Impact |
|---|---|
| 0-1 | None |
| 2-5 | Small |
| 6-10 | Moderate |
| 11-20 | Large |
| 21-30 | Extremely large |

The results were obtained from 147 patients, who were randomized and treated as described above. At baseline, 56% of patients reported a very large / extremely large disease effect as assessed by baseline DLQI scores, as shown below in Table 2:

**Table 2: Disease impact at baseline by treatment group**

| Disease Impact | Ada 40 mg eow | Ada 40 mg weekly | Placebo |
|---|---|---|---|
| Moderate | 27% | 24% | 39% |
| Large / Extremely Large | 60% | 60% | 50% |

By week 12, 85% of patients with large/extremely large disease impact at baseline who were randomized to adalimumab had no/small disease effect, compared with 1 (4%) placebo patient. More specifically, 85% of patients receiving ada 40 mg eow and 86% patients receiving ada 40 mg weekly had a change from large/extremely large impact at baseline to no/small impact at week 12. This level of improvement was maintained for up to 60 weeks of treatment with adalimumab as shown in Table 3.

**Table 3. Percentages of patients with change from large/extremely large impact at baseline to no/small impact at weeks 24, 36, and 60**

| Weeks | Ada 40 mg eow | Ada 40 mg weekly |
|---|---|---|
| 24 | 85% | 88% |
| 36 | 83% | 91% |
| 60 | 83% | 100% |

Of placebo patients with large/extremely large impact at baseline, 67% had improved to no/small effect by week 24 after switching to adalimumab at week 12. More specifically, the percentage of patients with a change from large/extremely large impact at baseline to no/small impact at week 24 was 67%; at week 36 was 79%; and at week 60 was 71%.

In conclusion, rapid reduction in disease impact was seen with adalimumab treatment of moderate to severe plaque psoriasis. Patients initially treated with placebo experienced a similar level of improvement after 12 weeks of treatment with adalimumab. Resolution of disease impact was maintained in most patients through 60 weeks of adalimumab treatment.

### Example 2: Efficacy and Safety of Adalimumab Treatment of Chronic Plaque Psoriasis in Patients Who Meet Some Criteria for Biological Interventions in Accordance with British Association ofDermatologists Guidelines

The British Association of Dermatologists (BAD) has published guidelines for the treatment of psoriasis with approved biologic interventions such as infliximab, etanercept, and efalizumab. This subanalysis study measured the efficacy of adalimumab in patients from the above study who qualified for biologic treatment under these guidelines.

The efficacy and safety of adalimumab in patients eligible for biologic therapy under selected BAD guidelines was evaluated. The selected BAD guidelines included a PASI ≥ 10 and a DLQI > 10. The efficacy outcome measures were PASI, PGA, and DLQI. Figure 1 shows the study design.

The following analytic methods were employed in this subanalysis: modified intention to treat analyses were preformed on all randomized patients receiving at least one dose of adalimumab; missing data were imputed using non-responder imputation (NRI) for PASI and PAG and observed data for DLQI; and a post-hoc analysis was conducted to examine key efficacy and safety outcomes for patients who were eligible for treatment with a biological agent as specified in the current BAD guidelines (PASI ≥ 10 and a DLQI > 10).

For the patients who met the selected BAD criteria, the baseline demographics and disease severity characteristics were similar across treatment groups. These characteristics were consistent with those of the overall patient population. Table 4 shows the overall baseline demographics and clinical characteristics of the patients enrolled in the overall study (not just specific to BAD).

**Table 4**

| | Placebo (N=52) | Adalimumab 40 mg eow (N=45) | Adalimumab 40 mg weekly (N=50) |
|---|---|---|---|
| Age, years (range) | 43 (20-70) | 46 (20-71) | 44 (24-86) |
| Duration of Ps, years (range) | 19 (1-40) | 21 (1-58) | 18 (2-48) |
| % Male | 65 | 71 | 66 |
| % Caucasian | 92 | 89 | 90 |
| Body Weight, kg (range) | 94 (50-147) | 93 (63-159) | 99 (42-149) |
| % BSA (range) | 28 (7-75) | 29 (6-58) | 25 (5-83) |
| PASI Score (range) | 16 (6-40) | 17 (5-39) | 15 (2-42) |
| DLQI Score | 12 | 13 | 14 |

Table 5 shows the overall baseline demographics and clinical characteristics for patients included in this subanalysis (those who met the above described selected BAD guidelines).

**Table 5**

| | Placebo (N=18) | Adalimumab 40 mg eow (N=26) | Adalimumab 40 mg weekly (N=23) |
|---|---|---|---|
| Age, years (range) | 44 (20-70) | 45 (20-71) | 42 (24-86) |
| Duration of Ps, years (range) | 19 (5-40) | 19 (1-58) | 19 (2-48) |
| % Male | 61 | 65 | 61 |
| % Caucasian | 89 | 85 | 91 |
| Body Weight, kg (range) | 89 (57-146) | 92 (70-159) | 104 (42-149) |
| % BSA (range) | 37 (1-75) | 31 (8-56) | 35 (10-83) |
| PASI Score (range) | 20 (11-34) | 19 (11-39) | 19 (11-42) |
| DLQI Score | 18 | 18 | 18 |

**Table 6. Overall PASI 75 Response Rates Up To Week 60**

| | | | | |
|---|---|---|---|---|
| | | | | |

| | | **Treatment** | **% of Patients** | |
|---|---|---|---|---|
| | **Week 12 n=147** | Placebo | 4 | |
| | | Placebo/Adalimumab 40mg eow | - | |
| | | Adalimumab 40mg eow | 53* | |
| | | Adalimumab 40 mg weekly | 80* | |
| | **Week 24 n=142** | Placebo | - | |
| | | Placebo/Adalimumab 40mg eow | 55 | |
| | | Adalimumab 40mg eow | 64 | |
| | | Adalimumab 40 mg weekly | 72 | |
| | **Week 36 n=142** | Placebo | - | |
| | | Placebo/Adalimumab 40mg eow | 62 | |
| | | Adalimumab 40mg eow | 64 | |
| | | Adalimumab 40 mg weekly | 68 | |
| | **Week 60 n=142** | Placebo | - | |
| | | Placebo/Adalimumab 40mg eow | 45 | |
| | | Adalimumab 40mg eow | 58 | |
| | | Adalimumab 40 mg weekly | 64 | |
| | **At Week 12, placebo patients received 80-mg loading dose, then 40 mg eow.** | | | |
| | Modified ITT, NRI. | | | |
| | *p<0.001 vs. placebo/adalimumab 40 eow group. | | | |
| | Patients with <PASI 50 response on or after Week 24 were eligible to receive OL weekly adalimumab rescue therapy. | | | |
| | Patients receiving rescue therapy were considered non-responders in this analysis. | | | |

As can be seen in Table 6, a substantial portion of all patients achieved and sustained a PASI 75 response up to Week 60 of adalimumab treatment. Also seen in Table 6, placebo patients who started adalimumab at Week 12 achieved rapid improvements in their PASI scores, as indicated by PASI 75 response rates at Week 24.

**Table 7. Overall DLQI Scores Up To Week 60**

| | | | | |
|---|---|---|---|---|
| | | | | |

| | | **Treatment** | **% of Patients** | |
|---|---|---|---|---|
| | **Week 12 n=140** | Placebo | 11 | |
| | | Placebo/Adalimumab 40mg eow | - | |
| | | Adalimumab 40mg eow | 4 | |
| | | Adalimumab 40 mg weekly | 3 | |
| | **Week 24 n=131** | Placebo | | |
| | | Placebo/Adalimumab 40mg eow | 3 | |
| | | Adalimumab 40mg eow | 5 | |
| | | Adalimumab 40 mg weekly | 3 | |
| | **Week 36 n=118** | Placebo | - | |
| | | Placebo/Adalimumab 40mg eow | 2 | |
| | | Adalimumab 40mg eow | 3 | |
| | | Adalimumab 40 mg weekly | 2 | |
| | **Week 60 n=106** | Placebo | - | |
| | | Placebo/Adalimumab 40mg eow | 2 | |
| | | Adalimumab 40mg eow | 4 | |
| | | Adalimumab 40 mg weekly | 1 | |
| | **At Week 12, placebo patients received 80-mg loading dose, then 40 mg eow.** | | | |
| | Modified ITT, observed. | | | |
| | Patients with <PASI 50 response on or after Week 24 were eligible to receive OL weekly adalimumab rescue therapy. | | | |
| | Patients receiving rescue therapy were considered non-responders in this analysis. | | | |

Table 7 shows the overall DLQI scores up to Week 60 for patients included in the subanalysis study. As can be seen in this figure, all patients demonstrated a significant improvement in DLQI scores up to Week 60 of adalimumab treatment. Also, placebo patients who started adalimumab at Week 12 achieved rapid improvements in DLQI scores.

Overall, substantial percentages of patients who met the selected BAD criteria achieved PASI 75 responses, comparable to PASI 75 response rates in the overall patient population (see Table 8). PASI improvements were largely sustained out to Week 60 (Table 8).

**Table 8. PASI 75 Response Rates Up To Week 60 in Patients With Baseline PASI ≥10 and DLOI > 10**

| | | | | | |
|---|---|---|---|---|---|
| | | | | | |

| | | **n** | **Treatment** | **% of Patients** | |
|---|---|---|---|---|---|
| | **Week 12** | 18 | Placebo | 0 | |
| | | 26 | Placebo/Adalimumab 40mg eow | - | |
| | | 23 | Adalimumab 40mg eow | 69 | |
| | | - | Adalimumab 40 mg weekly | 74 | |
| | **Week 24** | - | Placebo | - | |
| | | 15 | Placebo/Adalimumab 40mg eow | 50 | |
| | | 26 | Adalimumab 40mg eow | 77 | |
| | | 23 | Adalimumab 40 mg weekly | 78 | |
| | **Week 36** | - | Placebo | - | |
| | | 15 | Placebo/Adalimumab 40mg eow | 50 | |
| | | 26 | Adalimumab 40mg eow | 73 | |
| | | 23 | Adalimumab 40 mg weekly | 74 | |
| | **Week 60** | - | Placebo | - | |
| | | 15 | Placebo/Adalimumab 40mg eow | 44 | |
| | | 26 | Adalimumab 40mg eow | 65 | |
| | | 23 | Adalimumab 40 mg weekly | 65 | |
| | **At Week 12, placebo patients received 80-mg loading dose, then 40 mg eow.** | | | | |
| | Modified ITT, NRI. | | | | |
| | Patients with <PASI 50 response on or after Week 24 were eligible to receive OL weekly adalimumab rescue therapy. | | | | |
| | Patients receiving rescue therapy were considered non-responders in this analysis. | | | | |

Also, in the subset of patients who met the selected BAD criteria for biologic treatment, patients achieved and largely sustained clinical improvements as demonstrated by PASI 90 responses to Week 60 (Table 9). As can be seen in Table 10, there were significant percentages of patients who achieved a PGA score of "Clear" or "Almost Clear" and sustained these responses until Week 60.

**Table 9. PASI 90 Response Rates Up To Week 60 in Patients With Baseline PASI ≥10 and DLQI >10**

| | | | | | |
|---|---|---|---|---|---|
| | | | | | |

| | | **n** | **Treatment** | **% of Patients** | |
|---|---|---|---|---|---|
| | **Week 12** | 18 | Placebo | 0 | |
| | | 26 | Placebo/Adalimumab 40mg eow | - | |
| | | 23 | Adalimumab 40mg eow | 39 | |
| | | - | Adalimumab 40 mg weekly | 61 | |
| | **Week 24** | - | Placebo | - | |
| | | 15 | Placebo/Adalimumab 40mg eow | 28 | |
| | | 26 | Adalimumab 40mg eow | 54 | |
| | | 23 | Adalimumab 40 mg weekly | 70 | |
| | **Week 36** | - | Placebo | - | |
| | | 15 | Placebo/Adalimumab 40mg eow | 50 | |
| | | 26 | Adalimumab 40mg eow | 58 | |
| | | 23 | Adalimumab 40 mg weekly | 65 | |
| | **Week 60** | - | Placebo | - | |
| | | 15 | Placebo/Adalimumab 40mg eow | 39 | |
| | | 26 | Adalimumab 40mg eow | 42 | |
| | | 23 | Adalimumab 40 mg weekly | 48 | |
| | **At Week 12, placebo patients received 80-mg loading dose, then 40 mg eow.** | | | | |
| | Modified ITT, NRI. | | | | |
| | Patients with <PASI 50 response on or after Week 24 were eligible to receive OL weekly adalimumab rescue therapy. | | | | |
| | Patients receiving rescue therapy were considered non-responders in this analysis. | | | | |

**Table 10. PGA "Clear" or "Almost Clear" Up To Week 60 in Patients With Baseline PASI ≥10 and DLQI >10**

| | | | | | |
|---|---|---|---|---|---|
| | | | | | |

| | | **n** | **Treatment** | **% of Patients** | |
|---|---|---|---|---|---|
| | **Week 12** | 18 | Placebo | 0 | |
| | | 26 | Placebo/Adalimumab 40mg eow | - | |
| | | 23 | Adalimumab 40mg eow | 62 | |
| | | - | Adalimumab 40 mg weekly | 70 | |
| | **Week 24** | - | Placebo | - | |
| | | 15 | Placebo/Adalimumab 40mg eow | 33 | |
| | | 26 | Adalimumab 40mg eow | 69 | |
| | | 23 | Adalimumab 40 mg weekly | 74 | |
| | **Week 36** | - | Placebo | - | |
| | | 15 | Placebo/Adalimumab 40mg eow | 44 | |
| | | 26 | Adalimumab 40mg eow | 65 | |
| | | 23 | Adalimumab 40 mg weekly | 65 | |
| | **Week 60** | - | Placebo | - | |
| | | 15 | Placebo/Adalimumab 40mg eow | 39 | |
| | | 26 | Adalimumab 40mg eow | 54 | |
| | | 23 | Adalimumab 40 mg weekly | 52 | |
| | **At Week 12, placebo patients received 80-mg loading dose, then 40 mg eow.** | | | | |
| | Modified ITT, NRI. | | | | |
| | Patients with <PASI 50 response on or after Week 24 were eligible to receive OL weekly adalimumab rescue therapy. | | | | |
| | Patients receiving rescue therapy were considered non-responders in this analysis.. | | | | |

Adalimumab-treated patients showed rapid and sustained improvement in their DLQI scores (Table 11), with mean scores of 3.0 and 0.7 at Week 60 in the adalimumab every other week and adalimumab weekly groups respectively.

**Table 11. Change in DLQI Scores Up To Week 60 in Patients With Baseline PASI ≥10 and DLQI >10**

| | | | | | |
|---|---|---|---|---|---|
| | | | | | |

| | | **n** | **Treatment** | **Mean change from baseline** | |
|---|---|---|---|---|---|
| | **Week 12** | 15 | Placebo | -2 | |
| | | 26 | Placebo/Adalimumab 40mg eow | - | |
| | | 22 | Adalimumab 40mg eow | -15 | |
| | | - | Adalimumab 40 mg weekly | -16 | |
| | **Week 24** | - | Placebo | - | |
| | | 15 | Placebo/Adalimumab 40mg eow | -12 | |
| | | 25 | Adalimumab 40mg eow | -14 | |
| | | 20 | Adalimumab 40 mg weekly | -17 | |
| | **Week 36** | - | Placebo | - | |
| | | 13 | Placebo/Adalimumab 40mg eow | -14 | |
| | | 22 | Adalimumab 40mg eow | -15 | |
| | | 18 | Adalimumab 40 mg weekly | -18 | |
| | **Week 60** | | Placebo | - | |
| | | 12 | Placebo/Adalimumab 40mg eow | -12 | |
| | | 22 | Adalimumab 40mg eow | -15 | |
| | | 15 | Adalimumab 40 mg weekly | -17 | |
| | **At Week 12, placebo patients received 80-mg loading dose, then 40 mg eow.** | | | | |
| | Modified ITT, observed. | | | | |
| | Patients with <PASI 50 response on or after Week 24 were eligible to receive OL weekly adalimumab rescue therapy. | | | | |
| | Patients receiving rescue therapy were considered non-responders in this analysis. | | | | |

As can be seen in Table 12, withdrawal rates due to adverse events in this post-hoc analysis were comparable for patients treated with adalimumab every other week and adalimumab weekly.

**Table 12**

| | **Weeks 0-12** | | | **Weeks 12-60** | |
|---|---|---|---|---|---|
| **Event, n (%)** | **Placebo (N=18)** | **Adalimumab 40 mg eow (N=26)** | **Adalimumab 40 mg weekly (N=23)** | **Placebo/ Adalimumab 40 mg eow + Adalimumab 40 mg eow (N=41)** | **Adalimumab 40 mg weekly (N=23)** |
| **Any AE** | 13 (72) | 16 (62) | 19 (83) | 23 (56) | 11 (48) |
| **Serious AEs** | 0 (0) | 0 (0) | 2 (9) | 1 (2) | 0 (0) |
| **Any Infectious Serious AEs** | 0 (0) | 0 (0) | 0 (0) | 0 (0) | 0 (0) |
| **AEs leading to withdrawal** | 1 (6) | 0 (0) | 1 (4) | 2 (5) | 0 (0) |
| | | | | | |

Overall, patients with moderate to sever plaque psoriasis achieved sustained efficacy up to Week 60 of treatment with adalimumab. Adalimumab treatment significantly reduced the signs and symptoms and improved the quality of life in patients with psoriasis who meet some of the necessary criteria of the BAD guidelines for biologic treatment. The adverse events observed in this study were similar to those previously reported in adalimumab rheumatoid arthritis and psoriatic arthritis trials.

### Example 3: Recent History of Systemic or Biologic Therapy Does Not Adversely Affect Adalimumab Efficacy and Safety in Patients with Moderate to Severe Chronic Plaque Psoriasis

Psoriasis is a chronic, inflammatory proliferative disease of the skin that affects 1-3% of the general population (Greaves and Weinstein (1995) *N Engl J Med* 332: 581). Treatment of moderate to severe psoriasis with systemic therapy such as methotrexate or cyclosporine or biologic therapy such as efalizumab can be limited by lack of efficacy or precluded by side effects. Ultraviolet light therapy is often inconvenient.

Previous studies demonstrated adalimumab is effective in treating moderate to severe plaque psoriasis (Ps), with an acceptable safety profile. Some study patients had a recent (within past 12 months) history of exposure systemic or biologic (except TNF antagonists) therapy, but could enroll if systemic therapy stopped at least 4 wks prior to study (at least 12 wks for biologics). The impact of prior treatment with these agents on the safety and efficacy of patients receiving adalimumab is a practical concern to physicians and patients and was investigated in this subanalysis. Thus, the following study describes an analysis of the efficacy and safety of adalimumab treatment in psoriasis patients with or without recent exposure to systemic or biologic agents.

The objective of the study was to investigate the impact of prior treatment with systemic non-biologic or biologic therapies on the safety and efficacy of adalimumab in patients with psoriasis

The efficacy and safety of adalimumab was evaluated in a 12-week, double-blind, placebo-controlled trial, followed by a 48-week extension, conducted at 18 sites two different countries. Inclusion criteria for the study included the following parameters: ≥18 years of age; moderate to severe chronic plaque psoriasis ≥ 1 year; and affected BSA ≥5%. Exclusion criteria for the study included prior TNF-antagonist therapy and discontinuation of other systemic psoriasis therapies. Impact of prior biologics, other than TNF antagonists, on the safety and efficacy of adalimumab for psoriasis was measured in this subanalysisn study. Efficacy outcome measures included PASI and PGA. Analytical methods included the following:
◆ Modified intent-to-treat analyses were performed on all randomized patients receiving at least one dose of adalimumab
◆ Missing data were imputed using non-responder imputation
◆ Subanalyses were conducted on the outcomes of patients who were and were not exposed to systemic therapy within 12 months of study entry A diagram of the study design is shown in Figure 1.

Out of 148 patients enrolled in a double-blind, placebo (pbo)-controlled study, 147 patients received at least one dose of study medication in 1 of 3 randomized treatment arms: 1) pbo (n=52); 2) adalimumab 80 mg subcutaneous (sc) at Wk 0, then 40 mg sc every other wk (eow) starting at Wk 1 (n=45); and 3) adalimumab 80 mg sc at Wks 0 and 1, then 40 mg sc wkly (qw) starting at Wk 2 (n=50). Placebo patients were eligible at Week 12 to receive adalimumab 40 mg eow (placebo/adalimumab eow).

Baseline data were similar among randomization groups. Baseline demographics, disease severity characteristics, and recent history of systemic therapies were similar across the treatment groups. Table 13 shows the baseline demographics and clinical characteristics.

**Table 13: Baseline Demographics and Clinical Characteristics***

| | Placebo (N=52) | Adalimumab 40 mg eow (N=45) | Adalimumab 40 mg weekly (N=50) |
|---|---|---|---|
| Age, years (range) | 43 (20-70) | 46 (20-71) | 44 (24-86) |
| Duration of Ps, years (range) | 19.1 (1.0-39.8) | 20.5 (1.3-57.9) | 18.4 (1.7-47.7) |
| % Male | 65 | 71 | 66 |
| % Caucasian | 92 | 89 | 90 |
| Body Weight, kg (range) | 94 (50-147) | 93 (63-159) | 99 (42-149) |
| % BSA (range) | 28 (7-75) | 29 (6-58) | 25 (5-83) |
| PASI Score (range) | 16.0 (5.5-40.4) | 16.7 (5.4-39.0) | 14.5 (2.3-42.4) |
| % with PsA | 31 | 33 | 24 |

| | | | |
|---|---|---|---|
| * Mean values except percentages. Langley R, et al. *J Am Acad Dermatol* 2005;52(3 Suppl ):2. | | | |

Systemic non-biologic therapies taken by more than 2% of patients included methotrexate (16%), oral tazarotene (11%), cyclosporine (9%), and acitretin (4%). The only systemic biologic therapy taken by more than 2% of patients was efalizumab (15%) (prior use of TNF-antagonist therapy was an exclusion criterion for study entry). Table 14 shows clinical characteristics of the patients involved in the study, including recent past history of systemic therapy.

**Table 14: Clinical Characteristics: Recent Past History of Systemic Therapy***

| | Placebo (N=52) | Adalimumab 40 mg eow (N=45) | Adalimumab 40 mg weekly (N=50) |
|---|---|---|---|
| No Recent History of Systemic Therapy, n (%) | 28 (54) | 23 (51) | 25 (50) |
| Recent History of Systemic Non-biologic Therapy, n (%) | 19 (37) | 19 (42) | 22 (44) |
| Recent History of Systemic Biologic Therapy, n (%) | 8 (15) | 8 (18) | 9 (18) |

| | | | |
|---|---|---|---|
| *Within 12 months of study entry. | | | |

Percentages in each column do not sum to 100 because some patients had recent history of systemic non-biologic and biologic therapy. The numbers of patients exposed to multiple types of systemic therapy were too small to permit meaningful analysis of their outcomes.

The results show that substantial percentages of all patients achieved and sustained PASI 75 responses up to Week 60 of adalimumab treatment. In addition, placebo patients who started adalimumab at Week 12 attained clinically significant improvement in their PASI 75 response rates by Week 24. These results are described in Table 6.

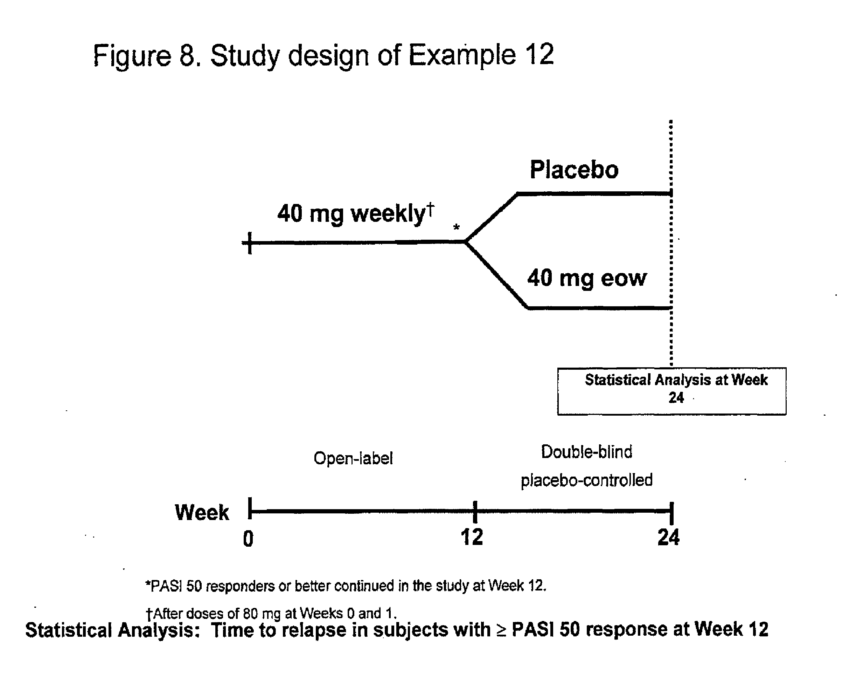

Patients with and without a recent history of systemic non-biologic or biologic therapy had similar percentages of PASI 75 responses at Week 24, which were largely sustained out to Week 60. Figures 8a and 8b shows PASI 75 response rates in patients stratified by recent history of systemic therapy at week 24 (Table 15) and week 60 (Table 16).

**Table 15. PASI 75 Response Rates at Week 24 in Patients Stratified by Recent History of Systemic Therapy**

| | | | | | |
|---|---|---|---|---|---|
| | | | | | |

| | | **n** | **Treatment** | **% Patients** | |
|---|---|---|---|---|---|
| | **Prior Systemic Use** | 17 | Placebo/Adalimumab 40mg eow | 47 | |
| | | 19 | Adalimumab 40mg eow | 68 | |
| | | 22 | Adalimumab 40 mg weekly | 73 | |
| | **Prior Biological Use** | 8 | Placebo/Adalimumab 40mg eow | 63 | |
| | | 8 | Adalimumab 40mg eow | 50 | |
| | | 9 | Adalimumab 40 mg weekly | 67 | |
| | **No Prior Use** | 25 | Placebo/Adalimumab 40mg eow | 56 | |
| | | 23 | Adalimumab 40mg eow | 65 | |
| | | 25 | Adalimumab 40 mg weekly | 76 | |
| **Modified ITT, NRI.** | | | | | |

**Table 16. PASI 75 Response Rates at Week 60 in Patients Stratified by Recent History of Systemic Therapy**

| | | | | | |
|---|---|---|---|---|---|
| | | | | | |

| | | **n** | **Treatment** | **% Patients** | |
|---|---|---|---|---|---|
| | **Prior Systemic Use** | 17 | Placebo/Adalimumab 40mg eow | 41 | |
| | | 19 | Adalimumab 40mg eow | 68 | |
| | | 22 | Adalimumab 40 mgweekly | 64 | |
| | **Prior Biological Use** | 8 | Placebo/Adalimumab 40mg eow | 63 | |
| | | 8 | Adalimumab 40mg eow | 63 | |
| | | 9 | Adalimumab 40 mg weekly | 78 | |
| | **No Prior Use** | 25 | Placebo/Adalimumab 40mg eow | 40 | |
| | | 23 | Adalimumab 40mg eow | 48 | |
| | | 25 | Adalimumab 40 mg weekly | 64 | |
| **Modified ITT, NRI.** | | | | | |

Patients with and without a recent history of systemic non-biologic or biologic therapy who received adalimumab 40 mg eow dosing achieved and largely sustained clinical improvement to Week 60, as measured by a variety of secondary efficacy variables. Table 17 shows secondary efficacy outcomes after 60 weeks of adalimumab 40 mg eow.

**Table 17**

| | PASI 50 (%) | PASI 90 (%) | PASI 100 (%) | % PGA "Clear"/ "Almost Clear" |
|---|---|---|---|---|
| No Prior Use | 65 | 30 | 17 | 44 |
| Prior Systemic Use | 68 | 37 | 11 | 47 |
| Prior Biologic Use | 63 | 38 | 13 | 38 |

Table 18 show PASI responses up to week 60. Overall, percent of PASI 75 responders in the (placebo/adalimumab eow)/adalimumab eow/adalimumab weekly arms were 55/64/72 at Wk 24 and 45/58/64 at Wk 60, respectively. For patients with prior biologic therapy (n=25), the rates were 63/50/67 at Week 24 and 63/63/78 at Week 60. For patients in neither of these groups (n=73), the rates were 56/65/76 at Week 24 and 40/48/64 at Week 60.

**Table 18: Percentages of PASI 75 responders for each subgroup at Wks 24 and 60**

| | Wk 24 | Wk 60 |
|---|---|---|
| | Pbo/ Adalimumab eow/ Adalimumab qw | Adalimumab eow (formerly Pbo)/ Adalimumab eow/ Adalimumab qw |
| Systemic, n=58 (%) | 47/68/73 | 41/68/64 |
| Biologic, n=25 (%) | 63/50/67 | 63/63/78 |
| Other, n=73 (%) | 56/65/76 | 40/48/64 |

Withdrawal rates due to adverse events were similarly low for patients treated with adalimumab eow and adalimumab weekly. All patients treated with adalimumab in the double-blind portion of the trial continued into the open-label extension. The percentage of patients who withdrew due to adverse events between Weeks 12 and 60 ranged from 3.3% to 10.0%

Serious adverse events (SAEs) were detected in 7 prior-systemic patients, 4 prior-biologic patients, and 5 patients without recent prior systemic or biologic therapy during the 60-week trial. Most SAEs did not appear to be related to adalimumab use. Table 19 shows adverse events by treatment period, while Tables 20 and 21 shows adverse events by subgroup (Table 20: weeks 0-12; Table 21: Weeks 12-60).

**Table 19: Adverse Events by Treatment Period**

| | **Weeks 0-12** | | | **Weeks 12-60** | |
|---|---|---|---|---|---|
| **Event, n (%)** | **Placebo (N=52)** | **Adalimumab 40 mg eow (N=46)** | **Adalimumab 40 mg weekly (N=50)** | **Placebo/ Adalimumab 40 mg eow + Adalimumab 40 mg eow (N=92)** | **Adalimumab 40 mg weekly** |
| **Any AE** | 35 (67) | 28 (62) | 39 (78) | 72 (78) | 39 (78) |
| **Serious AEs** | 0 (0) | 1 (2) | 4 (8) | 2 (2) | 7 (14) |
| **AEs leading to withdrawal** | 1 (1) | 2 (4) | 3 (6) | 3 (3) | 5 (10) |

**Table 20: Adverse Events by Subgroup: Weeks 0-12 - Results for Patients Stratified by Recent Past History**

| | Placebo | | | Adalimumab 40 mg eow | | | Adalimumab 40 mg weekly | | |
|---|---|---|---|---|---|---|---|---|---|
| | No Prior Use (n=28) | Prior Systemic Use (n=19) | Prior Biologic Use (n=8) | No Prior Use (n=23) | Prior Systemic Use (n=19) | Prior Biologic Use (n=8) | No Prior Use (n=25) | Prior Systemic Use (n=22) | Prior Biologic Use (n=9) |
| Any AE, n (%) | 18 (64) | 15 (79) | 5 (63) | 16 (70) | 11 (58) | 2 (25) | 20 (80) | 17 (77) | 6 (67) |
| Serious AE, n (%) | 0 (0) | 0 (0) | 0 (0) | 0 (0) | 1 (5) | 0 (0) | 1 (4) | 2 (9) | 2 (22) |
| AE's→ Withdrawal, n (%) | 0 (0) | 1 (5) | 0 (0) | 1 (4) | 1 (5) | 0 (0) | 1 (4) | 1 (5) | 2 (22) |

**Table 21: Adverse Events by Subgroup: Weeks 12-60 - Results for Patients Stratified by Recent Past History**

| | Placebo/Adalimumab 40 mg eow + Adalimumab 40 mg eow | | | Adalimumab 40 mg weekly | | |
|---|---|---|---|---|---|---|
| | No Prior Use (n=47) | Prior Systemic Use (n=35) | Prior Biologic Use (n=16) | No Prior Use (n=24) | Prior Systemic Use (n=21) | Prior Biologic Use (n=7) |
| Any AE (%) | 37 (79) | 28 (80) | 12 (75) | 19 (79) | 17 (81) | 6 (86) |
| Serious AE (%) | 0 (0) | 2 (6) | 2 (13) | 4 (17) | 2 (10) | 0 (0) |
| AE's→ Withdrawal (%) | 1 (2) | 2 (6) | 1 (6) | 2 (8) | 3 (14) | 0 (0) |

The most frequent adverse events, between weeks 12 and 60 are described in Table 22.

In conclusion, patients with moderate to severe psoriasis achieved sustained efficacy up to week 60 of treatment with adalimumab. Prior biologic or non-biologic therapy did not appear to adversely affect adalimumab efficacy or safety in patients with moderate to severe psoriasis. Finally, the types and rates of adverse events in this study were similar to those previously reported in adalimumab RA and PsA trials. In sum, this post-hoc analysis suggests that recent treatment with systemic or biologic therapy does not adversely affect adalimumab efficacy or safety in moderate to severe psoriasis patients.

**Table 22. Most frequent adverse events, between weeks 12 and 60**

| | | | | |
|---|---|---|---|---|
| | | | | |

| | **Event** | **Placebo/Adalimumab 40 mg eow + Adalimumab 40 mg eow (N=92)** | **Adalimumab 40 mg weekly (N=50)** | |
|---|---|---|---|---|
| | **Nasopharyngitis** | 13 (14.1) | 6 (12.0) | |
| | **URI, NOS** | 9 (9.8) | 7 (14.0) | |
| | **URI, viral NOS** | 4 (4.3) | 3 (6.0) | |
| | **Muscle strain** | 1 (1.1) | 4 (8.0) | |
| | **Blood CPK increased** | 5 (5.4) | 2 (4.0) | |
| | **Blood TG increased** | 7 (7.6) | 2 (4.0) | |
| | **Back pain** | 4 (4.3) | 4 (8.0) | |
| | **Skin papilloma** | 3 (3.3) | 5 (10.0) | |
| | **Headache** | 3 (3.3) | 6 (12.0) | |
| | **Urticari** | 0 (0.0) | 3 (6.0) | |
| | **URI=upper respiratory infection; NOS=not otherwise specified;** | | | |
| | **CPK=Creatine phosphokinase; TG=Triglycerides.** | | | |

### Example 4: Minimum Clinically Important Difference (MCID) in Dermatology Life Quality Index (DLQI) in Moderate to Severe Plaque Psoriasis Patients Treated with Adalimumab

Because psoriasis exerts substantial deleterious effects on physical function and quality of life (HRQOL) (see de *Arruda et al.* (2001) *Br J Dermatol* 144 (Suppl 58):33; Finlay (1998) *Seminars Cutan Med Surgery* 17:291; Rapp *et al.* (2001) *Br JDermatol* 145:610; and Wahl *et al.* (2000) *JAm Acad Dermatol* 43:803), patient-reported outcomes (PROs) help evaluate the beneficial effects of treatment, in addition to clinical endpoints.

The minimum clinically important difference (MCID) is defined as "the smallest difference in score that patients perceive as beneficial" (see Juniper *et al.* (1994) *J Clin Epidemiol* 47:81). While clinical trials of biologics in the treatment of psoriasis have included PROs - particularly the Dermatology Life Quality Index (DLQI) (see Feldman *et al.* (2004)) *Br J Dermatol* 150:317; Finlay *et al.* (2003) *Dermatology* 206:307; Gordon *et al.* (2003) *JAMA* 290:3073; Gottlieb *et al.* (2003) *Arch Dermatol* 139:1627*;* Leonardi *et al.* (2003) *NEngl JMed* 349:2014; and Menter *et al.* (2004) *J Drugs Dermatol* 3:27) - the clinical relevance and MCID for this instrument have not been established in moderate to severe plaque psoriasis.

Tumor necrosis factor (TNF) is a cytokine involved in inflammatory response and scientific evidence suggests it plays a fundamental role in the pathogenesis of psoriasis (Kreuger et al. (2004) Arch Dermatol 140:218; Kupper (2003) N Engl J Med 349:1987). Adalimumab is a monoclonal IgG antibody that contains only human peptide sequences. It binds with high specificity and affinity to soluble and membrane-bound TNF, thereby neutralizing the biological activities of TNF. The following describes a study which examined the DLQI as a secondary efficacy endpoint in a 12-week study (placebo-controlled trial) of two different dose regimens of adalimumab in moderate to severe plaque psoriasis.

The objective of the study was to estimate the sensitivity of DLQI to clinical changes associated with moderate to severe plaque psoriasis

Patients were randomized into one of the following treatment groups: 80 mg adalimumab at baseline (week 0) and 40 mg at week 1 followed by 40 mg every other week (eow) starting at week 3 (referred to as ada 40 mg eow); 80 mg adalimumab at baseline (week 0) and 80 mg at week 1 followed by 40 mg weekly starting at week 2 (referred to as ada 40 mg weekly); or placebo administered weekly beginning at baseline (referred to as placebo). Inclusion criteria included a diagnosis of moderate to severe chronic plaque psoriasis ≥ 1 year prior to entry, a psoriasis-affected body surface area (BSA) >5%, and no previous TNF-antagonist therapy.

PROs were assessed using the DLQI. The DLQI is a validated instrument used to assess dermatologic-related functional limitations. Characteristics of the DLQI include: ten items on an overall scoring range of 0-30; higher scores represent greater quality of life impairment and lower scores represent lower quality of life impairment; well-established properties of reliability and validity for the DLQI total score in a dermatology setting (see Badia et al. (1999) Br J Dermatol 141:698; Finlay et al. (1994) Clin Exp Dermatol 19:210; and Shikier et al. (2003) Health and Quality of Life Outcomes 1:53); and six subcategories: symptoms and feelings; daily activities; leisure; work/school; personal relationships; and treatment.

The Physician's Global Assessment (PGA), which was used as a clinical endpoint in the trial, was used to determine the MCID for DLQI. The PGA is a seven point scale used to measure disease severity from a physician's evaluation. Scoring under the PGA ranges from 1 (Clear) to 7 (Severe). Categories for the 7-point scale include the following:
Severe: very marked plaque elevation, scaling and/or erythema;
Moderate to severe: marked plaque elevation, scaling and/or erythema;
Moderate: moderate plaque elevation, scaling and/or erythema;
Mild to moderate: intermediate between moderate and mild;
Mild: slight plaque elevation, scaling and/or erythema;
Almost clear: intermediate between mild and clear; and
Clear: no signs of psoriasis (post-inflammatory hypopigmentation or hyperpigmentation could be present).

In this study, PGA was assessed at screening; baseline; week 1; week 2; week 4; week 8; and week 12/early termination; and follow up. The same investigator performed the assessment for each patient throughout the study. Statistical methods were performed according to the following summary. Analyses were performed on blinded data, combining results from all three groups. Changes in the PGA from baseline to week 12 were correlated with changes in DLQI total score. Mean changes in DLQI total score were calculated and compared for two sets of patients: those whose PGA scores improved by 1 or 2 points ("minimal responders") and those whose PGA scores stayed the same of declined or declines by 1 point "non-responders"). It should be noted that there is no universally accepted method to calculate MCID. One method to estimate the MCID is to calculate the difference between the mean DLQI changes for patients classified as "minimal responders" and the changes for patients classified as "non-responders." Other methods to calculate MCID are based on mean DLQI change corresponding to PASI improvement 25%-49%. MCID for DLQI was also calculated based on mean DLQI change corresponding to PASI improvement 50%-74% and based on three distributional methods: standard error of the mean (SEM) change in DLQI; upper limit of the 95% confidence interval of the SEM; and half of the standard deviation (SD) of the DLQI mean change. MCID was applied to demonstrated changes in DLQI total score from baseline to week 12 in the three randomized groups of this trial. All data were observed values, and patients who discontinued before the time point were not included in the analysis.

147 patients from 18 different sites enrolled in the study and received at least one dose of study medication. Data were available for 140 of the 147 patients at the end of the trial. Mean values for DLQI and PGA at baseline and week 12 are shown below in Table 23:

**Table 23: Mean (SD) of DLQI and PGA at Baseline and Week 12**

| | Baseline (N=147) | Week 12 (N=140) | Change² (N=140) |
|---|---|---|---|
| DLQI Total Score | 12.7 (7.2) | 5.3 (6.5) | -7.5 (7.8) |
| Physician's Global Assessment' (PGA) | 5.5 (0.8) | 3.4 (1.7) | -2.1 (1.9) |

| | | | |
|---|---|---|---|
| ¹Scored such that 1="Clear" to 7="Severe" ²Calculated only for patients with both baseline and week-12 scores (Numbers in parentheses are standard deviations (SD)). | | | |

Mean improvements in DLQI between "minimal responders" and "non-responders" based on PGA was significantly different (p<0.0001), as shown in Table 24.

**Table 24. Mean (SD) DLQI Change Score by PGA Response**

| | PGA Minimal Responder | PGA Non-Responder | Difference in Mean DLQI Total Score* |
|---|---|---|---|
| DLQI Total Score | -5.7 (5.7) | 0.0 (4.7) | 5.7 |

| | | | |
|---|---|---|---|
| ^{*} p < 0.0001 Note: Negative numbers indicate improvement, positive numbers indicate worsening in dermatologic-related functional limitations as measured by DLQI. | | | |

Based on other MCID calculations with PASI (2.3-4) (data not shown) and these MCID calculations with PGA (5.7) in the same study population, the MCID for DLQI was estimated to range between 2.3 and 5.7, with the most conservative estimate of MCID derived from analysis of the mean change in DLQI for PGA "minimal responders." Table 25 shows DLQI improvement at 12 weeks in each of the three arms of the study.

**Table 25. DLQI improvement at 12 weeks in each of the three arms of the study**

| | | | |
|---|---|---|---|
| | | | |

| | **Treatment** | **Mean Change** | |
|---|---|---|---|
| | Placebo | -1.3 | |
| | Adalimuma b 40mg eow | -10.8 | |
| | Adalimuma b 40mg weekly | -11.5* | |
| ***p<0.001 vs. Placebo** | | | |

In conclusion, through a variety of methods, the MCID for the DLQI was estimated to range between 2.3 and 5.7. Given that adalimumab-treated patients demonstrated mean change in DLQI of ≥ 10 points, which is well above the most conservative estimate of MCID for DLQI, treatment with adalimumab is associated with clinically important improvements in dermatologic-related functional limitations.

### Example 5: Enhanced Adalimumab Efficacy Following Dosage Escalation In Psoriasis Patients With Subtherapeutic Response To Every-Other-Week Adalimumab

Adalimumab is a fully human, monoclonal IgG₁ antibody against TNF. A 60-week, Phase II study demonstrated that adalimumab is effective in moderate to severe plaque psoriasis patients, with an acceptable safety profile (see Figure 1). Patients with a subtherapeutic (<PASI 50) response to adalimumab at Week 24 could increase their dosages from every other week (eow) to every week (qw). The objective of this study was to assess efficacy and safety of adalimumab weekly dosing in those psoriasis patients who had a subtherapeutic response to every-other-week adalimumab dosage

Thus, the present subanalysis evaluated adalimumab efficacy and safety in those patients who increased their dosages.

The efficacy and safety of adalimumab was evaluated in a 48-week extension trial conducted at 18 sites in the US and Canada. Prior to this open label study, patients were enrolled in a 12-week, double-blind, placebo-controlled trial. Inclusion criteria included patients being ≥18 years of age; having moderate to severe chronic plaque psoriasis ≥1 year; and affected BSA ≥5%. Exclusion criteria included prior TNF-antagonist therapy and discontinuation of other systemic psoriasis therapies. Efficacy outcome measures which were used included PASI, PGA, DLQI. The study design is shown in Figure 1. Baseline demographics and clinical characteristics are described in Table 4 above. In addition, the percentage of patients with PsA was 31 % placebo, 33% adalimumab 40 mg eow, and 24% adalimumab 40 mg weekly.

Randomized treatment arms included: 1) placebo (n=52); 2) adalimumab 80 mg subcutaneously (sc) at Week 0, then 40 mg sc eow starting at Week 1 (n=45); and 3) adalimumab 80 mg sc at Weeks 0 and 1, then 40 mg sc qw starting at Week 2 (n=50). At Week 12, placebo patients were switched to adalimumab 40 mg eow (placebo/eow). At Week 24 and thereafter, adalimumab eow patients with <PASI 50 improvement from baseline started adalimumab 40 mg qw. Skin and safety outcomes were assessed up to Week 60.

Of 148 patients enrolled in the double-blind, placebo-controlled study, 147 received at least one dose of study medication. At Week 24, PASI 50 responses were 77/73/80 and PASI 75 responses were 55/64/72 for the (placebo/eow)/eow/weekly arms, respectively.

On or after Week 24, 18 of the 47 patients in the placebo/adalimumab eow group and 12 of the 45 patients in the adalimumab eow group achieved a PASI <50 response, which qualified them for dosage escalation to adalimumab weekly (Table 26)

**Table 26. Number of Patients Who Qualified for Dosage Escalation at Each Timepoint**

| | Placebo/ Adalimumab 40 mg eow (n=18) | Adalimumab 40 mg eow (n=12) |
|---|---|---|
| Week 24 | 10 | 9 |
| Week 28 | 2 | 1 |
| Week 32 | 2 | 0 |
| Week 44 | 1 | 1 |
| Week 52 | 3 | 0 |
| Week 60 | 0 | 1 |

Patients whose dosages were increased to weekly dosing had baseline demographics and clinical characteristics similar to the overall patient population (Table 27).

**Table 27. Baseline Demographic and Clinical Characteristics for the Dosage Escalation Group***

| Characteristics | Dosage Escalation Group (n=30) |
|---|---|
| Age, years | 46.5 ±12.0 |
| Duration of Ps, years | 19.2 ± 12.8 |
| % Male | 63 |
| % Caucasian | 97 |
| Body Weight, kg | 97.4 ± 24.2 |
| % BSA ± SD | 23 ± 13.8 |
| PASI Score | 14.1 ± 6.1 |
| % with PsA | 37 |

| | |
|---|---|
| * Mean values ± SD except percentages. | |

After qualifying for dosage escalation, nearly 20% of patients achieved a PASI 75 response in their psoriasis at Week 60 (Table 28)

**Table 28. Efficacy Results at Week 60 for Dosage Escalation Group**

| | Dosage Escalation Group (n=30) |
|---|---|
| PASI 50 Response Rate (%) | 40 |
| PASI 75 Response Rate (%) | 17 |
| PASI 90 Response Rate (%) | 0 |
| PGA ("Clear" or "Almost Clear") (%) | 20 |

| | |
|---|---|
| ITT for patients qualifying for dosage escalation. Patients with missing PASI or PGA evaluations were considered non-responders. | |

Figure 2 shows the PASI improvement in patients after dosage escalation.

DLQI was evaluated at the time of dosage escalation and at Week 60 for 18 of the 30 patients who switched to weekly dosing. Mean improvement in DLQI for these patients was 2.1. One patient in the dosage escalation group experienced a serious adverse event, and two others withdrew from the study due to adverse events

In conclusion, the majority of psoriasis patients treated with adalimumab eow dosing had a sufficiently satisfactory clinical response and did not require dosage escalation. Adalimumab 40 mg eow dosing provided sustained, clinically significant improvement in ∼80% of psoriasis patients. mong patients whose dosages increased to 40 mg weekly, 40% achieved PASI 50, and no serious adverse events were observed. After qualifying for dosage escalation, nearly 20% of patients achieved at least 75% improvement in their psoriasis at Week 60. Adalimumab's safety profile in the population of patients who qualified for dosage escalation was consistent with the safety profile of adalimumab in rheumatoid arthritis clinical trials

### Example 6: Achievement of PASI 100 is Associated with Better Dermatology-Specific Patient Reported Outcomes Compared to Achievement of PASI 75-99

Adalimumab is a fully human anti-TNF monoclonal antibody efficacious in the treatment of psoriasis, based on results from a Phase II clinical study. The present subanalysis was performed to assess if there was additional benefit to patients who achieved PASI 100 as compared with the benefit experienced by patients who achieved PASI 75-99.

The efficacy and safety of adalimumab was evaluated in a 48-week extension trial conducted at 18 sites in the US and Canada. Prior to this open-label study, patients were enrolled in a 12-week, double-blind, placebo-controlled trial. Inclusion criteria included:
◆ ≥ 18 years of age
◆ Moderate to severe chronic plaque psoriasis ≥1 year
◆ Affected BSA ≥5%
Exclusion criteria included:
◆ Prior TNF-antagonist therapy
◆ Discontinuation of other systemic psoriasis therapies
Efficacy outcome was measured using PASI and DLQI responses/scores.
Analytical methods included the following:
◆ Analyses of clinical data were performed on a modified intention-to-treat population (ITT). Missing data were analyzed by non-responder imputation (NRI) for PASI scores. Patients whose dosages were increased on or after Week 24 were considered non-responders;
◆ Patients with similar percentage PASI improvement across treatment arms were pooled into four strata: PASI <50, PASI 50-74, PASI 75-99, and PASI 100; and
◆ Week-60 distribution of DLQI scores, mean DLQI scores, and mean change in DLQI from Week 0-60 were calculated within each stratum.

The study design is shown in Figure 1, and the baseline demographics and clinical characteristics are shown in Table 4.

Randomized treatment arms included: 1) placebo (n=52); 2) adalimumab 80 mg subcutaneously (sc) at Week 0, then 40 mg sc every other week (eow) starting at Week 1 (n=45); and 3) adalimumab 80 mg sc at Weeks 0 and 1, then 40 mg sc weekly starting at Week 2 (n=50). At Week 12, placebo patients were switched to adalimumab 40 mg eow (placebo→eow). Skin and patient reported outcomes were assessed up to Week 60.

**Table 29. PASI Response Rates at Week 60**

| | | | | |
|---|---|---|---|---|
| | | | | |

| | | **Treatment** | **% Patien ts** | |
|---|---|---|---|---|
| | **PASI 50** | Placebo/Adalimumab 40mg eow | 57 | |
| | | Adalimumab 40mg eow | 64 | |
| | | Adalimumab 40 mg weekly | 66 | |
| | **PASI 75** | Placebo/Adalimumab 40mg eow | 45 | |
| | | Adalimumab 40mg eow | 56 | |
| | | Adalimumab 40 mg weekly | 64 | |
| | **PASI 90** | Placebo/Adalimumab 40mg eow | 40 | |
| | | Adalimumab 40mg eow | 33 | |
| | | Adalimumab 40 mg weekly | 48 | |
| | **PASI 100** | Placebo/Adalimumab 40mg eow | 19 | |
| | | Adalimumab 40mg eow | 16 | |
| | | Adalimumab 40 mg weekly | 26 | |
| | **At Week 12, placebo patients started adalimumab 40 mg eow, after an initial 80-mg dose.** | | | |
| | Modified ITT, NRI. | | | |
| | Patients with PASI <50 response on or after Week 24 received open-label adalimumab weekly rescue therapy. | | | |
| | Patients receiving rescue therapy were considered non-responders in this analysis. | | | |

As shown in Table 29, 16-26% of patients who received at least one dose of adalimumab at baseline reported a PASI 100 response at Week 60. There were no marked differences in the baseline characteristics between patients with DLQI=0 and DLQI >0 within each PASI stratum, with the exception that a higher percentage of patients with concomitant psoriatic arthritis reported DLQI=0 (Table 30)

**Table 30. Baseline Characteristics by DLQI Score Among Key PASI Strata**

| | PASI 100 | | PASI 75-99 | |
|---|---|---|---|---|
| | DLQI=0 (n=23) | DLQI >0 (n=6) | DLQI=0 (n=20) | DLQI >0 (n=34) |
| Age (years) | 43.9 | 44.2 | 43.5 | 44.6 |
| % Male | 74 | 83 | 95 | 85 |
| Body Weight (kg) | 94.2 | 98.2 | 97.6 | 100.4 |
| Psoriasis Duration (years) | 17.1 | 23.4 | 19.9 | 20.1 |
| % PsA | 22 | 0 | 35 | 18 |
| PASI Score at Baseline | 13.3 | 13.6 | 17.2 | 19.3 |
| % BSA at Baseline | 23 | 23 | 29 | 36 |

| | | | | |
|---|---|---|---|---|
| Mean values except percentages | | | | |

At week 60, PASI 75-99/100 response rates were 38/17 for the (placebo→eow + eow) group and 38/26 for the weekly group (observed analysis). Pooling patients with similar % PASI improvement across treatment arms, the distribution of DLQI scores (lower score indicates better health status) at week 60 for patients with PASI<50, 50-74, 75-99, 100 are shown below in Table 31. Pooling across treatment arms, 48 of the total 106 patients receiving adalimumab at Week 60 achieved DLQI=0. A majority of patients (79%) with PASI 100 achieved a DLQI=0 at Week 60 compared with 37% in patients with PASI 75-99 and 28% in patients with 50-74.

**Table 31: Number and Percentage of Patients with DLQI Scores of 0, 1, 2, ≥3 by PASI Improvement at Week 60 (DLQI Scores By PASI Strata at Week 60)**

| PASI Improvement Group | DLQI = 0 n (%) | DLQI = 1 n (%) | DLQI = 2 n (%) | DLQI ≥ 3 n (%) |
|---|---|---|---|---|
| <50 (n=5) | 0 (0) | 1 (20.0) | 2 (40) | 2 (40) |
| 50-74(n=18) | 5 (28) | 3 (17) | 3 (17) | 7 (39) |
| 75-99(n=54) | 20 (37) | 19 (35) | 4 (7) | 11 (20) |
| 100 (n=29) | 23 (79) | 3 (10) | 3 (10) | 0 (0) |

Across all PASI strata, DLQI mean scores were substantially lower at Week 60 (Table 32). At Week 60, patients with PASI <50, 50-74, 75-99, and 100 had mean DLQI scores of 3.0, 3.3, 1.8, and 0.3, respectively with mean changes in DLQI from baseline of -6, -8, -11.5, and -11.1, respectively.

**Table 32. Mean Change in DLQI by PASI Response Rates at Week 60**

| | | | | |
|---|---|---|---|---|
| | | | | |

| | **PASI Response** | **Mean DLQI at Week 60** | **Mean ΔDLQI** | |
|---|---|---|---|---|
| | **PASI <50** | -6.0 | 3.0 | |
| | **PASI 50-74** | -8.8 | 3.3 | |
| | **PASI 75-99** | -11.5 | 1.8 | |
| | **PASI 100** | -11.1 | 0.3 | |
| | **Observed values.** | | | |
| | **MID^{1,2} (Minimum important difference) = -5** | | | |
| | **1Shikiar R, et al. *Health Qual Life Outcomes* 2006, in press.** | | | |
| | **2Khilji FA, et al. *Br J Dermatol* 2002, 147: 50 (abstract).** | | | |

Among those PASI 75-99 responders who had DLQI >0 at Week 60, approximately 50% experienced persistent symptoms of itching, soreness, pain, or stinging of the skin and approximately 20% experienced embarrassment/self-consciousness because of their skin condition.

Withdrawal rates due to adverse events were similarly low in all treatment groups. All patients treated with adalimumab in the placebo-controlled study continued into the extension study (Tables 22 and 33). The percentage of patients who withdrew due to adverse events from Weeks 12-60 ranged from 3-10% (Tables 22 and 33). A higher percentage of patients with PASI 100 and DLQI=0 had adverse events than did patients with PASI 100 and DLQI >0 (91% vs. 50%), but a lower percentage of patients with PASI 75-99 and DLQI=0 had adverse events than did patients with PASI 75-99 and DLQI >0 (80% vs. 91%).

**Table 33. Adverse Events bv Treatment Period**

| | | | | | | | |
|---|---|---|---|---|---|---|---|
| | | | | | | | |

| | | **Weeks 0-12** | | | **Weeks 12-60** | | |
|---|---|---|---|---|---|---|---|
| | **Event** | **Placebo (n=52) n (%)** | **Adalimum ab 40mg eow (n=45) n (%)** | **Adalimum ab 40 mg weekly (n=50) n (%)** | **Placebo/Adalimuma b 40mg eow + Adalimumab 40mg eow (n=92) n (%)** | **Adalimuma b 40 mg weekly (n=50) n (%)** | |
| | **Any AE** | 35 (67.3) | 28 (62.2) | 39 (78.0) | 72 (87.3) | 39 (78) | |
| | **Serious AE** | 0 (0) | 1 (2.2) | 4 (8.0) | 2 (2.2) | 7 (14) | |
| | **AEs leading to withdra wl** | 1 (1.9) | 2 (4.4) | 3 (6.0) | 3 (3.3) | 5 (10.0) | |
| | **Gordon KB et al., J. Am. Acad. Dermatol., Published online.** | | | | | | |
| | **DOI:10.1016/j.jaad.2006.05.027** | | | | | | |

In sum, approximately 45% of patients receiving adalimumab at Week 60 achieved DLQI=0. In this post-hoc subanalysis of a Phase II study, patients who achieved PASI 100 responses averaged lower DLQI scores than patients who achieved PASI 75-99 responses at Week 60. Long-term adalimumab treatment of psoriasis patients provided sustained, clinically significant improvement. Patients who achieved PASI 100 typically had superior dermatology-specific patient reported outcomes compared with patients who achieved PASI 75-99. The types and rates of adverse events in this study were similar to those previously reported in adalimumab rheumatoid arthritis and psoriatic arthritis trials

### Example 7: Use Of TNF Inhibitor In Patients With Hepatitis B Virus

Use of TNF blockers, including Humira, has been associated with reactivation of hepatitis B virus (HBV) in patients who are chronic carriers of this virus. In some instances, HBV reactivation occurring in conjunction with TNF blocker therapy has been fatal. The majority of these reports have occurred in patients concomitantly receiving other medications that suppress the immune system, which may also contribute to HBV reactivation. Patients at risk for HBV infection should be evaluated for prior evidence of HBV infection before initiating TNF blocker therapy. Prescribers should exercise caution in prescribing TNF blockers for patients identified as carriers of HBV. Adequate data are not available on the safety or efficacy of treating patients who are carriers of HBV with anti-viral therapy in conjunction with TNF blocker therapy to prevent HBV reactivation. Patients who are carriers of HBV and require treatment with TNF blockers should be closely monitored for clinical and laboratory signs of active HBV infection throughout therapy and for several months following termination of therapy. In patients who develop HBV reactivation, HUMIRA should be stopped and effective anti-viral therapy with appropriate supportive treatment should be initiated. The safety of resuming TNF blocker therapy after HBV reactivation is controlled is not known. Therefore, prescribers should exercise caution when considering resumption of HUMIRA therapy in this situation and monitor patients closely.

### Example 8: The Validity and Responsiveness of Three Quality of Life Measures in the Assessment of Psoriasis Patients: Results of a Phase II Study

### BACKGROUND

Moderate to severe plaque psoriasis has been demonstrated to have substantial impact on function limitations and psychosocial factors of patients with the disease [1-5]. Moreover, successful treatment of moderate to severe psoriasis - as assessed by improved physical functioning and reduction of signs and symptoms - has been shown to have a positive impact on social and psychological aspects of psoriasis [6-11].

Given the functional and psychosocial impact of the disease, studies of moderate to severe psoriasis patients often include both physician-assessed clinical endpoints and dermatology-specific patient-reported outcomes (PROs) to obtain a holistic view of the disease and treatment effects in patients [12]. Such practices are bolstered by the assertion of the Medical Advisory Board of the National Psoriasis Foundation (NPF) that, even more so than physical signs, such as the percentage of body surface area (BSA) affected by psoriasis, the severity of psoriasis is "first and foremost a quality-of-life (QOL) issue" [13]. The same values for percentage BSA involvement can result in very different degrees of impact for different patients, depending on the location of psoriatic plaques, the pain associated with the lesions and plaques, the extent of bleeding associated with the psoriatic lesions, and resulting functional limitations. The NPF Advisory Board suggests an alternative basis for defining mild, moderate, or severe psoriasis, predicated on QOL impacts of the disease. Similarly, the guidelines recently promulgated by the British Association of Dermatologists [14] for the use of biologics in psoriasis indicate that eligible patients must have a Psoriasis Area and Severity Index (PASI) score of at least 10 and a score on the Dermatology Life Quality Index (DLQI) [15] - a dermatology-specific validated PRO measure - of greater than 10.

A Phase II clinical trial of two dosages of adalimumab and placebo in the treatment of moderate to severe psoriasis provided an opportunity to further explore the psychometric characteristics -including responsiveness and minimum important differences - of the three PROs used in the trial: the DLQI; the general health-related QOL measure MOS Short Form 36 (SF-36) Health Survey [16]; and the general health status measure EuroQOL 5D (EQ-5D) [17,18]. Establishing the reliability, validity, and responsiveness of PRO measures is necessary for their use in support of labeling claims, according to an FDA draft guide to industry [19]. Reliability refers to the accuracy of a measure, while validity refers to the extent the measure actually is measuring what it purports to measure. Responsiveness is a component of validity and represents the PRO's capability to detect changes related to changes in the clinical status of patients or other relevant outcomes measures. Minimum important difference (MID) is related to responsiveness and provides guidance to those reviewing clinical trial results as to whether the statistically significant group differences or changes are clinically meaningful and important. Jaeschke and colleagues [20] define a minimal clinically important difference (MCID) (MID is used here instead of MCID to avoid confusion) as "the smallest difference in score ... which patients perceive as beneficial and which would mandate, in the absence of troublesome side-effects and excessive cost, a change in the patient's management." Estimation of the MID - using several different approaches - is also emphasized in the FDA guidance and is consistent with recently published recommendations of health outcomes researchers [21,22].

The objective of the study was to examine the relationships among the Dermatology Life Quality Index (DLQI), the Short Form 36 (SF-36), and the EuroQOL 5D (EQ-5D) and to assess their validity, responsiveness, and estimates of minimum important differences.

### METHODS

### Overview

A Phase II, randomized, double-blind, parallel group, placebo-controlled, multi-center clinical trial assessed the clinical efficacy and safety of two doses of subcutaneously administered adalimumab vs. placebo for 12 weeks in the treatment of patients with moderate to severe plaque psoriasis. This study provided the opportunity to evaluate the validity and responsiveness to change in clinical status of PROs instruments. Patients completed the DLQI, SF-36, and EQ-5D questionnaires at baseline and at 12 weeks. Blinded investigators assessed the Psoriasis Area and Severity Index (PASI) scores and the Physician's Global Assessment (PGA) scores of enrolled patients. The responsiveness of the measures to changes in the clinical endpoints from baseline to Week 12 was assessed. Estimates of minimum important differences (MID) were derived. All analyses were performed with blinded data; findings and conclusions were not biased based on treatment condition.

The objectives of the Phase II, randomized, double-blind, parallel group, placebo-controlled, multi-center clinical trial were to assess the clinical efficacy and safety of subcutaneously administered adalimumab vs. placebo using two dosage regimens for 12 weeks in the treatment of patients with moderate to severe plaque psoriasis. The study included a screening period, a blinded 12-week treatment period, and a 30-day follow-up visit for patients not completing 12 weeks of active treatment or not entering an extension study. Time between screening and baseline visits was not to exceed 28 days. The trial found that adalimumab was both safe and efficacious vs. placebo in the treatment of moderate to severe, chronic plaque psoriasis [23].

### Patients and Inclusion Criteria

Patients with a diagnosis of moderate to severe plaque psoriasis and an affected BSA of ≥5% for at least 1 year were eligible for the study. In addition to other inclusion criteria (e.g., age ≥18 years, willingness to give informed consent), patients had to be able to self-inject medication or have a designee or nurse who could inject the randomized assignment. Patients signed informed consent forms, and the study complied with FDA Good Clinical Practices, Health Protection Branch guidelines, and all other applicable ethical, legal, and regulatory requirements [23].

### Clinical Measures

For purposes of the analyses reported here, there were two primary clinical outcomes:

### Psoriasis Area and Severity Index

Frequently used as an endpoint in psoriasis clinical trials [24], the PASI [25] was the primary efficacy outcome in this trial. PASI is a composite index indicating the severity of the three main signs of psoriatic plaques (i.e., erythema, scaling, and thickness) and is weighted by the amount of coverage of these plaques in the four main body areas (head, trunk, upper extremities, and lower extremities). PASI scores range from 0-72, with higher scores indicating greater disease severity. PASI was assessed at screening and baseline, at Weeks 1, 2, 4, 8, and 12/Early Termination, and at the final follow-up visit.

### Physician's Global Assessment

The PGA is a seven-point scale used to measure the severity of disease at the time of the physician's evaluation. The seven disease categories are:
- *Severe:* very marked plaque elevation, scaling, and/or erythema
- *Moderate to Severe:* marked plaque elevation, scaling, and/or erythema
- *Moderate:* moderate plaque elevation, scaling, and/or erythema
- *Mild to moderate:* intermediate between moderate and mild
- *Mild:* slight plaque elevation, scaling, and/or erythema
- *Almost Clear:* intermediate between mild and clear
- *Clear:* no signs of psoriasis (post-inflammatory hypopigmentation or hyperpigmentation could be present).

The PGA scale is scored from 1 (Clear) to 7 (Severe). The PGA was assessed by the investigator at screening, baseline, and Weeks 1, 2, 4, 8, 12/Early Termination, and the follow-up visit. Each study site was to make every attempt to have the same investigator perform these assessments throughout the study for each patient.

### Patient-Reported Outcome Measures

Three PROs measures were used in the study and are the subject of the analyses reported here. All PROs measures were assessed at baseline and at Week 12 (or early termination, if applicable).

### Dermatology Life Quality Index

The DLQI was developed as a simple, compact, and practical questionnaire for use in dermatology clinical settings to assess limitations related to the impact of skin disease [15]. The instrument contains 10 items dealing with skin (e.g., Item 1: "Over the last week, how itchy sore, painful, or stinging has your skin been?"). The DLQI score ranges from 0-30, with "30" corresponding to the worst quality of life, and "0" corresponding to the best score. The DLQI has well-established properties of reliability and validity in the dermatology setting [15,26-28].

### Short Form 36 Health Survey

The SF-36 is a 36-item general health status instrument often used in clinical trials and health services research [16]. It consists of eight domains: Physical Function, Role Limitations-Physical, Vitality, General Health Perceptions, Bodily Pain, Social Function, Role Limitations-Emotional, and Mental Health. Two overall summary scores can be obtained - a Physical Component Summary (PCS) score and a Mental Component Summary (MCS) score [29]. The PCS and MCS scores range from 0-100, with higher scores indicating better health. The SF-36 has been used in a wide variety of studies involving psoriasis, including descriptive studies [30] and clinical research studies [6,7], and has demonstrated good reliability and validity. Internal consistency for most SF-36 domains is greater than 0.70. The SF-36 has been shown to discriminate between known groups in a variety of diseases, is reproducible, and is responsive to longitudinal clinical changes.

### EuroQOL 5D

The EQ-5D [17,18] is a six-item, preference-based instrument designed to measure general health status. The EQ-5D has two sections: The first consists of five items to assess degree of physical functioning (mobility, self-care, usual activities, pain/discomfort, and anxiety/depression). Items are rated on a three-point scale ranging from "No Problem" to "Extreme Problem" or "Unable to Do." Each pattern of scores for the five items is linked to an index score that has a value ranging from 0-1, indicating the health utility of that person's health status. The specific linkage can differ from country to country, reflecting differences in cultures to the item responses. The second section is the sixth item on the EQ-5D, which is a visual analog scale with endpoints of "100" or "Best Imaginable Health," and "0" or "Worst Imaginable Health." It offers a simple method for the respondents to indicate how good or bad their health statuses are "today." The score is taken directly from the patients' responses.

### Statistical Methods

Validity of the PRO measures was assessed in several ways. First, an assessment was made of the concurrent validity of scales and subscales (i.e., the extent to which PRO measures are correlated with one another). As a disease-specific PRO measure, the DLQI was expected to correlate moderately to extremely well with general PRO measures. Another important aspect of validity in this study was to assess the extent to which the PRO measures correlated with the clinical endpoints - PASI and PGA-both at baseline and at Week 12.

Responsiveness of PRO measures was assessed via two approaches. First, changes in these measures from baseline to Week 12 were correlated with changes in the PASI or PGA over the 12-week course of treatment within the trial. Concurrent improvement in both clinical measures and PRO measures was expected to result in positive correlations. The second approach to assessing responsiveness involved categorizing patients into responder groups based on the changes in their PASI scores from baseline to Week 12. This was done in two ways. First, a responder was defined as a patient with >75% improvement in PASI (consistent with the definition of success with the primary efficacy variable), and a non-responder was defined as a patient with a PASI improvement <50% (consistent with the definition of failure for a secondary efficacy variable). Tests of mean differences in improvement on the PRO measures were completed between the two groups. Secondly, in support of the estimation of the MID, discussed below, patients were further categorized by degree of PASI response, and assessed differences among these four groups: PASI improvement <25%; PASI improvement 25-49%; PASI improvement 50-74%; and PASI improvement ≥75%. Analyses of variance tests were performed among these four groups for changes in PRO measures.

In accordance with the FDA draft guidance [19] and consistent with recent recommendations from PRO researchers [21,22], five methods were used to estimate MIDs of the PROs. The PRO change score corresponding to PASI 25-PASI 49 was the first estimate of MID, called MID-1. This was based on the assumption that patients would perceive a PASI improvement of 25% as beneficial. The trial did not provide data to test this assumption (e.g., there was no rating by patients of their overall improvements). A second estimate, MID-2, was based on the PRO change score corresponding to a PASI improvement between 50-74%. The PASI 50 is seen as clinically relevant, and, as such, this degree of improvement served as a secondary efficacy endpoint in this trial. A third method for estimating MID relied on the association of changes in the PRO measure with changes in the PGA. A non-responder was defined as a patient with a PGA change score of either "0" (no change) or "1" (slight increase in severity of disease) from baseline to Week 12. A minimal responder was defined as a patient whose PGA improved by either 1 or 2 points from baseline to Week 12. The third estimate of MID, MID-3, was the difference in the PRO score between non-responders and minimal responders.

In addition, two distributional methods were used to support the anchor-based MID estimates for the PROs [21,22]. Based on evidence by Wyrwich and associates [31,32], the standard error of measurement (SEM) can be used to approximate the MID. The SEM, which describes the error associated with the measure, was estimated by the standard deviation of the measure multiplied by the square root of 1 minus its reliability coefficient. Finally, there has been discussion [33] concerning a number of studies demonstrating that one-half of the standard deviation of a measure represents the upper limit of the MID [22]. In estimating the SEM for the SF-36 and the EQ-5D, reliability estimates from the literature were used. The SEM for the DLQI incorporated the reliability estimated from the trial data, which was consistent with what has been found in the literature for this instrument [27].

Finally, it is important to note that all analyses were performed with blinded data (i.e., the statuses of patients with respect to their assigned treatment groups were not known).

### RESULTS

### Overview

The dermatology-specific DLQI was highly correlated to clinical endpoints at baseline and at Week 12, and was the most responsive PRO to changes in endpoints. Compared with the SF-36, the EQ-5D index score and VAS scores were generally more highly correlated with clinical endpoints, but displayed about the same degree of responsiveness. The most responsive SF-36 scales were the Bodily Pain and Social Functioning scales. Estimates of the MID for the DLQI ranged from 2.3-5.7 and for the SF-36 Physical Component Summary (PCS) score ranged from 2.5-3.9.

### Patient Demographics and Clinical Characteristics

A total of 147 patients enrolled and received at least one dose of study medication at 18 sites in the United States and Canada. Blinded data were available for the PROs for 147 patients at baseline and 140 patients at Week 12. Since the focus of these analyses were on the psychometric properties of the PROs rather than with efficacy, observed cases were employed rather than last observation carried forward or other methods for treating missing observations at the end of trial. The mean age of the patients enrolled in the trial was 44.2 years, two-thirds were male, and the preponderance were white (Table 34).

**Table 34. Baseline Demographic Characteristics**

| Characteristic | | (N=147) |
|---|---|---|
| | | |

| Age | | |
|---|---|---|
| | Mean (SD) | 44.2 (12.7) |
| | | |

| Gender | | |
|---|---|---|
| | Female n (%) | 48 (32.7%) |
| | Male n (%) | 99 (67.3%) |
| | | |

| Race | | |
|---|---|---|
| | White n (%) | 133 (90.5%) |
| | Black n (%) | 4 (2.7%) |
| | Asian n (%) | 5 (3.4%) |
| | Other n (%) | 5 (3.4%) |
| | | |

### Clinical Endpoints

The results for the PASI and the PGA at baseline and Week 12, as well as the change from baseline to Week 12, are displayed in Table 35. The mean PASI at baseline was 15.7, which decreased by 8.9 points (improvement) to 6.8 by Week 12. The mean PGA at baseline was 5.5 (i.e., midway between "Moderate" and "Moderate to Severe"), and decreased (improved) by 2.1 points to 3.4 by Week 12 (i.e., between "Mild" and "Mild to Moderate"). In evaluating the improvement in the two clinical endpoints, it is important to keep in mind that these analyses included pooled placebo and active treatment groups.

**Table 35. Mean (Standard Deviation) of PASI and PGA at Baseline and Week 12, and Change from Baseline to Week 12**

| Measure | Baseline | Week 12 | Change² |
|---|---|---|---|
| | | | |
| PASI | 15.69 (7.34) | 6.84 (7.77) | -8.87 (8.41) |
| | | | |
| PGA¹ | 5.48 (0.81) | 3.36 (1.74) | -2.14 (1.87) |

| | | | |
|---|---|---|---|
| ¹ Scored such that 1="Clear" to 7="Severe." ²Change scores are computed only for the 140 patients with scores at baseline and Week 12; sample size at baseline=147. | | | |

### Patient-Reported Outcome Measures

The results for the DLQI, SF-36, and EQ-5D at baseline and Week 12, and the change from baseline are shown in Table 36. Based on blinded data, mean PRO measures improved during the course of the trial (a decrease in DLQI scores indicates an improvement; an increase in the SF-36 and EQ-5D indicates improvement). The greatest improvement in a DLQI item occurred for the first item, assessing how "itchy, sore, painful, or stinging" the person's skin felt. Similarly, as shown in Table 19, the greatest improvement among the SF-36 scales was for Bodily Pain, although there were improvements in each of the SF-26 scales using adalimumab treatment for psoriasis. The largest improvement among the five EQ-5D dimensions occurred for the Pain/ Discomfort dimension. Given these findings, it appears that improvement in pain and discomfort is the most pronounced among all PRO measures assessed.

**Table 36. Mean (Standard Deviation) of PROs at Baseline and Week 12, and Change from Baseline to Week 12**

| Measure | | Baseline | Week 12 | Change¹ |
|---|---|---|---|---|
| DLQI Total Score | | 12.71 (7.18) | 5.28 (6.49) | -7.45 (7.78) |

| SF-36 | | | | |
|---|---|---|---|---|
| | Physical Functioning | 79.26 (24.95) | 84.43 (22.62) | 5.63 (22.25) |
| | Role-Physical | 72.45 (37.56) | 82.50 (34.67) | 9.64 (40.30) |
| | Bodily Pain | 59.58 (25.37) | 75.87 (24.89) | 16.59 (26.90) |
| | General Health | 66.7 (20.6) | 72.69 (20.79) | 6.10 (16.25) |
| | Vitality | 53.89 (22.81) | 61.04 (23.18) | 7.60 (20.88) |
| | Social Functioning | 74.49 (27.56) | 85.98 (23.63) | 11.16 (27.80) |
| | Role- Emotional | 76.03 (35.39) | 85.48 (30.77) | 8.39 (37.02) |
| | Mental Health | 69.39 (19.30) | 77.43 (17.73) | 8.14 (18.57) |
| | Physical Summary Score (PCS) | 47.93 (10.23) | 51.24 (9.51) | 3.47 (9.30) |
| | Mental Summary Score (MCS) | 47.30 (11.23) | 51.36 (10.08) | 3.94 (11.04) |

| EQ-5D | | | | |
|---|---|---|---|---|
| | Index Score | 0.66 (0.28) | 0.82 (0.23) | 0.16 (0.29) |
| | VAS Overall Health | 72.25 (20.67) | 81.22 (17.26) | 9.35 (20.71) |

| | | | | |
|---|---|---|---|---|
| ¹Change scores are computed only for patients with scores at baseline and Week 12; this number varied between 138 and 140, depending on the specific measure, as compared with the 147 patients at baseline. | | | | |

The reliability of the DLQI, as assessed by coefficient alpha, was 0.89 at baseline and 0.92 at Week 12, indicating that this is a highly reliable measure, and in line with previous findings [27, 28].

### Relationships Among Patient-Reported Outcome Measures

Table 37 displays the correlations among PRO measures at baseline and at Week 12, as well as the correlations among changes in these measures from baseline to Week 12. There were a few trends evident form this data. First, all measures were statistically significantly inter-correlated. Second, with respect to the relationship between the DLQI and the SF-36, the DLQI correlated the greatest with the Bodily Pain and Social Functioning domains, both at baseline and at Week 12, and, for changes in these scores over the course of the trial. Third, the DLQI correlated highly with the EQ-5D index score, and these correlations were consistently higher than the correlations with the EQ-5D visual analog scale (VAS) scores. Fourth, the EQ-5D index score tended to correlate greatest with the Bodily Pain domain of the SF-36. Finally, the scores tended to be more highly correlated at the end of the trial than at baseline, consistent with previous findings [28].

**Table 37. Correlations¹ among PROs at Baseline and Week 12, and Change from Baseline to Week 12**

| Measure | | Baseline | | | Week 12 | | | Change | | |
|---|---|---|---|---|---|---|---|---|---|---|
| | | DLQI Total | EQ-5D Index | EQ-5D VAS | DLQI Total | EQ-5D Index | EQ-5D VAS | DLQI Total | EQ-5D Index | EQ-5D VAS |
| *DLQI* | | | | | | | | | | |
| | Total Score | 1.00 | -0.51 | -0.35 | 1.00 | -0.71 | -0.58 | 1.00 | -0.53 | -0.46 |

| *SF-36* | | | | | | | | | | |
|---|---|---|---|---|---|---|---|---|---|---|
| | Physical Functioning | -0.44 | 0.58 | 0.35 | -0.41 | 0.61 | 0.49 | -0.29 | 0.47 | 0.32 |
| | Role-Physical | -0.45 | 0.64 | 0.38 | -0.57 | 0.67 | 0.50 | -0.47 | 0.51 | 0.45 |
| | Bodily Pain | -0.55 | 0.73 | 0.45 | -0.61 | 0.76 | 0.56 | -0.66 | 0.64 | 0.53 |
| | General Health | -0.24** | 0.39 | 0.47 | -0.38 | 0.59 | 0.69 | -0.33 | 0.46 | 0.46 |
| | Vitality | -0.31 | 0.43 | 0.48 | -0.43 | 0.62 | 0.60 | -0.46 | 0.37 | 0.48 |
| | Social Functioning | -0.69 | 0.52 | 0.46 | -0.68 | 0.74 | 0.60 | -0.68 | 0.50 | 0.56 |
| | Role-Emotional | -0.41 | 0.45 | 0.42 | -0.56 | 0.67 | 0.53 | -0.50 | 0.41 | 0.48 |
| | Mental Health | -0.44 | 0.46 | 0.50 | -0.56 | 0.66 | 0.67 | -0.52 | 0.49 | 0.56 |
| | Physical Summary Score (PCS) | -0.41 | 0.64 | 0.36 | -0.46 | 0.65 | 0.52 | -0.41 | 0.56 | 0.39 |
| | Mental Summary Score (MCS) | -0.45 | 0.39 | 0.49 | -0.58 | 0.66 | 0.63 | -0.59 | 0.42 | 0.57 |

| *EQ-5D* | | | | | | | | | | |
|---|---|---|---|---|---|---|---|---|---|---|
| | Index Score | -0.51 | 1.00 | 0.39 | -0.71 | 1.00 | 0.63 | -0.53 | 1.00 | 0.39 |
| | VAS-General Health | -0.35 | 0.39 | 1.00 | -0.58 | 0.63 | 1.00 | -0.46 | 0.39 | 1.00 |

| | | | | | | | | | | |
|---|---|---|---|---|---|---|---|---|---|---|
| ¹All correlations were significant at p<0.001, unless otherwise noted. *p≤0.05, **p≤0.01 , ns=non-significant. | | | | | | | | | | |

### Correlations with Clinical Endpoints

Table 38 displays correlations of PRO measures with the two clinical assessments - PASI score and PGA - at baseline (first two columns of data) and at Week 12 (second two columns). In addition to almost uniformly greater correlations at Week 12 vs. at baseline - consistent with previous findings [28] - one can also note that both the DLQI and EQ-5D index score tended to be more highly correlated with the two clinical endpoints than any of the SF-36 domains. The SF-36 scales with the strongest association with clinical endpoints are Social Functioning and Bodily Pain.

**Table 38. Correlations¹ between PROs and Clinical Endpoints at Baseline and Week 12, and Change from Baseline to Week 12**

| **Measure** | | **Baseline** | | **Week 12** | | **Change** | |
|---|---|---|---|---|---|---|---|
| | | **PASI** | **PGA** | **PASI** | **PGA** | **PASI** | **PGA** |
| DLQI Total Score | | 0.31 | 0.29 | 0.67 | 0.65 | 0.69 | 0.71 |

| *SF-36* | | | | | | | |
|---|---|---|---|---|---|---|---|
| | Physical Functioning | -0.32 | -0.14 ns | -0.28 | -0.25** | -0.38 | -0.14 ns |
| | Role-Physical | -0.22** | -0.14 ns | -0.41 | -0.37 | -0.42 | -0.31 |
| | Bodily Pain | -0.36 | -0.19* | -0.47 | -0.42 | -0.60 | -0.44 |
| | General Health | -0.08 ns | 0.05 ns | -0.34 | -0.33 | -0.34 | -0.24** |
| | Vitality | -0.15 ns | -0.06 ns | -0.37 | -0.37 | -0.38 | -0.31 |
| | Social Functioning | -0.23** | -0.21** | -0.46 | -0.38 | -0.44 | -0.43 |
| | Role-Emotional | -0.16 ns | -0.06 ns | -0.37 | -0.29 | -0.39 | -0.36 |
| | Mental Health | -0.17* | -0.09 ns | -0.46 | -0.38 | -0.43 | -0.36 |
| | Physical Summary | -0.28 | -0.13 ns | -0.35 | -0.33 | -0.45 | -0.25** |
| | Mental Summary | -0.12 ns | -0.08 ns | -0.44 | -0.36 | -0.40 | -0.42 |

| *EQ-5D* | | | | | | | |
|---|---|---|---|---|---|---|---|
| | Index Score | -0.40 | -0.31 | -0.60 | -0.51 | -0.57 | -0.44 |
| | VAS-General Health | -0.24** | -0.09 ns | -0.52 | -0.43 | -0.43 | -0.38 |
| | | | | | | | |
| PASI | | 1.00 | 0.59 | 1.00 | 0.83 | 1.00 | 0.75 |

| | | | | | | | |
|---|---|---|---|---|---|---|---|
| ¹ All correlations were significant at p<0.001, unless otherwise noted.*p≤0.05, **p<0.01, ns=non-significant. | | | | | | | |

### Responsiveness of the Patient-Reported Outcome Measures

An important attribute for a PRO measure is responsiveness to change in the clinical status of a patient (i.e., as a patient's disease improves, the PRO measures also improve). The last two columns of Table 5 display the correlations between changes in PRO measures used in the trial and changes in PASI scores and the PGA from baseline to Week 12. These data demonstrate that the DLQI is the most responsive of the PRO measures. The correlations between changes over the course of the trial in the DLQI total score and changes in the PASI score (r=0.69, p<0.001) and PGA (r=0.71, p<0.001) approach the correlation between changes in the two clinical measures themselves (r=0.75, p<0.001). In addition, the DLQI is the only one of the PRO measures to demonstrate equal responsiveness to PGA and PASI scores. The correlation between changes in the EQ-5D index score and the two clinical assessments was r=-0.57 (p<0.001) for changes in the PASI to r=-0.44 for changes in the PGA (p<0.001). Similarly, the correlations between changes in all but one of the SF-36 scores *and* changes in the PGA were smaller than correlations between changes in the SF-36 and the PASI.

A second way to assess responsiveness was to contrast patients who were defined as clinical responders with those characterized as non-responders. Given that the primary endpoint in the trial was defined as the percentage of patients achieving a PASI 75 response (i.e., ≥75% improvement in PASI from. baseline) by Week 12, a responder was defined as a patient with a PASI75 response. A non-responder was a patient with <PASI 50, since some of the secondary endpoints in the trial used this cut-off. The results of these analyses are displayed in Table 39. DLQI total scores for responders improved by 12.17 points, while scores of non-responders improved by 1.77 points. This difference was statistically significant (t=9.0; p<0.0001). All the PRO measures except for the SF-36 Physical Functioning domain were responsive, as defined by a statistically significant difference between responders and non-responders. The DLQI was the most responsive of the PRO measures, as evidenced by the size of the t-statistic and the effect size. The responsiveness of the EQ-5D index and VAS scores were generally the same as several of the SF-36 domain scores.

**Table 39. Change in PRO Measures among Responder¹ Groups**

| Change in Measure | Mean Change Score for Responders (n=66) | Mean Change Score for Non-Responders (n=53) | Difference | t-value | P Value | Effect Size |
|---|---|---|---|---|---|---|
| DLQI Total Score | -12.17 (6.78) | -1.77 (5.52) | -10.39 | 9.0 | <.0001 | 0.40 |

| *SF-36* | | | | | | |
|---|---|---|---|---|---|---|
| Physical Functioning | 9.12 (23.50) | 3.52 (20.19) | 5.59 | 1.4 | 0.1724 | 0.01 |
| Role-Physical | 20.08 (35.69) | -5.19 (44.76) | 25.26 | 3.4 | 0.0008 | 0.08 |
| Bodily Pain | 26.47 (27.40) | 4.21 (22.74) | 22.26 | 4.7 | <.0001 | 0.15 |
| General Health | 8.87 (15.62) | 1.47 (17.77) | 7.39 | 2.4 | 0.0178 | 0.04 |
| Vitality | 13.01 (22.58) | 1.13 (18.20) | 11.87 | 3.1 | 0.0024 | 0.07 |
| Social Functioning | 21.59 (28.13) | -2.59 (25.04) | 24.19 | 4.9 | <.0001 | 0.16 |
| Role-Emotional | 19.70 (32.54) | -9.62 (36.95) | 29.31 | 4.6 | <.0001 | 0.14 |
| Mental Health | 14.55 (17.77) | -0.38 (18.15) | 14.92 | 4.5 | <.0001 | 0.14 |
| Physical Summary Score (PCS) | 5.35 (9.67) | 1.47 (9.08) | 3.88 | 2.2 | 0.0287 | 0.03 |
| Mental Summary Score (MCS) | 8.03 (10.59) | -2.03 (10.42) | 10.06 | 5.1 | <.0001 | 0.17 |

| *EQ-5D* | | | | | | |
|---|---|---|---|---|---|---|
| Index Score | 0.25 (0.30) | 0.04 (0.26) | 0.22 | 4.2 | <.0001 | 0.12 |
| VAS General Health | 15.69 (18.96) | 1.92 (23.24) | 13.77 | 3.5 | 0.0006 | 0.09 |

| | | | | | | |
|---|---|---|---|---|---|---|
| ¹ Responder is defined as PASI improvement ≥75%; non-responder is defined as PASI improvement<50 | | | | | | |

While the estimates of responsiveness displayed in the last two columns of Table 38 take into account the full range of PASI change scores and their relationship to PRO change scores, the responsiveness analysis in Table 39 places patients in two categories - responders and non-responders. Table 36 defines *four* categories of responders: responders, defined as those with PASI improvements ≥75%; "partial responders," those with PASI improvement 50-74%, inclusively; "near responders," those with PASI improvement 25-49%, inclusively; and non-responders, with <PASI25. One-way analyses of variance were performed among these groups for each of the PRO measures. As can be seen by the size of the f-statistics, the DLQI was the most responsive of the PRO measures. In fact, only the DLQI was able to demonstrate statistically significant differences between responders and partial responders based on post-hoc significance tests among the four responder groups. These results for the DLQI total score with respect to differences among responder groups were similar to those reported previously in the literature, except that the improvement in DLQI total scores displayed in Table 40 was larger for each of the responder groups than for the equivalent responder groups described by Shikiar and colleagues in a study of efalizumab [28]. As was the case for the data displayed in Table 39, the responsiveness of the EQ-5D index and VAS scores were generally the same as for most of the SF-36 scores. Finally, both the SF-36 MCS and PCS scores were responsive, but the MCS was more responsive, indicating that the impact of the disease was both physical and mental, with the latter perhaps being more prominent for this study population.

**Table 40. PRO Change Scores Corresponding to Levels of PASI Improvement**

| Change in Measure | PASI Improvement <25% (n=31) | PASI Improvement 25-49% (n=22) | PASI Improvement 50-74% (n=21) | PASI Improve ment ≥75% (n=66) | Overall F Value | p Values¹ |
|---|---|---|---|---|---|---|
| DLQI Total Score | -0.16 (5.41) | -4.05 (4.95) | -6.95 (5.71) | -12.17 (6.78) | 30.4*** | 2**,3***, 5***,6* |

| *SF-36* | | | | | | |
|---|---|---|---|---|---|---|
| Physical Functioning | 2.15 (17.67) | 5.45 (23.60) | 0.02 (22.36) | 9.12 (23.50) | 1.2 | |
| Role-Physical | -11.29 (39.18) | 3.41 (51.35) | 14.29 (31.20) | 20.08 (35.69) | 4.9** | 3** |
| Bodily Pain | -1.03 (21.42) | 11.59 (22.96) | 16.76 (22.74) | 26.47 (27.40) | 9.0*** | 3*** |
| General Health | -0.61 (17.33) | 4.41 (18.37) | 9.19 (11.29) | 8.87 (15.62) | 2.8* | |
| Vitality | -1.45 (16.29) | 4.77 (20.44) | 6.90 (17.43) | 13.01 (22.58) | 3.8* | 3* |
| Social Functioning | -3.23 (23.71) | -1.70 (27.36) | 13.10 (17.44) | 21.59 (28.13) | 8.7*** | 3*** 5** |
| Role-Emotional | -11.11 (36.44) | -7.58 (38.40) | 17.46 (34.35) | 19.70 (32.54) | 7.6*** | 2*, 3**, 5* |
| Mental Health | -0.77 (18.08) | 0.18 (18.66) | 9.52 (13.53) | 14.55 (17.77) | 7.2*** | 3**,5* |
| Physical Summary | -0.31 (7.18) | 3.91 (10.88) | 2.57 (7.78) | 5.35 (9.67) | 2.7* | |
| Mental Summary | -2.19 (9.86) | -1.82 (11.38) | 6.05 (6.90) | 8.03 (10.59) | 9.9*** | 2*, 3***, 5** |

| *EQ-5D* | | | | | | |
|---|---|---|---|---|---|---|
| Index Score | -0.01 (0.26) | 0.10 (0.24) | 0.20 (0.21) | 0.25 (0.30) | 7.1*** | 3*** |
| VAS General Health | 0.58 (24.31) | 3.82 (22.07) | 8.43 (11.24) | 15.69 (18.96) | 4.8** | 3** |
| Mean PASI Improvement² | 0.94 (4.07) | -6.24 (2.99) | -8.94 (3.47) | -14.33 (7.65) | | |

| | | | | | | |
|---|---|---|---|---|---|---|
| ¹Pairwise comparisons between means were performed using Scheffe's test adjusting for multiple comparisons. 1=improvement <25% vs. improvement 25-49%, 2=improvement <25% vs. improvement 50-74%, 3=improvement <25% vs. improvement ≥75%, 4=improvement 25-49% vs. improvement 50-74%, 5=improve 25-49% vs. improvement ≥75%, and 6=improvement 50-74% vs. improvement ≥75%. *p<0.05, **p<0.01, ***p<0.001. ²Negative change scores indicate improvement | | | | | | |

### Estimates of Minimum Important Differences

There is no one best way to estimate the MID for a PRO measure [21,34]. Table 41 contains three different anchor-based methods for estimating the MID based on data from this study. MID-1 contains the estimate obtained from the scores from the "near-responders," shown as the PASI 25-PASI 49 group in Table 40; MID-2 contains the estimate corresponding to "partial responders" in the same table 40. MID-3 corresponds to the difference between non-responders for the PGA (defined as patients who had no change in score or a decrease in score by one point on this 7-point scale) and minimal responders for this same measure (defined as patients who improved by 1 or 2 points). The distribution-based estimates, the SEM and one-half the standard deviation of baseline scores are also reported in Table 41.

**Table 41. Estimates of MCID for PRO Measures**

| Change in Measure | MID-1: PASI Improvement 25-49% | MID-2: PASI Improvement 50-74% | MID-3: Difference Between Non-Responders (n=34) and Minimal Responders (n=41) on PGA | SEM | 0.5 SD |
|---|---|---|---|---|---|
| DLQI Total Score | -4.05 (4.95) | -6.95 (5.71) | -5.69 | 2.33 | 3.59 |

| *SF-36* | | | | | |
|---|---|---|---|---|---|
| Physical Functioning¹ | N/A | N/A | N/A | N/A | N/A |
| Role-Physical | 3.41 (51.35) | 14.29 (31.20) | 10.51 | 15.02 | 18.78 |
| Bodily Pain | 11.59 (22.96) | 16.76 (22.74) | 9.05 | 10.76 | 12.69 |
| General Health | 4.41 (18.37) | 9.19 (11.29) | 4.97 | 9.67 | 10.31 |
| Vitality | 4.77 (20.44) | 6.90 (17.43) | 6.54 | 8.22 | 11.40 |
| Social Functioning | -1.70 (27.36) | 13.10 (17.44) | 13.62 | 10.67 | 13.78 |
| Role-Emotional | -7.58 (38.40) | 17.46 (34.35) | 24.71 | 14.59 | 17.70 |
| Mental Health | 0.18 (18.66) | 9.52 (13.53) | 4.90 | 6.10 | 9.65 |
| Physical Summary Score (PCS) | 3.91 (10.88) | 2.57 (7.78) | 0.51 | 2.71 | 5.12 |
| Mental Summary Score (MCS) | -1.82 (11.38) | 6.05 (6.90) | 6.61 | 3.89 | 5.61 |

| *EQ-5D* | | | | | |
|---|---|---|---|---|---|
| Index Score | 0.10 (0.24) | 0.20 (0.21) | 0.09 | 0.22 | 0.14 |
| VAS-General Health | 3.82 (22.07) | 8.43 (11.24) | 4.59 | N/A | 10.34 |

| | | | | | |
|---|---|---|---|---|---|
| Note: MID-1 corresponds to the score for the PASI 25-49 group; MID-2 corresponds to the score for the PASI50-74; for MID-3 and MID-4, reliability estimates for computing SEM were obtained from the data in this study for the DLQI and from estimates found in the literature for the SF-36 and EQ-5D. ¹MID estimates are not provided for the SF-36 Physical Function domain since there were not significant differences among responder groups. | | | | | |

Estimates for the DLQI MID ranged from 4.05 (for MID-1) to 6.95 (for MID-2), while the SEM was 2.33 and one-half standard deviation was 3.59. The MID results for the SF-36 PCS ranged from 0.51 (for MID-3) to 3.91 (for MID-1), with the SEM estimated as 2.71 and one-half standard deviation estimates as 5.12. For the MCS, the MID estimates included a decrease of 1.82 points based on a PASI improvement of 25-49%, but the other two MIDs were 6.05 and 6.61, respectively. The SEM for the MCS was 3.89 and one-half standard deviation was 5.61. Consistent with the MCS findings, decreases were observed for the Role-Emotional and Social Functioning domains for the MID-1 definition. The differences between non-responders and minimal responders ranged from 4.90 for Mental Health to 24.71 for Social Functioning (Table 41). The results for the EQ-5D index score demonstrated an MID ranging from 0.09 (for MID-3) to 0.20 (for MID-2). For the EQ-5D VAS, the available estimates ranged from 3.82 (MID-1) to 8.43 (MID-3).

### DISCUSSION

A Phase II randomized clinical trial of adalimumab in moderate to severe plaque psoriasis provided the opportunity to evaluate the validity and responsiveness to clinical change of three PRO assessment instruments - one dermatology-specific instrument and two general health status instruments - all used as endpoints in the study. All analyses were performed on a blinded basis, since the main focus of these secondary analyses was on the psychometric qualities of the PRO instruments.

The present study further establishes the reliability and validity of the DLQI and its responsiveness to change in the clinical status of patients over the course of a 12-week clinical trial, confirming previous findings [28]. Changes in the DLQI total score demonstrated significant and sizeable correlations with independently obtained physician-assessed changes in the clinical statuses of patients. This indicates that the alleviation of psoriatic signs, as determined by clinical assessments, results in significant and marked improvement in dermatologic-related functional limitations and quality of life in patients with moderate to severe plaque psoriasis. Based on this study, the DLQI is a psychometrically sound and responsive measure of psoriasis-specific outcomes that captures more comprehensively the impact of clinical signs and symptoms on patient well-being.

We used both the PASI and the PGA, as well as two distributional approaches to derive estimates of the MID of the DLQI. These estimates ranged from 2.33-6.95, although the PASI 50 was too conservative for estimating the minimum change that patients will find beneficial. Therefore, we the estimate based on PASI improvement of 25-49% or between non-responders and minimal responders provided a better estimate of MID for this study. Therefore, the results indicate that the MID is in the range of approximately 2.3-5.7, which is slightly higher than the range of estimates derived from Shikiar et al. [28] in an analysis of two clinical trials involving another psoriasis therapy. The distributional approaches resulted in the lowest estimates of MID for the DLQI, but it should be noted that the distributional approach to estimating the MID is considered supportive of the anchor-based methods [22,35]. For example, the one-half standard deviation estimate is certainly clinically meaningful, but is likely not a minimum magnitude of change. Finally, the range of estimates incorporates another previous estimate of the MID of the DLQI of 5.0 [36].

Two general PRO measures were used in this study. In general, the EQ-5D index and VAS scores demonstrated higher correlations than the SF-36 scale scores with the clinical endpoints (Table 34). However, the responsiveness of these two EQ-5D scores were generally the same as the responsiveness of most of the SF-36 scores. Although most of the SF-36 scores showed improvements associated with clinical outcomes, the MCS, Social Functioning, and Role-Emotional domain scores demonstrated decreases in the PASI 25-49% group. These findings may have been driven by several outliers and the relatively small sample size for this group. Alternatively, given that Bodily Pain and other physical domains may be more related to the signs and symptoms of psoriasis than Role-Limitations and Social Functioning, small improvements in PASI scores may not be directly associated with changes in these PRO domains. That is, larger changes in clinical outcomes may be needed to significantly impact the areas of physical function and well-being. This idea seems to be supported by the observed changes in the PASI 50-74% and other analyses. However, the SF-36 domain and summary scores demonstrated consistently reasonable validity and were correlated with clinical endpoints and DLQI scores.

The SF-36 PCS and MCS scores demonstrated good evidence of validity and responsiveness in this sample of patients with moderate to severe plaque psoriasis. There were demonstrable associations between changes in PASI score categories and changes in PCS scores, with the largest improvements seen in the PASI75 responder groups. The MID estimates for the PCS were in the range of 0.51-3.91, with the best estimate at approximately 2.5 points. The SEM estimate (2.71) also supports this range of MID values for the PCS. The MID findings for the MCS were somewhat weaker, but there is evidence that a change of 4-6 points is certainly clinically meaningful. The MID for the EQ-5D index score was in the range of 0.09-0.22.

Given the impact of psoriasis on the functional ability of patients the importance attached to assessing physical function in psoriasis patients, the results of the present study provide positive support for the use of a dermatology-specific health-related PRO measure, the DLQI, in the assessment of psoriasis and responses to treatment. In addition, the correlation of SF-36 and DLQI indicates that disease-related changes in the SF-36 are largely dependent on two specific domains, Bodily Pain and Social Functioning. It appears that the DLQI total score, as a single index score, adequately captures the functional and psychosocial impact of moderate to severe plaque psoriasis. Further, the DLQI does so in a way that is more responsive than the general health-related quality of life measures used to assess changes in patients' underlying clinical statuses.

In conclusion, the findings of this study highlight the importance of capturing PRO measures in clinical trials of moderate to severe plaque psoriasis. This analysis provides additional evidence supporting the psychometric qualities and responsiveness of the DLQI as a disease-specific measure of PROs in psoriasis. The DLQI MID was determined as ranging from 2.3-5.7 points. The study further shows that adalimumab was effective at improving DLQI, SF-36, and EQ-5D scores in patients with psoriasis.

### Example 9: Adalimumab Efficacy and Safety Compared with Methotrexate and Placebo in Patients with Moderate to Severe Psoriasis

The following study was the first to directly compare the clinical efficacy, safety, and tolerability of a biologic (adalimumab) vs. a traditional systemic agent (methotrexate or MTX) in the treatment of moderate to severe chronic plaque psoriasis. A published international consensus statement recommends that for patients with moderate to severe psoriasis, "equal consideration" should be given to traditional systemic therapy (e.g., MTX, phototherapy, or biologics) (Sterry et al. Brit J Dermatol. 2004;151(Suppl 69):3-17). Resistance to using biologics as first-line therapy has rested in part on the absence of data demonstrating superiority of a biologic to a traditional systemic agent in a direct, head-to-head study.

The objective of this study was to compare the clinical efficacy, safety, and tolerability of adalimumab vs. MTX and vs. placebo in the treatment of moderate to severe chronic plaque psoriasis

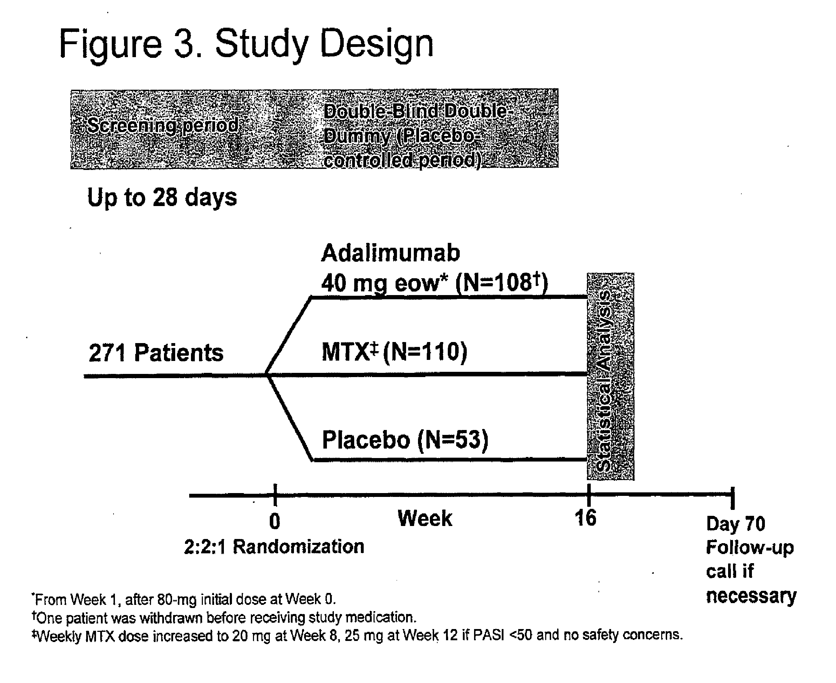

The study was a multi-center, 16-week, randomized controlled Phase III trial. The study design is shown in Figure 3. The main inclusion criteria included:
■ Clinical diagnosis of psoriasis for ≥1 year
■ Affected body surface area (BSA) ≥10%
■ Psoriasis Area and Severity Index (PASI) ≥10

The main exclusion criteria was previous use of MTX or systemic anti-TNF therapy. The study required a washout period, including the following:
■ 2 weeks for topical and phototherapy
■ 4 weeks for non-biologic systemic therapies
■ 12 weeks for biologic therapies

Efficacy was measured according to the following:
■ PASI, PGA (Physician's Global Assessment)
■ Primary endpoint was PASI 75 response rate at Week 16
Safety measures was examined using adverse events and laboratory parameters

The adalimumab dosage regimen (subcutaneous) included an 80 mg (two 40 mg injections) at Week 0 (baseline), followed by 40 mg every other week (eow) from Week 1 until Week 15. The MTX dosage regimen included the following:
- 7.5 mg at Weeks 0 and 1 (weekly MTX dose adjusted to ALT, AST, WBC count, platelet count and serum creatinine from Week 2 until Week 15, and reduced or withheld if deemed appropriate by the safety assessor);
- 10 mg at Weeks 2 and 3 (weekly MTX dose adjusted to ALT, AST, WBC count, platelet count and serum creatinine from Week 2 until Week 15, and reduced or withheld if deemed appropriate by the safety assessor);
- 15 mg from Weeks 4-15 (Weekly MTX dose increased to 20 mg at Week 8, 25 mg at Week 12 if PASI <50 and there were no safety concerns).

Analytical methods included the following:
■ Intention-to-treat analyses were performed for all randomized patients
■ Missing data were analyzed using non-responder imputation (NRI)
■ PASI 75 at Week 16 (primary endpoint) was analyzed using adjusted Cochran-Mantel-Haenszel test.

Baseline characteristics were similar across treatment groups and consistent with expectations for patients with moderate to severe chronic plaque psoriasis (Table 42)

**Table 42 Baseline Demographics and Clinical Characteristics**

| | Placebo (N=53) | MTX (N=110) | Adalimumab (N=108†) |
|---|---|---|---|
| Age (yrs) | 40.7 ± 11.43 | 41.6 ± 11.98 | 42.9 ± 12.57 |
| Duration of Psoriasis (mos) | 225.3 ± 104.2 | 226.5 ± 122.1 | 214.8 ± 121.1 |
| % Male | 66.0 | 66.4 | 64.8 |
| Body Weight (kg) | 82.6 ± 19.91 | 83.1 ± 17.50 | 81.7 ± 19.98 |
| % BSA | 28.4 ± 16.09 | 32.4 ± 20.60 | 33.6 ± 19.88 |
| PASI score | 19.2 ± 6.89 | 19.4 ± 7.39 | 20.2 ± 7.53 |
| % with Psoriatic Arthritis | 20.8 | 17.3 | 21.3 |

| | | | |
|---|---|---|---|
| *Mean values ± SD except % Male and % with Psoriatic Arthritis. †One patient was withdrawn before receiving any study medication. | | | |

PASI 75 response rate for adalimumab-treated patients was significantly superior to PASI 75 response rates for MTX-treated and placebo-treated patients at Week 16 (primary endpoint) (Table 43).

**Table 43. PASI 75 Response Rates**

| | | | | | |
|---|---|---|---|---|---|
| | | | | | |

| | | **n** | **Treatment** | **% Patients** | |
|---|---|---|---|---|---|
| | **Week 4** | 53 | Placebo | 4 | |
| | | 110 | Methotrexate | 3 | |
| | | 108 | Adalimumab | 23*† | |
| | **Week 8** | 53 | Placebo | 13 | |
| | | 110 | Methotrexate | 9 | |
| | | 108 | Adalimumab | 62‡ | |
| | **Week 12** | 53 | Placebo | 15 | |
| | | 110 | Methotrexate | 25 | |
| | | 108 | Adalimumab | 77‡ | |
| | **Week 16** | 53 | Placebo | 19 | |
| | | 110 | Methotrexate | 36 | |
| | | 108 | Adalimumab | 80‡ | |
| ***p=0.001, †p<0.001, both vs. placebo; ‡p<0.001 vs. MTX.** | | | | | |
| **ITT, patients with missing PASI scores were considered non-responders** | | | | | |

**Table 44. PASI 90 Response Rates**

| | | | | | |
|---|---|---|---|---|---|
| | | | | | |

| | | **n** | **Treatment** | **% Patients** | |
|---|---|---|---|---|---|
| | **Week 4** | 53 | Placebo | 0 | |
| | | 110 | Methotrexate | 1 | |
| | | 108 | Adalimumab | 7* | |
| | **Week 8** | 53 | Placebo | 4 | |
| | | 110 | Methotrexate | 3 | |
| | | 108 | Adalimumab | 27†‡ | |
| | **Week 12** | 53 | Placebo | 8 | |
| | | 110 | Methotrexate | 9 | |
| | | 108 | Adalimumab | 48†‡ | |
| | **Week 16** | 53 | Placebo | 11 | |
| | | 110 | Methotrexate | 14 | |
| | | 108 | Adalimumab | 52†‡ | |
| ***p<0.05 vs. MTX; †p<0.001 vs. placebo; ‡p<0.001 vs. MTX.** | | | | | |
| **ITT, patients with missing PASI scores were considered non-responders.** | | | | | |

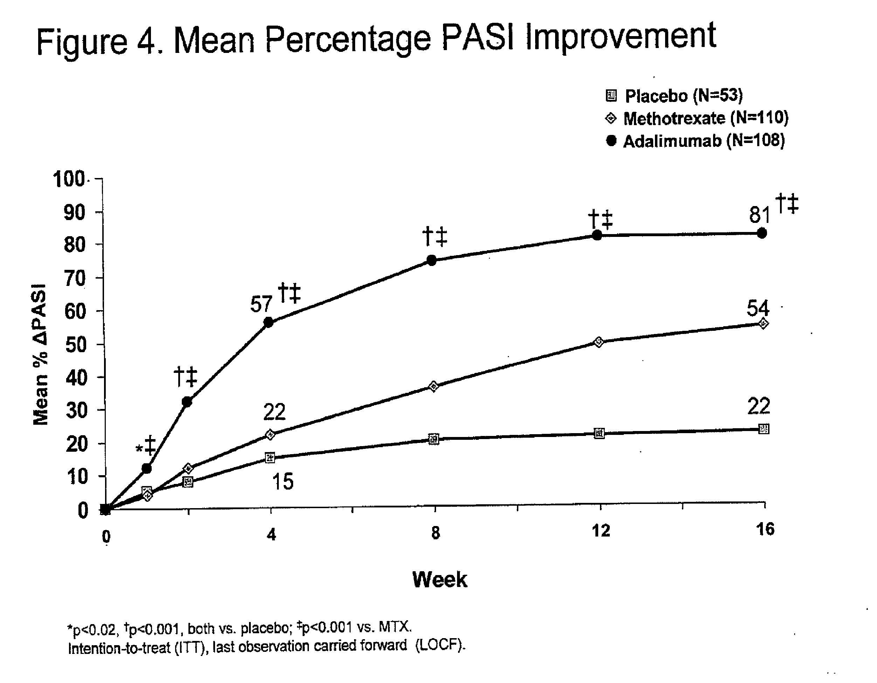

Adalimumab-treated patients achieved a significantly superior PASI 90 response rate vs. MTX-treated and placebo-treated patients at Week 16 (Table 44). Mean percentage PASI improvement for adalimumab-treated patients was rapid, with a mean percentage PASI improvement of 57% achieved at Week 4 (Figure 4). The percentage of adalimumab-treated patients who achieved PGA "Clear" or "Minimal" was similar to the percentage of adalimumab-treated patients who achieved PASI 75 or greater improvement (Table 45).

**Table 45. PGA "Clear" or "Minimal" Responses**

| | | | | | |
|---|---|---|---|---|---|
| | | | | | |

| | | **n** | **Treatment** | **% Patients** | |
|---|---|---|---|---|---|
| | **Week 4** | 53 | Placebo | 0 | |
| | | 110 | Methotrexate | 1 | |
| | | 108 | Adalimumab | 7*† | |
| | **Week 8** | 53 | Placebo | 4 | |
| | | 110 | Methotrexate | 3 | |
| | | 108 | Adalimumab | 27‡§ | |
| | **Week 12** | 53 | Placebo | 8 | |
| | | 110 | Methotrexate | 9 | |
| | | 108 | Adalimumab | 48‡§ | |
| | **Week 16** | 53 | Placebo | 11 | |
| | | 110 | Methotrexate | 14 | |
| | | 108 | Adalimumab | 521‡§ | |
| ***p<0.01 vs. placebo; †p<0.01 vs. MTX; ‡ p<0.001 vs. placebo; §p<0.001 vs. MTX.** | | | | | |
| **ITT, patients missing PGA scores were counted as not achieving PGA "Clear" or "Minimal".** | | | | | |

There were no significant differences in the incidences of adverse events reported for adalimumab-treated vs. MTX-treated and vs. placebo-treated patients (Tables 46 and 47). The most frequent adverse events were nasopharyngitis (21-28%) and headache (9-13%) (Table 47)

**Table 46. Adverse Events**

| Event, n (%) | Placebo (N=53) | MTX (N=110) | Adalimumab (N=107*) |
|---|---|---|---|
| Any AE | 42 (79) | 89 (81) | 79 (74) |
| AnySAE | 1 (2) | 1 (1) | 2 (2) |
| Any AE leading to discontinuation | 1 (2) | 6 (6) | 1 (1) |
| Any infectious AE | 23 (43) | 46 (42) | 51 (48) |
| Any infectious SAE | 0 (0) | 0 (0) | 0 (0) |

| | | | |
|---|---|---|---|
| *Does not include patient who was withdrawn from study before receiving any study medication. AE=adverse events. SAE=serious adverse events. SAE were a calculus of right uretero-pelvic junction in one placebo patient, hepatitis secondary to methotrexate in one MTX patient, pancreatitis in one adalimumab patient, and an increase in a benign ovarian cyst in one adalimumab patient. | | | |

**Table 47. Common Adverse Events in ≥5% of CHAMPION Patient Population**

| Event, n (%) | Placebo (N=53) | MTX (N=110) | Adalimumab (N=107*) |
|---|---|---|---|
| Nausea | 4 (8) | 8 (7) | 4 (4) |
| Nasopharyngitis | 11 (21) | 26 (24) | 30 (28) |
| Rhinitis | 4 (8) | 4 (4) | 3 (3) |
| Viral Infection | 1 (2) | 6 (6) | 0 (0) |
| Gamma-Glutamyltransferase Increased | 3 (6) | 0 (0) | 2 (2) |
| Arthralgia | 1 (2) | 5 (5) | 6 (6) |
| Headache | 5 (9) | 12 (11) | 14 (13) |
| Rhinorrhea | 3 (6) | 0 (0) | 3 (3) |
| Pruritus | 6 (11) | 2 (2) | 4 (4) |

| | | | |
|---|---|---|---|
| *One patient was withdrawn from study before receiving any study medication. | | | |

In conclusion, adalimumab demonstrated significantly superior efficacy in the treatment of moderate to severe psoriasis vs. MTX and vs. placebo - PASI 75 at Week 16: 80% for adalimumab vs. 36% for MTX and vs. 19% for placebo. Furthermore, response to adalimumab was rapid. Adalimumab was well-tolerated, with a safety profile in this psoriasis trial comparable to its profile in RA clinical trials. The results of CHAMPION suggest a paradigm shift in the treatment of moderate to severe psoriasis, from "equal consideration" to "first-line consideration" for biologics

### REFERENCES

1. de Arruda LH, De Moraes AP: The impact of psoriasis on quality of life. Br J Dermatol 2001, 144:33-36.
2. Finlay AY: Quality of life assessments in dermatology. Semin Cutan Med Surg 1998, 17:291-296.
3. Rapp SR, Cottrell CA, Leary MR: Social coping strategies associated with quality of life decrements among psoriasis patients. Br J Dermatol 2001, 145:610-616.
4. W ahl A: The impact of psoriasis on psychosocial life domains. A review. Scand J Caring Sci 1997, 11:243-249.
5. Weiss SC, Kimball AB, Liewehr DJ, Blauvelt A, Turner ML, Emanuel EJ: Quantifying the harmful effect of psoriasis on health-related quality of life. J Am Acad Dermatol 2002, 47:512-518.
6. Feldman SR, Menter A, Koo JY: Improved health-related quality of life following a randomized controlled trial of alefacept treatment in patients with chronic plaque psoriasis. Br JDermatol 2004, 150:317-326.
7. Finlay AY, Salek MS, Haney J: Intramuscular alefacept improves health-related quality of life in patients with chronic plaque psoriasis. Dermatology 2003, 206:307-315.
8. Gordon KB, Papp KA, Hamilton TK, Walicke PA, Dummer W, Li N, Bresnahan BW, Menter A: Efalizumab for patients with moderate to severe plaque psoriasis: a randomized controlled trial. JAMA 2003, 290:3073-3080.
9. Gottlieb AB, Matheson RT, Lowe N, Krueger GG, Kang S, Goffe BS, Gaspari AA, Ling M, Weinstein GD, Nayak A, Gordon KB, Zitnik R: A randomized trial of etanercept as monotherapy for psoriasis. Arch Dermatol 2003, 139:1627-1632; discussion 1632.
10. Leonardi CL, Powers JL, Matheson RT, Goffe BS, Zitnik R, Wang A, Gottlieb AB: Etanercept as monotherapy in patients with psoriasis. N Engl J Med 2003, 349:2014-2022.
11. Menter A, Kosinski M, Bresnahan BW, Papp KA, Ware JE, Jr.: Impact of efalizumab on psoriasis-specific patient-reported outcomes. Results from three randomized, placebo-controlled clinical trials of moderate to severe plaque psoriasis. J Drugs Dermatol 2004, 3:27-38.
12. Kirby B, Richards HL, Woo P, Hindle E, Main CJ, Griffiths CE: Physical and psychologic measures are necessary to assess overall psoriasis severity. J Am Acad Dermatol 2001, 45:72-76.
13. Krueger GG, Feldman SR, Camisa C, Duvic M, Elder JT, Gottlieb AB, Koo J, Krueger JG, Lebwohl M, Lowe N, Menter A, Morison WL, Prystowsky JH, Shupack JL, Taylor JR, Weinstein GD, Barton TL, Rolstad T, Day RM: Two considerations for patients with psoriasis and their clinicians: what defines mild, moderate, and severe psoriasis? What constitutes a clinically significant improvement when treating psoriasis? J Am Acad Dermatol 2000, 43:281-285.
14. Smith CH, Anstey AV, Barker JN, Burden AD, Chalmers RJ, Chandler D, Finlay AY, Grifitths CE, Jackson K, McHugh NJ, McKenna KE, Reynolds NJ, Ormerod AD: British Association of Dermatologists guidelines for use of biological interventions in psoriasis 2005. Br J Dermatol 2005, 153:486-497.
15. Finlay AY, Khan GK: Dermatology Life Quality Index (DLQI)--a simple practical measure for routine clinical use. Clin Exp Dermatol 1994, 19:210-216.
16. Ware JE, Snow KK, Kosinski MK, Gandek B: SF-36 Health Survey: Manual and Interpretation Guide. Boston: The Health Institute, New England Medical Center; 1993.
17. EuroQol Group: EuroQol--a new facility for the measurement of health-related quality of life. Health Policy 1990, 16:199-208.
18. Kind P: The EuroQol Instrument: An index of health-related quality of life. In Quality of Life and Pharmacoeconomics in Clinical Trials. 2nd edition. Edited by Spilker B. Philadelphia: Lippincott-Raven Publishers; 1996: 191-201
19. Food and Drug Administration: Guidance for industry. Patient-reported outcome measures: use in medical product development to support labeling claims. Draft. February 2006.
20. Jaeschke R, Singer J, Guyatt GH. Measurement of health status. Ascertaining the minimal clinically important difference. Control Clin Trials. 1989;10:407-415.
21. Wyrwich KW, Bullinger M, Aaronson N, Hays RD, Patrick DL, Symonds T, Sloan JA. Estimating clinically significant differences in quality of life outcomes. Qual Life Res 2005; 14: 285-295.
22. Sloan JA, Dueck A, Erickson PA, Guess H, Revicki DA, Santanello NC. Analysis and interpretation of patient reported outcome results. Presented at the FDA Guidance on Patient Reported Outcomes: Discussion, Dissemination and Operationalization, Chantilly, Virgina, February 2006.
23. Gordon KB, Langley RG, Leonardi C, Toth D, Menter MA, Kang S, Heffernan M, Miller B, Hamlin R, Lim L, Zhong J, Hoffman R, Okun MM: Clinical response to adalimumab treatment in patients with moderate to severe psoriasis patients: Double-blind, randomized controlled trial and open-label extension study. J Am Acad Dermatol, in proqress.
24. Ashcroft DM, Wan Po AL, Williams HC, Griffiths CE: Clinical measures of disease severity and outcome in psoriasis: a critical appraisal of their quality. Br J Dermatol 1999, 141:185-191.
25. Fredriksson T, Pettersson U: Severe psoriasis-oral therapy with a new retinoid. Dermatologica 1978, 157:238-244.
26. Badia X, Mascaro JM, Lozano R: Measuring health-related quality of life in patients with mild to moderate eczema and psoriasis: clinical validity, reliability and sensitivity to change of the DLQI. The Cavide Research Group. Br J Dermatol 1999, 141:698-702.
27. Lewis V, Finlay AY: 10 years experience of the Dermatology Life Quality Index (DLQI). J Investig Dermatol Symp Proc 2004, 9:169-180.
28. Shikiar R, Bresnahan BW, Stone SP, Thompson C, Koo J, Revicki DA: Validity and reliability of patient reported outcomes used in Psoriasis: results from two randomized clinical trials. Health Qual Life Outcomes 2003, 1:53.
29. Ware JE, Kosinski MK, Keller SD: SF-36 Physical and Mental Health Summary Scales: A User's Manual. Boston: The Health Institute, New England Medical Center; 1994.
30. Wahl A, Loge JH, Wiklund I, Hanestad BR: The burden of psoriasis: a study concerning health-related quality of life among Norwegian adult patients with psoriasis compared with general population norms. J Am Acad Dermatol 2000, 43:803-808.
31. Wyrwich KW, Nienaber NA, Tierney WM, Wolinsky FD: Linking clinical relevance and statistical significance in evaluating intra-individual changes in health-related quality of life. Med Care 1999, 37:469-478.
32. Wyrwich KW, Tierney WM, Wolinsky FD: Further evidence supporting an SEM-based criterion for identifying meaningful intra-individual changes in health-related quality of life. J Clin Epidemiol 1999, 52:861-873.
33. Norman GR, Sloan JA, Wyrwich KW: Interpretation of changes in health-related quality of life: the remarkable universality of half a standard deviation. Med Care 2003, 41:582-592.
34. Beaton DE, Boers M, Wells GA: Many faces of the minimal clinically important difference (MCID): a literature review and directions for future research. Curr Opin Rheumatol 2002, 14:109-114.
35. Hays RD, Farivar SS, Liu H: Approaches and recommendations for estimating minimally important differences for health-related quality of life measures. Journal of Chronic Obstructive Pulmonary Disease 2005, 2:63-67.
36. Khilji FA, Gonzalez M, Finlay AY: Clinical meaning of change in Dermatology Life Quality Index. Br J Dermatol 2002, 147:50 (abstract).
37. Kosinski M, Zhao SZ, Dedhiya S, Osterhaus JT, Ware JE, Jr.: Determining minimally important changes in generic and disease-specific health-realted quality of life questionnaires in clinical trials of rheumatoid arthritis. Arthritis & Rheumatism 2000, 43:1478-1487.
38. Chren MM, Lasek RJ, Quinn LM, Mostow EN, Zyzanski SJ: Skindex, a quality-of-life measure for patients with skin disease: reliability, validity, and responsiveness. J Invest Dermatol 1996, 107:707-713.
39. McKenna SP, Lebwohl M, Kahler KN: Development of the US PSORIQoL: a psoriasis-specific measure of quality of life. Int J Dermatol 2005, 44:462-469.

### Example 10: Efficacy and Safety of a 120-Week Open-Label Extension Study in Patients With Moderate to Severe Chronic Plaque Psoriasis

Psoriasis is a chronic, inflammatory proliferative disease of the skin that affects 1-3% of general population (Greaves and Weinstein N Engl J Med 1995; 332:581-8). Treatment of moderate to severe psoriasis with systemic agents such as methotrexate or cyclosporine can be limited by lack of efficacy or precluded by dose dependent side effects, and ultraviolet light therapy is often inconvenient

The objective of the following study was to determine the long-term (120 weeks) efficacy and safety of adalimumab in patients with moderate to severe plaque psoriasis.

The study includes a Phase III open-label extension (OLE) study of adalimumab in which patients with moderate to severe chronic plaque psoriasis (PASI≥12, PGA of at least moderate, BSA≥10%) who completed the lead-in 60-week Phase II adalimumab clinical trials could enroll. This example summarizes results in patients treated with adalimumab eow for up to 120 weeks (up to 60 weeks in the preceding Phase II studies and for up to 72 weeks in the Phase III extension trial). The 120-week efficacy and safety of adalimumab 40 mg every other week (eow) was evaluated in an open-label extension trial (III) that followed a 48-week extension trial (II.B), which was conducted at 18 sites. Prior to the II.B study, patients were enrolled in a 12-week, double-blind, placebo-controlled trial (II.A). Inclusion criteria included: moderate to severe chronic plaque psoriasis: ≥1 year and affected BSA: ≥5%. Exclusion criteria included prior TNF-antagonist therapy. As observed analysis was conducted to assess efficacy and safety over the 120-week treatment period.

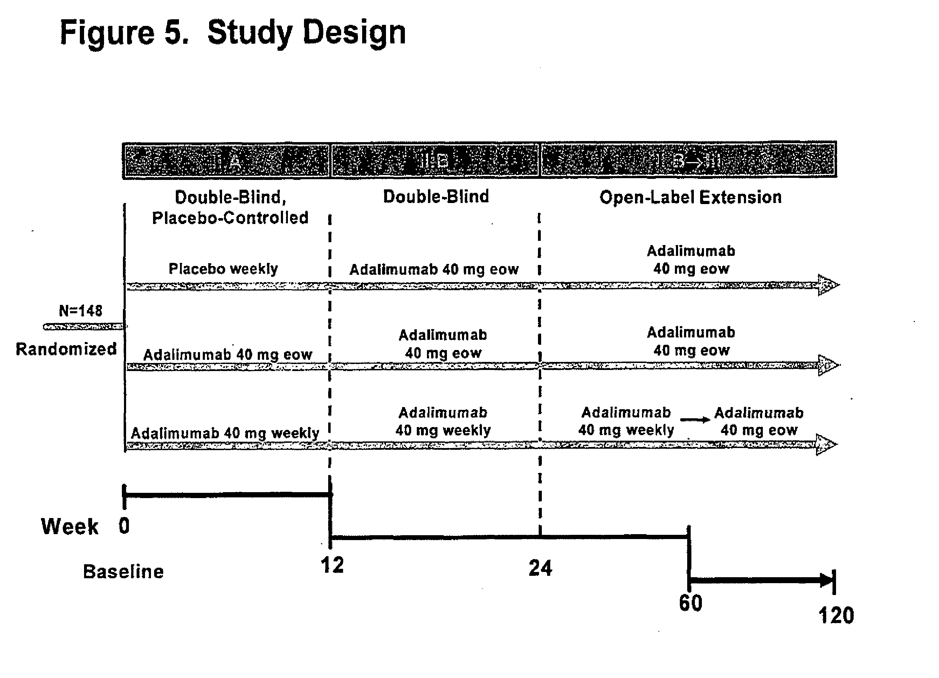

Patients who had completed the lead-in studies could enroll in the open-label extension (OLE) trial, during which they received adalimumab 40 mg every other week (eow). After 24 weeks of OL therapy, patients with an inadequate response (<PASI 50 relative to baseline of II.A - see Study Design of Figure 5) could increase to adalimumab 40 mg weekly. PASI responses and PGA scores were analyzed as observed. Patients who underwent dose escalation, and patients randomized at Week 0 to adalimumab 40 mg weekly are not included in this analysis. Thus, patients whose dosages increased were counted as non-responders from the time of dosage escalation. Adverse events were collected throughout the 120-week period.

A total of 92 patients were enrolled in Study II.A (see Figure 5) and received at least one dose of adalimumab 40 mg eow during the 120-week observation period. Baseline demographics and clinical characteristics for patients randomized to placebo or to adalimumab 40 mg eow in Study II.A were typical for patients with moderate to severe chronic plaque psoriasis (Table 48)

**Table 48. Baseline Demographics and Clinical Characteristics**

| | Adalimumab 40 mg eow (n=92) |
|---|---|
| Age (years) | 45.1 |
| Duration of Ps (years) | 19.7 |
| % Male | 70.7 |
| % Caucasian | 90.2 |
| Body Weight (kg) | 94.7 |
| % BSA | 28.6 |
| PASI Score | 16.3 |
| % with PsA | 29.3 |

| | |
|---|---|
| Mean values except % Male, % Caucasian, and % with Ps. | |

Fifty-three percent (49/92) of patients were evaluable for efficacy and safety after more than 2 years of continuous adalimumab 40 mg eow dosing (Table 49). Week 0 corresponds to the beginning of adalimumab therapy

**Table 49. Number of Evaluable Patients Over 120 Weeks**

| Time | No. of Evaluable Patients |
|---|---|
| Week 0 | 92 |
| Week 4 | 92 |
| Week 8 | 91 |
| Week 12 | 89 |
| Week 16 | 79 |
| Week 24 | 75 |
| Week 36 | 64 |
| Week 48 | 60 |
| Week 60 | 57 |
| Week 72 | 52 |
| Week 84 | 52 |
| Week 96 | 53 |
| Week 108 | 50 |
| Week 120 | 49 |

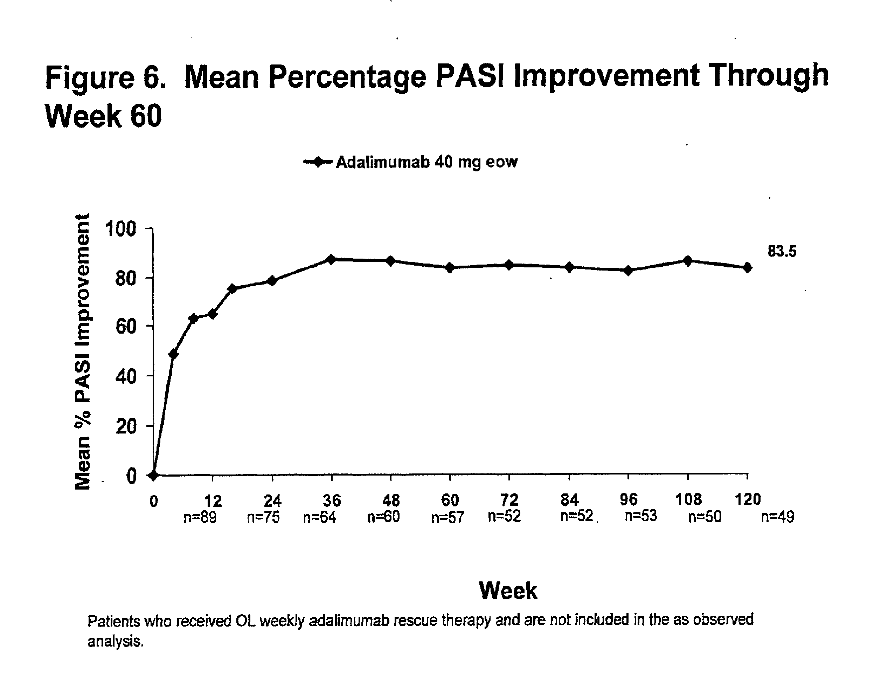

Among patients who continued to receive adalimumab 40 mg eow, PASI responses peaked at Week 48 and were generally maintained up to Week 120 of adalimumab therapy (Table 50). Among patients who continued to receive adalimumab 40 mg eow, mean percentage PASI improvement peaked at Week 36 and was generally maintained to Week 120 (Figure 6)

**Table 50. PASI Response Rates Up To Week 120**

| | | | | |
|---|---|---|---|---|
| | | | | |

| | | **PASI response** | **% Patients** | |
|---|---|---|---|---|
| | **Week 12** | PASI 50 | 78.7 | |
| | | PASI 75 | 56.2 | |
| | | PASI 90 | 29.2 | |
| | | PASI 100 | 11.2 | |
| | **Week 24** | PASI 50 | 86.7 | |
| | | PASI 75 | 74.7 | |
| | | PASI 90 | 52 | |
| | | PASI 100 | 20 | |
| | **Week 48** | PASI 50 | 96.7 | |
| | | PASI 75 | 81.7 | |
| | | PASI 90 | 61.7 | |
| | | PASI 100 | 31.7 | |
| | **Week 72** | PASI 50 | 96.2 | |
| | | PASI 75 | 73.1 | |
| | | PASI 90 | 53.8 | |
| | | PASI 100 | 25 | |
| | **Week 96** | PASI 50 | 92.5 | |
| | | PASI 75 | 75.5 | |
| | | PASI 90 | 52.8 | |
| | | PASI 100 | 24.5 | |
| | **Week 120** | PASI 50 | 93.9 | |
| | | PASI 75 | 77.6 | |
| | | PASI 90 | 53.1 | |
| | | PASI 100 | 28.6 | |
| | **Patients who received OL weekly adalimumab rescue therapy and are not included in the as observed analysis.** | | | |

The percentage of patients who reported malignant, serious, and serious infectious adverse events were low and stable over the surveillance period (Table 51). The malignant adverse event reported was a squamous cell carcinoma of the neck in an ex-smoker who had been noted to have lymphadenopathy at screening. The serious adverse events reported were squamous cell carcinoma (case described above), fall, and dyspepsia. No cases of tuberculosis, demyelinating disorder, lupus-like syndrome, lymphoma, or congestive heart failure were noted. The percentage of patients who reported malignant, serious, and serious infectious adverse events were low and stable over the surveillance period. The malignant adverse event reported was a squamous cell carcinoma of the neck in an ex-smoker who had been noted to have lymphadenopathy at screening. The serious adverse events reported were squamous cell carcinoma (case described above), fall, and dyspepsia. No cases of tuberculosis, demyelinating disorder, lupus-like syndrome, lymphoma, or congestive heart failure were noted.

**Table 51. Patients Experiencing Adverse Events over 120 Weeks**

| | Week 0-24 n=92 n (%) | Week 25-48 n=78 n (%) | Week 49-72 n=62 n (%) | Week 73-96 n=55 n (%) | Week 97-120 n=53 n (%) |
|---|---|---|---|---|---|
| Any AE | 69 (75.0) | 33 (42.3) | 28 (45.2) | 27 (49.1) | 2 (47.2) |
| Serious AEs | 2 (2.2) | 0 (0.0) | 0 (0.0) | 0 (0.0) | 1 (1.9) |
| Infectious AEs | 28 (30.4) | 22 (28.2) | 17 (27.4) | 15 (27.3) | 7 (13.2) |
| Serious Infectious AEs | 0 (0.0) | 0 (0.0) | 0 (0.0) | 0 (0.0) | 0 (0.0) |
| Malignant AEs | 1 (1.1) | 0 (0.0) | 0 (0.0) | 0 (0.0) | 0 (0.0) |
| AEs leading to withdrawal | 4 (4.3) | 0 (0.0) | 0 (0.0) | 0 (0.0) | 0 (0.0) |

Adverse event profile of adalimumab was stable over the 120-week treatment period (Table 52). Blood tests were performed under non-fasting conditions

**Table 52. Common Adverse Events >5% in Any 24-Week Period**

| Adverse Events | Week 0-24 n=92 n (%) | Week 25-48 n=78 n (%) | Week 49-72 n=62 n (%) | Week 73-96 n=55 n (%) | Week 97-120 n=53 n (%) |
|---|---|---|---|---|---|
| Nasopharyngitis | 9 (9.8) | 5 (6.4) | 1 (1.6) | 3 (5.5) | 2 (3.8) |
| Increased triglycerides | 7 (7.6) | 1 (1.3) | 1 (1.6) | 1 (1.8) | 0 (0.0) |
| Headache | 7 (7.6) | 0 (0.0) | 0 (0.0) | 0 (0.0) | 0 (0.0) |
| Upper respiratory tract infection | 6 (6.5) | 3 (3.8) | 2 (3.2) | 2 (3.6) | 0 (0.0) |
| Diarrhea | 5 (5.4) | 1 (1.3) | 1 (1.6) | 0 (0.0) | 3 (5.7) |
| Hypercholesterolemia | 4 (4.3) | 0 (0.0) | 1 (1.6) | 3 (5.5) | 1 (1.9) |
| Sinusitis | 1 (1.1) | 1 (1.3) | 1 (1.6) | 3 (5.5) | 1 (1.9) |

In conclusion, patients with moderate to severe psoriasis achieved sustained efficacy up to Week 120 of treatment with adalimumab. The types of adverse events in this study were similar to those previously reported in adalimumab rheumatoid arthritis and psoriatic arthritis trials. The percentage of patients diagnosed with adverse events appeared stable over the 120-week observation period

### Example 11: Short- and Long-Term Efficacy and Safety of Adalimumab in a Pivotal Phase III Study in Adult Patients With Moderate to Severe Chronic Plaque Psoriasis

Psoriasis is a chronic, immune-mediated, inflammatory skin disease that affects 1-3% of general population (Greaves and Weinstein, N Engl J Med. 1995; 332:581-8). Treatment of moderate to severe psoriasis with systemic agents such as cyclosporine or methotrexate can be limited by lack of efficacy or precluded by dose-dependent adverse events. In addition, ultraviolet light therapy is often inconvenient.

This example describes a Phase III trial of adalimumab conducted at 81 sites, in adult patients with moderate to severe chronic plaque psoriasis. The objective of the study was to compare the efficacy and safety of adalimumab with placebo during an initial placebo-controlled, double-blind trial period, and to determine if improvement with adalimumab is sustained during open-label treatment. This study also sought to determine whether discontinuation of adalimumab therapy is associated with a loss of adequate clinical response, and to determine the adverse event (AE) profile of adalimumab-treated patients over a 52-week period.

The main inclusion criteria for this study included a clinical diagnosis of psoriasis for ≥6 months and affected body surface area (BSA) ≥10% and PASI ≥12. The main exclusion criteria included prior use of systemic or biologic anti-TNF therapy. The washout period included two weeks for topical agents and UVB, 4 weeks for PUVA and non-biologic systemic therapies, and 12 weeks for all biologic therapies. The two co-primary endpoints were:
- Percentage of patients achieving PASI ≥75 at Week 16
- Percentage of patients, among Week 33 PASI ≥75 responders, losing an adequate response after Week 33, characterized by:
   - PASI<50 (relative to baseline) and
   - At least a 6 point increase in PASI

Safety measures included the following:
- AEs and laboratory parameters
- Percentages of patients and rates (events/patient-year) of AEs for adalimumab in Period A were compared with those of placebo in period A, and with those of adalimumab-treated patients over the entire 52-week study period

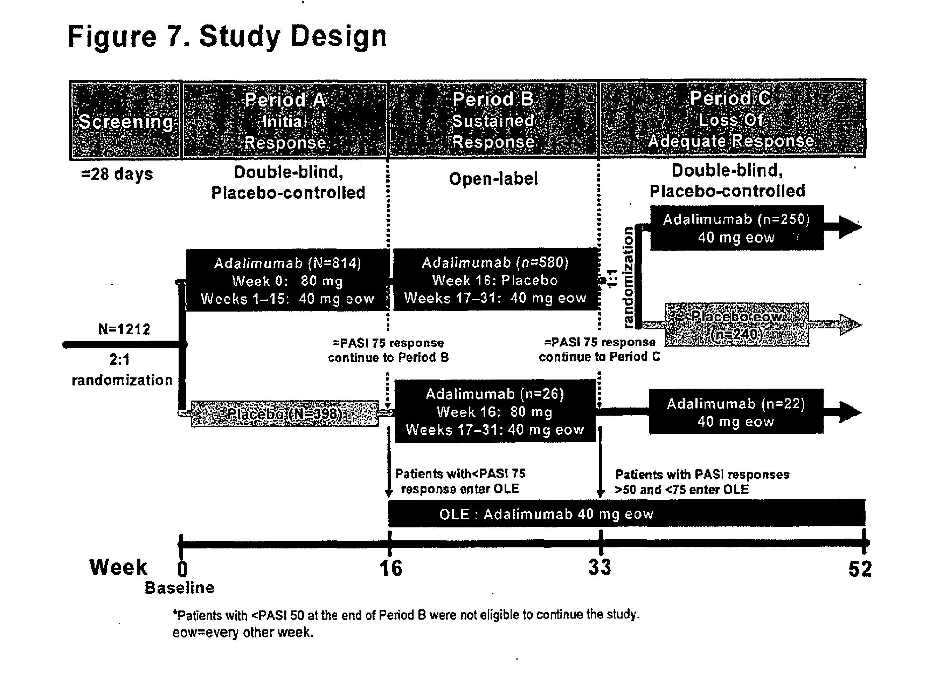

As described in the study design of Figure 7, there were three treatment periods, Periods A, B, and C.

Period A was double-blind, placebo-controlled, and lasted from weeks 0-16. During Period A, patients were randomized 2:1 to adalimumab or placebo. Patients in the adalimumab arm received: Week 0: 80 mg subcutaneously (sc) and Weeks 1-15: 40 mg every other week (eow). At Week 16 patients who achieved ≥PASI 75 improvement continued into Period B, while those patients having <PASI 75 improvement entered the open label extension (OLE) study (Figure 7).

Period B was open-label and lasted from Weeks 17-33. Adalimumab was administered at 40 mg every other week (eow) subcutaneously (sc). Patients who achieved ≥PASI 75 response at Week 33 entered Period C. Those who had a PASI 50-<75 response at the end of Period B entered the OLE, while those with a <PASI 50 response discontinued the study.

Period C was a double-blind, placebo-controlled which was held from weeks 34-52. Patients randomized to adalimumab treatment in Period A and who achieved ≥PASI 75 at Week 33 were re-randomized 1:1 to either continue adalimumab 40 mg eow or receive placebo treatment. Patients randomized to placebo treatment in Period A and who received adalimumab in Period B, continued adalimumab treatment in Period C if they achieved a ≥PASI 75 in Period B

Various analytical methods were used in the study. Intention-to-treat (ITT) analyses were performed for all randomized patients in Periods A and C. Binary variables were analyzed with non-responder imputation (NRI) in Period A. In order to assess efficacy beyond Week 16, efficacy outcomes for all patients who had been randomized to adalimumab at Week 0 were assessed in Period B and in the OLE. Week 24 results from NRI analysis include pooled efficacy outcomes from OLE and Period B. After Week 24, non-overlapping OLE and Period B study visits prevent complete pooling of efficacy outcomes. Continuous variables were analyzed as observed during Period B

Baseline characteristics were similar across treatment groups and consistent with expectations for patients with moderate to severe chronic plaque psoriasis (Table 53)

**Table 53. Baseline Demographics and Clinical Characteristics**

| | Placebo (N=398) | Adalimumab (N=814) |
|---|---|---|
| Age (years) | 45.4 ± 13.4 | 44.1 ± 13.2 |
| % Male | 64.6 | 67.1 |
| % Caucasian | 90.2 | 91.2 |
| Duration of Ps (years) | 18.4 ± 11.94 | 18.1 ± 11.91 |
| Body Weight (kg) | 94.1 ± 23.1 | 92.3 ± 22.9 |
| BSA (%) | 25.6 ± 14.76 | 25.8 ± 15.51 |
| PASI Score | 18.8 ± 7.09 | 19.0 ± 7.08 |
| % with PsA | 28.4 | 27.5 |

| | | |
|---|---|---|
| Mean values ± SD except % Male, % Caucasian and % with psoriatic arthritis. | | |

Adalimumab-treated patients achieved rapid, and significantly superior, PASI 75 response rates vs. placebo-treated patients from Week 4, the first time point evaluated, and every visit through Week 16 (Table 54). PASI 75 response rates were sustained through open-label treatment to Week 24. Week 24 results include both Period B and OLE efficacy outcomes. Pooling of efficacy outcomes after this time point was not possible because of disparate Period B and OLE study visit schedules (Table 54).

**Table 54. PASI 75 Response Rates at Weeks 0-24**

| | | | | | |
|---|---|---|---|---|---|
| | | | | | |

| | | **n** | **Treatment** | **% Patients** | |
|---|---|---|---|---|---|
| | **Week 4** | 398 | Placebo | 1.3 | |
| | | 814 | Adalimumab | 18.9* | |
| | **Week 8** | 398 | Placebo | 3.0 | |
| | | 814 | Adalimumab | 54.1* | |
| | **Week 12** | 398 | Placebo | 4.8 | |
| | | 814 | Adalimumab | 67.7* | |
| | **Week 16** | 398 | Placebo | 6.5 | |
| | | 814 | Adalimumab | 71.0* | |
| | **Week 24** | 398 | Placebo | - | |
| | | 814 | Adalimumab | 71.0* | |
| ***p<0.001, adalimumab vs. placebo.** | | | | | |
| **ITT; Patients with missing PASI scores were considered non-responders. Week 24 results represent pooling of efficacy outcomes from Period B and OLE.** | | | | | |
| **Weeks 4 to 16 were double-blind, placebo-controlled. Week 24 was open-label.** | | | | | |

**Table 55. PASI 100 Response Rates at Weeks 0-24**

| | | | | | |
|---|---|---|---|---|---|
| | | | | | |

| | | **n** | **Treatment** | **% Patients** | |
|---|---|---|---|---|---|
| | **Week 4** | 398 | Placebo | 0.3 | |
| | | 814 | Adalimumab | 0.9 | |
| | **Week 8** | 398 | Placebo | 0.3 | |
| | | 814 | Adalimumab | 7.1* | |
| | **Week 12** | 398 | Placebo | 0.3 | |
| | | 814 | Adalimumab | 14.4* | |
| | **Week 16** | 398 | Placebo | 0.8 | |
| | | 814 | Adalimumab | 20* | |
| | **Week 24** | 398 | Placebo | - | |
| | | 814 | Adalimumab | 22.5* | |
| ***p<0.001, adalimumab vs. placebo.** | | | | | |
| **ITT; Patients with missing PASI scores were considered non-responders. Week 24 results represent pooling of efficacy outcomes from Period B and OLE.** | | | | | |
| **Weeks 4 to 16 were double-blind, placebo-controlled. Week 24 was open-label.** | | | | | |

PASI 100 responses were sustained from Week 16 to Week 24 during open-label adalimumab treatment (Table 55).

Only patients who achieved ≥PASI 75 responses entered Period B. For patients who entered Period B, mean percentage PASI improvement achieved at Week 16 was maintained throughout Period B (Table 56).

**Table 56. Mean Percentage PASI Improvement During Period B in Adalimumab-Treated Patients***

| | | | | |
|---|---|---|---|---|
| | | | | |

| | | **n** | **% PASI improvement** | |
|---|---|---|---|---|
| | **Week 16** | 574 | 91.9 | |
| | **Week 24** | 577 | 91.2 | |
| | **Week 33** | 551 | 89.3 | |
| *** Relative to baseline for patients who were randomized to adalimumab at Week 0 and entered Period B. As observed.** | | | | |

Clinical characteristics were similar between patients re-randomized to adalimumab and placebo in Period C. The proportion of patients losing an adequate response during Period C was significantly lower for patients re-randomized to continue adalimumab therapy (Table 57).

**Table 57. Loss of Adequate Response by Week 52**

| | | | | |
|---|---|---|---|---|
| | | | | |

| | **Treatment** | **n** | **% Patients** | |
|---|---|---|---|---|
| | Placebo in period C | 240 | 28.4 | |
| | Adalimuma b in period C | 250 | 4.9* | |
| | | | | |
| ***p<0,001, adalimumab vs. placebo.** | | | | |
| **Primary endpoint: Proportion of patients experiencing an event (loss of adequate response) on or before Week 52.** | | | | |
| **An event was defined as the more stringent of** | | | | |
| **a) a PASI <50 (relative to baseline) or** | | | | |
| **b) a ≥6 point increase in PASI from weeks 34 to 52 relative to week 33** | | | | |
| **ITT; Missing Week 52 PASI assessment imputed as an event because of lack of efficacy or study drug toxicity.** | | | | |

Adalimumab Treatment Group comprised all patients who received at least one dose of adalimumab, with adverse event rate for this group calculated for the entire 52 week study period (See table 58). Percentages of patients and rates of AEs and of infectious AEs were higher among adalimumab- vs. placebo-treated patients. The percentage of patients with serious AEs was comparable among placebo- and adalimumab-treated patients in Period A (1.8%). The rates of serious AEs in the adalimumab-treated patients of Period A were consistent with serious AE rates throughout the study. The vast majority of Infectious AEs in Period A were not severe. Severe infectious AE rates were comparable between patients receiving adalimumab and those receiving placebo. Percentages of patients and rates of AEs leading to withdrawal were lower for adalimumab-treated patients vs. placebo treated patients

**Table 58. Safety Results**

| | Percentage of AEs During Period A | | Rates of AEs during Period A and for the Adalimumab Treatment Group | | |
|---|---|---|---|---|---|
| | Period A* Placebo (N=398) (%) | Period A* Adalimumab (N=814) (%) | Period A* Placebo (120.7 PYs) (Event/PY) | Period A* Adalimumab (250.2 PYs) (Event/PY) | Adalimumab Treatment Group† (540.5 PYs) (Event/PY) |
| Any AE | 55.5 | 62.2‡ | 4.127 | 4.616 | 3.991 |
| Serious AEs | 1.8 | 1.8 | 0.058 | 0.068 | 0.061 |
| Serious Infectious AEs | 1.0 | 0.6 | 0.033 | 0.028 | 0.022 |
| Infectious AEs | 22.4 | 28.9‡ | 0.878 | 1.259 | 1.203 |
| Severe Infectious AEs | 0.8 | 0.7 | 0.025 | 0.036 | 0.030 |
| AEs leading to withdrawal | 2.0 | 1.7 | 0.124 | 0.072 | 0.078 |

| | | | | | |
|---|---|---|---|---|---|
| *Includes safety data up to 70 days after last dose for patients not continuing into Period B. †Includes safety data up to 70 days after last dose of adalimumab for all patients who received at least one dose of adalimumab in REVEAL. Adverse events experienced by patients re-randomized to placebo in Period C (within 70 days after last dose of adalimumab in Period B) are included in this group. ‡p<0.05, adalimumab vs. placebo. | | | | | |

From Table 59 it can be seen that no lymphomas were diagnosed in this study. The percentages of patients with non-melanoma skin cancers and the percentages of patients with all other types of malignancies (excluding non-melanoma skin cancers and lymphomas) were comparable among placebo- and adalimumab-treated patients in Period A, and for Period A vs. the entire 52-week study. One case of oral candidiasis (opportunistic infection) was diagnosed, and one case of presumptive tuberculosis (-ve AFB and -ve culture, with clinical course suggestive of tuberculosis) was diagnosed in a patient who was PPD+ve at baseline and who was non-compliant with INH prophylaxis. No cases of rebound were noted among patients re-randomized to placebo in Period C.

**Table 59. Adverse Events of Interest**

| | Percentage of AEs during Period A | | Rates of AEs during Period A and for the Adalimumab Treatment Group | | |
|---|---|---|---|---|---|
| | Period A* Placebo (N=398) | Period A* Adalimumab (N=814) | Period A* Placebo (120.7 PY) | Period A* Adalimumab (250.2 PY) | Adalimumab Treatment Group† (540.5 PY) |
| | % | % | Event/PY | Event/PY | Event/PY |
| Tuberculosis (TB) | 0 | 0 | 0 | 0 | 0.002 |
| Opportunistic Infections, excluding TB | 0 | 0 | 0 | 0 | 0.002 |
| Congestive Heart Failure | 0 | 1 | 0 | 0.004 | 0.002 |
| Allergic Reaction | 0 | 1 | 0 | 0.004 | 0.002 |
| Injection Site Reaction | 5.3 | 6.9 | 0.215 | 0.276 | 0.017 |
| Malignancies, excluding NMSC + lymphoma | 0.3 | 0.2 | 0.008 | 0.008 | 0.004 |
| Non-melanoma skin cancers | 0.3 | 0.5 | 0.008 | 0.016 | 0.013 |
| Lymphoma | 0 | 0 | 0 | 0 | 0 |
| Lupus-like Syndrome | 0 | 0 | 0 | 0 | 0 |
| Demyelinating Disorder | 0 | 0 | 0 | 0 | 0 |

| | | | | | |
|---|---|---|---|---|---|
| *Includes safety data up to 70 days after last dose for patients not continuing into Period B. †Includes safety data up to 70 days after last dose of adalimumab for all patients who received at least one dose of adalimumab in REVEAL. Adverse events experienced by patients re-randomized to placebo in Period C (within 70 days after last dose of adalimumab in Period B) are included in this group. | | | | | |

From Table 60 it can be seen that during Period A, infectious AEs experienced by more than 2% of adalimumab-treated patient were upper respiratory tract infections (7.2%), nasopharyngitis (5.3%), and sinusitis (2.7%). The most common adverse events reported in the Adalimumab Treatment Group were upper respiratory tract infections, nasopharyngitis, and headache.

**Table 60. Common Adverse Events >5%: Period A**

| | Placebo (N=398) n (%) | Adalimumab (N=814) n (%) |
|---|---|---|
| Upper Respiratory Tract Infection | 14 (3.5) | 59 (7.2) |
| Nasopharyngitis | 26 (6.5) | 45 (5.3) |

| | | |
|---|---|---|
| Includes safety data up to 70 days after last dose for patients not continuing into period B. | | |

In conclusion, treatment with adalimumab 40 mg eow is efficacious for patients with moderate to severe psoriasis. Efficacy is sustained during open-label adalimumab treatment. Discontinuation of adalimumab is associated with loss of adequate response Percentages of serious AEs, serious infectious AEs, and malignancies were comparable between placebo- and adalimumab-treated patients during Period A. Rates of serious AEs, serious infectious AEs, and malignancies were low over the 52-week duration of the study.

### Example 12: Efficacy and Safety of Adalimumab Treatment in Patients with Moderate to Severe Psoriasis Patients: A Double-Blind, Randomized Clinical Trial

It is necessary to assess the efficacy and safety of adalimumab therapy for patients with moderate to severe plaque psoriasis and evaluate the duration of treatment response after withdrawal from or dosage reduction of adalimumab therapy. Accordingly, the objective of this clinical trial was to investigate the time to relapse after either withdrawal of adalimumab or continued adalimumab therapy (but at a dosage lower [40 mg eow] than initially used) in patients with moderate to severe chronic plaque psoriasis who had achieved a Psoriasis Area and Severity Index (PASI) response of at least 50% after 12 weeks of open-label 40-mg weekly therapy.

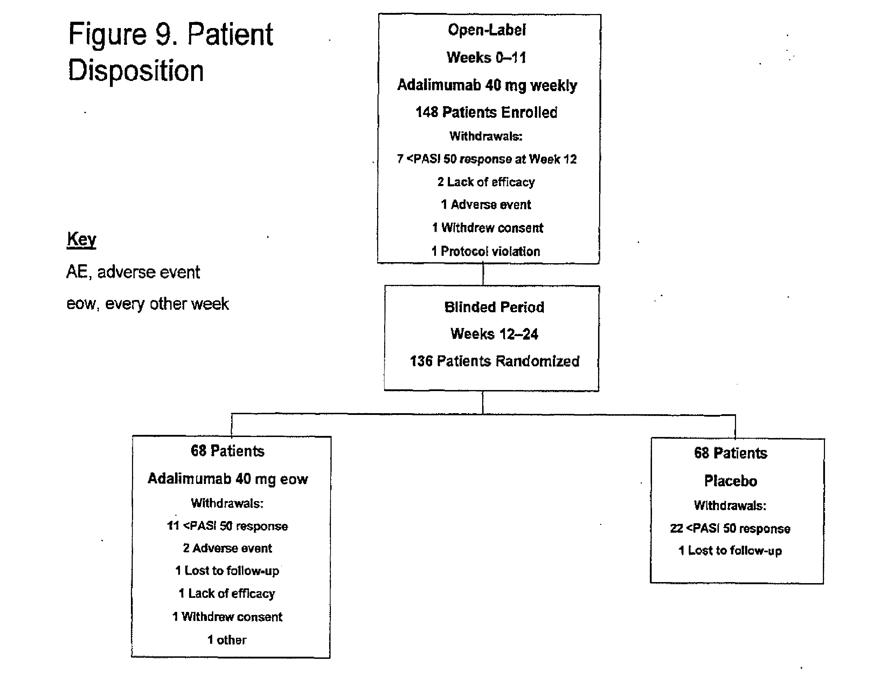

In this multicenter, randomized, double-blind, placebo-controlled study, patients with moderate to severe plaque psoriasis received 12-week, open-label therapy with subcutaneous adalimumab, consisting of 80 mg of adalimumab at Weeks 0 and 1, followed by 40 mg weekly Weeks 2-11. At Week 12, patients who had an improvement in Psoriasis Area and Severity Index (PASI) score of ≥50% were randomized to blinded therapy and received either adalimumab 40 mg every other week (eow) or placebo for an additional 12 weeks. A diagram of the study design is shown in Figure 8. During the double-blind period, 33.8% (23/68) of placebo patients discontinued early, vs. 25% (17/68) of adalimumab 40 mg eow patients (Figure 9). This trial also featured a 52-week, follow-up period, during which no patients received injections in order to collect additional information regarding treatment-free relapse.

Baseline data were similar among randomization groups. Baseline demographics, disease severity characteristics, and recent history of systemic therapies were similar across the treatment groups. Table 61 shows the baseline demographics and clinical characteristics.

**Table 61: Baseline Demographics and Clinical Characteristics**

| **Characteristic** | **All patients (baseline)** | **Randomized patients (Week 12)** | |
|---|---|---|---|
| | **(N=148)** | **Placebo (N=68)** | **Adalimumab 40 mg eow (N=68)** |
| Age, years | 44 (18-69) | 45 (19-69) | 43 (18-64) |
| Male, % | 63 | 66 | 56 |
| Caucasian, % | 94 | 96 | 91 |
| Body weight, kg | 92 (47-155) | 90 (55-155) | 90 (47-150) |
| Duration of psoriasis, years | 20 (2-52) | 20 (2-45) | 21 (2-52) |
| BSA affected, % | 25 (5-99) | 25 (5-91) | 25 (5-99) |
| PASI score | 16.4 (8-46) | 16.3 (8-39) | 16.4 (8-46) |
| History of psoriatic arthritis, % | 29.0% | 26.5% | 35.3% |
| PGA, % | | | |
| Severe psoriasis | 13 | 16 | 10 |
| Moderate to severe psoriasis | 40 | 41 | 38 |

| | | | |
|---|---|---|---|
| Values represent means and ranges unless otherwise specified. BSA=body surface area; PASI=Psoriasis Area and Severity Index; PGA=Physician's Global Assessment; eow=every other week. | | | |

During open-label treatment with adalimumab 40 mg weekly, most patients experienced clinically significant improvements in their psoriasis (Table 62). Clinical response was rapid, with a PASI 50 response rate of 28% at Week 2 of therapy. Ultimately, a PASI 50 response rate was observed in the vast majority of patients, with 92% (136/148) reaching PASI 50 at Week 12. In addition, PASI 75 and PASI 90 responses were achieved by 76.4% (113/148) and 47.3% (70/148) of patients, respectively. The percentage of patients who achieved a PGA "Clear" or "Minimal" was 66% (98/148) at Week 12. Patients with a recent history of treatment with biologic agents (within the past year), including TNF antagonists, had PASI 50 response rates similar to those of biologic-naïve patients. To this point, 12/14 patients exposed to etanercept, 4/4 patients exposed to infliximab, 6/6 exposed to alefacept, and 14/15 exposed to efalizumab achieved ≥PASI 50 responses and were randomized to placebo or adalimumab eow at Week 12.

**Table 62: PASI Response During Qpen-Label Therapy, Weeks 0-12***

| | **Week** | **Patients (%)** |
|---|---|---|
| **PASI50** | 4 | 59 |
| | 8 | 84 |
| | 12 | 92 |
| **PASI75** | 4 | 26 |
| | 8 | 55 |
| | 12 | 30 |
| **PASI90** | 4 | 9 |
| | 8 | 30 |
| | 12 | 47 |

| | | |
|---|---|---|
| *Patients with missing scores swere considered non-responders. | | |

During the double-blind, placebo-controlled period (Weeks 12-24), the majority of patients in both groups sustained >PASI 50 scores vs. baseline. The point estimate of the hazard ratio of the risk of relapse for patients continuing on adalimumab was 0.7 (95% CI of 0.37-1.34), with a hazard ratio below 1 signifying lower risk of relapse than would be observed in the overall psoriasis population.

At Week 24, greater percentages of patients who had been randomized to adalimumab 40 mg eow sustained efficacy response, as assessed by several efficacy measures, compared with patients who had been randomized to adalimumab withdrawal (Table 63). A greater percentage of patients randomized to adalimumab 40 mg eow (54.4% [37/68]) achieved PGA "Clear" or "Minimal" at Week 24 vs. patients who were withdrawn from adalimumab (39.7% [27/68]) (p=0.069). For patients who achieved ≥PASI 75 at Week 12, the relapse rate (loss of PASI 50 response relative to baseline) at Week 24 was 17.2% for patients randomized to adalimumab eow and 23.6% for patients randomized to placebo.

**Table 63: Percentages of patients achieving at least 75% and 90% Improvements in PASI at Week 24***

| | | **Patients (%)** |
|---|---|---|
| **Placebo** | **PASI50** | 66 |
| | **PASI75** | 49 |
| | **PASI90** | 28 |
| **Adalimumab 40 mg eow** | **PASI50** | 78 |
| | **PASI75** | 68** |
| | **PASI90** | 47** |

| | | |
|---|---|---|
| *Patients with missing scores were considered non-responders. **p<0.05 vs. placebo | | |

Adalimumab therapy was generally well-tolerated (Table 64). Of the 148 patients who received at least one dose of adalimumab, three withdrew because of an adverse event. The most frequently reported adverse events were nasopharyngitis and upper respiratory infection. The incidences of specific adverse events during Weeks 12-24 were similar between the placebo and adalimumab eow groups, with the exception of reports of arthralgias in the adalimumab group (7.4%), which were characterized as mild to moderate. Across the open-label and placebo-controlled periods of the trial, adverse events were most often mild or moderate and typically deemed unrelated or probably unrelated to treatment by the investigators.

**Table 64. Adverse events by treatment group during the open-label (Weeks 0-11) and double-blind (Weeks 12-24) treatment periods**

| **Event, n (%)** | **Open-label period** | **Double-blind period** | |
|---|---|---|---|
| | **40 mg weekly (N=148)** | **Placebo (N=68)** | **40 mg eow (N=68)** |
| Any AE | 105 (70.9) | 36 (52.9) | 46 (67.6) |
| Any SAE | 2 (1.4) | 0 | 2 (2.9) |
| Any infectious SAE | 1 (0.7) | 0 | 2 (2.9) |
| Any AEs leading to discontinuation | 1 (0.7) | 0 | 2 (2.9) |
| Adverse Events* | | | |
| Upper respiratory tract infection | 9 (6.1) | 6 (8.8) | 10 (14.7) |
| Injection site reaction | 17 (11.5) | 1 (1.5) | 4 (5.9) |
| Nasopharyngitis | 11 (7.4) | 7 (10.3) | 3 (4.4) |
| Headache | 11 (7.4) | 0 | 1 (1.5) |
| Arthralgia | 4 (2.7) | 0 | 5 (7.4) |

| | | | |
|---|---|---|---|
| All data are for the types of adverse events that occurred in ≥5% of patients in any group. AE=adverse event; eow=every other week; SAE=serious adverse event; eow=every other week. | | | |

Four patients experienced serious adverse events during the 24-week trial. The two serious adverse events in the open-label period were cellulitis due to insect bite and limb pain, which were attributed by the investigator to fissures in the psoriatic plaques of the lower extremities. The two serious adverse events in the placebo-controlled period were diverticulitis and post-operative wound infection after Mohs' surgery for a basal cell carcinoma of the left hand with flap repair (both events in the adalimumab 40 eow group).

Three patients discontinued prematurely primarily because of an adverse event: one during the open-label period and two after randomization to adalimumab 40 mg eow. During the initial open-label period, one patient who experienced cellulitis subsequently developed acute renal failure, which the investigator ascribed to antibiotic treatment and deemed not related to study drug. Another patient in the open-label period developed pneumonia. This event was deemed possibly related to study drug. However, the patient's primary reason for discontinuation was withdrawal of consent. During the second, blinded period of the trial, a patient experienced a post-operative wound infection and subsequently developed a basal cell carcinoma and squamous cell carcinoma of the skin. Both of these events were deemed mild and considered possibly related to study drug by the investigator.

No patients developed tuberculosis or other opportunistic infections, lymphoma, melanoma, malignancies other than non-melanoma skin cancers, demyelinating disorders, lupus-like syndromes, or congestive heart failure. One patient in the adalimumab weekly/placebo group developed a herpes simplex infection, which was deemed a flare of a pre-existing herpes infection and was considered probably not related to study medication by the investigator. The patient who developed three non-melanoma skin cancers during the study previously had a keratoacanthoma excised 2 years earlier.

Three patients were classified by investigators as "rebounders." One patient had a baseline PASI score of 19.5, which increased to 27.3 by Week 4 of the open-label period. However, the patient did not discontinue, and, upon continued therapy, achieved a PASI score of 6.9 at Week 12. Another patient had a baseline PASI score of 17.1 and was assessed as having a treatment-emergent AE of "psoriasis aggravated" during the treatment-free follow-up period, at which time the patient's PASI score was 1.6. A third patient had a baseline PASI of 31.8 and was assessed as having a treatment-emergent AE of "worsening of psoriasis" during the treatment-free follow-up period, at which time this patient's PASI score was 15.5. None of the three patients met the study's prespecified definition of rebound (ie, PASI score≥125% of Week-0 PASI score or new generalized pustular or erythrodermic psoriasis any time after Week 12 and within 90 days of last dose of study drug).

The majority of patients had no clinically significant laboratory abnormalities or changes from baseline during the 24-week period. No patients discontinued because of laboratory abnormalities. No patients had alanine aminotransferase (ALT) elevations greater than 2.5 times the upper limit of normal at Week 12. One patient in the adalimumab eow group had an ALT elevation greater than 2.5 times the upper limit of normal (212.0 U/ml) at Week 24. At the 30-day follow-up visit, the patient's ALT value was 125.0 U/ml. The patient was a self-reported moderate drinker.

In conclusion, weekly adalimumab therapy rapidly improved psoriasis during an initial 12-week period. Improvement was sustained in most, but not all patients, despite dosage reduction to every other week. No patients randomized to adalimumab withdrawal (placebo at Week 12) experienced rebound, and most maintained >PASI50 improvement, relative to baseline, during the 3 months following adalimumab discontinuation. Overall, greater efficacy rates were observed for patients randomized to adalimumab 40 mg eow than for patients randomized to adalimumab withdrawal.

In addition, the present invention further relates to the following iteins;
1. Use of a human TNFα antibody in the manufacture of a medicament for the treatment of psoriasis in a subject comprising
   administering an initial loading dose of a human TNFα antibody to the subject at week 0,
   administering a second dose of the human TNFα antibody to the subject, wherein the second dose is about half the dose amount of the loading dose, and
   administering at least one a maintenance dose to the subject, wherein the maintenance dose is about half the dose amount of the second dose,
   such that psoriasis is treated.
2. The use of item 1, wherein the initial dose is given in its entirety on one day or is divided over 2 days.
3. The use of item 1 or 2, wherein the second dose is administered to the subject about two weeks after the first dose.
4. The use of any one of items 1-3, wherein the maintenance dose is administered to the subject about two weeks after the second dose.
5. The use of any one of items 1-4, wherein the maintenance dose is administered on a biweekly dosing regimen.
6. A method of determining the efficacy of a human TNFα antibody for treating psoriasis in a subject comprising
   determining a Psoriasis Area Severity Index (PASI) 75 response of a patient population having psoriasis and a baseline PASI < 10 and who was administered the human TNFα antibody,
   wherein a PASI 75 response is achieved in at least about 77% of the patient population indicates that the human TNFα antibody is an effective human TNFα antibody for the treatment of psoriasis in a subject.
7. The method of item 6, further comprising administering the effective human TNFα antibody to a subject to treat psoriasis.
8. The method of item 6 or 7, wherein a PASI 75 response is achieved in at least about 80% of the patient population indicates that the human TNFα antibody is an effective human TNFα antibody for the treatment of psoriasis in a subject.
9. The method of item 6 or 7, wherein a PASI 75 response is achieved in at least about 85% of the patient population indicates that the human TNFα antibody is an effective human TNFα antibody for the treatment of psoriasis in a subject.
10. The method of item 6 or 7, wherein a PASI 75 response is achieved in at least about 88% of the patient population indicates that the human TNFα antibody is an effective human TNFα antibody for the treatment of psoriasis in a subject.
11. The method of item6 or 7, wherein a PASI 75 response is achieved in at least about 90% of the patient population indicates that the human TNFα antibody is an effective human TNFα antibody for the treatment of psoriasis in a subject.
12. Use of a human TNFα antibody in the manufacture of a medicament for the treatment of psoriasis comprising administering an effective amount of a human TNFα antibody to the subject such that psoriasis is treated, wherein the effective human TNFα antibody was previously identified as achieving a PASI 75 response in at least about 77% of a patient population having psoriasis and a baseline PASI < 10.
13. The use of item12, wherein the effective human TNFα antibody was previously identified as achieving a PASI 75 response in at least about 80% of a patient population having psoriasis and a baseline PASI < 10.
14. The use of item 12, wherein the effective amount of the human.TNFα antibody was previously identified as achieving a PASI 75 response in at least about 85% of a patient population having psoriasis and a baseline PASI < 10.
15. The use of item 12, wherein the effective amount of the human TNFα antibody was previously identified as achieving a PASI 75 response in at least about 88% of a patient population having psoriasis and a baseline PASI < 10.
16. The use of item 12, wherein the effective amount of the human TNFα antibody was previously identified as achieving a PASI 75 response in at least about 90% of a patient population having psoriasis and a baseline PASI < 10.
17. A method of determining the efficacy of a human TNFα antibody for achieving a clinical response in psoriasis in a subject comprising
   determining a Psoriasis Area Severity Index (PASI) 90 response of a patient population having psoriasis and a baseline PASI < 10 and who was administered the human TNFα antibody,
   wherein a PASI 90 response is achieved in at least about 63% of the patient population indicates that the human TNFα antibody is an effective human TNFα antibody for achieving a clinical response in psoriasis in a subject.
18. The method of item17. further comprising administering the effective human TNFα antibody to a subject to achieve a clinical response in psoriasis.
19. The method of item17 or 18, wherein a PASI 90 response is achieved in at least about 65% of the patient population indicates that the human TNFα antibody is an effective human TNFα antibody for achieving a clinical response in psoriasis in a subject.
20. The method of item 17 or 18, wherein a PASI 90 response is achieved in at least about 68% of the patient population indicates that the human TNFα antibody is an effective human TNFα antibody for achieving a clinical response in psoriasis in a subject.
21. The method of item 17 or 18, wherein a PASI 90 response is achieved in at least about 69% of the patient population indicates that the human TNFα antibody is an effective human TNFα antibody for achieving a clinical response in psoriasis in a subject.
22. The method of item 17 or 18, wherein a PASI 90 response is achieved in at least about 70% of the patient population indicates that the human TNFα antibody is an effective human TNFα antibody for achieving a clinical response in psoriasis in a subject.
23. The method of item 17 or 18, wherein a PASI 90 response is achieved in at least about 75% of the patient population indicates that the human TNFα antibody is an effective human TNFα antibody for achieving a clinical response in psoriasis in a subject.
24. The method of item 17 or 18, wherein a PASI 90 response is achieved in at least about 80% of the patient population indicates that the human TNFα antibody is an effective human TNFα antibody for achieving a clinical response in psoriasis in a subject.
25. A method of achieving a clinical response in psoriasis in a subject comprising administering an effective amount of a human TNFα antibody to the subject such that a clinical response in psoriasis is achieved, wherein the effective amount of the human TNFα antibody was previously identified as achieving a PASI 90 response in at least about 63% of a patient population having psoriasis and a baseline PASI < 10.
26. The method of item 25, wherein the effective amount of the human TNFα antibody was previously identified as achieving a PASI 90 response in at least about 65% of a patient population having psoriasis and a baseline PASI < 10.
27. The method of item25, wherein the effective amount of the human TNFα antibody was previously identified as as achieving a PASI 90 response in at least about 68% of a patient population having psoriasis and a baseline PASI < 10.
28. The method of item 25, wherein the effective amount of the human TNFα antibody was previously identified as as achieving a PASI 90 response in at least about 70% of a patient population having psoriasis and a baseline PASI < 10.
29. The method of item25, wherein the effective amount of the human TNFα antibody was previously identified as as achieving a PASI 90 response in at least about 75% of a patient population having psoriasis and a baseline PASI < 10.
30. The method ofitem 25, wherein the effective amount of the human TNFα antibody was previously identified as as achieving a PASI 90 response in at least about 80% of a patient population having psoriasis and a baseline PASI < 10.
31. A method of determining the efficacy of a human TNFα antibody, or antigen-binding portion thereof, for achieving a clinical response in psoriasis in a subject comprising
   determining a Psoriasis Area Severity Index (PASI) 100 response of a patient population having psoriasis and who was administered the human TNFα antibody, or antigen-binding portion thereof,
   wherein a PASI 100 response is achieved in at least about 36% of the patient population indicates that the human TNFα antibody, or antigen-binding portion thereof, is an effective human TNFα antibody, or antigen-binding portion thereof, for achieving a clinical response in psoriasis in a subject.
32. The method of item 31, further comprising administering the effective human TNFα antibody, or antigen-binding portion thereof, to a subject.
33. The method of item 31 or 32, wherein a PASI 100 response is achieved in at least about 38% of the patient population indicates that the human TNFα antibody, or antigen-binding portion thereof, is an effective human TNFα antibody, or antigen-binding portion thereof, for achieving a clinical response in psoriasis in a subject.
34. The method of item 31 or 32, wherein a PASI 100 response is achieved in at least about 40% of the patient population indicates that the human TNFα antibody, or antigen-binding portion thereof, is an effective human TNFα antibody, or antigen-binding portion thereof, for achieving a clinical response in psoriasis in a subject.
35. The method of item 31 or 32, wherein a PASI 100 response is achieved in at least about 45% of the patient population indicates that the human TNFα antibody, or antigen-binding portion thereof, is an effective human TNFα antibody, or antigen-binding portion thereof, for achieving a clinical response in psoriasis in a subject.
36. The method of item 31 or 32, wherein a PASI 100 response is achieved in at least about 48% of the patient population indicates that the human TNFα antibody, or antigen-binding portion thereof, is an effective human TNFα antibody, or antigen-binding portion thereof, for achieving a clinical response in psoriasis in a subject.
37. The method of item 31 or 32, wherein a PASI 100 response is achieved in at least about 50% of the patient population indicates that the human TNFα antibody, or antigen-binding portion thereof, is an effective human TNFα antibody, or antigen-binding portion thereof, for achieving a clinical response in psoriasis in a subject.
38. A method of determining the efficacy of a human TNFα antibody to treat psoriasis in a subject comprising
   determining a Physician's Global Assessment (PGA) score ofa patient population having psoriasis who was administered the human TNFα antibody,
   wherein a PGA score of "clear" or "almost clear" in at least about 77% of the patient population indicates that the human TNFα antibody is an effective human TNFα antibody for treating psoriasis in a subject.
39. The method of item38, further comprising administering the effective human TNFα antibody to a subject having psoriasis.
40. The method of item38 or 39, wherein a PGA score of "clear" or "almost clear" in at least about 80% of the patient population indicates that the human TNFα antibody is an effective human TNFα antibody for treating psoriasis in a subject.
41. Use of a human TNFα antibody in the manufacture of a medicament for the treatment of psoriasis comprising administering an effective amount of a human TNFα antibody to the subject, wherein the effective human TNFα antibody was previously identified as maintaining an PGA score of "clear" or "almost clear" in at least about 77% of a patient population having psoriasis.
42. The use of item 41, wherein the effective human TNFα antibody was previously identified as maintaining an PGA score of "clear" or "almost clear" in at least about 80% of a patient population having psoriasis.
43. The methods and uses of any one of items 1-42, wherein the human TNFα antibody is a human TNFα antibody or antigen-binding portion thereof.
44. The methods and uses of item 43, wherein the human TNFα antibody, or an antigen-binding portion thereof, dissociates from human TNFα with a K_{d} of I x 10⁻⁸ M or less and a K_{off} rate constant of 1 x 10⁻³ s⁻¹ or less, both determined by surface plasmon resonance, and neutralizes human TNFα cytotoxicity in a standard *in vitro* L929 assay with an IC₅₀ of 1 x 10⁻⁷ M or less.
45. The methods and uses of item44, wherein the human TNFα antibody, or an antigen-binding portion thereof, has the following characteristics:
   a) dissociates from human TNFα with a K_{off} rate constant of 1 x 10⁻³ s⁻¹ or less, as determined by surface plasmon resonance;
   b) has a light chain CDR3 domain comprising the amino acid sequence of SEQ ID NO: 3, or modified from SEQ ID NO: 3 by a single alanine substitution at position 1, 4, 5, 7 or 8 or by one to five conservative amino acid substitutions at positions 1, 3, 4, 6, 7, 8 and/or 9;
   c) has a heavy chain CDR3 domain comprising the amino acid sequence of SEQ ID NO: 4, or modified from SEQ ID NO: 4 by a single alanine substitution at position 2, 3, 4, 5, 6, 8, 9, 10 or 11 or by one to five conservative amino acid substitutions at positions 2, 3, 4, 5, 6, 8, 9, 10, 11 and/or 12.
46. The methods and uses of item 44, wherein the human TNFα antibody, or an antigen-binding portion thereof, comprises a light chain variable region (LCVR) having a CDR3 domain comprising the amino acid sequence of SEQ ID NO: 3, or modified from SEQ ID NO: 3 by a single alanine substitution at position 1, 4, 5, 7 or 8, and comprises a heavy chain variable region (HCVR) having a CDR3 domain comprising the amino acid sequence of SEQ ID NO: 4, or modified from SEQ ID NO: 4 by a single alanine substitution at position 2, 3, 4, 5, 6, 8, 9, 10 or 11.
47. The methods and uses of item44, wherein the human TNFα antibody, or an antigen-binding portion thereof, comprises a light chain variable region (LCVR) comprising the amino acid sequence of SEQ ID NO: 1 and a heavy chain variable region (HCVR) comprising the amino acid sequence of SEQ ID NO: 2.
48. The methods and uses of item 44, wherein the human TNFα antibody, or an antigen-binding portion thereof, is adalimumab.
49. The methods and uses of any one of items 44-48, wherein the human TNFα antibody, or an antigen-binding portion thereof, was administered to the patient population on a biweekly dosing regimen.
50. The methods and uses of item 49, wherein the human TNFα antibody, or an antigen-binding portion thereof, was administered in a dose of about 40 mg.
51. A method of achieving a clinical response in psoriasis in a subject comprising administering an effective amount of a human TNFα. antibody, or antigen-binding portion thereof, to the subject such that a clinical response in psoriasis is achieved, wherein the effective human TNFα antibody, or antigen-binding portion thereof, was previously identified as achieving a PASI 75 response in at least about 77% of a patient population having psoriasis.
52. The method of item 51, wherein the effective human TNFα antibody, or antigen-binding portion thereof, was previously identified as achieving a PASI 75 response in at least about 78% of a patient population having psoriasis.
53. The method of item 51, wherein the effective amount of the human TNFα antibody, or antigen-binding portion thereof, was previously identified as achieving a PASI 75 response in at least about 80% of a patient population having psoriasis.
54. The method of item51, wherein the effective amount of the human TNFα antibody, or antigen-binding portion thereof, was previously identified as achieving a PASI 75 response in at least about 85% of a patient population having psoriasis.
55. The method of item 51, wherein the effective amount of the human TNFα antibody, or antigen-binding portion thereof, was previously identified as achieving a PASI 75 response in at least about 88% of a patient population having psoriasis.
56. The method of item51, wherein the effective amount of the human TNFα antibody, or antigen-binding portion thereof, was previously identified as achieving a PASI 75 response in at least about 90% of a patient population having psoriasis.
57. Use of a human TNFα antibody, or antigen-binding portion thereof, in the manufacture of a medicament for treating psoriasis in a subject.
58. Use of a human TNF a antibody in the manufacture of a medicament for the treatment of psoriasis comprising
   administering an initial loading dose of a human TNFα antibody or antigen-binding portion thereof, to the subject at week 0,
   administering a second dose of the human TNFα antibody or antigen-binding portion thereof, to the subject, wherein the second dose is about half the dose amount of the loading dose, and
   administering at least one a maintenance dose of the human TNFα antibody or antigen-binding portion thereof, to the subject, wherein the maintenance dose is about half the dose amount of the second dose,
   such that psoriasis is treated.
59. The use of item 58, wherein the initial dose is given in its entirety on one day or is divided over 2 days.
60. The use of item 58 or 59, wherein the second dose is administered to the subject about two weeks after the first dose.
61. The use of any one of items 58-60, wherein the maintenance dose is administered to the subject about two weeks after the second dose.
62. The use of any one of items 58-61, wherein the maintenance dose is administered on a biweekly dosing regimen.
63. The methods or uses of any one of items51-62, wherein the human TNFα antibody, or an antigen-binding portion thereof, dissociates from human TNFα. with a K_{d} of 1 x 10⁻⁸ M or less and a K_{off} rate constant of 1 x 10⁻³ s⁻¹ or less, both determined by surface plasmon resonance, and neutralizes human TNFα cytotoxicity in a standard *in vitro* L929 assay with an IC₅₀ of 1 x 10⁻⁷ M or less.
64. The methods or uses of any one of items 51-62, wherein the human TNFα antibody, or an antigen-binding portion thereof, has the following characteristics:
   a) dissociates from human TNFα with a K_{off} rate constant of 1 x 10⁻³ s⁻¹ or less, as determined by surface plasmon resonance;
   b) has a light chain CDR3 domain comprising the amino acid sequence of SEQ ID NO: 3, or modified from SEQ ID NO: 3 by a single alanine substitution at position 1, 4, 5, 7 or 8 or by one to five conservative amino acid substitutions at positions 1, 3, 4, 6, 7, 8 and/or 9;
   c) has a heavy chain CDR3 domain comprising the amino acid sequence of SEQ ID NO: 4, or modified from SEQ ID NO: 4 by a single alanine substitution at position 2, 3, 4, 5, 6, 8, 9, 10 or 11 or by one to five conservative amino acid substitutions at positions 2, 3, 4, 5, 6, 8, 9, 10, 11 and/or 12.
65. The methods or uses of any one of items 51-62, wherein the human TNFα antibody, or an antigen-binding portion thereof, comprises a light chain variable region (LCVR) having a CDR3 domain comprising the amino acid sequence of SEQ ID NO: 3, or modified from SEQ ID NO: 3 by a single alanine substitution at position 1, 4, 5, 7 or 8, and comprises a heavy chain variable region (HCVR) having a CDR3 domain comprising the amino acid sequence of SEQ ID NO: 4, or modified from SEQ ID NO: 4 by a single alanine substitution at position 2, 3, 4, 5, 6, 8, 9, 10 or 11.
66. The methods or uses of any one of items 51-62, wherein the human TNFα antibody, or an antigen-binding portion thereof, comprises a light chain variable region (LCVR) comprising the amino acid sequence of SEQ ID NO: 1 and a heavy chain variable region (HCVR) comprising the amino acid sequence of SEQ ID NO: 2.
67. The methods or uses of any one of items 51-62, wherein the human TNFα antibody, or an antigen-binding portion thereof, is adalimumab.
68. An article of manufacture comprising a human TNFα antibody and a package insert, wherein the package insert indicates the recommended human TNFα antibody dose regimen for adult patients with psoriasis is 80 mg at week 0, 80 mg at week 1, followed by 40 mg every other week beginning at week 3.
69. The article of item 68, wherein the human TNFα antibody is a human TNFα antibody, or an antigen-binding portion thereof.
70. The article of item 69, wherein the human TNFα antibody, or an antigen-binding portion thereof, dissociates from human TNFα with a K_{d} of 1 x 10⁻⁸ M or less and a K_{off} rate constant of 1 x 10⁻³ s⁻¹ or less, both determined by surface plasmon resonance, and neutralizes human TNFα cytotoxicity in a standard *in vitro* L929 assay with an IC₅₀ of 1 x 10⁻⁷ M or less.
71. The article of item 68, wherein the human TNFα antibody, or an antigen-binding portion thereof, comprises a light chain variable region (LCVR) having a CDR3 domain comprising the amino acid sequence of SEQ ID NO: 3, or modified from SEQ ID NO: 3 by a single alanine substitution at position 1, 4, 5, 7 or 8, and comprises a heavy chain variable region (HCVR) having a CDR3 domain comprising the amino acid sequence of SEQ ID NO: 4, or modified from SEQ ID NO: 4 by a single alanine substitution at position 2, 3, 4, 5, 6, 8, 9, 10 or 11.
72. The article of item 68, wherein the human TNFα antibody, or an antigen-binding portion thereof, is adalimumab.
73. An article of manufacture which comprising adalimumab and a package insert, wherein the package insert indicates that the adalimumab may be used to treat psoriasis in patients who have had an inadequate response to conventional therapy and/or who have lost response to or are intolerant to infliximab.
74. An article of manufacture comprising
   a) a packaging material;
   b) a human TNFα antibody, or antigen-binding portion thereof; and
   c) a package insert contained within the packaging material indicating that aminosalicylates, corticosteroids, and/or immunomodulatory agent, e.g., 6-mercaptopurine and azathioprine, may be continued during treatment with the TNFα antibody, or antigen-binding portion thereof.
75. An article of manufacture comprising:
   a) a packaging material;
   b) a human TNFα antibody, and
   c) a label or package insert contained within the packaging material indicating that a history of systemic or biologic therapy does not adversely effect efficacy of the human TNFα antibody in patients and/or that administration of the human TNFα antibody is safe in patients with a history of systemic or biologic therapy.
76. An article of manufacture comprising:
   a) a packaging material;
   b) a human TNFα antibody, and
   c) a label or package insert contained within the packaging material indicating that a use of TNF blockers have been associated with reactivation of hepatitis B virus (HBV) in patients who are chronic carriers of the virus.
77. An article of manufacture comprising:
   a) a packaging material;
   b) a human TNFα antibody, and
   c) a label or package insert contained within the packaging material indicating that an adverse event which has been reported in the use of the human TNFα antibody is angioneurotic edema.
78. An article of manufacture comprising:
   a) a packaging material;
   b) a human TNFα antibody, and
   c) a label or package insert contained within the packaging material indicating that the human TNFα antibody may be used as a first line treatment for the treatment of psoriasis.
79. An article of manufacture comprising:
   a) a packaging material;
   b) a human TNFα antibody, and
   c) a label or package insert contained within the packaging material indicating that the human TNFα antibody may be used for the treatment of psoriasis without methotrexate.
80. An article of manufacture comprising:
   a) a packaging material;
   b) a human TNFα antibody, and
   c) a label or package insert contained within the packaging material indicating that the human TNFα antibody was found to be more effective than methotrexate as a first line treatment and/or that the human TNFα antibody has significantly superior efficacy for the treatment of moderate to severe psoriasis versus methotrexate.
81. A method of decreasing a DLQI score of a subject having psoriasis by at least about 10 points comprising administering a human TNFα antibody to the subject, such that the DLQI score is decreased by at least about 10 points.
82. Use of a human TNFα antibody in the treatment of a subtherapeutic response in a subject having psoriasis comprising administering a human TNFα antibody to the subject at an increased dosing rate which is about twice as frequent as the original dosing rate.
83. The use of item 82, wherein the increased dosing rate is weekly.
84. The use of item 82, wherein the subtherapeutic response comprises < PASI 50 improvement from baseline (week 0) and a determined time period following baseline.
85. The use of item 84, wherein the determined time period following baseline is at least about 24 weeks.
86. A method of decreasing a DLQI score of a subject having psoriasis from a large/extremely large score to a no or small impact score comprising administering a human TNFα antibody to the subject, such that the DLQI score is decreased from the large/extremely large score to the no or small impact score.
87. A method of decreasing a DLQI score of a subject having psoriasis by at least about 10 points comprising administering a human TNFα antibody to the subject, such that the DLQI score is decreased by at least about 10 points.
88. A method of decreasing a PASI score of a subject having psoriasis by at least about 8 points comprising administering a human TNFα antibody to the subject, such that the PASI score is decreased by at least about 8 points.
89. A method of decreasing a PGA score of a subject having psoriasis by at least about 2 points comprising administering a human TNFα antibody to the subject, such that the PGA score is decreased by at least about 2 points.
90. A method for determining the efficacy of a TNFα inhibitor for improving the functional limitations of a subject having psoriasis comprising
   determining an improvement in a Dermatology Life Quality Index (DLQI) score from a patient population who was administered the TNFα inhibitor,
   wherein a DLQI score of no or small impact in at least about 83% of the patient population indicates that the TNFα inhibitor is efficacious for improving the functional limitations of human subjects having psoriasis.
91. The methods or uses of any one of items 84-90, wherein the subject has a PASI score of ≥ 10 and a DLQI score >10

## Claims

1. An isolated anti-TNFα antibody, or antigen binding portion thereof, for use in treating psoriasis, wherein the anti-TNFα antibody, or antigen binding portion thereof, is administered to a subject for 16 weeks,
wherein if the subject achieves a Psoriasis Area and Severity Index (PASI) ≥75 response after the 16 weeks, the anti-TNFα antibody, or antigen binding portion thereof, is administered to the subject for an additional 17 weeks for a total of 33 weeks; and
wherein if the subject achieves a PASI ≥75 response after the 33 weeks, the anti-TNFα antibody, or antigen binding portion thereof, is administered to the subject for an additional 19 weeks, and
wherein the anti-TNFα antibody, or antigen binding portion thereof, comprises a light chain variable region (LCVR) having a CDR1 domain comprising the amino acid sequence of SEQ ID NO: 7, a CDR2 domain comprising the amino acid sequence of SEQ ID NO: 5, and a CDR3 domain comprising the amino acid sequence of SEQ ID NO: 3; and a heavy chain variable region (HCVR) having a CDR1 domain comprising the amino acid sequence of SEQ ID NO: 8, a CDR2 domain comprising the amino acid sequence of SEQ ID NO: 6, and a CDR3 domain comprising the amino acid sequence of SEQ ID NO: 4.

2. An isolated human anti-TNFα antibody, or antigen binding portion thereof, for use in treating psoriasis in a subject who failed to respond to, or who have a contraindication to, or are intolerant to, other systemic psoriasis therapy, and wherein the anti-TNFa antibody, or antigen binding portion thereof, comprises a light chain variable region (LCVR) having a CDR1 domain comprising the amino acid sequence of SEQ ID NO: 7, a CDR2 domain comprising the amino acid sequence of SEQ ID NO: 5, and a CDR3 domain comprising the amino acid sequence of SEQ ID NO: 3; and a heavy chain variable region (HCVR) having a CDR1 domain comprising the amino acid sequence of SEQ ID NO: 8, a CDR2 domain comprising the amino acid sequence of SEQ ID NO: 6, and a CDR3 domain comprising the amino acid sequence of SEQ ID NO: 4.

3. The anti-TNFα antibody, or antigen binding portion thereof, of claim 2, wherein the other systemic psoriasis therapy is methotrexate or infliximab.

4. An anti-TNFα antibody, or antigen binding portion thereof, for use in treating psoriasis for at least 96 weeks, wherein the anti-TNFa antibody, or antigen binding portion thereof, is administered to a subject, wherein a PASI75 response is achieved, and wherein the anti-TNFα antibody, or antigen binding portion thereof, comprises a light chain variable region (LCVR) having a CDR1 domain comprising the amino acid sequence of SEQ ID NO: 7, a CDR2 domain comprising the amino acid sequence of SEQ ID NO: 5, and a CDR3 domain comprising the amino acid sequence of SEQ ID NO: 3; and a heavy chain variable region (HCVR) having a CDR1 domain comprising the amino acid sequence of SEQ ID NO: 8, a CDR2 domain comprising the amino acid sequence of SEQ ID NO: 6, and a CDR3 domain comprising the amino acid sequence of SEQ ID NO: 4.

5. The anti-TNFa antibody, or antigen binding portion thereof, of any one of claims 1-4, wherein the human TNFα antibody, or an antigen-binding portion thereof, is administered to the subject on a biweekly dosing regimen.

6. The anti-TNFa antibody, or antigen binding portion thereof, of any one of claims 1-4, wherein the human TNFα antibody, or an antigen-binding portion thereof, is administered at an initial dose of 80 mg followed by 40 mg every other week starting one week after the initial dose.

7. An anti-TNFα antibody, or antigen binding portion thereof, for use in treating psoriasis, wherein the anti-TNFα antibody, or antigen binding portion thereof, is administered for a period of 24 weeks to a subject at a first dosage, and if the subject achieves a Psoriasis Area and Severity Index (PASI) <50 response at 24 weeks, the anti-TNFa antibody, or antigen binding portion thereof, an escalated dosage of the anti-TNFα antibody, or antigen binding portion thereof, is administered to the subject.

8. The anti-TNFα antibody, or antigen binding portion thereof, of claim 7, wherein first dosage is 40 mg every other week, and the escalated dosage is 40 mg every week.

9. The antibody of any one of claims 1-8, wherein the human anti-TNFα antibody, or antigen-binding portion thereof, comprises a light chain variable region (LCVR) comprising the amino acid sequence of SEQ ID NO: 1 and a heavy chain variable region (HCVR) comprising the amino acid sequence of SEQ ID NO: 2.

10. The antibody of any one of claims 1-8, wherein the human anti-TNFα antibody, or antigen-binding portion thereof, is adalimumab, or an antigen binding portion thereof.
